# EUROPEAN PATENT APPLICATION

(11) **EP 4 349 828 A1**
(43) Date of publication of application: **10.04.2024**
(21) Application number: 22810495.6
(22) Date of filing: 23.05.2022
(51) Int. Cl.: C07D 403/02, C07D 403/04, C07D 239/84, C07D 401/04, C07D 487/04, C07D 498/04, A61K 31/41, A61K 31/4188, A61K 31/498, A61K 31/4985, A61P 35/00

(54) **NITROGEN-CONTAINING HETEROCYCLIC COMPOUND, PREPARATION METHOD THEREFOR AND APPLICATION THEREOF**

(30) Priority: 24.05.2021 CN 202110567504; 29.09.2021 CN 202111153102; 07.01.2022 CN 202210017287
(71) Applicant: Shanghai Yingli Pharmaceutical Co. Ltd., Shanghai 201203 (CN)
(72) Inventor: XU, Zusheng, Shanghai 201203 (CN); LOU, Yangtong, Shanghai 201203 (CN); SHEN, Jian, Shanghai 201203 (CN); XIE, Tiegang, Shanghai 201203 (CN); SUN, Qingrui, Shanghai 201203 (CN); CHEN, Li, Shanghai 201203 (CN); ZENG, Kun, Shanghai 201203 (CN); JIN, Xin, Shanghai 201203 (CN)
(74) Representative: Studio Torta S.p.A.
(86) International application number: PCT/CN2022/094364
(87) International publication number: WO 2022/247770

(57) **Abstract**

A nitrogen-containing heterocyclic compound, a preparation method thereof and an application thereof. A nitrogen-containing heterocyclic compound represented by formula I, a pharmaceutically acceptable salt thereof, a stereoisomer thereof, a tautomer thereof, or an isotopic compound thereof. The nitrogen-containing heterocyclic compound is expected to be used for the treatment and/or prevention of multiple diseases related to Ras.

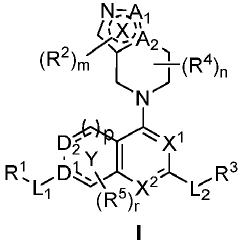

## Description

The present application claims the priority of Chinese patent application 202110567504.1 filed on May 24, 2021, Chinese patent application 202111153102.3 filed on September 29, 2021, and Chinese patent application 202210017287.3 filed on January 7, 2022. The contents of the aforementioned Chinese patent applications are incorporated into the present application by reference in their entirety.

### Technical field

The present disclosure relates to a nitrogen-containing heterocyclic compound, a method for preparing the same and a use of the same.

### Background

Ras (Rat sarcoma viral oncogene) was first found in rat sarcoma. There are three members in the ras gene family of mammals, namely H-ras (HRAS), K-ras (KRAS) and N-ras (NRAS), where the fourth exon of K-ras has variants A and B. Ras gene is widely found in various eukaryotes such as mammals, fruit flies, fungi, nematodes and yeasts, and is expressed at various levels in different tissues, where H-Ras is mainly expressed in skin and skeletal muscle, K-Ras is mainly expressed in colon and thymus, and N-Ras is expressed at a high level in testis. Ras protein regulates and controls signal transduction by switching the binding to GTP/GDP as a molecular switch in cell signal transduction, thereby regulating the life processes such as proliferation, differentiation, senescence and apoptosis of cells.

The mutated forms of RAS are closely associated with the occurrence and development of human tumors, and are present in about 30% of human tumors. KRAS mutation is most common and accounts for approximately 85% of cases, and NRAS and HRAS account for 12% and 3%, respectively. KRAS mutation is mainly found in pancreatic, colorectal and lung cancers, NRAS mutation is common in melanoma and acute myelogenous leukemia, and HRAS mutation is common in bladder and head and neck cancers. Ras proto-oncogene mutation occurs mainly by point mutation. More than 150 different Ras point mutations have been found, with mutations in the glycine at positions 12 and 13 and glutamine at position 61 being the most common.

For decades, efforts have been made to develop small molecule inhibitors targeting Ras. Scientists have been hoping to develop competitive inhibitors of GTP that act directly on the Ras protein. However, this has not been successful because of the strong affinity between GTP and Ras (pmol/L level), the high concentration of GTP in cells (0.5 mM), the lack of a pocket in the RAS protein structure that facilitates the binding of small molecules, and the like. In recent years, some advances have been made in drug development using the allosteric site of K-Ras G12C mutant. In 2013, a team of researchers reported the discovery about K-Ras G12C small molecule inhibitors (Nature, 2013, 503, 548-551). They identified a novel binding pocket located below the molecular switch II region from K-Ras G12C mutant. Those inhibitors bind to the allosteric pocket and covalently bind to nearby Cys12, thereby selectively inhibiting the activation of K-Ras G12C. Other researchers have reported KRAS inhibitors with cellular activity (Science, 2016, 351, 604-608). Compound AMG510 from Amgen, studied in a clinical trial began in 2018, is the first small molecule inhibitor directly targeting KRAS to enter clinical studies, and has received accelerated marketing approval from the U.S. FDA in May 2021.

In conclusion, after decades of unremitting efforts, the understanding of Ras has been gradually improved, but only one drug for KRAS G12C mutation has been marketed so far, and there is no particularly effective treatment for other different mutations. The search for compounds with better inhibitory effects on Ras remains a hot and difficult research area in new drug development.

### Content of the present invention

The technical problem to be solved by the present disclosure is the lack of effective drugs serving as Ras inhibitors for clinical treatment in the prior art. Therefore, the present disclosure provides a nitrogen-containing heterocyclic compound, a method for preparing the same and use of the same, and the nitrogen-containing heterocyclic compound is expected to treat and/or prevent various RAS-related diseases.

The present disclosure solves the above problem by the following technical schemes.

The present disclosure provides a nitrogen-containing heterocyclic compound of formula I, a pharmaceutically acceptable salt thereof, a stereoisomer thereof, a tautomer thereof or an isotopically labeled compound thereof: 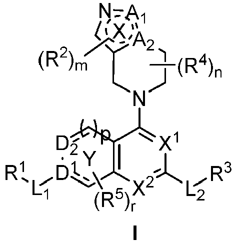 wherein " = " represents a single or double bond; 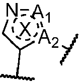
is nitrogen-containing 5-membered heteroaryl; A₁ is CH, O or N; A₂ is C or N;
m is 0, 1 or 2;
R² is -CN, C₁₋₆ alkyl, C₂₋₆ alkenyl, C₂₋₆ alkynyl, C₁₋₆ alkyl substituted with one or more R²⁻¹, halogen, -OR^{2a}, -C(=O)R^{2b}, -NR^{2c1}R^{2c2}, -C(=O)OR^{2d}, -C(=O)NR^{2e1}R^{2e2}, C₃₋₁₀ cycloalkyl, C₃₋₁₀ cycloalkyl substituted with one or more R²⁻², "4- to 10-membered heterocycloalkyl containing 1-3 heteroatoms independently selected from O and N", "4- to 10-membered heterocycloalkyl containing 1-3 heteroatoms independently selected from O and N" substituted with one or more R²⁻³, C₆₋₂₀ aryl, C₆₋₂₀ aryl substituted with one or more R²⁻⁴, "5- to 12-membered heteroaryl containing 1-4 heteroatoms selected from O, S and N", or "5- to 12-membered heteroaryl containing 1-4 heteroatoms selected from O, S and N" substituted with one or more R²⁻⁵; provided that when multiple substituents are present, the substituents are the same or different;
R²⁻¹, R²⁻², R²⁻³, R²⁻⁴ and R²⁻⁵ are independently halogen, hydroxyl, cyano, C₁₋₆ alkyl, C₂₋₆ alkenyl, C₂₋₆ alkynyl, C₁₋₆ alkyl-O-, -C(=O)R³¹, -NR³²R³³, -C(=O)OR³⁴ or -C(=O)NR³⁵R³⁶;
R^{2a}, R^{2b}, R^{2c1}, R^{2c2}, R^{2d}, R^{2e1} and R^{2e2} are independently hydrogen or C₁₋₆ alkyl;
R³¹, R³², R³³, R³⁴, R³⁵ and R³⁶ are independently hydrogen or C₁₋₆ alkyl;
n is 0, 1, 2, 3, 4, 5 or 6;
R⁴ is independently C₁₋₆ alkyl, C₁₋₆ alkyl substituted with one or more R⁴⁻¹, C₁₋₆ alkyl-O-, O=, -C(=O)OR^{4a} or -C(=O)NR^{4b}R^{4c}; or, when n is 2, 3, 4, 5 or 6, two optional R⁴ are connected, together with the atoms on the ring to which they are attached, independently form 3- to 8-membered carbocyclic ring or 3- to 8-membered heterocyclic ring containing 1-3 heteroatoms selected from O, S and N;
R⁴⁻¹ is independently halogen, cyano, hydroxyl, C₁₋₆ alkyl-O-, -NR⁴ⁱR^{4j}, -C(=O)OR^{4d} or - C(=O)NR^{4e}R^{4f};
R^{4a}, R^{4b}, R^{4c}, R^{4d}, R^{4e}, R^{4f}, R⁴ⁱ and R^{4j} are independently hydrogen or C₁₋₆ alkyl;
p is 0 or 1; 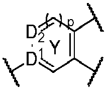
is phenyl, "5- to 7-membered heterocycloalkenyl containing 1-3 heteroatoms independently selected from O, S and N", "5- to 7-membered heteroaryl containing 1-3 heteroatoms independently selected from O, S and N" or 5- to 7-membered cycloalkenyl; wherein D¹ is C, CH or N; D₂ is 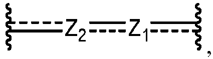 wherein Z₁ and Z₂ are independently a bond, CH, CH₂, O, S, N or NH;
r is 0, 1, 2, 3, 4, 5 or 6;
R⁵ is independently halogen or C₁₋₆ alkyl;
X¹ and X² are independently CR^{b} or N, and X¹ and X² are not both CR^{b};
L₁ is a bond, -C(=O)- or C₁₋₆ alkylene;
R¹ is C₆₋₂₀ aryl, C₈₋₁₁ benzocycloalkenyl, "5- to 12-membered heteroaryl containing 1-4 heteroatoms selected from O, S and N", C₆₋₂₀ aryl substituted with one or more R¹⁻¹ or "5- to 12-membered heteroaryl containing 1-4 heteroatoms selected from O, S and N" substituted with one or more R¹⁻²; provided that when multiple substituents are present, the substituents are the same or different;
R^{b}, R¹⁻¹ and R¹⁻² are independently halogen, -OR^{c}, cyano, -C(=O)R¹¹, -NR¹²R¹³, -C(=O)OR¹⁴, -C(=O)NR¹⁵R¹⁶, C₁₋₆ alkyl, C₂₋₆ alkenyl, C₂₋₆ alkynyl, C₃₋₁₀ cycloalkyl, "5- to 7-membered heterocycloalkyl containing 1 or 2 heteroatoms independently selected from O and N", C₆₋₂₀ aryl, "5- to 7-membered heteroaryl containing 1 or 2 heteroatoms independently selected from O and N", C₁₋₆ alkyl substituted with one or more R¹⁻¹⁻¹, C₁₋₆ alkyl-O- substituted with one or more R¹⁻¹⁻², C₃₋₁₀ cycloalkyl substituted with one or more R¹⁻¹⁻³, "5- to 7-membered heterocycloalkyl containing 1 or 2 heteroatoms independently selected from O and N" substituted with one or more R¹⁻¹⁻⁴, C₆₋₂₀ aryl substituted with one or more R¹⁻¹⁻⁵, or "5- to 7-membered heteroaryl containing 1 or 2 heteroatoms independently selected from O and N" substituted with one or more R¹⁻¹⁻⁶; provided that when multiple substituents are present, the substituents are the same or different; or, when the number of R¹⁻¹ or R¹⁻² is more than one, two optional R¹⁻¹ or R¹⁻² are connected, together with the atoms on the ring to which they are attached, independently form 3- to 8-membered cyclic olefin;
R^{c}, R¹² and R¹³ are independently hydrogen, C₁₋₆ alkyl, C(=O)R^{c1}, -C(=O)OR^{c2}, - C(=O)NR^{c3}R^{c4} or -SO₂R^{c5}; R^{c1}, R^{c2}, R^{c3} , R^{c4} and R^{c5} are independently hydrogen, C₁₋₆ alkyl, C₃₋₁₀ cycloalkyl, "5- to 7-membered heterocycloalkyl containing 1 or 2 heteroatoms independently selected from O and N", C₆₋₂₀ aryl, "5- to 7-membered heteroaryl containing 1 or 2 heteroatoms independently selected from O and N", C₁₋₆ alkyl substituted with one or more R⁴⁻¹⁻¹, C₃₋₁₀ cycloalkyl substituted with one or more R⁴⁻¹⁻², "5- to 7-membered heterocycloalkyl containing 1 or 2 heteroatoms independently selected from O and N" substituted with one or more R⁴⁻¹⁻³, C₆₋₂₀ aryl substituted with one or more R⁴⁻¹⁻⁴, or, "5- to 7-membered heteroaryl containing 1 or 2 heteroatoms independently selected from O and N" substituted with one or more R⁴⁻¹⁻⁵; provided that when multiple substituents are present, the substituents are the same or different;
R¹⁻¹⁻¹, R¹⁻¹⁻², R¹⁻¹⁻³, R¹⁻¹⁻⁴, R¹⁻¹⁻⁵, R¹⁻¹⁻⁶, R⁴⁻¹⁻¹, R⁴⁻¹⁻², R⁴⁻¹⁻³, R⁴⁻¹⁻⁴ and R⁴⁻¹⁻⁵ are independently cyano, halogen, hydroxyl, C₁₋₆ alkyl-O-, C₁₋₆ alkyl, -C(=O)R²¹, -NR²²R²³, -C(=O)OR²⁴ or - C(=O)NR²⁵R²⁶;
R¹¹, R²¹, R²², R²³, R¹⁴, R²⁴, R¹⁵, R²⁵, R¹⁶ and R²⁶ are independently hydrogen or C₁₋₆ alkyl;
L₂ is a bond, C₁₋₆ alkylene, -C(=O)-, -O(R^{L-1})ₙ₁-, -S(R^{L-2})ₙ₂- or -NR^{L-3}(R^{L-4})ₙ₃-; R^{L-1}, R^{L-2} and R^{L-4} are independently C₁₋₆ alkylene; R^{L-3} is hydrogen or C₁₋₆ alkyl; n1, n2 and n3 are independently 0 or 1;
R³ is C₃₋₁₂ cycloalkyl, C₃₋₁₂ cycloalkyl substituted with one or more R³⁻¹, "4- to 12-membered heterocycloalkyl containing 1-3 heteroatoms selected from O, S and N", "4- to 12-membered heterocycloalkyl containing 1-3 heteroatoms selected from O, S and N" substituted with one or more R³⁻², C₁₋₆ alkyl, C₁₋₆ alkyl substituted with one or more R³⁻³, -OR^{d}, -SR^{d1}, -NR^{e1}R^{e2} or - C(=O)NR^{e3}R^{e4}; provided that when multiple substituents are present, the substituents are the same or different;
R³⁻¹, R³⁻² and R³⁻³ are independently C₁₋₆ alkyl, C₁₋₆ alkyl substituted with one or more R³⁻¹⁻¹, hydroxyl, C₁₋₆ alkyl-O-, halogen, O=, -NR^{e5}R^{e6} or -C(=O)NR^{e7}R^{e8};
R^{d}, R^{d1}, R^{e1}, R^{e2}, R^{e3} and R^{e4} are independently hydrogen, C₁₋₆ alkyl, C₃₋₁₀ cycloalkyl, "4- to 10-membered heterocycloalkyl containing 1-3 heteroatoms independently selected from O and N", or C₁₋₆ alkyl substituted with one or more R³⁻¹⁻²;
R³⁻¹⁻¹ and R³⁻¹⁻² are independently deuterium, cyano, halogen, hydroxyl, C₁₋₆ alkyl-O-, - C(=O)R^{e9}, -NR^{e10}R^{e11}, -C(=O)OR^{e12} or -C(=O)NR^{e13}R^{e14};
R^{e5}, R^{e6}, R^{e7}, R^{e8}, R^{e9}, R^{e10}, R^{e11}, R^{e12}, R^{e13} and R^{e14} are independently hydrogen or C₁₋₆ alkyl.

In a certain embodiment, with regard to a nitrogen-containing heterocyclic compound of formula I, a pharmaceutically acceptable salt thereof, a stereoisomer thereof, a tautomer thereof or an isotopically labeled compound thereof, some groups are as defined as follows, and the unmentioned group definitons are described in any one of the embodiments of the present disclosure (this content is hereinafter referred to simply as "in a certain embodiment"). Wherein, each R³⁻¹⁻¹ is independently deuterium(D).

In a certain embodiment, R³⁻¹⁻² is independently deuterium(D).

In a certain embodiment, m is 0 or 1.

In a certain embodiment, R² is -CN, C₁₋₆ alkyl, C₁₋₆ alkyl substituted with one or more R²⁻¹, halogen, -NR^{2c1}R^{2c2}, -C(=O)NR^{2e1}R^{2e2}, C₃₋₁₀ cycloalkyl, C₆₋₂₀ aryl or "5- to 12-membered heteroaryl containing 1-4 heteroatoms selected from O, S and N".

In a certain embodiment, R²⁻¹ is hydroxyl.

In a certain embodiment, R^{2c1}, R^{2c2}, R^{2e1} and R^{2e2} are independently hydrogen.

In a certain embodiment, n is 0 or 1.

In a certain embodiment, R⁴ is independently C₁₋₆ alkyl, C₁₋₆ alkyl substituted with one or more R⁴⁻¹, or C₁₋₆ alkyl-O-.

In a certain embodiment, R⁴⁻¹ is independently cyano.

In a certain embodiment, D¹ is C or N.

In a certain embodiment, in D₂, either of Z₁ and Z₂ is CH, CH₂, O, S or N, and the other is a bond;

In a certain embodiment, r is 0 or 1.

In a certain embodiment,

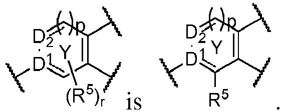

In a certain embodiment, when p is 1, 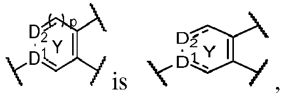 which is phenyl, 6-membered heterocycloalkenyl containing 1-2 heteroatoms independently selected from O and N, 6-membered cycloalkenyl, or "6-membered heteroaryl containing 1-2 heteroatoms selected from O, S and N"; when p is 0, 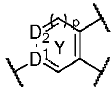 is thiophenyl.

In a certain embodiment, R⁵ is independently halogen;

In a certain embodiment, X¹ and X² are independently N;

In a certain embodiment, L₁ is a bond or -C(=O)-;

In a certain embodiment, R¹ is C₆₋₂₀ aryl substituted with one or more R¹⁻¹, C₈₋₁₁ benzocycloalkenyl, "5- to 12-membered heteroaryl containing 1-4 heteroatoms selected from O, S and N" or "5- to 12-membered heteroaryl containing 1-4 heteroatoms selected from O, S and N" substituted with one or more R¹⁻²;

In a certain embodiment, R¹⁻¹ is independently halogen, -NR¹²R¹³, hydroxyl, -OR^{c}, C₁₋₆ alkyl, C₂₋₆ alkynyl, C₃₋₁₀ cycloalkyl, C₁₋₆ alkyl substituted with one or more R¹⁻¹⁻¹, or C₁₋₆ alkyl-O-substituted with one or more R¹⁻¹⁻², or when the number of R¹⁻¹ is more than one, two optional R¹⁻¹ are connected, together with the atoms on the ring to which they are attached, independently form 3- to 8-membered cyclic olefin.

In a certain embodiment, R^{c} is hydrogen, C₁₋₆ alkyl, -C(=O)R^{c1}, -C(=O)OR^{c2} or - C(=O)NR^{c3}R^{c4}.

In a certain embodiment, in R^{c}, R^{c1} is C₁₋₆ alkyl, C₁₋₆ alkyl substituted with one or more R⁴⁻¹⁻¹, or C₆₋₂₀ aryl substituted with one or more R⁴⁻¹⁻⁴; R⁴⁻¹⁻¹ is -NR²²R²³, R²² and R²³ are independently hydrogen; R⁴⁻¹⁻⁴ is independently -NR²²R²³ or C₁₋₆ alkyl, R²² and R²³ are independently hydrogen.

In a certain embodiment, in R^{c}, R^{c2} is C₁₋₆ alkyl.

In a certain embodiment, in R^{c}, R^{c3} and R^{c4} are independently hydrogen, C₁₋₆ alkyl, C₆₋₂₀ aryl substituted with one or more R⁴⁻¹⁻⁴.

In a certain embodiment, in R^{c}, R⁴⁻¹⁻¹ is -NR²²R²³, R²² and R²³ are independently hydrogen.

In a certain embodiment, in R^{c}, R⁴⁻¹⁻⁴ is independently -NR²²R²³ or C₁₋₆ alkyl, R²² and R²³ are independently hydrogen.

In a certain embodiment, R¹² and R¹³ are independently hydrogen, C(=O)R^{c1}, -C(=O)OR^{c2} or -SO₂R^{c5}.

In a certain embodiment, in R¹² and R¹³, R^{c1}, R^{c2} and R^{c5} are independently C₁₋₆ alkyl.

In a certain embodiment, R¹⁻² is C₁₋₆ alkyl.

R¹⁻¹⁻¹ is independently halogen.

In a certain embodiment, R¹⁻¹⁻² is independently C₁₋₆ alkyl-O-.

In a certain embodiment, L₂ is a bond or -O(R^{L-1})ₙ₁-.

In a certain embodiment, R^{L-1} is independently C₁₋₆ alkylene.

In a certain embodiment, n1 is 0 or 1.

In a certain embodiment, R³ is C₃₋₁₂ cycloalkyl substituted with one or more R³⁻¹, "4- to 12-membered heterocycloalkyl containing 1-3 heteroatoms selected from O, S and N", "4- to 12-membered heterocycloalkyl containing 1-3 heteroatoms selected from O, S and N" substituted with one or more R³⁻², C₁₋₆ alkyl or -NR^{e1}R^{e2}; for example, "4- to 12-membered heterocycloalkyl containing 1-3 heteroatoms selected from O, S and N", "4- to 12-membered heterocycloalkyl containing 1-3 heteroatoms selected from O, S and N" substituted with one or more R³⁻², C₁₋₆ alkyl or -NR^{e1}R^{e2}.

In a certain embodiment, R³⁻¹ is independently -NR^{e5}R^{e6} or C₁₋₆ alkyl substituted with one or more R³⁻¹⁻¹, R³⁻¹⁻¹ is -NR^{e10}R^{e11}. R^{e5}, R^{e6}, R^{e10} and R^{e11} are independently C₁₋₆ alkyl.

In a certain embodiment, R³⁻² is independently C₁₋₆ alkyl, C₁₋₆ alkyl substituted with one or more R³⁻¹⁻¹, halogen, -NR^{e5}R^{e6} or O=.

In a certain embodiment, R^{e5} and R^{e6}are independently C₁₋₆ alkyl, R³⁻¹⁻¹ is halogen or deuterium.

In a certain embodiment, R^{e1} and R^{e2} are independently C₁₋₆ alkyl.

In a certain embodiment, in 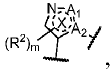 A₁ is CH or N, and A₂ is N; or, A₁ is O, and A₂ is C; or, A₁ is NH, and A₂ is C.

In a certain embodiment, when m is 1, 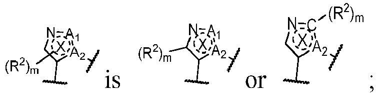 preferably, when 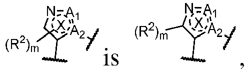 R² is -CN, C₁₋₆ alkyl, halogen, -C(=O)NR^{2e1}R^{2e2}, C₃₋₁₀ cycloalkyl, C₆₋₂₀ aryl or "5- to 12-membered heteroaryl containing 1-4 heteroatoms selected from O, S and N"; when 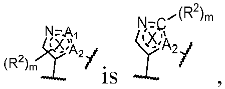 R² is -CN, C₁₋₆ alkyl, C₁₋₆ alkyl substituted with one or more R²⁻¹, halogen, -NR^{2c1}R^{2e2}, C₃₋₁₀ cycloalkyl or C₆₋₂₀ aryl; more preferably, in 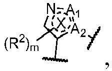 when R² is - CN, C₁₋₆ alkyl, halogen, C₃₋₁₀ cycloalkyl or C₆₋₂₀ aryl, and m is 1,

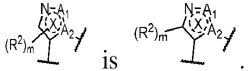

In a certain embodiment, when n is 1, 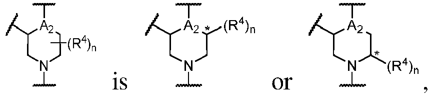 wherein 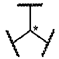 represents R configuration, S configuration or a mixture thereof; when 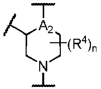 is 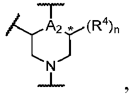 R⁴ is C₁₋₆ alkyl substituted with one or more R⁴⁻¹, or C₁₋₆ alkyl-O-; when 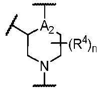 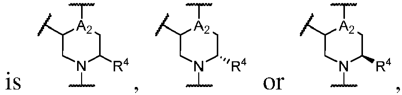 and n is 1, R⁴ is C₁₋₆ alkyl.

In a certain embodiment, in 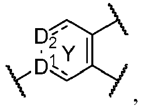 D¹ is C, and in D₂, either of Z₁ and Z₂ is CH or N, and the other is a bond; or D¹ is CH, and in D₂, either of Z₁ and Z₂ is O or CH₂, and the other is a bond; or D¹ is N, and in D₂, either of Z₁ and Z₂ is CH₂, and the other is a bond.

In a certain embodiment, in 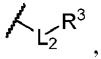 when L₂ is a bond, R³ is "4- to 12-membered heterocycloalkyl containing 1-3 heteroatoms independently selected from O, S and N"; or, when L₂ is -O(R^{L-1})ₙ₁-, R³ is C₃₋₁₂ cycloalkyl substituted with one or more R³⁻¹, "4- to 12-membered heterocycloalkyl containing 1-3 heteroatoms independently selected from O, S and N" substituted with one or more R³⁻², C₁₋₆ alkyl or -NR^{e1}R^{e2}, for example, "4- to 12-membered heterocycloalkyl containing 1-3 heteroatoms independently selected from O, S and N" substituted with one or more R³⁻², C₁₋₆ alkyl or -NR^{e1}R^{e2}; preferably, when R³ is "4- to 12-membered heterocycloalkyl containing 1-3 heteroatoms independently selected from O, S and N" substituted with one or more R³⁻², R³⁻² is C₁₋₆ alkyl.

In a certain embodiment, in 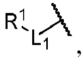 when L₁ is a bond, R¹ is C₆₋₂₀ aryl substituted with one or more R¹⁻¹, Cs-n benzocycloalkenyl, "5- to 12-membered heteroaryl containing 1-4 heteroatoms independently selected from O, S and N" or "5- to 12-membered heteroaryl containing 1-4 heteroatoms independently selected from O, S and N" substituted with one or more R¹⁻²; or, when L₁ is -C(=O)-, R¹ is C₆₋₂₀ aryl substituted with one or more R¹⁻¹.

In a certain embodiment, in 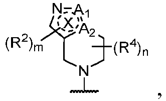 m and n are independently 0 or 1, and 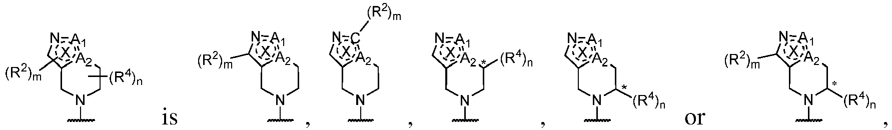 wherein 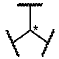 represents R configuration, S configuration or a mixture thereof.

In a certain embodiment, when m is 1, n is 0, and 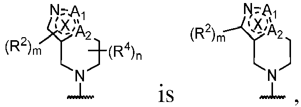 R² is -CN, C₁₋₆ alkyl, halogen, -C(=O)NR^{2e1}R^{2e2}, C₃₋₁₀ cycloalkyl, C₆₋₂₀ aryl, or "5- to 12-membered heteroaryl containing 1-4 heteroatoms independently selected from O, S and N".

In a certain embodiment, when m is 1, n is 0, and 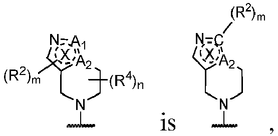 R² is -CN, C₁₋₆ alkyl, C₁₋₆ alkyl substituted with one or more R²⁻¹, halogen, -NR^{2c1}R^{2c2}, C₃₋₁₀ cycloalkyl or C₆₋₂₀ aryl.

In a certain embodiment, when m is 0, n is 1, and 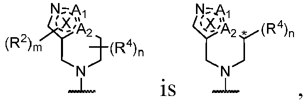 R⁴ is C₁₋₆ alkyl substituted with one or more R⁴⁻¹, or C₁₋₆ alkyl-O-.

In a certain embodiment, preferably, in 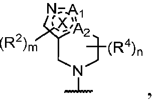 when R² is -CN, C₁₋₆ alkyl, halogen, C₃₋₁₀ cycloalkyl or C₆₋₂₀ aryl, m is 1, and n is 0,

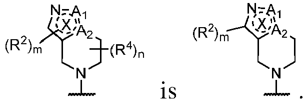

In a certain embodiment, when m is 0, n is 1, and 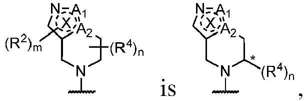 R⁴ is C₁₋₆ alkyl; preferably, when R⁴ is C₁₋₆ alkyl, and n is 1, 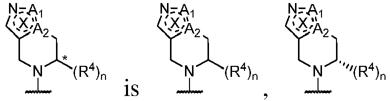 or

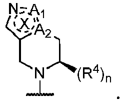

In a certain embodiment, when m is 1, n is 1, and 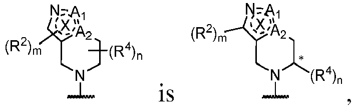 R⁴ is C₁₋₆ alkyl; preferably, when R⁴ is C₁₋₆ alkyl, and n is 1,

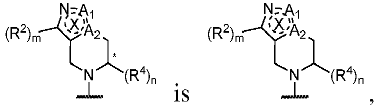

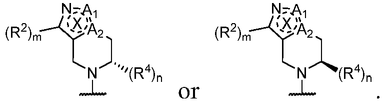

In a certain embodiment, when R² is C₁₋₆ alkyl or C₁₋₆ alkyl substituted with one or more R²⁻¹, the C₁₋₆ alkyl and the C₁₋₆ alkyl in the C₁₋₆ alkyl substituted with one or more R²⁻¹ are C₁₋₄ alkyl, for example, methyl, ethyl, *n*-propyl, isopropyl, *n*-butyl, *sec*-butyl, isobutyl or *tert-*butyl*,* and for another example, methyl.

In a certain embodiment, when R² is C₂₋₆ alkenyl, the C₂₋₆ alkenyl is C₂₋₃ alkenyl, for example, vinyl, propenyl or allyl.

In a certain embodiment, when R² is C₂₋₆ alkynyl, the C₂₋₆ alkynyl is C₂₋₃ alkynyl, for example, ethynyl, propynyl or propargyl.

In a certain embodiment, when R² is halogen, the halogen is fluorine, chlorine, bromine or iodine, for example, bromine.

In a certain embodiment, when R² is C₃₋₁₀ cycloalkyl or C₃₋₁₀ cycloalkyl substituted with one or more R²⁻², the C₃₋₁₀ cycloalkyl and the C₃₋₁₀ cycloalkyl in the C₃₋₁₀ cycloalkyl substituted with one or more R²⁻² are C₃-C₆ cycloalkyl, for example, cyclohexyl, cyclopentyl, cyclobutyl or cyclopropyl, and for another example, cyclopropyl.

In a certain embodiment, when R² is "4- to 10-membered heterocycloalkyl containing 1-3 heteroatoms independently selected from O and N" or "4- to 10-membered heterocycloalkyl containing 1-3 heteroatoms independently selected from O and N" substituted with one or more R²⁻³, the "4- to 10-membered heterocycloalkyl containing 1-3 heteroatoms independently selected from O and N" and the "4- to 10-membered heterocycloalkyl containing 1-3 heteroatoms independently selected from O and N" in the" 4- to 10-membered heterocycloalkyl containing 1-3 heteroatoms independently selected from O and N" substituted with one or more R²⁻³ can be "4-to 6-membered heterocycloalkyl containing 1-3 heteroatoms independently selected from O and N", it can also be "4- to 6-membered heterocycloalkyl containing 1 heteroatom being O or N".

In a certain embodiment, when R² is C₆₋₂₀ aryl or C₆₋₂₀ aryl substituted with one or more R²⁻⁴, the C₆₋₂₀ aryl and the C₆₋₂₀ aryl in the C₆₋₂₀ aryl substituted with one or more R²⁻⁴ can be C₆₋₁₀ aryl, for example, phenyl or naphthyl, and for another example, phenyl.

In a certain embodiment, when R² is "5- to 12-membered heteroaryl containing 1-4 heteroatoms independently selected from O, S and N" or "5- to 12-membered heteroaryl containing 1-4 heteroatoms independently selected from O, S and N" substituted with one or more R²⁻⁵, the "5- to 12-membered heteroaryl containing 1-4 heteroatoms independently selected from O, S and N" and the "5- to 12-membered heteroaryl containing 1-4 heteroatoms independently selected from O, S and N" in the "5- to 12-membered heteroaryl containing 1-4 heteroatoms independently selected from O, S and N" substituted with one or more R²⁻⁵ are "5- to 6-membered heteroaryl containing 1 heteroatom being one of O, S or N", for example, pyridinyl, and for another example,

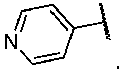

In a certain embodiment, when R²⁻¹, R²⁻², R²⁻³, R²⁻⁴ and R²⁻⁵ are independently halogen, the halogen is fluorine, chlorine, bromine or iodine.

In a certain embodiment, when R²⁻¹, R²⁻², R²⁻³, R²⁻⁴ and R²⁻⁵ are independently C₁₋₆ alkyl, the C₁₋₆ alkyl is C₁₋₄ alkyl, for example, methyl, ethyl, n-propyl, isopropyl, n-butyl, sec-butyl, isobutyl or *tert-butyl,* and for another example, methyl.

In a certain embodiment, when R²⁻¹, R²⁻², R²⁻³, R²⁻⁴ and R²⁻⁵ are independently C₂₋₆ alkenyl, the C₂₋₆ alkenyl is C₂₋₃ alkenyl, for example, vinyl, propenyl or allyl.

In a certain embodiment, when R²⁻¹, R²⁻², R²⁻³, R²⁻⁴ and R²⁻⁵ are independently C₂₋₆ alkynyl, the C₂₋₆ alkynyl is C₂₋₃ alkynyl, for example, ethynyl, propynyl or propargyl.

In a certain embodiment, when R²⁻¹, R²⁻², R²⁻³, R²⁻⁴ and R²⁻⁵ are independently C₁₋₆ alkyl-O-, the C₁₋₆ alkyl in the C₁₋₆ alkyl-O- is C₁₋₄ alkyl, for example, methyl, ethyl, *n*-propyl, isopropyl, *n-*butyl, *sec-*butyl, isobutyl or *tert*-butyl*,* and for another example, methyl.

In a certain embodiment, when R^{2a}, R^{2b}, R^{2c1}, R^{2c2}, R^{2d}, R^{2e1} and R^{2e2} are independently C₁₋₆ alkyl, the C₁₋₆ alkyl is C₁₋₄ alkyl, for example, methyl, ethyl, *n*-propyl, isopropyl, *n*-butyl, *sec-*butyl, isobutyl or *tert-butyl,* and for another example, methyl.

In a certain embodiment, when R³¹, R³², R³³, R³⁴, R³⁵ and R³⁶ are independently C₁₋₆ alkyl, the C₁₋₆ alkyl is C₁₋₄ alkyl, for example, methyl, ethyl, *n*-propyl, isopropyl, *n*-butyl, *sec*-butyl, isobutyl or *tert-butyl,* and for another example, methyl.

In a certain embodiment, when R⁴ is C₁₋₆ alkyl or C₁₋₆ alkyl substituted with one or more R⁴⁻¹, the C₁₋₆ alkyl and the C₁₋₆ alkyl in the C₁₋₆ alkyl substituted with one or more R⁴⁻¹ are C₁₋₄ alkyl, for example, methyl, ethyl, *n*-propyl, isopropyl, *n*-butyl, *sec*-butyl, isobutyl or *tert-*butyl*,* and for another example, methyl.

In a certain embodiment, when R⁴ is C₁₋₆ alkyl-O-, the C₁₋₆ alkyl in the C₁₋₆ alkyl-O- can be C₁₋₄ alkyl, for example, methyl, ethyl, *n*-propyl, isopropyl, *n*-butyl, *sec*-butyl, isobutyl or *tert-butyl,* and for another example, methyl.

In a certain embodiment, when n is 2, 3, 4, 5 or 6, and two optional R⁴ are connected, together with the atoms on the ring to which they are attached, independently form 3- to 8-membered carbocyclic ring, the 3- to 8-membered carbocyclic ring is 3- to 6-membered carbocyclic ring, and the carbocyclic ring can be a monocyclic or bridged ring.

In a certain embodiment, when n is 2, 3, 4, 5 or 6, and two optional R⁴ are connected, together with the atoms on the ring to which they are attached, independently form "3- to 8-membered heterocyclic ring containing 1-3 heteroatoms independently selected from O, S and N", the "3- to 8-membered heterocyclic ring containing 1-3 heteroatoms independently selected from O, S and N" is "3- to 6-membered heterocyclic ring containing 1-3 heteroatoms independently selected from O, S and N", for example, "3- to 6-membered heterocyclic ring containing 1 heteroatom being O, S or N".

In a certain embodiment, when R⁴⁻¹ is independently halogen, the halogen is fluorine, chlorine, bromine or iodine.

In a certain embodiment, when R⁴⁻¹ is independently C₁₋₆ alkyl-O-, the C₁₋₆ alkyl in the C₁₋₆ alkyl-O- is C₁₋₄ alkyl, for example, methyl, ethyl, *n*-propyl, isopropyl, *n*-butyl, *sec*-butyl, isobutyl or *tert*-butyl*.*

In a certain embodiment, when R^{4a}, R^{4b}, R^{4c}, R^{4d}, R^{4e}, R^{4f}, R⁴ⁱ and R^{4j} are independently C₁₋₆ alkyl, the C₁₋₆ alkyl is C₁₋₄ alkyl, for example, methyl, ethyl, *n*-propyl, isopropyl, *n*-butyl, *sec-*butyl, isobutyl or *tert*-butyl*.*

In a certain embodiment, when p is 0,

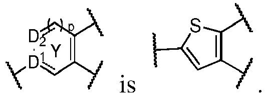

In a certain embodiment, when 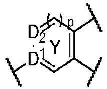 is "5- to 7-membered heterocycloalkenyl containing 1-3 heteroatoms independently selected from O, S and N", the "5- to 7-membered heterocycloalkenyl containing 1-3 heteroatoms independently selected from O, S and N" is "5- to 7-membered heterocycloalkenyl containing 1-3 heteroatoms independently selected from O and N", for example, "5- to 6-membered heterocycloalkenyl containing 1-2 heteroatoms independently selected from O and N", for another example, "6-membered heterocycloalkenyl containing 1 heteroatom being O or N", for yet another example, "5- to 6-membered heterocycloalkenyl containing 1 heteroatom being O or N", for still yet another example, 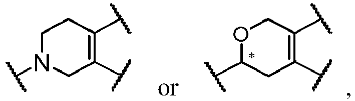 and for further yet another example, 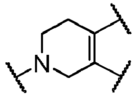 or

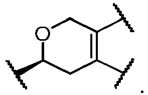

In a certain embodiment, when 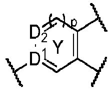 is "5- to 7-membered heteroaryl containing 1-3 heteroatoms independently selected from O, S and N", the "5- to 7-membered heteroaryl containing 1-3 heteroatoms independently selected from O, S and N" is "6-membered heteroaryl containing 1-2 heteroatoms independently selected from O, S and N", for example, "6-membered heteroaryl containing 1 heteroatom being N", for another example, pyridinyl, and for yet another example,

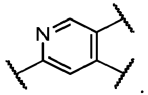

In a certain embodiment, when 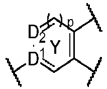 is 5- to 7-membered cycloalkenyl, the 5- to 7-membered cycloalkenyl is 5- to 6-membered cycloalkenyl, for example, cyclopentenyl or cyclohexenyl.

In a certain embodiment, when R⁵ is independently halogen, the halogen is fluorine, chlorine, bromine or iodine, for example, fluorine.

In a certain embodiment, when R⁵ is independently C₁₋₆ alkyl, the C₁₋₆ alkyl is C₁₋₄ alkyl, for example, methyl, ethyl, *n*-propyl, isopropyl, *n*-butyl, *sec*-butyl, isobutyl or *tert*-butyl*.*

In a certain embodiment, when L₁ is C₁₋₆ alkylene, the C₁₋₆ alkylene can be -CH₂-, -CH₂CH₂-, -CH₂CH₂CH₂-, -CH(CH₃)CH₂-, -CH₂CH₂CH₂CH₂-, -CH(CH₃)CH₂CH₂-, -CH₂CH(CH₃)CH₂- or - C(CH₃)₂CH₂-.

In a certain embodiment, when R¹ is C₆₋₂₀ aryl or C₆₋₂₀ aryl substituted with one or more R¹⁻¹, the C₆₋₂₀ aryl and the C₆₋₂₀ aryl in the C₆₋₂₀ aryl substituted with one or more R¹⁻¹ are C₆₋₁₀ aryl, for example, phenyl or naphthyl.

In a certain embodiment, when R¹ is Cs-n benzocycloalkenyl, the Cs-n benzocycloalkenyl is benzocyclobutenyl, benzocyclopentenyl or benzocyclohexenyl(for example, 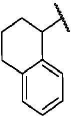 ).

In a certain embodiment, when R¹ is "5- to 12-membered heteroaryl containing 1-4 heteroatoms independently selected from O, S and N" or "5- to 12-membered heteroaryl containing 1-4 heteroatoms independently selected from O, S and N" substituted with one or more R¹⁻², the "5- to 12-membered heteroaryl containing 1-4 heteroatoms independently selected from O, S and N" and the "5- to 12-membered heteroaryl containing 1-4 heteroatoms independently selected from O, S and N" in the "5- to 12-membered heteroaryl containing 1-4 heteroatoms independently selected from O, S and N" substituted with one or more R¹⁻² are "5- to 9-membered heteroaryl containing 1-2 heteroatoms independently selected from O, S and N", for example, "5-to 9-membered heteroaryl containing 1 heteroatom being N", for another example, quinolinyl, and for yet another example, 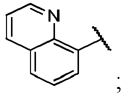 for another example, "9-membered heteroaryl containing 2 heteroatoms independently selected from O, S and N", for another example, indazolyl, and for yet another example,

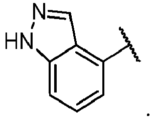

In a certain embodiment, when R^{b}, R¹⁻¹ and R¹⁻² are independently halogen, the halogen is fluorine, chlorine, bromine or iodine, for example, fluorine or chlorine.

In a certain embodiment, when R^{b}, R¹⁻¹ and R¹⁻² are independently C₁₋₆ alkyl or C₁₋₆ alkyl substituted with one or more R¹⁻¹⁻¹, the C₁₋₆ alkyl and the C₁₋₆ alkyl in the C₁₋₆ alkyl substituted with one or more R¹⁻¹⁻¹ are C₁₋₄ alkyl, for example, methyl, ethyl, *n*-propyl, isopropyl, *n*-butyl, *sec-*butyl, isobutyl or *tert-*butyl*.*

In a certain embodiment, when R^{b}, R¹⁻¹ and R¹⁻² are independently C₁₋₆ alkyl substituted with one or more R¹⁻¹⁻¹, the more R¹⁻¹⁻¹ is 2 or 3 R¹⁻¹⁻¹.

In a certain embodiment, when R^{b}, R¹⁻¹ and R¹⁻² are independently C₂₋₆ alkenyl, the C₂₋₆ alkenyl is C₂₋₃ alkenyl, for example, vinyl, propenyl or allyl.

In a certain embodiment, when R^{b}, R¹⁻¹ and R¹⁻² are independently C₂₋₆ alkynyl, the C₂₋₆ alkynyl is C₂₋₃ alkynyl, for example, ethynyl, propynyl or propargyl.

In a certain embodiment, when R^{b}, R¹⁻¹ and R¹⁻² are independently C₁₋₆ alkyl-O- substituted with one or more R¹⁻¹⁻², the C₁₋₆ alkyl in the C₁₋₆ alkyl-O- and the C₁₋₆ alkyl-O- substituted with one or more R¹⁻¹⁻² are C₁₋₄ alkyl, for example, methyl, ethyl, *n*-propyl, isopropyl, *n*-butyl, *sec*-butyl, isobutyl or *tert*-butyl*,* and for another example, methyl.

In a certain embodiment, when R^{b}, R¹⁻¹ and R¹⁻² are independently C₃₋₁₀ cycloalkyl or C₃₋₁₀ cycloalkyl substituted with one or more R¹⁻¹⁻³, the C₃₋₁₀ cycloalkyl and the C₃₋₁₀ cycloalkyl in the C₃₋₁₀ cycloalkyl substituted with one or more R¹⁻¹⁻³ are C₃-C₆ cycloalkyl, for example, cyclohexyl, cyclopentyl, cyclobutyl or cyclopropyl, and for another example, cyclopropyl.

In a certain embodiment, when R^{b}, R¹⁻¹ and R¹⁻² are independently "5- to 7-membered heterocycloalkyl containing 1 or 2 heteroatoms independently selected from O and N" or "5- to 7-membered heterocycloalkyl containing 1 or 2 heteroatoms independently selected from O and N" substituted with one or more R¹⁻¹⁻⁴, the "5- to 7-membered heterocycloalkyl containing 1 or 2 heteroatoms independently selected from O and N" and the "5- to 7-membered heterocycloalkyl containing 1 or 2 heteroatoms independently selected from O and N" in the "5- to 7-membered heterocycloalkyl containing 1 or 2 heteroatoms independently selected from O and N" substituted with one or more R¹⁻¹⁻⁴ are "5- to 6-membered heterocycloalkyl containing 1 or 2 heteroatoms independently selected from O and N", for example, "5- to 6-membered heterocycloalkyl containing 1 heteroatom being O or N".

In a certain embodiment, when R^{b}, R¹⁻¹ and R¹⁻² are independently C₆₋₂₀ aryl or C₆₋₂₀ aryl substituted with one or more R¹⁻¹⁻⁵, the C₆₋₂₀ aryl and the C₆₋₂₀ aryl in the C₆₋₂₀ aryl substituted with one or more R¹⁻¹⁻⁵ are C₆₋₁₀ aryl, for example, phenyl or naphthyl.

In a certain embodiment, when R^{b}, R¹⁻¹ and R¹⁻² are independently "5- to 7-membered heteroaryl containing 1 or 2 heteroatoms independently selected from O and N" or "5- to 7-membered heteroaryl containing 1 or 2 heteroatoms independently selected from O and N" substituted with one or more R¹⁻¹⁻⁶, the "5- to 7-membered heteroaryl containing 1 or 2 heteroatoms independently selected from O and N" and the "5- to 7-membered heteroaryl containing 1 or 2 heteroatoms independently selected from O and N" in the "5- to 7-membered heteroaryl containing 1 or 2 heteroatoms independently selected from O and N" substituted with one or more R¹⁻¹⁻⁶ are "5- to 6-membered heteroaryl containing 1 or 2 heteroatoms independently selected from O and N", for example, "5- to 6-membered heteroaryl containing 1 heteroatom being O or N".

In a certain embodiment, when two optional R¹⁻¹ or R¹⁻² are connected, together with the atoms on the ring to which they are attached, independently form 3- to 8-membered cyclic olefin, the 3- to 8-membered cyclic olefin is cyclobutene, cyclopentene or cyclohexene.

In a certain embodiment, when R^{c}, R¹² and R¹³ are independently C₁₋₆ alkyl, the C₁₋₆ alkyl is methyl, ethyl, *n*-propyl, isopropyl, *n*-butyl, *sec*-butyl, isobutyl or *tert-*butyl*.*

In a certain embodiment, when R^{c1} is C₁₋₆ alkyl substituted with -NH₂, the R^{c1} is C₅ alkyl substituted with -NH₂, for example, 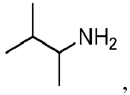 for another example,

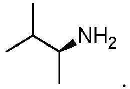

In a certain embodiment, when R² is C₁₋₃ alkyl, the C₁₋₃ alkyl is methyl, ethyl, n-propyl or isopropyl.

In a certain embodiment, when R^{c1}, R^{c2}, R^{c3}, R^{c4} and R^{c5} are independently C₁₋₆ alkyl or C₁₋₆ alkyl substituted with one or more R⁴⁻¹⁻¹, the C₁₋₆ alkyl and the C₁₋₆ alkyl in the C₁₋₆ alkyl substituted with one or more R⁴⁻¹⁻¹ are methyl, ethyl, *n*-propyl, isopropyl, *n*-butyl, *sec*-butyl, isobutyl or *tert-*butyl*.*

In a certain embodiment, when R^{c1}, R^{c2}, R^{c3}, R^{c4} and R^{c5} are independently C₃₋₁₀ cycloalkyl or C₃₋₁₀ cycloalkyl substituted with one or more R⁴⁻¹⁻², the C₃₋₁₀ cycloalkyl and the C₃₋₁₀ cycloalkyl in the C₃₋₁₀ cycloalkyl substituted with one or more R⁴⁻¹⁻² are cyclohexyl, cyclopentyl, cyclobutyl or cyclopropyl.

In a certain embodiment, when R^{c1}, R^{c2}, R^{c3}, R^{c4} and R^{c5} are independently C₆₋₂₀ aryl or C₆₋₂₀ aryl substituted with one or more R⁴⁻¹⁻⁴, the C₆₋₂₀ aryl and the C₆₋₂₀ aryl in the C₆₋₂₀ aryl substituted with one or more R⁴⁻¹⁻⁴ are C₆₋₁₀ aryl, for example, phenyl or naphthyl.

In a certain embodiment, when R¹⁻¹⁻¹, R¹⁻¹⁻², R¹⁻¹⁻³, R¹⁻¹⁻⁴, R¹⁻¹⁻⁵, R¹⁻¹⁻⁶, R⁴⁻¹⁻¹, R⁴⁻¹⁻², R⁴⁻¹⁻³, R⁴⁻¹⁻⁴ and R⁴⁻¹⁻⁵ are independently halogen, the halogen is fluorine, chlorine, bromine or iodine.

In a certain embodiment, when R¹⁻¹⁻¹, R¹⁻¹⁻², R¹⁻¹⁻³, R¹⁻¹⁻⁴, R¹⁻¹⁻⁵, R¹⁻¹⁻⁶, R⁴⁻¹⁻¹, R⁴⁻¹⁻², R⁴⁻¹⁻³, R⁴⁻¹⁻⁴ and R⁴⁻¹⁻⁵ are independently C₁₋₆ alkyl-O-, the C₁₋₆ alkyl in the C₁₋₆ alkyl-O- is C₁₋₄ alkyl, for example, methyl, ethyl, *n*-propyl, isopropyl, *n-*butyl, *sec*-butyl, isobutyl or *tert-*butyl*.*

In a certain embodiment, when R¹⁻¹⁻¹, R¹⁻¹⁻², R¹⁻¹⁻³, R¹⁻¹⁻⁴, R¹⁻¹⁻⁵, R¹⁻¹⁻⁶, R⁴⁻¹⁻¹, R⁴⁻¹⁻², R⁴⁻¹⁻³, R⁴⁻¹⁻⁴ and R⁴⁻¹⁻⁵ are independently C₁₋₆ alkyl, the C₁₋₆ alkyl is C₁₋₄ alkyl, for example, methyl, ethyl, *n*-propyl, isopropyl, *n*-butyl, *sec*-butyl, isobutyl or *tert-*butyl*.*

In a certain embodiment, when R¹¹, R²¹, R²², R²³, R¹⁴, R²⁴, R¹⁵, R²⁵, R¹⁶ and R²⁶ are independently C₁₋₆ alkyl, the C₁₋₆ alkyl is C₁₋₄ alkyl, for example, methyl, ethyl, *n*-propyl, isopropyl, *n*-butyl, *sec*-butyl, isobutyl or *tert-butyl.*

In a certain embodiment, when L₂ is C₁₋₆ alkylene, the C₁₋₆ alkylene is -CH₂-, -CH₂CH₂-, - CH₂CH₂CH₂-, -CH(CH₃)CH₂-, -CH₂CH₂CH₂CH₂-, -CH(CH₃)CH₂CH₂-, -CH₂CH(CH₃)CH₂- or - C(CH₃)₂CH₂-.

In a certain embodiment, when R^{L-1}, R^{L-2} or R^{L-4} is independently C₁₋₆ alkylene, the C₁₋₆ alkylene can be C₁₋₄ alkylene, for example -CH₂-, -CH₂CH₂-, -CH₂CH₂CH₂-, -CH(CH₃)CH₂-, - CH₂CH₂CH₂CH₂-, -CH(CH₃)CH₂CH₂-, -CH₂CH(CH₃)CH₂- or -C(CH₃)₂CH₂-, for another example, - CH₂-,-CH₂CH₂-,-CH₂CH₂CH₂-or-CH(CH₃)CH₂-.

In a certain embodiment, when R^{L-3} is C₁₋₆ alkyl, the C₁₋₆ alkyl can be C₁₋₄ alkyl, for example, methyl, ethyl, *n*-propyl, isopropyl, *n*-butyl, *sec*-butyl, isobutyl or *tert*-butyl*.*

In a certain embodiment, when R³ is C₃₋₁₂ cycloalkyl or C₃₋₁₂ cycloalkyl substituted with one or more R³⁻¹, the C₃₋₁₂ cycloalkyl and the C₃₋₁₂ cycloalkyl in the C₃₋₁₂ cycloalkyl substituted with one or more R³⁻¹ is C₃₋₁₀ cycloalkyl, the C₃₋₁₂ cycloalkyl can be a monocyclic alkyl, a bridged cycloalkyl or a spiral cycloalkyl, for example, cyclopropyl.

In a certain embodiment, when R³ is "4- to 12-membered heterocycloalkyl containing 1-3 heteroatoms selected from O, S and N", the "4- to 12-membered heterocycloalkyl containing 1-3 heteroatoms selected from O, S and N" is "4- to 8-membered heterocycloalkyl containing 1-3 heteroatoms independently selected from O, S and N", it also can be "5- to 8-membered heterocycloalkyl containing 1-2 heteroatoms independently selected from O and N".

In a certain embodiment, when R³ is "4- to 12-membered heterocycloalkyl containing 1-3 heteroatoms selected from O, S and N", the "4- to 12-membered heterocycloalkyl containing 1-3 heteroatoms selected from O, S and N" is a monocyclic cycloalkyl, a spiral cycloalky or a fused cycloalkyl; for example, azetidinyl, pyrrolidinyl, tetrahydrofuryl, hexahydro-1*H*-pyrrolizinyl, 7-azaspiro[3.5]nonanyl, 3-azaspiro[5.5]undecanyl, morphinyl or piperidinyl, for another example, 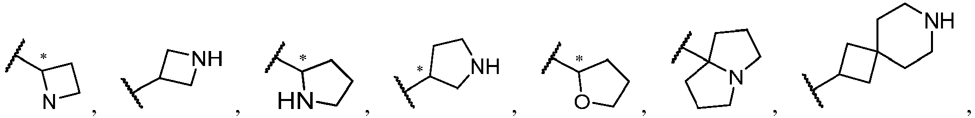 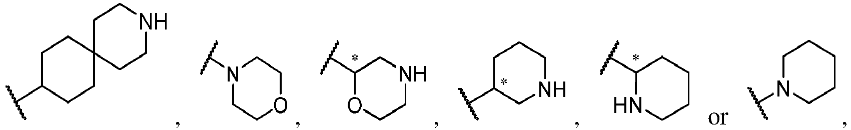 and for yet another example,

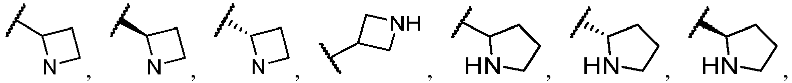

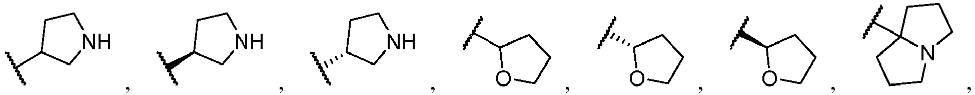

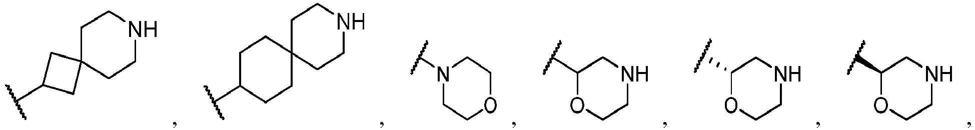

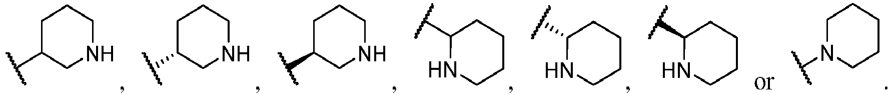

In a certain embodiment, when R³ is "4- to 12-membered heterocycloalkyl containing 1-3 heteroatoms selected from O, S and N" substituted with one or more R³⁻², the "4- to 12-membered heterocycloalkyl containing 1-3 heteroatoms selected from O, S and N" in the "4- to 12-membered heterocycloalkyl containing 1-3 heteroatoms selected from O, S and N" substituted with one or more R³⁻² is "5- to 11-membered heterocycloalkyl containing 1-2 heteroatoms selected from O and N".

In a certain embodiment, when R³ is "4- to 12-membered heterocycloalkyl containing 1-3 heteroatoms selected from O, S and N" substituted with one or more R³⁻², the "4- to 12-membered heterocycloalkyl containing 1-3 heteroatoms selected from O, S and N" is a monocyclic alkyl, a spiral cycloalky or fused cycloalkyl; the "4- to 12-membered heterocycloalkyl containing 1-3 heteroatoms independently selected from O, S and N" substituted with one or more R³⁻² is, for example, 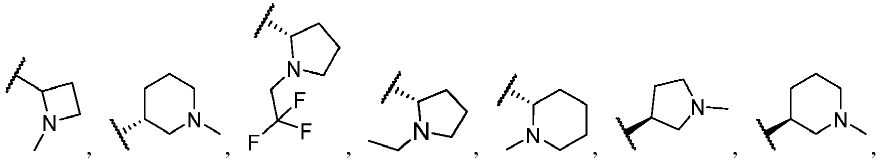 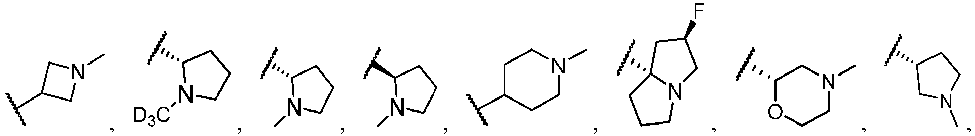 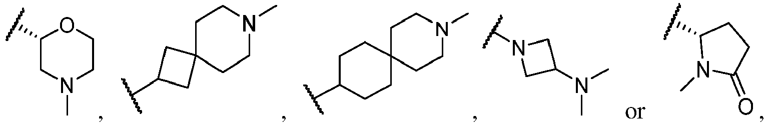 for another example, 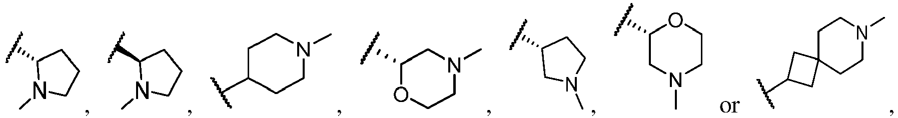 and for yet another example,

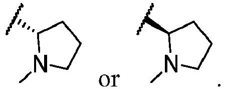

In a certain embodiment, when R³ is "4- to 12-membered heterocycloalkyl containing 1-3 heteroatoms independently selected from O, S and N" substituted with one or more R³⁻², the R³ is 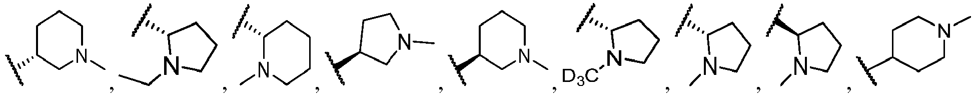 or 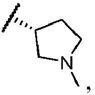 for example,

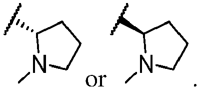

In a certain embodiment, when R³ is C₁₋₆ alkyl or C₁₋₆ alkyl substituted with one or more R³⁻³, the C₁₋₆ alkyl and the C₁₋₆ alkyl in the C₁₋₆ alkyl substituted with one or more R³⁻³ are C₁₋₄ alkyl, for example, methyl, ethyl, *n*-propyl, isopropyl, *n*-butyl, *sec*-butyl, isobutyl or *tert-*butyl*,* for another example, methyl.

In a certain embodiment, when R³⁻¹, R³⁻² and R³⁻³ are independently C₁₋₆ alkyl or C₁₋₆ alkyl substituted with one or more R³⁻¹⁻¹, the C₁₋₆ alkyl and the C₁₋₆ alkyl in the C₁₋₆ alkyl substituted with one or more R³⁻¹⁻¹ are C₁₋₄ alkyl, for example, methyl, ethyl, *n*-propyl, isopropyl, *n*-butyl, *sec-*butyl, isobutyl or *tert-*butyl*.*

In a certain embodiment, when R³⁻¹, R³⁻² and R³⁻³ are independently C₁₋₆ alkyl-O-, the C₁₋₆ alkyl in the C₁₋₆ alkyl-O- is C₁₋₄ alkyl, for example, methyl, ethyl, *n*-propyl, isopropyl, *n*-butyl, *sec*-butyl, isobutyl or *tert*-butyl.

In a certain embodiment, when R³⁻¹, R³⁻² and R³⁻³ are independently halogen, the halogen is fluorine, chlorine, bromine or iodine, for example, fluorine.

In a certain embodiment, when R^{d}, R^{d1}, R^{e1}, R^{e2}, R^{e3} and R^{e4} are independently C₁₋₆ alkyl or C₁₋₆ alkyl substituted with one or more R³⁻¹⁻², the C₁₋₆ alkyl and the C₁₋₆ alkyl in the C₁₋₆ alkyl substituted with one or more R³⁻¹⁻² are C₁₋₄ alkyl, for example, methyl, ethyl, n-propyl, isopropyl, *n*-butyl, *sec*-butyl, isobutyl or *tert-*butyl*,* for another example, methyl.

In a certain embodiment, when R^{d}, R^{d1}, R^{e1}, R^{e2}, R^{e3} and R^{e4} are independently C₃₋₁₀ cycloalkyl, the C₃₋₁₀ cycloalkyl is C₃₋₆ cycloalkyl, for example, cyclohexyl, cyclopentyl, cyclobutyl or cyclopropyl, for another example, cyclopropyl.

In a certain embodiment, when R^{d}, R^{d1}, R^{e1}, R^{e2}, R^{e3} and R^{e4} are independently "4- to 10-membered heterocycloalkyl containing 1-3 heteroatoms independently selected from O and N", the "4- to 10-membered heterocycloalkyl containing 1-3 heteroatoms independently selected from O and N" is "4- to 6-membered heterocycloalkyl containing 1-3 heteroatoms independently selected from O and N", for example, "4- to 6-membered heterocycloalkyl containing 1-2 heteroatoms independently selected from O and N".

In a certain embodiment, when R³⁻¹⁻¹ and R³⁻¹⁻² are independently halogen, the halogen is fluorine, chlorine, bromine or iodine.

In a certain embodiment, when R³⁻¹⁻¹ and R³⁻¹⁻² are independently C₁₋₆ alkyl-O-, the C₁₋₆ alkyl in the C₁₋₆ alkyl-O- is C₁₋₄ alkyl, for example, methyl, ethyl, n-propyl, isopropyl, n-butyl, *sec-*butyl, isobutyl or *tert*-butyl*.*

In a certain embodiment, when R^{e5}, R^{e6}, R^{e7}, R^{e8}, R^{e9}, R^{e10}, R^{e11}, R^{e12}, R^{e13} and R^{e14} are independently C₁₋₆ alkyl, the C₁₋₆ alkyl is C₁₋₄ alkyl, for example, methyl, ethyl, *n*-propyl, isopropyl, *n*-butyl, *sec-*butyl, isobutyl or *tert-*butyl*.*

In a certain embodiment, 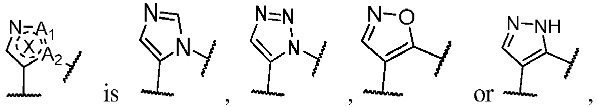 for example,

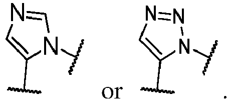

In a certain embodiment, R² is -CH₃, 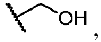 -NH₂, -CN, 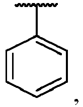 F, -Br, -Cl, 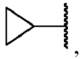 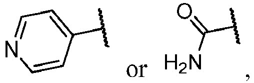 for example, -CH₃, 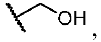 -NH₂, -CN, 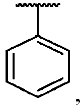 -Br or 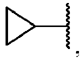 and for another example, -CH₃, 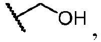 -CN, -Br or

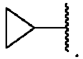

In a certain embodiment, 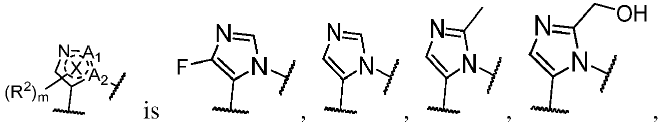 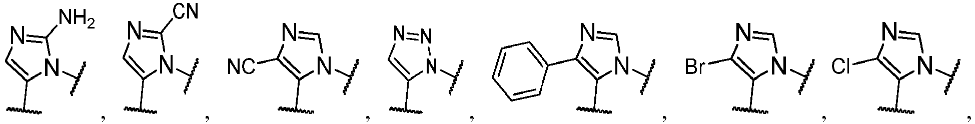 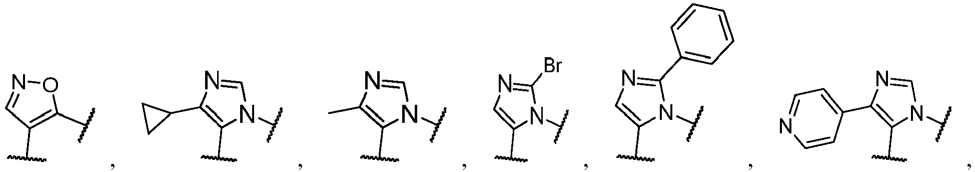 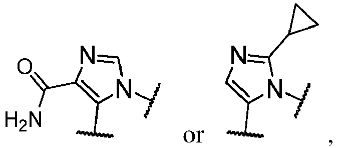 for example, 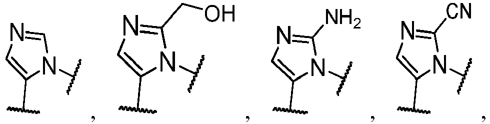 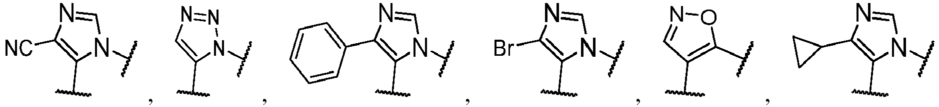 or 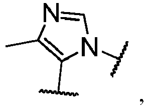 and for another example,

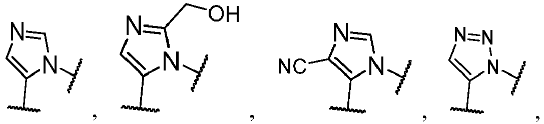

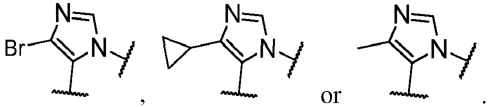

In a certain embodiment,

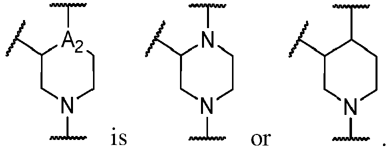

In a certain embodiment, R⁴ is -CH₃, 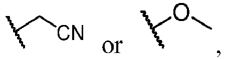 for example, -CH₃ or

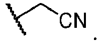

In a certain embodiment, 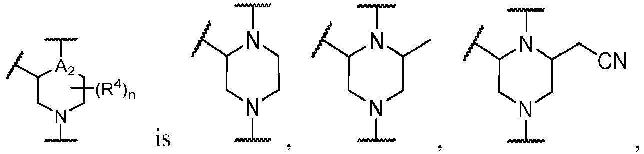 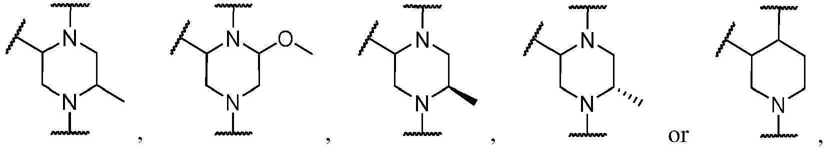 for example, 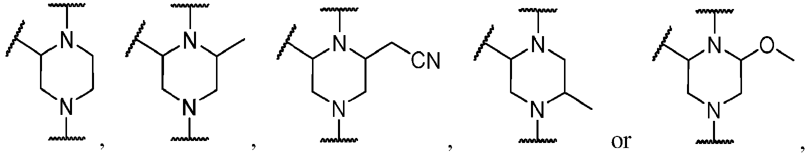 and for another example,

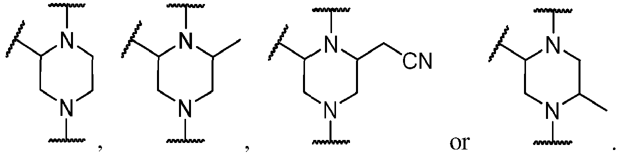

In a certain embodiment, 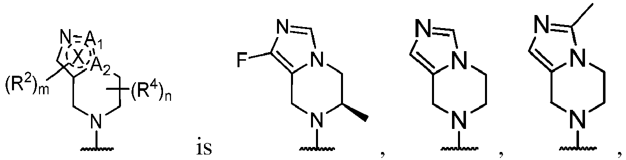 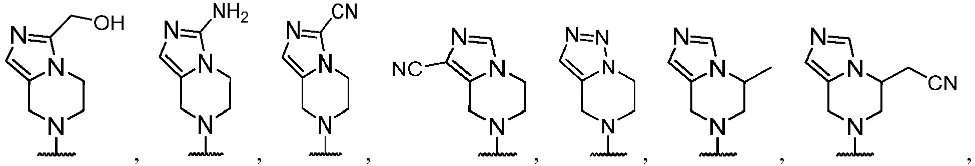 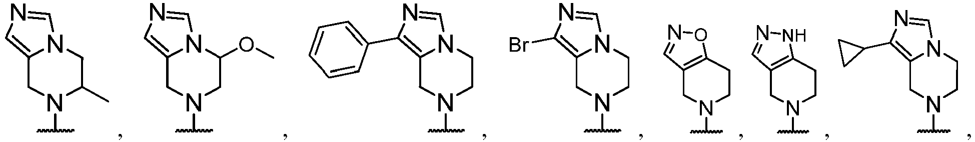 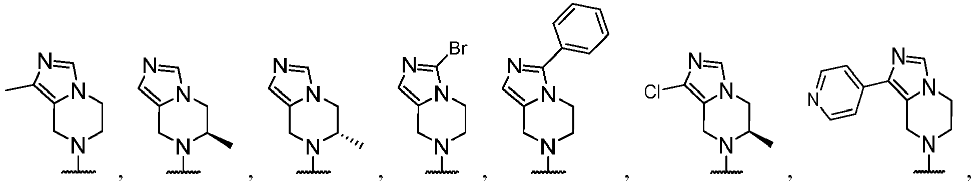 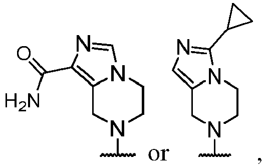 for example 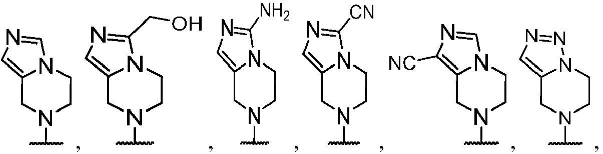 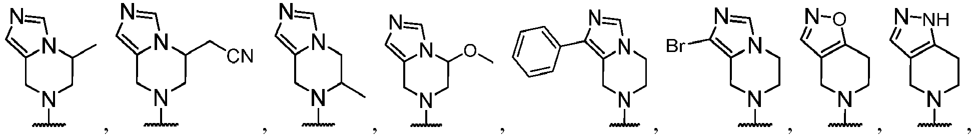 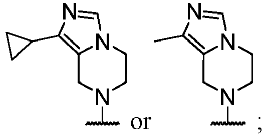 for another example,

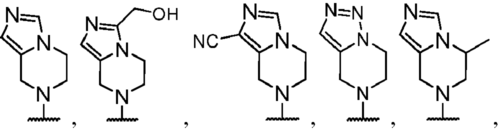

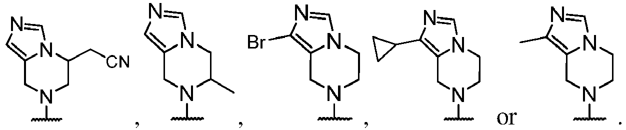

In a certain embodiment, 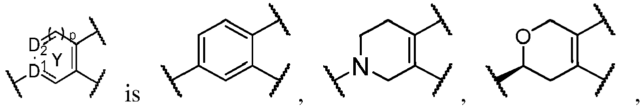 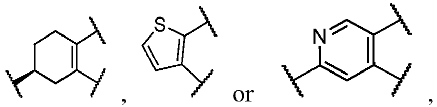 for example, 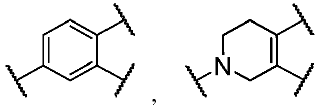 or 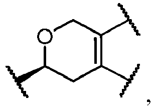 for another example, 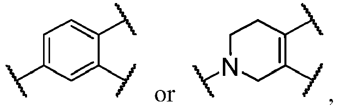 and for yet another example,

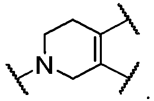

In a certain embodiment, 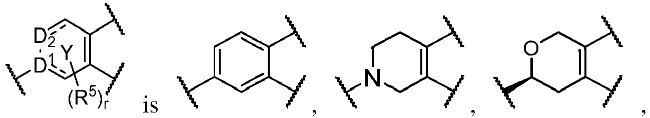 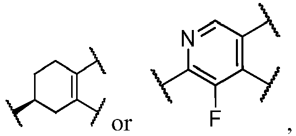 for example, 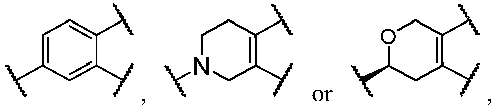 for another example, 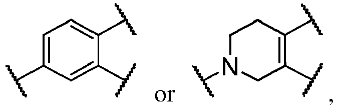 and for yet another example,

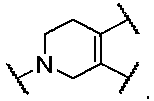

In a certain embodiment,

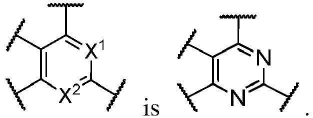

In a certain embodiment, 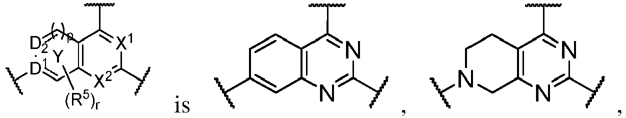 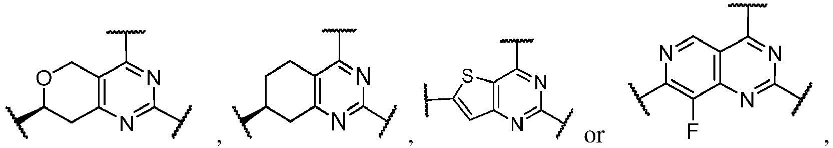 for example, 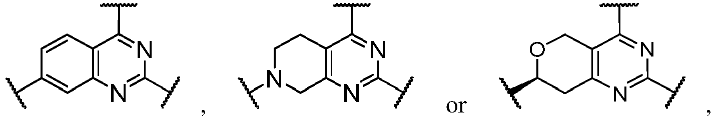 for another example, 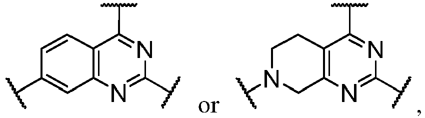 and for yet another example,

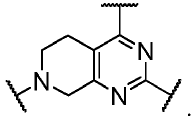

In a certain embodiment, R^{b}, R¹⁻¹ and R¹⁻² are independently hydroxyl, F, Cl, CF₃, methyl, methyl-O- , cyclopropyl, isopropyl, NH₂-,

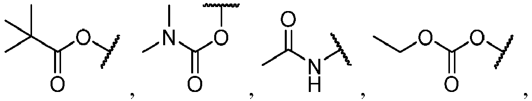

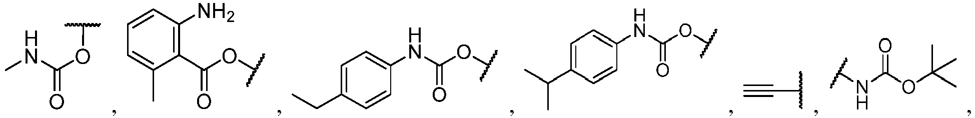

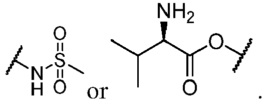

In a certain embodiment, R¹ is 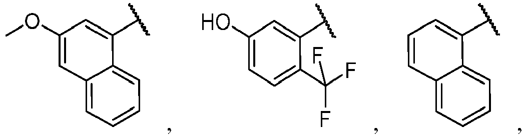 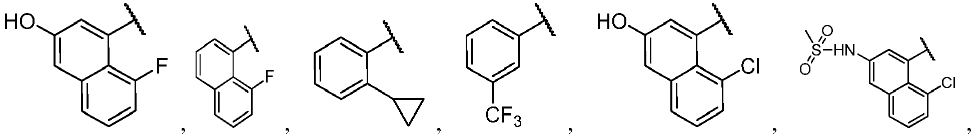 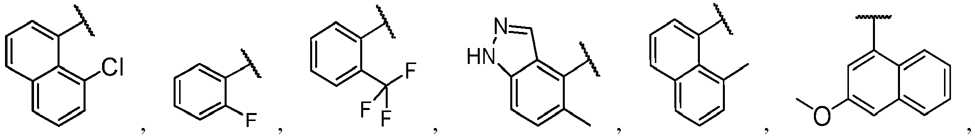 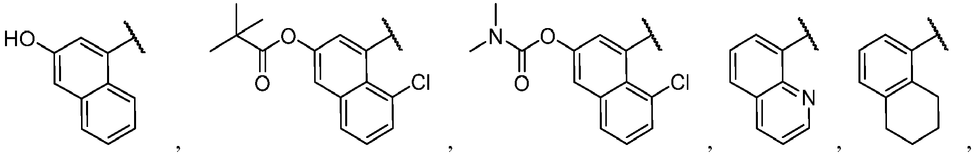 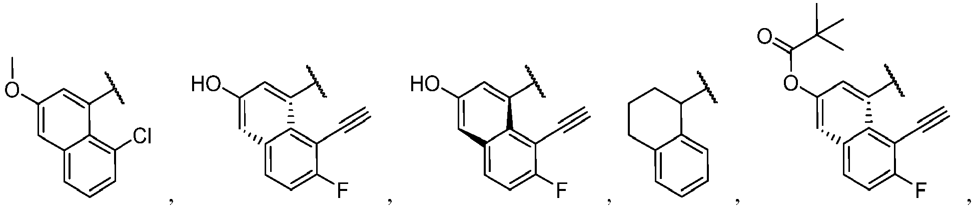 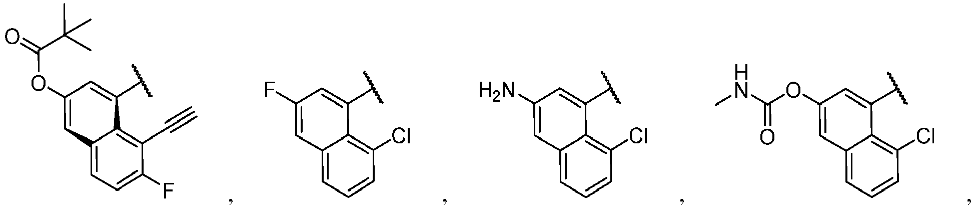 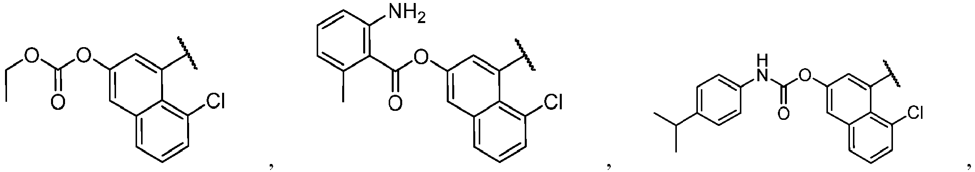 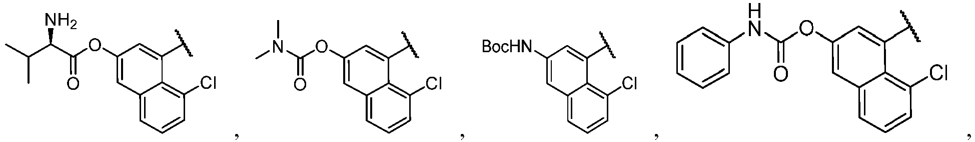 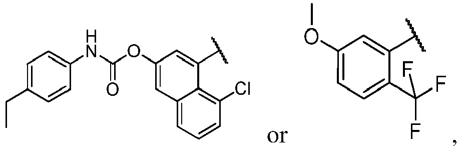 for example,

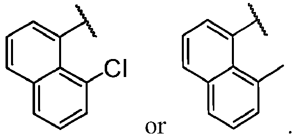

In a certain embodiment, 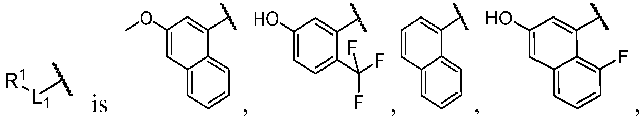 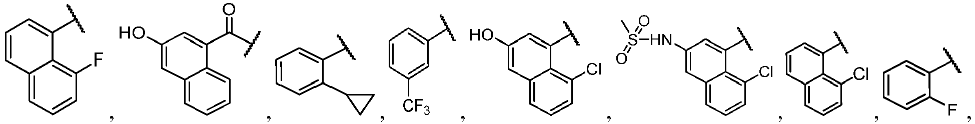 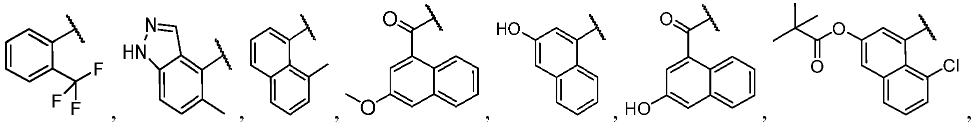 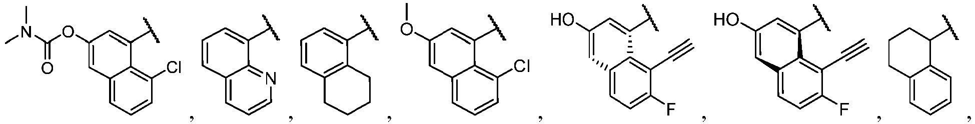 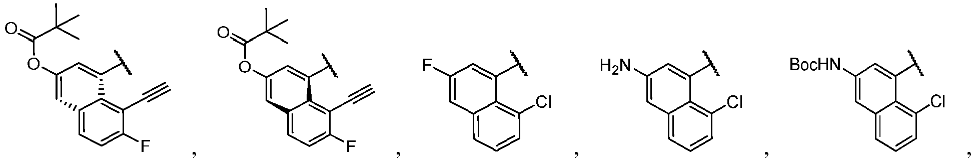 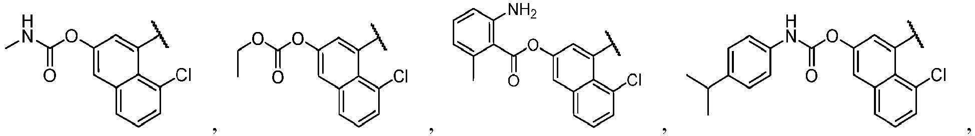 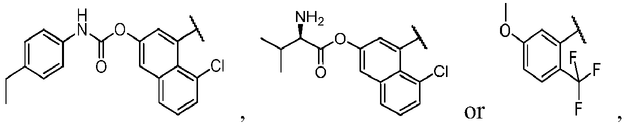 for example, 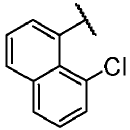 or

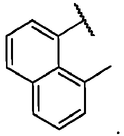

In a certain embodiment, L₂ is 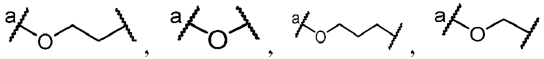 or 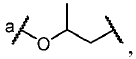 with position a connected to position b of ring

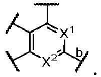

In a certain embodiment, R³⁻¹⁻¹ and R³⁻¹⁻² are independently deuterium (D), F or -N(CH₃)₂.

In a certain embodiment, R³⁻¹, R³⁻² and R³⁻³ are independently F, O=, 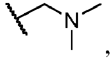 methyl, ethyl, 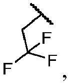 -CD₃ or -N(CH₃)₂.

In a certain embodiment, R³ is methyl, 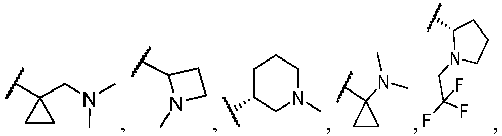 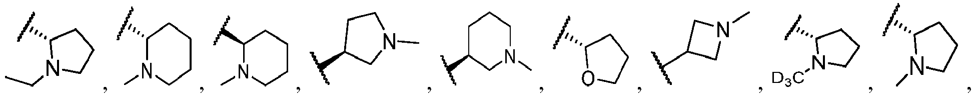 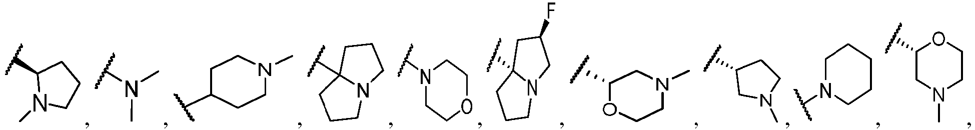 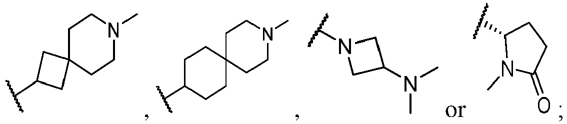 for example, methyl, 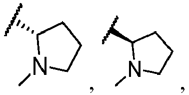 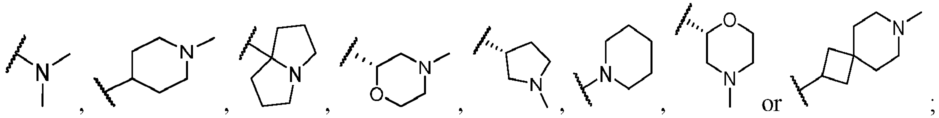 for another example,

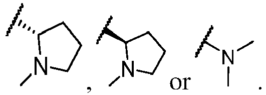

In a certain embodiment, 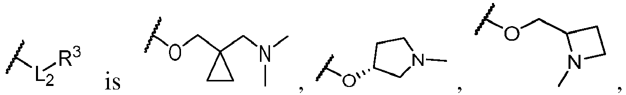 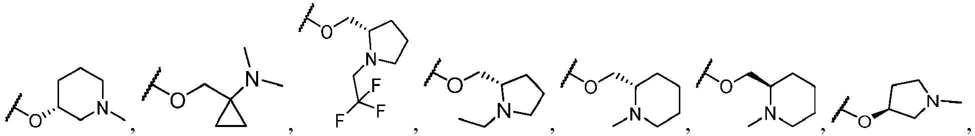 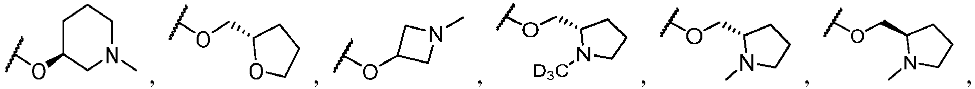 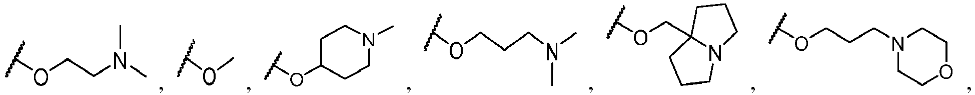 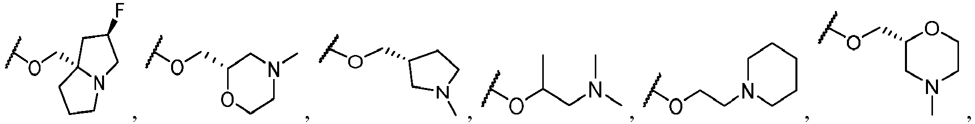 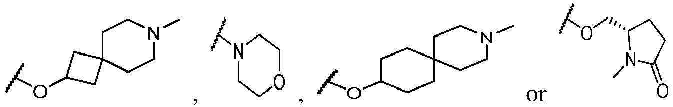 ,for example, 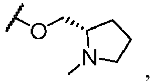 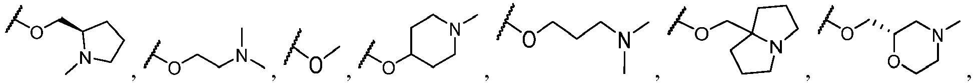 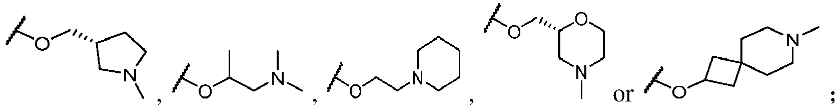 for another example

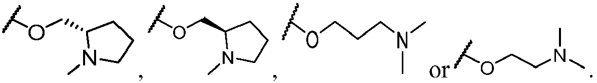

In a certain embodiment, the nitrogen-containing heterocyclic compound of formula I, having the structure shown below: 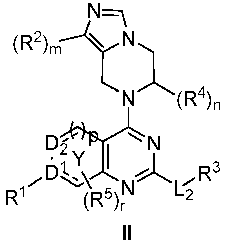 wherein, "=-=" represents a single or double bond;
m is 0 or 1;
R² is -CN, C₁₋₆ alkyl, halogen, C₃₋₁₀ cycloalkyl or C₆₋₂₀ aryl;
n is 0 or 1;
R⁴ is independently C₁₋₆ alkyl, or C₁₋₆ alkyl substituted with one or more R⁴⁻¹;
R⁴⁻¹ is independently halogen;
p is 0 or 1; 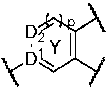
is phenyl, "6-membered heterocycloalkenyl containing 1 heteroatom selected from O and N", cyclohexenyl, pyridyl or thiophenyl; wherein D¹ is C, CH or N; D₂ is 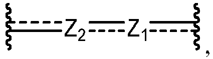 wherein Z₁ and Z₂ are independently a bond, CH, CH₂, O, S or N;
r is 0 or 1;
R⁵ is independently halogen or C₁₋₂ alkyl;
R¹ is C₆₋₂₀ aryl, "5- to 12-membered heteroaryl containing 1-4 heteroatoms selected from O, S and N", C₆₋₂₀ aryl substituted with one or more R¹⁻¹ or "5- to 12-membered heteroaryl containing 1-4 heteroatoms selected from O, S and N" substituted with one or more R¹⁻²; provided that when multiple substituents are present, the substituents are the same or different;
R¹⁻¹ and R¹⁻² are independently halogen, -OR^{c}, -NR¹²R¹³, C₁₋₆ alkyl, C₁₋₆ alkyl-O- substituted with one or more R¹⁻¹⁻²; provided that when multiple substituents are present, the substituents are the same or different;
R¹⁻¹⁻² is C₁₋₆ alkyl-O-;
R^{c} is hydrogen, C₁₋₆ alkyl, C(=O)R^{c1}, -C(=O)OR^{c2}, or -C(=O)NR^{c3}R^{c4}; R^{c1} is C₁₋₆ alkyl, C₁₋₆ alkyl substituted with -NH₂, C₆₋₂₀ aryl, or, C₆₋₂₀ aryl substituted with one or more R⁴⁻¹⁻⁴; R^{c3} and R^{c4} are independently C₆₋₂₀ aryl, or, C₆₋₂₀ aryl substituted with one or more R⁴⁻¹⁻⁴.
R⁴⁻¹⁻⁴ is halogen, C₁₋₆ alkyl or -NH₂;
R^{c2} is C₁₋₆ alkyl;
R¹² and R¹³are independently hydrogen, C₁₋₆ alkyl, C(=O)R^{c1}, or, -SO₂R^{c5}; R^{c1} and R^{c5} are independently C₁₋₆ alkyl;
L₂ is -O(R^{L-1})ₙ₁-; R^{L-1} is C₁₋₆ alkylene; n1 is 0 or 1;
R³ is "4- to 12-membered heterocycloalkyl containing 1-3 heteroatoms selected from O, S and N", "4- to 12-membered heterocycloalkyl containing 1-3 heteroatoms selected from O, S and N" substituted with one or more R³⁻², or -NR^{e1}R^{e2}; provided that when multiple substituents are present, the substituents are the same or different;
R³⁻² is independently C₁₋₆ alkyl, C₁₋₆ alkyl substituted with one or more R³⁻¹⁻¹ or halogen;
R³⁻¹⁻¹ is independently deuterium or halogen;
R^{e1} and R^{e2} are independently hydrogen or C₁₋₆ alkyl.

In a certain embodiment, the nitrogen-containing heterocyclic compound of formula I as described above, having the structure shown below: 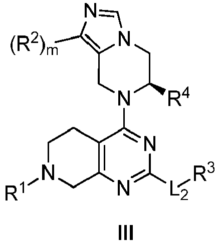
m is 0 or 1;
R² is -CN, C₁₋₃ alkyl or halogen;
R⁴ is independently C₁₋₆ alkyl;
R¹ is naphthyl, quinolinyl, naphthyl substituted with one or more R¹⁻¹ or, quinolinyl substituted with one or more R¹⁻²; provided that when multiple substituents are present, the substituents are the same or different;
R¹⁻¹ and R¹⁻² are independently halogen, -OR^{c}, -NR¹²R¹³, C₁₋₆ alkyl, or C₁₋₆ alkyl-O-substituted with one or more R¹⁻¹⁻²; provided that when multiple substituents are present, the substituents are the same or different;
R¹⁻¹⁻² is C₁₋₆ alkyl-O-;
R^{c} is hydrogen, C₁₋₆ alkyl, C(=O)R^{c1}, -C(=O)OR^{c2}, or -C(=O)NR^{c3}R^{c4}; R^{c1} is C₁₋₆ alkyl, C₁₋₆ alkyl substituted with -NH₂, C₆₋₂₀ aryl, or, C₆₋₂₀ aryl substituted with one or more R⁴⁻¹⁻⁴; R^{c3} and R^{c4} are independently C₆₋₂₀ aryl, or, C₆₋₂₀ aryl substituted with one or more R⁴⁻¹⁻⁴; R⁴⁻¹⁻⁴ is independently halogen, C₁₋₆ alkyl or -NH₂;
R^{c2} is C₁₋₆ alkyl;
R¹² and R¹³ are independently hydrogen, C₁₋₆ alkyl, C(=O)R^{c1} or -SO₂R^{c5}; R^{c1} and R^{c5} are independently C₁₋₆ alkyl;
L₂ is -O(R^{L-1})ₙ₁-; R^{L1} is C₁₋₆ alkylene; n1 is 1;
R³ is "5- to 6-membered heterocycloalkyl containing 1 heteroatom of N" substituted with one or more R³⁻², or -NR^{e1}R^{e2}; provided that when multiple substituents are present, the substituents are the same or different;
R³⁻² is independently C₁₋₂ alkyl, or C₁₋₆ alkyl substituted with one or more R³⁻¹⁻¹;
R³⁻¹⁻¹ is independently deuterium;
R^{e1} and R^{e2} are independently hydrogen or C₁₋₆ alkyl.

In a certain embodiment, the nitrogen-containing heterocyclic compound of formula I as described above, having the structure shown below: 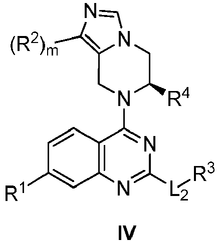
m is 0 or 1;
R² is -CN, C₁₋₃ alkyl or halogen;
R⁴ is C₁₋₆ alkyl;
R¹ is naphthyl, quinolinyl, naphthyl substituted with one or more R¹⁻¹ or, quinolinyl substituted with one or more R¹⁻²; provided that when multiple substituents are present, the substituents are the same or different;
R¹⁻¹ and R¹⁻² are independently halogen, -OR^{c}, -NR¹²R¹³, C₁₋₆ alkyl, or, C₁₋₆ alkyl-O-substituted with one or more R¹⁻¹⁻²; provided that when multiple substituents are present, the substituents are the same or different;
R¹⁻¹⁻² is C₁₋₆ alkyl-O-;
R^{c} is hydrogen, C₁₋₆ alkyl, C(=O)R^{c1}, -C(=O)OR^{c2}, or -C(=O)NR^{c3}R^{c4}; R^{c1} is C₁₋₆ alkyl, C₁₋₆ alkyl substituted with -NH₂, C₆₋₂₀ aryl, or, C₆₋₂₀ aryl substituted with one or more R⁴⁻¹⁻⁴; R^{c3} and R^{c4} are independently C₆₋₂₀ aryl, or, C₆₋₂₀ aryl substituted with one or more R⁴⁻¹⁻⁴; R⁴⁻¹⁻⁴ is halogen, C₁₋₆ alkyl or -NH₂; R^{c2} is C₁₋₆ alkyl;
R¹² and R¹³ are independently hydrogen, C₁₋₆ alkyl, C(=O)R^{c1}, or, -SO₂R^{c5}; R^{c1} and R^{c5} are independently C₁₋₆ alkyl;
L₂ is -O(R^{L-1})ₙ₁-; R^{L-1} is C₁₋₆ alkylene; n1 is 1;
R³ is "5- to 6-membered heterocycloalkyl containing 1heteroatom of N" substituted with R³⁻², or -NR^{e1}R^{e2};
R³⁻² is C₁₋₂ alkyl;
R^{e1} and R^{e2} are independently hydrogen or C₁₋₆ alkyl.

In a certain embodiment, the nitrogen-containing heterocyclic compound of formula I, the pharmaceutically acceptable salt thereof, the stereoisomer thereof, the tautomer thereof or the isotopically labeled compound thereof as described above, nitrogen-containing heterocyclic compound of formula I is defined as solution 1, solution 2 or solution 3:

### solution 1:

A nitrogen-containing heterocyclic compound of formula I, a pharmaceutically acceptable salt thereof, a stereoisomer thereof, a tautomer thereof or an isotopically labeled compound thereof: 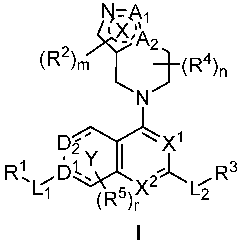 wherein "=-=" represents a single or double bond; 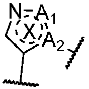
is nitrogen-containing 5-membered heteroaryl; A₁ is CH, O or N; A₂ is C or N; m is 0, 1 or 2;
R² is -CN, C₁₋₆ alkyl, C₂₋₆ alkenyl, C₂₋₆ alkynyl, C₁₋₆ alkyl substituted with one or more R²⁻¹, halogen, -OR^{2a}, -C(=O)R^{2b}, -NR^{2c1}R^{2c2}, -C(=O)OR^{2d}, -C(=O)NR^{2e1}R^{2e2}, C₃₋₁₀ cycloalkyl, C₃₋₁₀ cycloalkyl substituted with one or more R²⁻², "4- to 10-membered heterocycloalkyl containing 1-3 heteroatoms selected from O and N", "4- to 10-membered heterocycloalkyl containing 1-3 heteroatoms selected from O and N" substituted with one or more R²⁻³, C₆₋₂₀ aryl, C₆₋₂₀ aryl substituted with one or more R²⁻⁴, "5- to 12-membered heteroaryl containing 1-4 heteroatoms selected from O, S and N", or "5- to 12-membered heteroaryl containing 1-4 heteroatoms selected from O, S and N" substituted with one or more R²⁻⁵; provided that when multiple substituents are present, the substituents are the same or different;
R²⁻¹, R²⁻², R²⁻³, R²⁻⁴ and R²⁻⁵ are independently halogen, hydroxyl, cyano, C₁₋₆ alkyl, C₂₋₆ alkenyl, C₂₋₆ alkynyl, C₁₋₆ alkyl-O-, -C(=O)R³¹, -NR³²R³³, -C(=O)OR³⁴ or -C(=O)NR³⁵R³⁶;
R^{2a}, R^{2b}, R^{2c1}, R^{2e2}, R^{2d}, R^{2e1} and R^{2e2} are independently hydrogen or C₁₋₆ alkyl;
R³¹, R³², R³³, R³⁴, R³⁵ and R³⁶ are independently hydrogen or C₁₋₆ alkyl;
n is 0, 1, 2, 3, 4, 5 or 6;
R⁴ is independently C₁₋₆ alkyl, C₁₋₆ alkyl substituted with one or more R⁴⁻¹, C₁₋₆ alkyl-O-, O=, -C(=O)OR^{4a} or -C(=O)NR^{4b}R^{4c}; or, when n is 2, 3, 4, 5 or 6, two optional R⁴ are connected, together with the atoms on the ring to which they are attached, independently form 3- to 8-membered carbocyclic ring or 3- to 8-membered heterocyclic ring containing 1-3 heteroatoms selected from O, S and N;
R⁴⁻¹ is independently halogen, cyano, hydroxyl, C₁₋₆ alkyl-O-, -NR⁴ⁱR^{4j}, -C(=O)OR^{4d} or - C(=O)NR^{4e}R^{4f};
R^{4a}, R^{4b}, R^{4c}, R^{4d}, R^{4e}, R^{4f}, R⁴ⁱ and R^{4j} are independently hydrogen or C₁₋₆ alkyl; 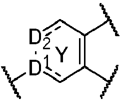
is phenyl, "5- to 7-membered heterocycloalkenyl containing 1-3 heteroatoms selected from O, S and N", "5- to 7-membered heteroaryl containing 1-3 heteroatoms selected from O, S and N" or 5- to 7-membered cycloalkenyl; wherein D¹ is C, CH or N; D₂ is 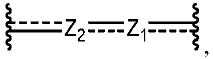 wherein Z₁ and Z₂ are independently a bond, CH, CH₂, O, S, N or NH;
r is 0, 1, 2, 3, 4, 5 or 6;
R⁵ is independently halogen or C₁₋₆ alkyl;
X¹ and X² are independently CR^{b} or N, and X¹ and X² are not both CR^{b};
L₁ is a bond, -C(=O)- or C₁₋₆ alkylene;
R¹ is C₆₋₂₀ aryl, "5- to 12-membered heteroaryl containing 1-4 heteroatoms selected from O, S and N", C₆₋₂₀ aryl substituted with one or more R¹⁻¹ or "5- to 12-membered heteroaryl containing 1-4 heteroatoms selected from O, S and N and substituted with one or more R¹⁻²"; provided that when multiple substituents are present, the substituents are the same or different;
R^{b}, R¹⁻¹ and R¹⁻² are independently halogen, hydroxyl, cyano, -C(=O)R¹¹, -NR¹²R¹³, - C(=O)OR¹⁴, -C(=O)NR¹⁵R¹⁶, C₁₋₆ alkyl, C₂₋₆ alkenyl, C₂₋₆ alkynyl, C₁₋₆ alkyl-O-, C₃₋₁₀ cycloalkyl, "5- to 7-membered heterocycloalkyl containing 1 or 2 heteroatoms selected from O and N", C₆₋₂₀ aryl, "5- to 7-membered heteroaryl containing 1 or 2 heteroatoms selected from O and N", C₁₋₆ alkyl substituted with one or more R¹⁻¹⁻¹, C₁₋₆ alkyl-O- substituted with one or more R¹⁻¹⁻², C₃₋₁₀ cycloalkyl substituted with one or more R¹⁻¹⁻³, "5- to 7-membered heterocycloalkyl containing 1 or 2 heteroatoms selected from O and N substituted with one or more R¹⁻¹⁻⁴, C₆₋₂₀ aryl substituted with one or more R¹⁻¹⁻⁵, or "5- to 7-membered heteroaryl containing 1 or 2 heteroatoms selected from O and N" substituted with one or more R¹⁻¹⁻⁶; provided that when multiple substituents are present, the substituents are the same or different;
R¹⁻¹⁻¹, R¹⁻¹⁻², R¹⁻¹⁻³, R¹⁻¹⁻⁴, R¹⁻¹⁻⁵ and R¹⁻¹⁻⁶ are independently cyano, halogen, hydroxyl, C₁₋₆ alkyl-O-, C₁₋₆ alkyl, -C(=O)R²¹, -NR²²R²³, -C(=O)OR²⁴ or -C(=O)NR²⁵R²⁶;
R¹¹, R²¹, R¹², R²², R¹³, R²³, R¹⁴, R²⁴, R¹⁵, R²⁵, R¹⁶ and R²⁶ are independently hydrogen or C₁₋₆ alkyl;
L₂ is a bond, C₁₋₆ alkylene, -C(=O)-, -O(R^{L-1})ₙ₁-, -S(R^{L-2})ₙ₂- or -NR^{L-3}(R^{L-4})ₙ₃-; R^{L-1}, R^{L-2} and R^{L-4} are independently C₁₋₆ alkylene; R^{L-3} is hydrogen or C₁₋₆ alkyl; n1, n2 and n3 are independently 0 or 1;
R³ is C₃₋₁₂ cycloalkyl, C₃₋₁₂ cycloalkyl substituted with one or more R³⁻¹, "4- to 12-membered heterocycloalkyl containing 1-3 heteroatoms selected from O, S and N", "4- to 12-membered heterocycloalkyl containing 1-3 heteroatoms selected from O, S and N" substituted with one or more R³⁻², C₁₋₆ alkyl, C₁₋₆ alkyl substituted with one or more R³⁻³, -OR^{d}, -SR^{d1}, -NR^{e1}R^{e2} or - C(=O)NR^{e3}R^{e4}; provided that when multiple substituents are present, the substituents are the same or different;
R³⁻¹, R³⁻² and R³⁻³ are independently C₁₋₆ alkyl, C₁₋₆ alkyl substituted with one or more R³⁻¹⁻¹, hydroxyl, C₁₋₆ alkyl-O-, halogen, O=, -NR^{e5}R^{e6} or -C(=O)NR^{e7}R^{e8};
R^{d}, R^{d1}, R^{e1}, R^{e2}, R^{e3} and R^{e4} are independently hydrogen, C₁₋₆ alkyl, C₃₋₁₀ cycloalkyl, "4- to 10-membered heterocycloalkyl containing 1-3 heteroatoms selected from O and N", or C₁₋₆ alkyl substituted with one or more R³⁻¹⁻²;
R³⁻¹⁻¹ and R³⁻¹⁻² are independently cyano, halogen, hydroxyl, C₁₋₆ alkyl-O-, -C(=O)R^{e9}, - NR^{e10}R^{e11}, -C(=O)OR^{e12} or -C(=O)NR^{e13}R^{e14};
R^{e5}, R^{e6}, R^{e7}, R^{e8}, R^{e9}, R^{e10}, R^{e11}, R^{e12}, R^{e13} and R^{e14} are independently hydrogen or C₁₋₆ alkyl.

### Solution 2:

A nitrogen-containing heterocyclic compound of formula I, a pharmaceutically acceptable salt thereof, a stereoisomer thereof, a tautomer thereof or an isotopically labeled compound thereof: 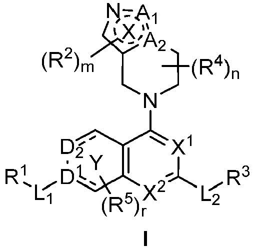 wherein "=-=" represents a single or double bond; 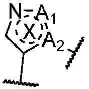
is nitrogen-containing 5-membered heteroaryl; A₁ is CH, O or N; A₂ is C or N; m is 0, 1 or 2;
R² is -CN, C₁₋₆ alkyl, C₂₋₆ alkenyl, C₂₋₆ alkynyl, C₁₋₆ alkyl substituted with one or more R²⁻¹, halogen, -OR^{2a}, -C(=O)R^{2b}, -NR^{2c1}R^{2c2}, -C(=O)OR^{2d}, -C(=O)NR^{2e1}R^{2e2}, C₃₋₁₀ cycloalkyl, C₃₋₁₀ cycloalkyl substituted with one or more R²⁻², "4- to 10-membered heterocycloalkyl containing 1-3 heteroatoms selected from O and N", "4- to 10-membered heterocycloalkyl containing 1-3 heteroatoms selected from O and N" substituted with one or more R²⁻³, C₆₋₂₀ aryl, C₆₋₂₀ aryl substituted with one or more R²⁻⁴, "5- to 12-membered heteroaryl containing 1-4 heteroatoms selected from O, S and N", or "5- to 12-membered heteroaryl containing 1-4 heteroatoms selected from O, S and N" substituted with one or more R²⁻⁵; provided that when multiple substituents are present, the substituents are the same or different;
R²⁻¹, R²⁻², R²⁻³, R²⁻⁴ and R²⁻⁵ are independently halogen, hydroxyl, cyano, C₁₋₆ alkyl, C₂₋₆ alkenyl, C₂₋₆ alkynyl, C₁₋₆ alkyl-O-, -C(=O)R³¹, -NR³²R³³, -C(=O)OR³⁴ or -C(=O)NR³⁵R³⁶;
R^{2a}, R^{2b}, R^{2c1}, R^{2e2}, R^{2d}, R^{2e1} and R^{2e2} are independently hydrogen or C₁₋₆ alkyl;
R³¹, R³², R³³, R³⁴, R³⁵ and R³⁶ are independently hydrogen or C₁₋₆ alkyl;
n is 0, 1, 2, 3, 4, 5 or 6;
each R⁴ is independently C₁₋₆ alkyl, C₁₋₆ alkyl substituted with one or more R⁴⁻¹, C₁₋₆ alkyl-O-, O=, -C(=O)OR^{4a} or -C(=O)NR^{4b}R^{4c}; or, when n is 2, 3, 4, 5 or 6, two optional R⁴ are connected, together with the atoms on the ring to which they are attached, independently form 3- to 8-membered carbocyclic ring or 3- to 8-membered heterocyclic ring containing 1-3 heteroatoms selected from O, S and N;
R⁴⁻¹ is independently halogen, cyano, hydroxyl, C₁₋₆ alkyl-O-, -NR⁴ⁱR^{4j}, -C(=O)OR^{4d} or - C(=O)NR^{4e}R^{4f};
R^{4a}, R^{4b}, R^{4c}, R^{4d}, R^{4e}, R^{4f}, R⁴ⁱ and R^{4j} are independently hydrogen or C₁₋₆ alkyl; 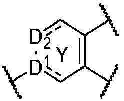
is phenyl, "5- to 7-membered heterocycloalkenyl containing 1-3 heteroatoms selected from O, S and N", "5- to 7-membered heteroaryl containing 1-3 heteroatoms selected from O, S and N" or 5- to 7-membered cycloalkenyl; wherein D¹ is C, CH or N; D₂ is 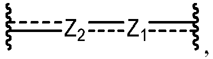 wherein Z₁ and Z₂ are independently a bond, CH, CH₂, O, S, N or NH;
r is 0, 1, 2, 3, 4, 5 or 6;
R⁵ is independently halogen or C₁₋₆ alkyl;
X¹ and X² are independently CR^{b} or N, and X¹ and X² are not both CR^{b};
L₁ is a bond, -C(=O)- or C₁₋₆ alkylene;
R¹ is C₆₋₂₀ aryl, "5- to 12-membered heteroaryl containing 1-4 heteroatoms selected from O, S and N", C₆₋₂₀ aryl substituted with one or more R¹⁻¹ or "5- to 12-membered heteroaryl containing 1-4 heteroatoms selected from O, S and N and substituted with one or more R¹⁻²"; provided that when multiple substituents are present, the substituents are the same or different;
R^{b}, R¹⁻¹ and R¹⁻² are independently halogen, hydroxyl, cyano, -C(=O)R¹¹, -NR¹²R¹³, - C(=O)OR¹⁴, -C(=O)NR¹⁵R¹⁶, C₁₋₆ alkyl, C₂₋₆ alkenyl, C₂₋₆ alkynyl, C₁₋₆ alkyl-O-, C₃₋₁₀ cycloalkyl, "5- to 7-membered heterocycloalkyl containing 1 or 2 heteroatoms selected from O and N", C₆₋₂₀ aryl, "5- to 7-membered heteroaryl containing 1 or 2 heteroatoms selected from O and N", C₁₋₆ alkyl substituted with one or more R¹⁻¹⁻¹, C₁₋₆ alkyl-O- substituted with one or more R¹⁻¹⁻², C₃₋₁₀ cycloalkyl substituted with one or more R¹⁻¹⁻³, "5- to 7-membered heterocycloalkyl containing 1 or 2 heteroatoms selected from O and N" substituted with one or more R¹⁻¹⁻⁴, C₆₋₂₀ aryl substituted with one or more R¹⁻¹⁻⁵, or "5- to 7-membered heteroaryl containing 1 or 2 heteroatoms selected from O and N" substituted with one or more R¹⁻¹⁻⁶; provided that when multiple substituents are present, the substituents are the same or different;
R¹⁻¹⁻¹, R¹⁻¹⁻², R¹⁻¹⁻³, R¹⁻¹⁻⁴, R¹⁻¹⁻⁵ and R¹⁻¹⁻⁶ are independently cyano, halogen, hydroxyl, C₁₋₆ alkyl-O-, C₁₋₆ alkyl, -C(=O)R²¹, -NR²²R²³, -C(=O)OR²⁴ or -C(=O)NR²⁵R²⁶;
R¹¹, R²¹, R¹², R²², R¹³, R²³, R¹⁴, R²⁴, R¹⁵, R²⁵, R¹⁶ and R²⁶ are independently hydrogen or C₁₋₆ alkyl;
L₂ is a bond, C₁₋₆ alkylene, -C(=O)-, -O(R^{L-1})ₙ₁-, -S(R^{L-2})ₙ₂- or -NR^{L-3}(R^{L-4})ₙ₃-; R^{L-1}, R^{L-2} and R^{L-4} are independently C₁₋₆ alkylene; R^{L-3} is hydrogen or C₁₋₆ alkyl; n1, n2 and n3 are independently 0 or 1;
R³ is C₃₋₁₂ cycloalkyl, C₃₋₁₂ cycloalkyl substituted with one or more R³⁻¹, "4- to 12-membered heterocycloalkyl containing 1-3 heteroatoms selected from O, S and N", "4- to 12-membered heterocycloalkyl containing 1-3 heteroatoms selected from O, S and N" substituted with one or more R³⁻², C₁₋₆ alkyl, C₁₋₆ alkyl substituted with one or more R³⁻³, -OR^{d}, -SR^{d1}, -NR^{e1}R^{e2} or - C(=O)NR^{e3}R^{e4}; provided that when multiple substituents are present, the substituents are the same or different;
R³⁻¹, R³⁻² and R³⁻³ are independently C₁₋₆ alkyl, C₁₋₆ alkyl substituted with one or more R³⁻¹⁻¹, hydroxyl, C₁₋₆ alkyl-O-, halogen, O=, -NR^{e5}R^{e6} or -C(=O)NR^{e7}R^{e8};
R^{d}, R^{d1}, R^{e1}, R^{e2}, R^{e3} and R^{e4} are independently hydrogen, C₁₋₆ alkyl, C₃₋₁₀ cycloalkyl, "4- to 10-membered heterocycloalkyl containing 1-3 heteroatoms selected from O and N", or C₁₋₆ alkyl substituted with one or more R³⁻¹⁻².
R³⁻¹⁻¹ and R³⁻¹⁻² are independently deuterium, cyano, halogen, hydroxyl, C₁₋₆ alkyl-O-, - C(=O)R^{e9}, -NR^{e10}R^{e11}, -C(=O)OR^{e12} or -C(=O)NR^{e13}R^{e14};
R^{e5}, R^{e6}, R^{e7}, R^{e8}, R^{e9}, R^{e10}, R^{e11}, R^{e12}, R^{e13} and R^{e14} are independently hydrogen or C₁₋₆ alkyl.

### Solution 3:

A nitrogen-containing heterocyclic compound of formula I, a pharmaceutically acceptable salt thereof, a stereoisomer thereof, a tautomer thereof or an isotopically labeled compound thereof: 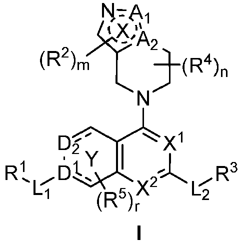 wherein "=-=" represents a single or double bond; 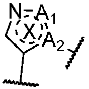
is nitrogen-containing 5-membered heteroaryl; A₁ is CH, O or N; A₂ is C or N;
m is 0, 1 or 2;
R² is -CN, C₁₋₆ alkyl, C₂₋₆ alkenyl, C₂₋₆ alkynyl, C₁₋₆ alkyl substituted with one or more R²⁻¹, halogen, -OR^{2a}, -C(=O)R^{2b}, -NR^{2c1}R^{2c2}, -C(=O)OR^{2d}, -C(=O)NR^{2e1}R^{2e2}, C₃₋₁₀ cycloalkyl, C₃₋₁₀ cycloalkyl substituted with one or more R²⁻², "4- to 10-membered heterocycloalkyl containing 1-3 heteroatoms independently selected from O and N", "4- to 10-membered heterocycloalkyl containing 1-3 heteroatoms independently selected from O and N" substituted with one or more R²⁻³, C₆₋₂₀ aryl, C₆₋₂₀ aryl substituted with one or more R²⁻⁴, "5- to 12-membered heteroaryl containing 1-4 heteroatoms selected from O, S and N", or "5- to 12-membered heteroaryl containing 1-4 heteroatoms selected from O, S and N" substituted with one or more R²⁻⁵; provided that when multiple substituents are present, the substituents are the same or different;
R²⁻¹, R²⁻², R²⁻³, R²⁻⁴ and R²⁻⁵ are independently halogen, hydroxyl, cyano, C₁₋₆ alkyl, C₂₋₆ alkenyl, C₂₋₆ alkynyl, C₁₋₆ alkyl-O-, -C(=O)R³¹, -NR³²R³³, -C(=O)OR³⁴ or -C(=O)NR³⁵R³⁶;
R^{2a}, R^{2b}, R^{2c1}, R^{2e2}, R^{2d}, R^{2e1} and R^{2e2} are independently hydrogen or C₁₋₆ alkyl;
R³¹, R³², R³³, R³⁴, R³⁵ and R³⁶ are independently hydrogen or C₁₋₆ alkyl;
n is 0, 1, 2, 3, 4, 5 or 6;
each R⁴ is independently C₁₋₆ alkyl, C₁₋₆ alkyl substituted with one or more R⁴⁻¹, C₁₋₆ alkyl-O-, O=, -C(=O)OR^{4a} or -C(=O)NR^{4b}R^{4c}; or, when n is 2, 3, 4, 5 or 6, two optional R⁴ are connected, together with the atoms on the ring to which they are attached, independently form 3- to 8-membered carbocyclic ring or 3- to 8-membered heterocyclic ring containing 1-3 heteroatoms selected from O, S and N;
R⁴⁻¹ is independently halogen, cyano, hydroxyl, C₁₋₆ alkyl-O-, -NR⁴ⁱR^{4j}, -C(=O)OR^{4d} or - C(=O)NR^{4e}R^{4f};
R^{4a}, R^{4b}, R^{4c}, R^{4d}, R^{4e}, R^{4f}, R⁴ⁱ and R^{4j} are independently hydrogen or C₁₋₆ alkyl; 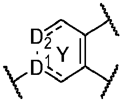
is phenyl, "5- to 7-membered heterocycloalkenyl containing 1-3 heteroatoms independently selected from O, S and N", "5- to 7-membered heteroaryl containing 1-3 heteroatoms independently selected from O, S and N" or 5- to 7-membered cycloalkenyl; wherein D¹ is C, CH or N; D₂ is 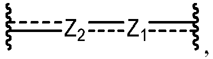 wherein Z₁ and Z₂ are independently a bond, CH, CH₂, O, S, N or NH;
r is 0, 1, 2, 3, 4, 5 or 6;
R⁵ is independently halogen or C₁₋₆ alkyl;
X¹ and X² are independently CR^{b} or N, and X¹ and X² are not both CR^{b};
L₁ is a bond, -C(=O)- or C₁₋₆ alkylene;
R¹ is C₆₋₂₀ aryl, C₈₋₁₁ benzocycloalkenyl, "5- to 12-membered heteroaryl containing 1-4 heteroatoms selected from O, S and N", C₆₋₂₀ aryl substituted with one or more R¹⁻¹ or "5- to 12-membered heteroaryl containing 1-4 heteroatoms selected from O, S and N" substituted with one or more R¹⁻²; provided that when multiple substituents are present, the substituents are the same or different;
R^{b}, R¹⁻¹ and R¹⁻² are independently halogen, -OR^{c}, cyano, -C(=O)R¹¹, -NR¹²R¹³, -C(=O)OR¹⁴, -C(=O)NR¹⁵R¹⁶, C₁₋₆ alkyl, C₂₋₆ alkenyl, C₂₋₆ alkynyl, C₃₋₁₀ cycloalkyl, "5- to 7-membered heterocycloalkyl containing 1 or 2 heteroatoms independently selected from O and N", C₆₋₂₀ aryl, "5- to 7-membered heteroaryl containing 1 or 2 heteroatoms independently selected from O and N", C₁₋₆ alkyl substituted with one or more R¹⁻¹⁻¹, C₁₋₆ alkyl-O- substituted with one or more R¹⁻¹⁻², C₃₋₁₀ cycloalkyl substituted with one or more R¹⁻¹⁻³, "5- to 7-membered heterocycloalkyl containing 1 or 2 heteroatoms independently selected from O and N" substituted with one or more R¹⁻¹⁻⁴, C₆₋₂₀ aryl substituted with one or more R¹⁻¹⁻⁵, or "5- to 7-membered heteroaryl containing 1 or 2 heteroatoms independently selected from O and N" substituted with one or more R^{1-1-6,} provided that when multiple substituents are present, the substituents are the same or different; or, when the number of R¹⁻¹ or R¹⁻² is more than one, two optional R¹⁻¹ or R¹⁻² are connected, together with the atoms on the ring to which they are attached, independently form 3- to 8-membered cyclic olefin;
R^{c}, R¹² and R¹³ are independently hydrogen, C₁₋₆ alkyl, C(=O)R^{c1}, -C(=O)OR^{c2}, or - C(=O)NR^{c3}R^{c4}; R^{c1}, R^{e2}, R^{c3} and R^{c4} are independently hydrogen, C₁₋₆ alkyl, C₃₋₁₀ cycloalkyl, "5-to 7-membered heterocycloalkyl containing 1 or 2 heteroatoms independently selected from O and N", C₆₋₂₀ aryl, "5- to 7-membered heteroaryl containing 1 or 2 heteroatoms independently selected from O and N", C₁₋₆ alkyl substituted with one or more R⁴⁻¹⁻¹, C₃₋₁₀ cycloalkyl substituted with one or more R⁴⁻¹⁻², "5- to 7-membered heterocycloalkyl containing 1 or 2 heteroatoms independently selected from O and N" substituted with one or more R⁴⁻¹⁻³, C₆₋₂₀ aryl substituted with one or more R⁴⁻¹⁻⁴, or, "5- to 7-membered heteroaryl containing 1 or 2 heteroatoms independently selected from O and N" substituted with one or more R⁴⁻¹⁻⁵; provided that when multiple substituents are present, the substituents are the same or different;
R¹⁻¹⁻¹, R¹⁻¹⁻², R¹⁻¹⁻³, R¹⁻¹⁻⁴, R¹⁻¹⁻⁵, R¹⁻¹⁻⁶, R⁴⁻¹⁻¹, R⁴⁻¹⁻², R⁴⁻¹⁻³, R⁴⁻¹⁻⁴ and R⁴⁻¹⁻⁵ are independently cyano, halogen, hydroxyl, C₁₋₆ alkyl-O-, C₁₋₆ alkyl, -C(=O)R²¹, -NR²²R²³, -C(=O)OR²⁴ or - C(=O)NR²⁵R²⁶;
R¹¹, R²¹, R²², R²³, R¹⁴, R²⁴, R¹⁵, R²⁵, R¹⁶ and R²⁶ are independently hydrogen or C₁₋₆ alkyl;
L₂ is a bond, C₁₋₆ alkylene, -C(=O)-, -O(R^{L-1})ₙ₁-, -S(R^{L-2})ₙ₂- or -NR^{L-3}(R^{L-4})ₙ₃-; R^{L-1}, R^{L-2} and R^{L-4} are independently C₁₋₆ alkylene; R^{L-3} is hydrogen or C₁₋₆ alkyl; n1, n2 and n3 are independently 0 or 1;
R³ is C₃₋₁₂ cycloalkyl, C₃₋₁₂ cycloalkyl substituted with one or more R³⁻¹, "4- to 12-membered heterocycloalkyl containing 1-3 heteroatoms selected from O, S and N", "4- to 12-membered heterocycloalkyl containing 1-3 heteroatoms selected from O, S and N" substituted with one or more R³⁻², C₁₋₆ alkyl, C₁₋₆ alkyl substituted with one or more R³⁻³, -OR^{d}, -SR^{d1}, -NR^{e1}R^{e2} or - C(=O)NR^{e3}R^{e4}; provided that when multiple substituents are present, the substituents are the same or different;
R³⁻¹, R³⁻² and R³⁻³ are independently C₁₋₆ alkyl, C₁₋₆ alkyl substituted with one or more R³⁻¹⁻¹, hydroxy, C₁₋₆ alkyl-O-, halogen, O=, -NR^{e5}R^{e6} or -C(=O)NR^{e7}R^{e8};
R^{d}, R^{d1}, R^{e1}, R^{e2}, R^{e3} and R^{e4} are independently hydrogen, C₁₋₆ alkyl, C₃₋₁₀ cycloalkyl, "4- to 10-membered heterocycloalkyl containing 1-3 heteroatoms independently selected from O and N", or C₁₋₆ alkyl substituted with one or more R³⁻¹⁻²,
R³⁻¹⁻¹ and R³⁻¹⁻² are independently deuterium, cyano, halogen, hydroxyl, C₁₋₆ alkyl-O-, - C(=O)R^{e9}, -NR^{e10}R^{e11}, -C(=O)OR^{e12} or -C(=O)NR^{e13}R^{e14};
R^{e5}, R^{e6}, R^{e7}, R^{e8}, R^{e9}, R^{e10}, R^{e11}, R^{e12}, R^{e13} and R^{e14} are independently hydrogen or C₁₋₆ alkyl.

In a certain embodiment, the nitrogen-containing heterocyclic compound of formula I can have any one of the following structures:

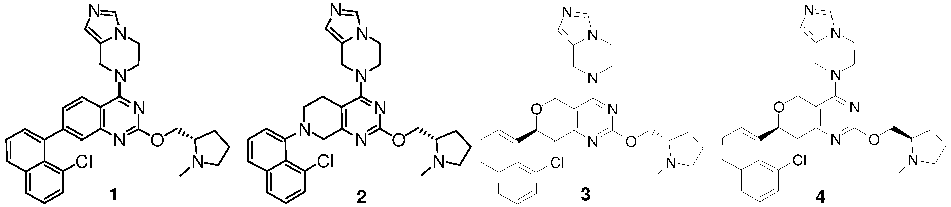

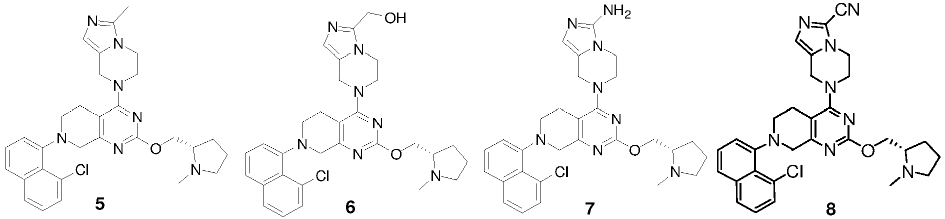

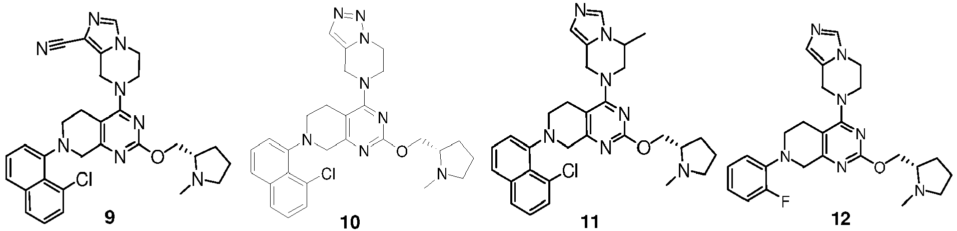

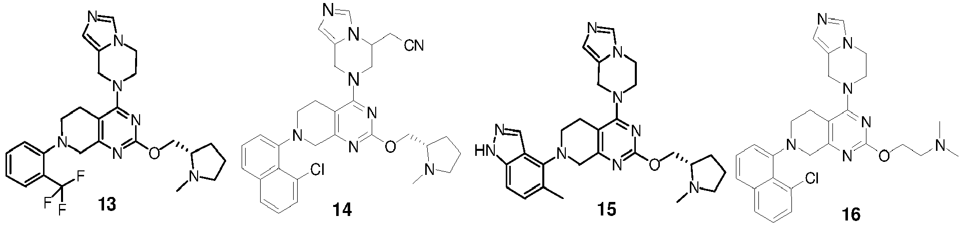

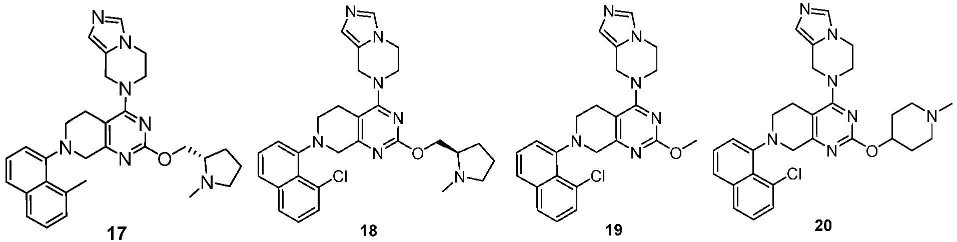

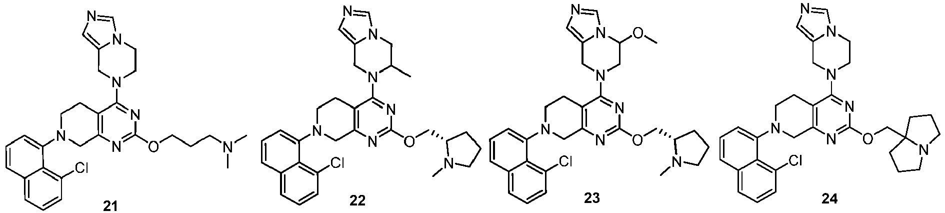

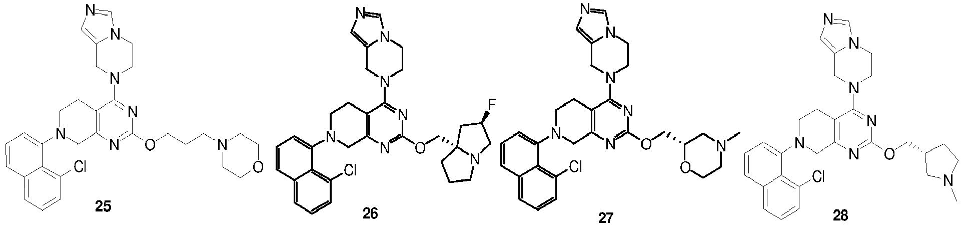

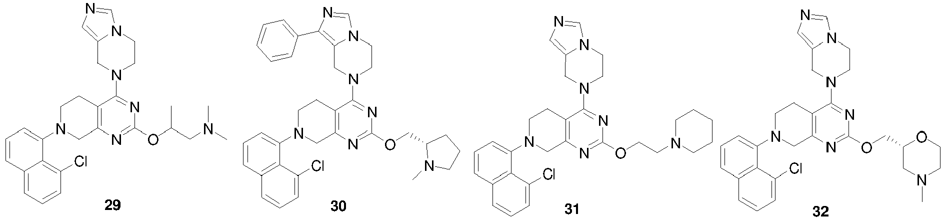

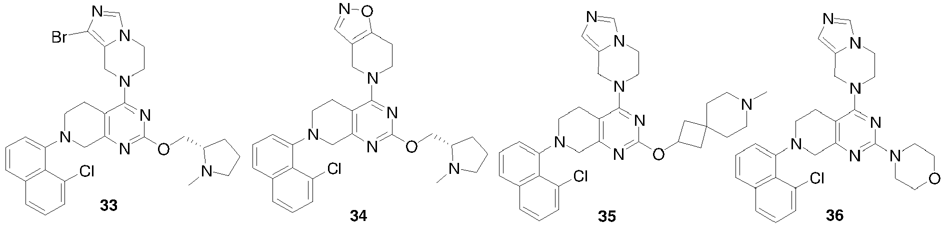

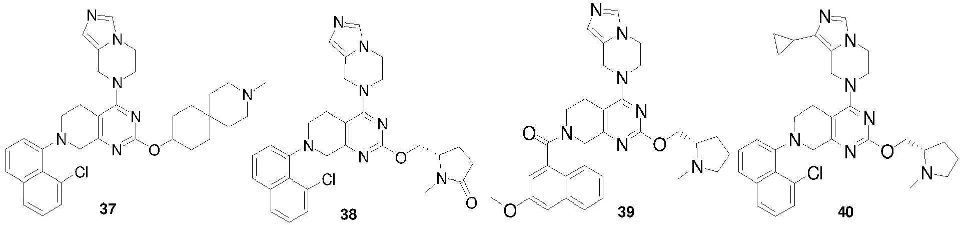

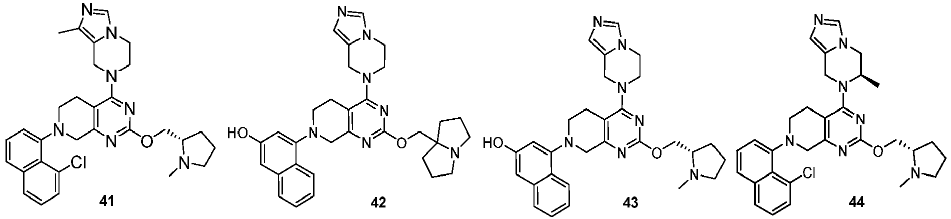

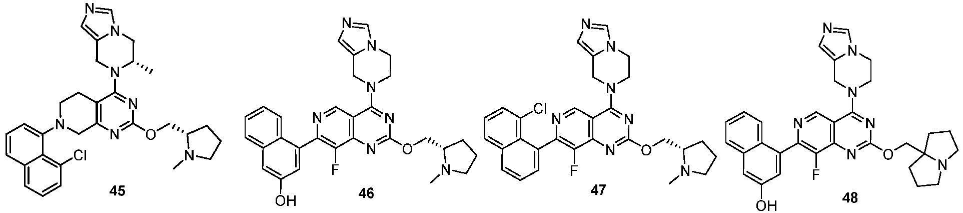

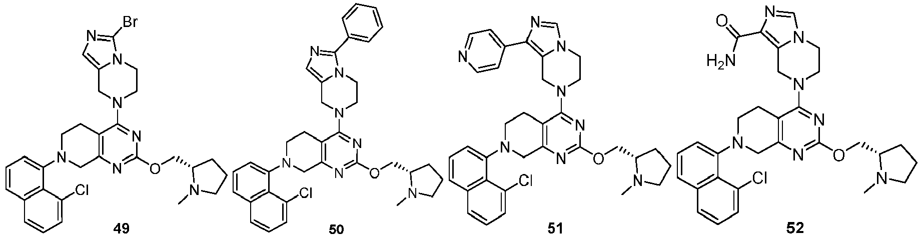

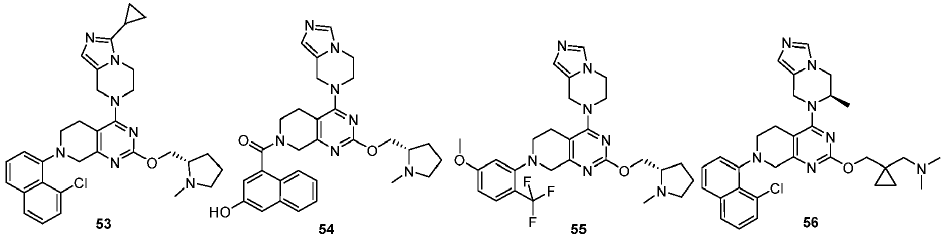

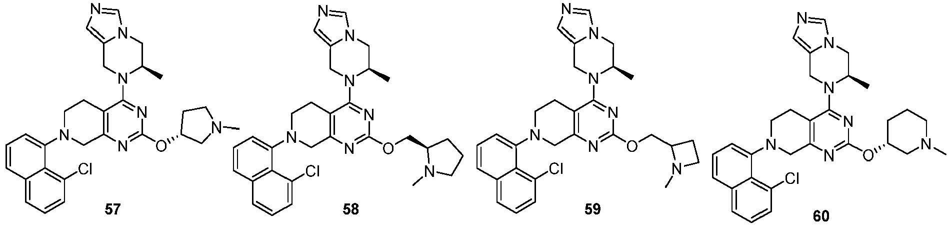

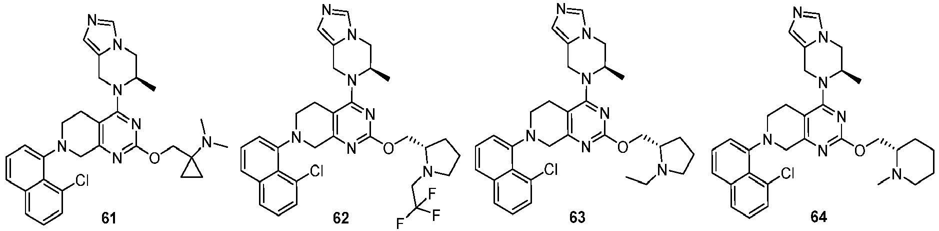

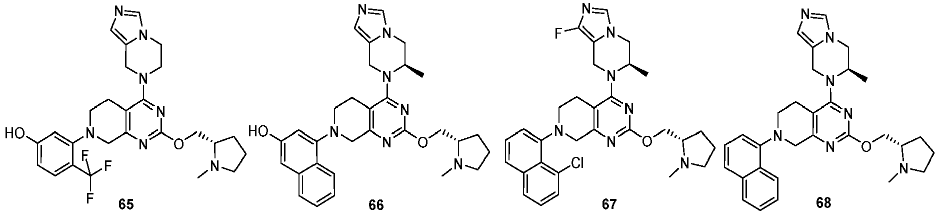

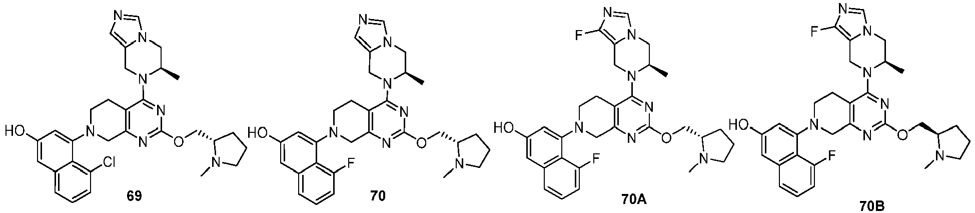

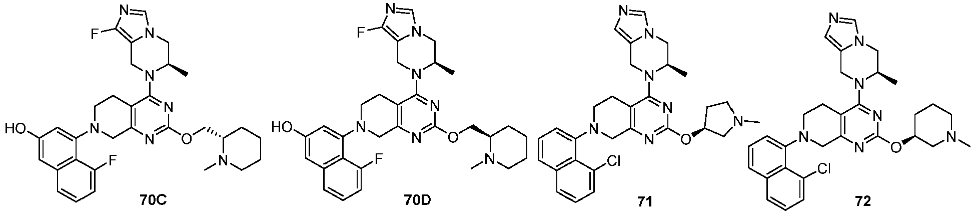

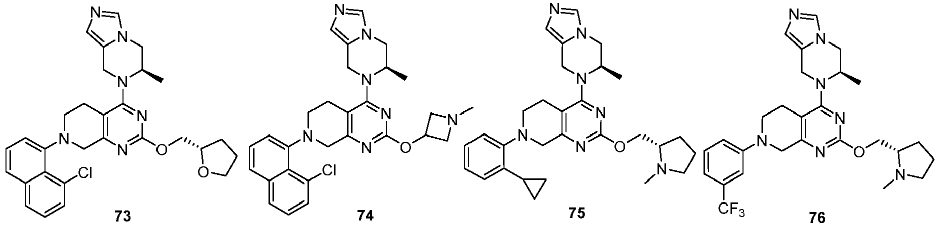

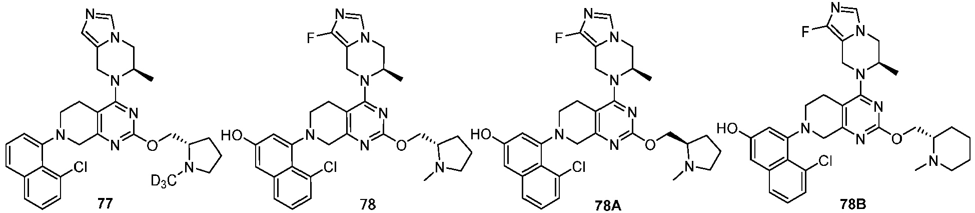

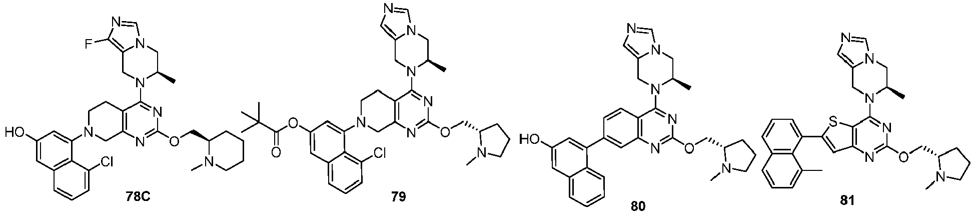

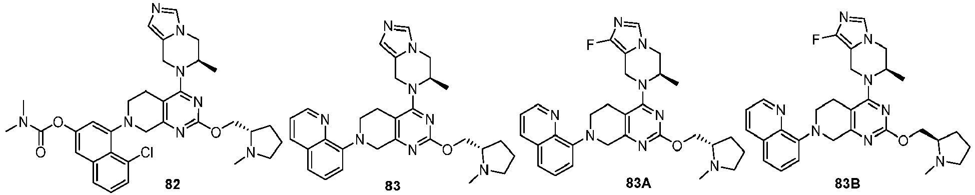

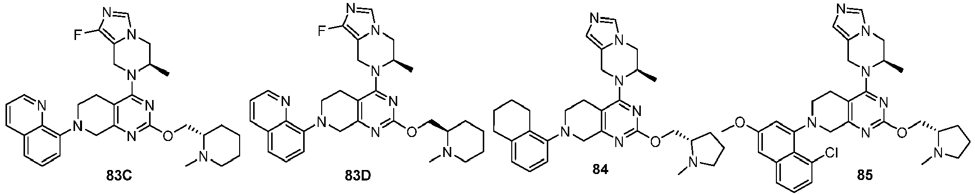

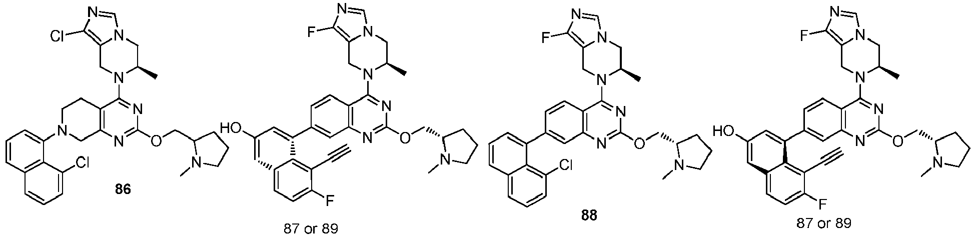

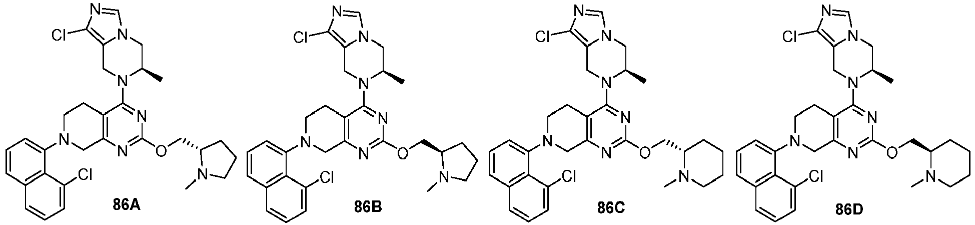

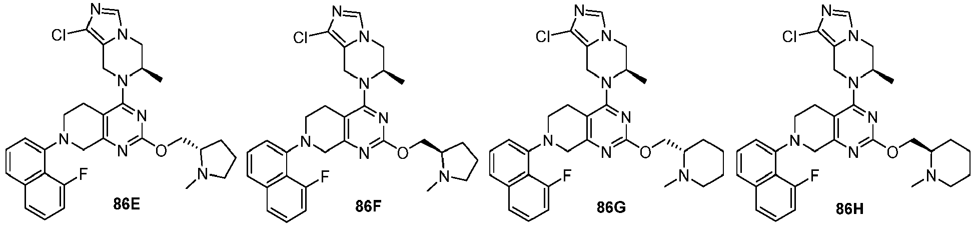

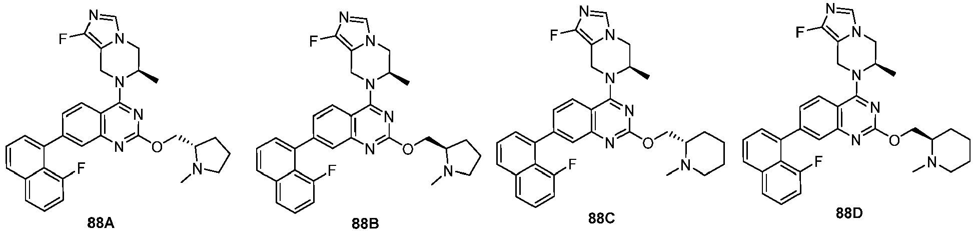

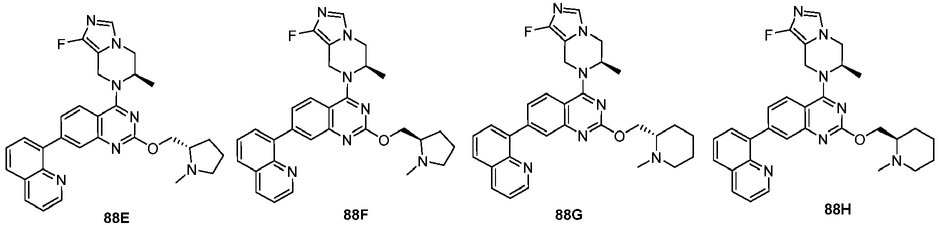

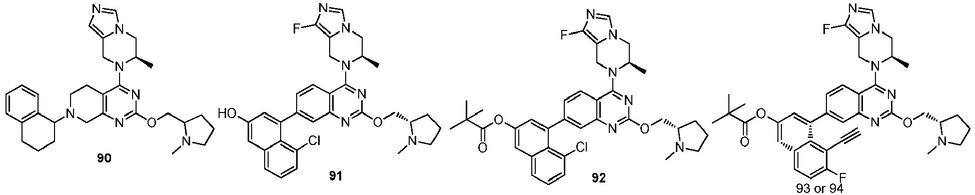

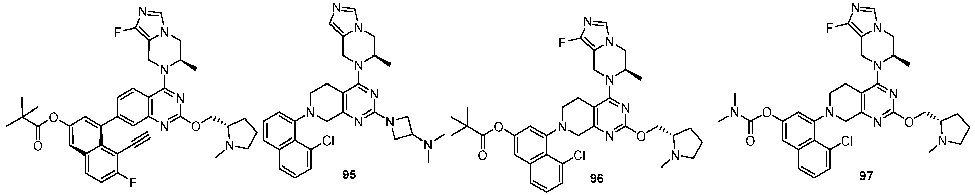

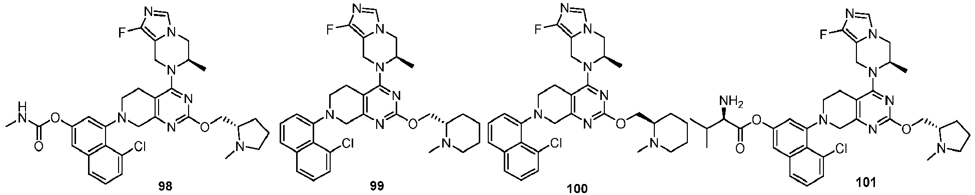

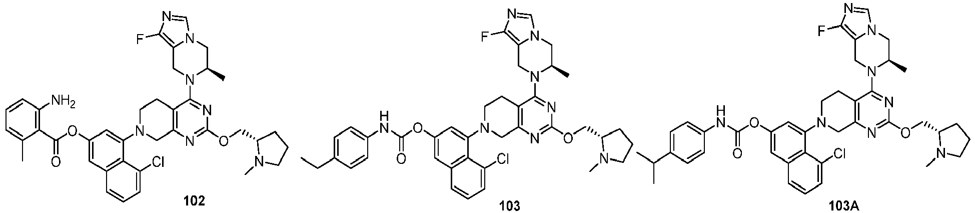

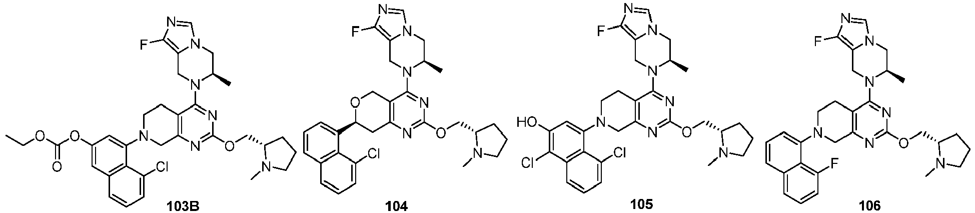

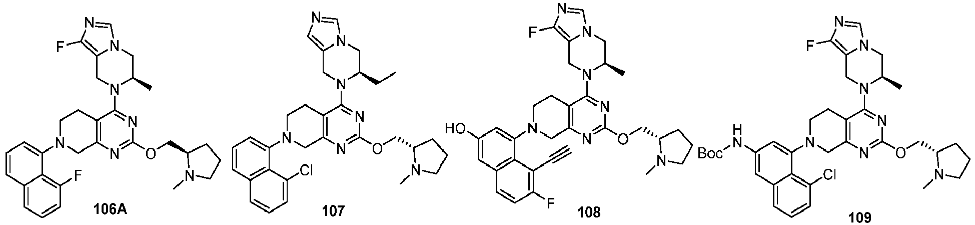

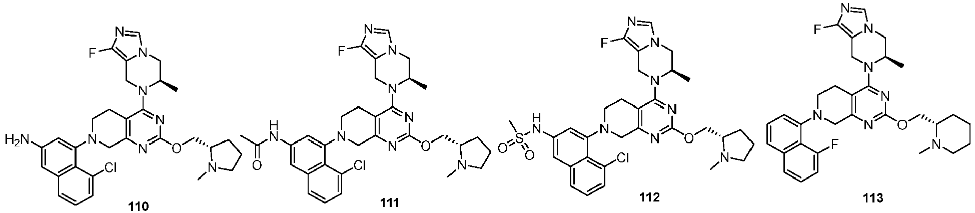

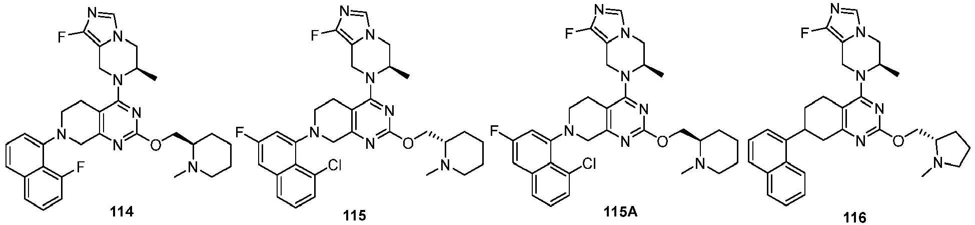

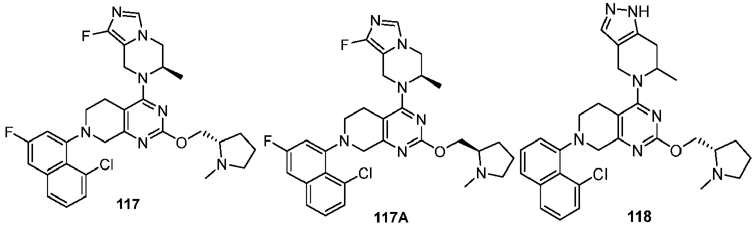

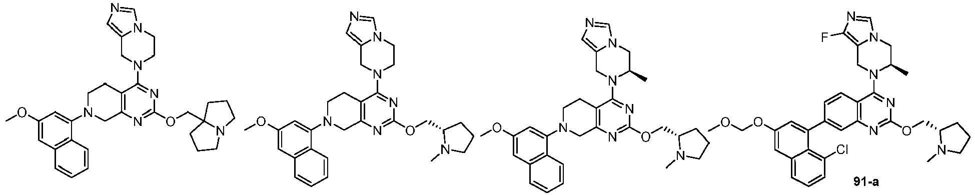

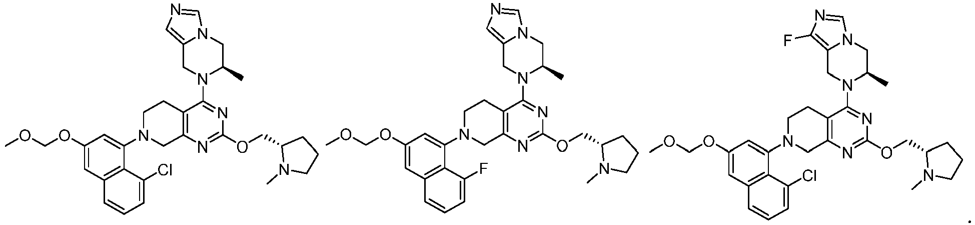

In a certain embodiment, the pharmaceutically acceptable salt of the nitrogen-containing heterocyclic compound of formula I is trifluoroacetate having any one of the following structures:

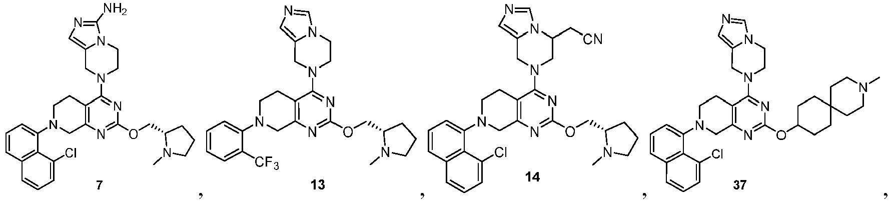

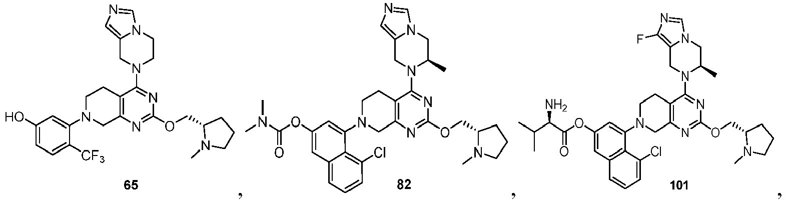

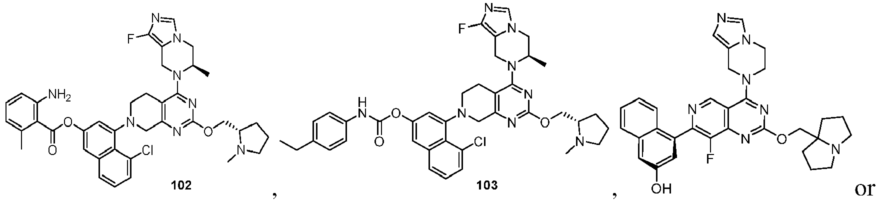

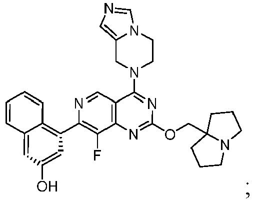

In a certain embodiment, the pharmaceutically acceptable salt of the nitrogen-containing heterocyclic compound of formula I is formate having the following structure:

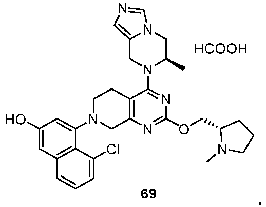

The stereoisomer of the nitrogen-containing heterocyclic compound of formula I has any one of the following structures:

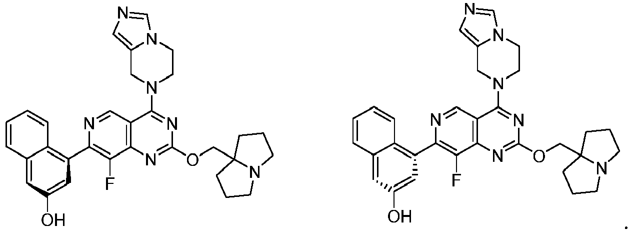

The present disclosure also provides a method for preparing the nitrogen-containing heterocyclic compound of formula I described above, which is route I, route II, route III, route IV, route V or route VI: 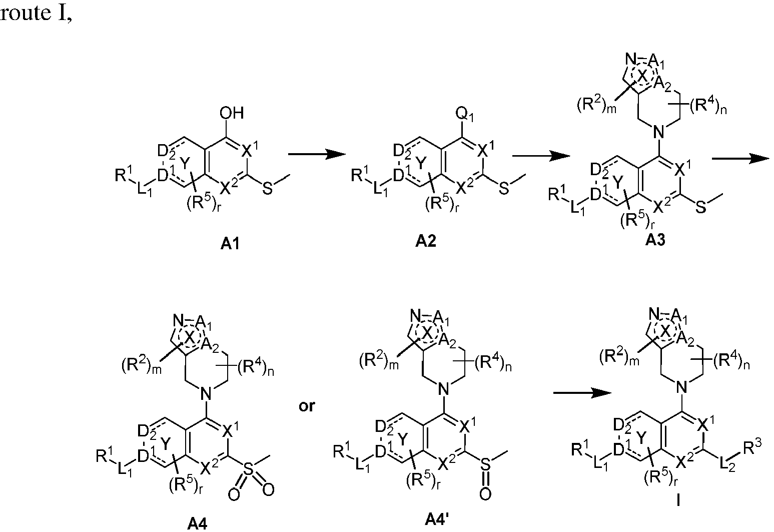
wherein R¹, R², R³, R⁴, R⁵, A₁, A₂, D¹, D₂, L₁, L₂, X¹, X², m, n, p and r are as defined above, and Q₁ is a leaving group; the route I comprises the following steps: converting hydroxyl of compound A1 into a leaving group to obtain A2, converting A2 into A3 by nucleophilic substitution reaction, oxidizing A3 into A4 or A4', and converting A4 or A4' into compound I by nucleophilic substitution reaction; 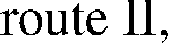 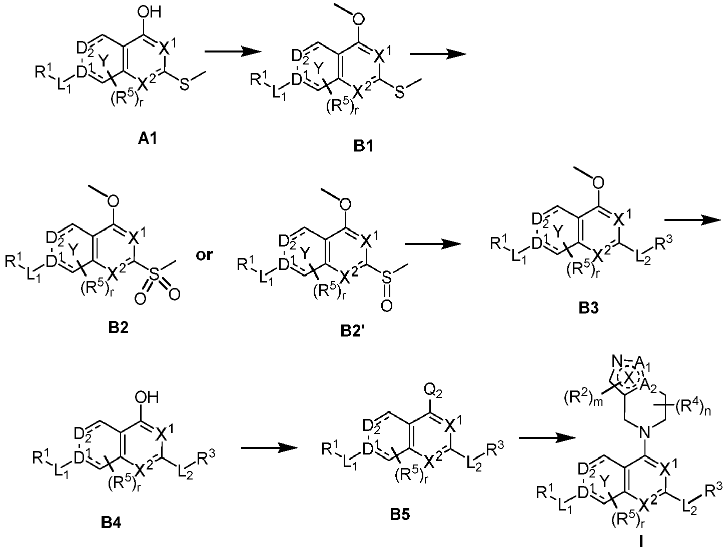
wherein R¹, R², R³, R⁴, R⁵, A₁, A₂, D¹, D₂, L₁, L₂, X¹, X², m, n, p and r are as defined above, and Q₂ is a leaving group (such as OTf or Cl); the route II comprises the following steps: protecting compound A1 with Me group to obtain B1, oxidizing B1 to obtain B2 or B2', converting B2 or B2' into B3 by nucleophilic substitution reaction or other ways, converting B3 into B4 by demethylation protection, converting hydroxyl in B4 into a leaving group to obtain B5, and converting B5 into compound I by nucleophilic substitution reaction or other ways; 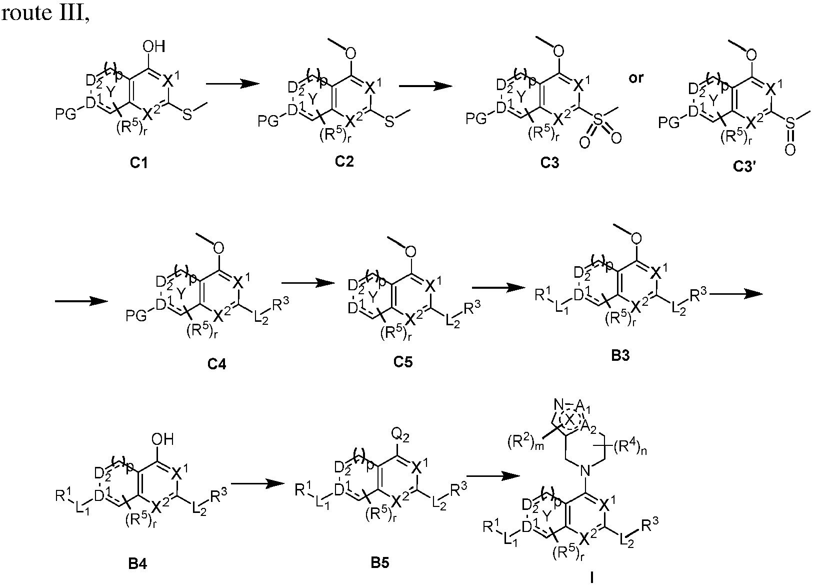
wherein R¹, R², R³, R⁴, R⁵, A₁, A₂, D¹, D₂, L₁, L₂, X¹, X², m, n, p and r are as defined above, PG and Q₂ are independently a leaving group (such as OTf or Cl); PG is H or an amino protecting group such as THP, Boc or Cbz; the route III comprises the following steps: protecting compound C1 with Me group to obtain C2, oxidizing C2 to obtain C3 or C3', converting C3 or C3' into C4 by nucleophilic substitution reaction, removing the protecting group from C4 to obtain C5, converting C5 into B3 by nucleophilic substitution, coupling reaction or other ways, converting B3 into B4 by demethylation protection, converting hydroxyl in B4 into a leaving group to obtain B5, and converting B5 into compound I by nucleophilic substitution reaction; 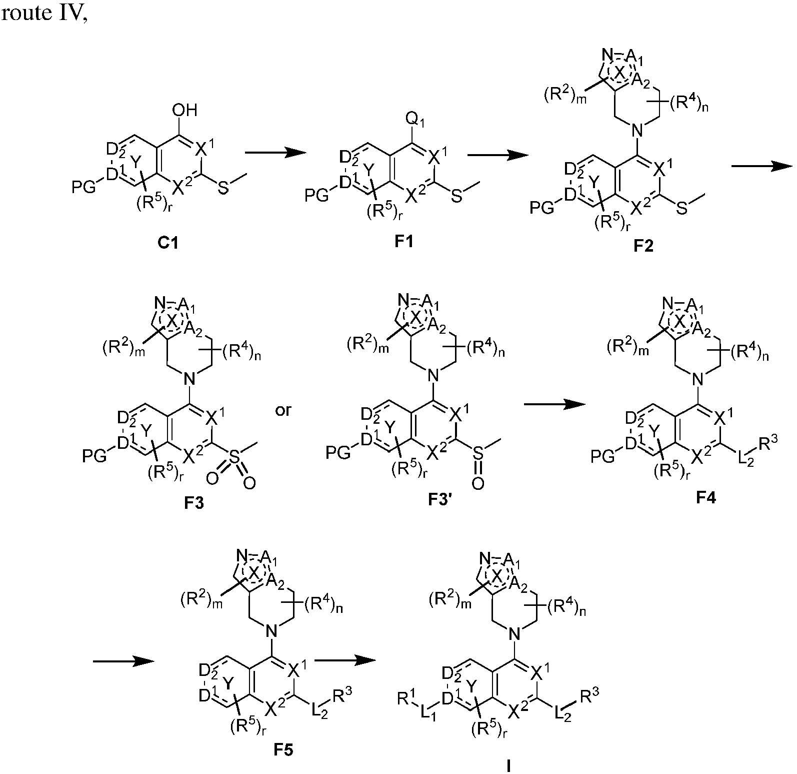
wherein R¹, R², R³, R⁴, R⁵, A₁, A₂, D¹, D₂, L₁, L₂, X¹, X², m, n, p and r are as defined above, Q₁ are independently a leaving group (such as OTf or Cl); PG is H or an amino protecting group such as THP, Boc or Cbz; the route IV comprises the following steps: converting hydroxyl in compound C1 into a leaving group to obtain F1, converting F1 into F2 by nucleophilic substitution reaction, oxidizing F2 to obtain F3 or F3', converting F3 or F3' into F4 by nucleophilic substitution reaction, removing protecting group from F4 to obtain F5, and converting F5 into compound I by nucleophilic substitution or coupling reaction; 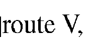 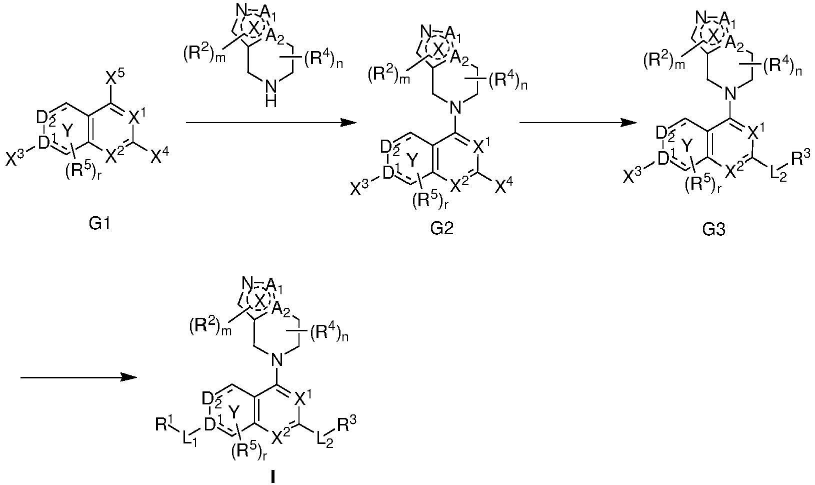
wherein R¹, R², R³, R⁴, R⁵, A₁, A₂, D¹, D₂, L₁, L₂, X¹, X², m, n, p and r are as defined above, and X³, X⁴ and X⁵ are independently a leaving group such as OTf, Cl or Br; the route V comprises the following steps: converting compound G1 into G2 by nucleophilic substitution reaction, converting G2 into G3 by nucleophilic substitution reaction, and converting G3 into compound I by nucleophilic substitution or coupling reaction; 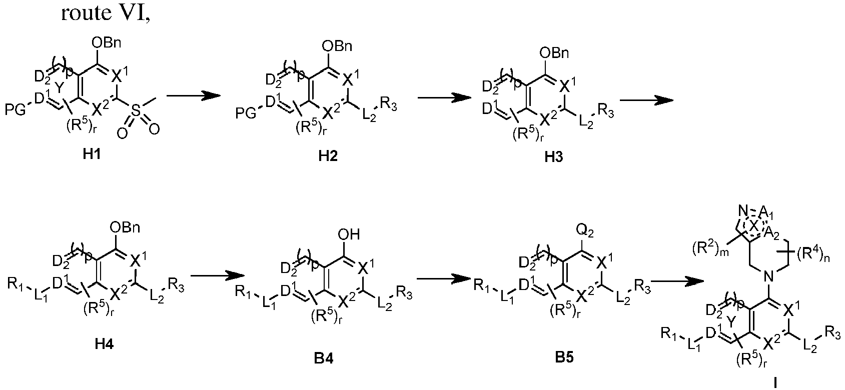
wherein R¹, R², R³, R⁴, R⁵, A₁, A₂, D¹, D₂, L₁, L₂, X¹, X², m, n, p and r are as defined above, and Q₂ is a leaving group such as OTf or Cl; the route VI comprises the following steps: converting compound H1 into H2 by nucleophilic substitution reaction, converting H2 into H3 by removing protecting group, and converting H3 into H4 by by nucleophilic substitution or coupling reaction, H4 is converted into B5 by removing protecting group benzyl, hydroxyl in the B4 is converted into B5, and B5 is converted into compound I by nucleophilic substitution reaction.

The conditions and steps used for the chemical reactions involved in the various reaction routes described in the present disclosure can be determined with reference to the conditions and steps of such reactions that are conventional in the art, in particular with reference to the following documents: R. Larock, Comprehensive Organic Transformations, VCH Publishers (1989); T. W. Greene and P. G. M. Wuts, Protective Groups in Organic Synthesis, 3rd ED., John Wiley and Sons (1999); L. Fieser and M. Fieser, Fieser and Fieser's Reagents for Organic Synthesis, John Wiley and Sons (1994); L. Paquette, ed., Encyclopedia of Reagents for Organic Synthesis, John Wiley and Sons (1995) and subsequent versions thereof.

The contents of which are incorporated herein by reference in their entireties. The compounds obtained by the above method can be further modified at the peripheral position with reference to the related methods in the above documents to obtain other target compounds of the present disclosure.

The present disclosure also provides a compound of formula T1, T2, T3, T4, T5, T6, T7, T8, T2', T6', 45-b, 44-b, 67-a, 67-b, 67-c or 67-d: 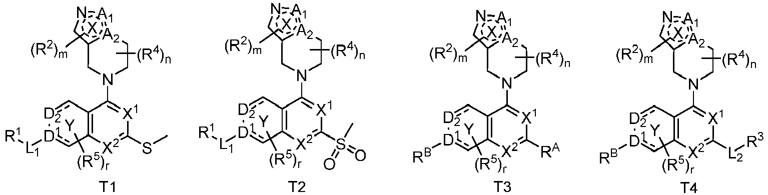 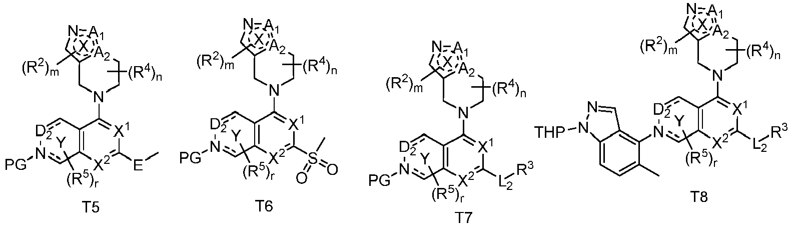 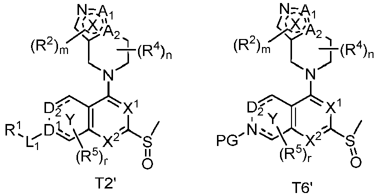 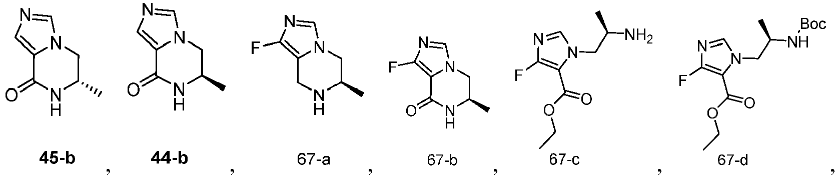
wherein A₁, A₂, R², R⁴, X¹, X², L₂, R³, D¹, D₂, L₁, R¹, R⁵, m, n and r are as defined above;
R^{A} and R^{B} are independently a leaving group (such as Cl, Br or OTf), and PG is H or an amino protecting group (such as THP, Boc or Cbz); E is O or S.

In a certain embodiment, the compound of formula T1, T2, T3, T4, T5, T6, T7, T8, T2' or T6' is any one of the following compounds:

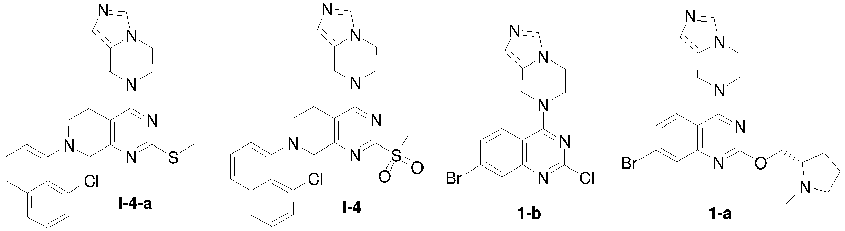

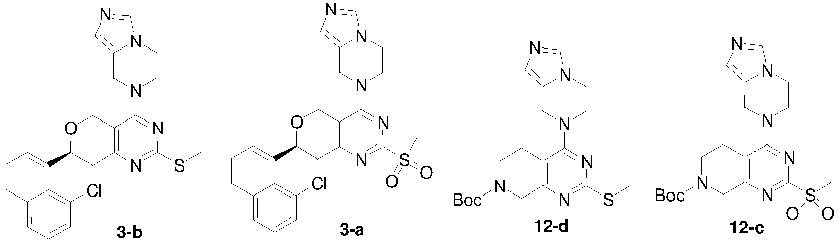

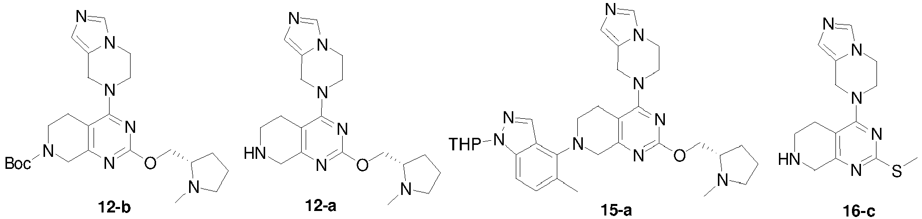

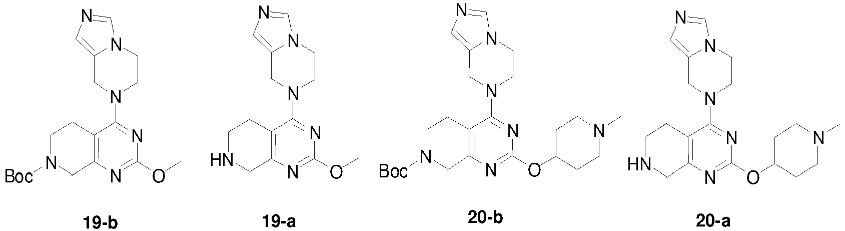

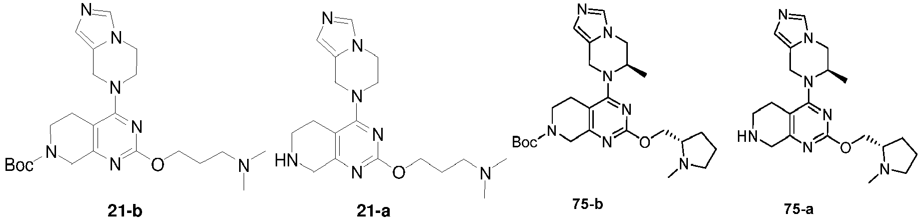

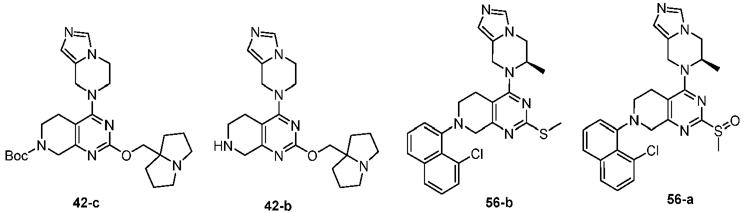

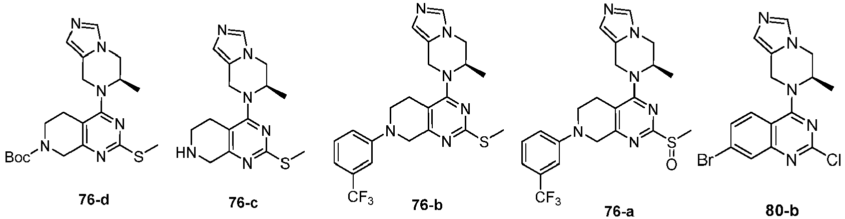

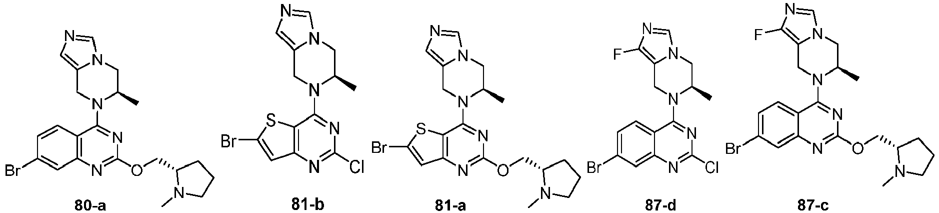

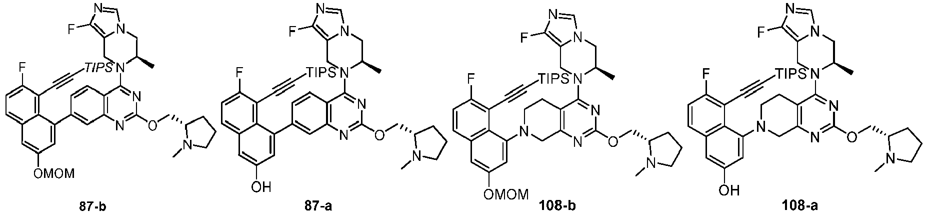

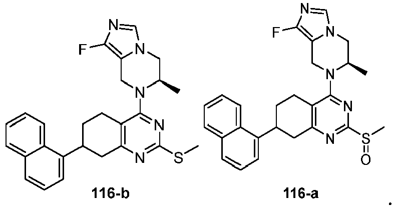

The present disclosure also provides a pharmaceutical composition comprising a substance **A** and a pharmaceutically acceptable excipient, wherein the substance **A** is a therapeutically effective amount of the nitrogen-containing heterocyclic compound of formula **I,** the pharmaceutically acceptable salt thereof, the stereoisomer thereof, the tautomer thereof or the isotopically labeled compound thereof as described above.

The present disclosure also provides a use of substance A in manufacturing an RAS inhibitor, the substance **A** is the nitrogen-containing heterocyclic compound of formula **I,** the pharmaceutically acceptable salt thereof, the stereoisomer thereof, the tautomer thereof or the isotopically labeled compound thereof as described above.

In the use of substance A in manufacturing an RAS inhibitor, the RAS is wild type or mutated forms of RAS; The mutated forms of RAS is, for example, a KRAS, HRAS or NRAS mutation, wherein the KRAS mutation can be a G12, G13 or Q61 mutation, for example, KRAS G12C, KRAS G12D, KRAS G12S, KRAS G12A, KRAS G12V or KRAS G13D, and for another example, KRAS G12C, KRAS G12D or KRAS G12V; the HRAS mutation can be a G12, G13 or Q61 mutation, for example, HRAS G12C, HRAS G12D, HRAS G12S, HRAS G12A, HRAS G12V or HRAS G13D; the NRAS mutation can be a G12, G13 or Q61 mutation, for example, NRAS G12C, NRAS G12D, NRAS G12S, NRAS G12A, NRAS G12V or NRAS G13D; the mutated forms of RAS is, for another example, KRAS G12C.

The present disclosure also provides a use of substance A in manufacturing a medicament, the medicament is used for treating or preventing an RAS-related disease; the substance **A** is the nitrogen-containing heterocyclic compound of formula **I,** the pharmaceutically acceptable salt thereof, the stereoisomer thereof, the tautomer thereof or the isotopically labeled compound thereof as described above.

In the use of substance A in manufacturing a medicament, the RAS is wild type or mutated forms of RAS. The mutated forms of RAS is, for example, a KRAS, HRAS or NRAS mutation, wherein the KRAS mutation can be a G12, G13 or Q61 mutation, for example, KRAS G12C, KRAS G12D, KRAS G12S, KRAS G12A, KRAS G12V or KRAS G13D, and for another example, KRAS G12C, KRAS G12D or KRAS G12V; the HRAS mutation can be a G12, G13 or Q61 mutation, for example, HRAS G12C, HRAS G12D, HRAS G12S, HRAS G12A, HRAS G12V or HRAS G13D; the NRAS mutation can be a G12, G13 or Q61 mutation, for example, NRAS G12C, NRAS G12D, NRAS G12S, NRAS G12A, NRAS G12V or NRAS G13D; the mutated forms of RAS is, for another example, KRAS G12C.

In the use of substance A in manufacturing a medicament, the RAS-related disease is, for example, cancer. The cancer is selected from the group consisting of colon cancer, appendiceal cancer, pancreatic cancer, MYH-related polyposis, hematologic cancer, breast cancer, endometrial cancer, gallbladder cancer, bile duct cancer, prostate cancer, lung cancer, brain cancer, ovarian cancer, cervical cancer, testicular cancer, kidney cancer, head or neck cancer, bone cancer, skin cancer, rectal cancer, liver cancer, esophageal cancer, stomach cancer, thyroid cancer, bladder cancer, lymphoma, leukemia and melanoma.

The present disclosure also provides a use of substance A in manufacturing a medicament, the medicament is used for treating or preventing cancer; The substance **A** is the nitrogen-containing heterocyclic compound of formula **I,** the pharmaceutically acceptable salt thereof, the stereoisomer thereof, the tautomer thereof or the isotopically labeled compound thereof as described above. The cancer is, for example, one or more of colon cancer, appendiceal cancer, pancreatic cancer, MYH-related polyposis, hematologic cancer, breast cancer, endometrial cancer, gallbladder cancer, bile duct cancer, prostate cancer, lung cancer, brain cancer, ovarian cancer, cervical cancer, testicular cancer, kidney cancer, head or neck cancer, bone cancer, skin cancer, rectal cancer, liver cancer, esophageal cancer, stomach cancer, thyroid cancer, bladder cancer, lymphoma, leukemia and melanoma.

The term "more" refers to 2, 3, 4 or 5.

The term "pharmaceutically acceptable salt" refers to a salt of the compound disclosed herein which is prepared using relatively safe and pharmaceutically acceptable acids or bases. When the compound disclosed herein contains a relatively acidic functional group, a base addition salt can be obtained by contacting a neutral form of the compound with a sufficient amount of a pharmaceutically acceptable base in a pure solution or a suitable inert solvent. Pharmaceutically acceptable base addition salts include, but are not limited to: lithium salt, sodium salt, potassium salt, calcium salt, aluminum salt, magnesium salt, zinc salt, bismuth salt, ammonium salt, and diethanolamine salt. When the compound disclosed herein contains a relatively basic functional group, an acid addition salt can be obtained by contacting a neutral form of the compound with a sufficient amount of a pharmaceutically acceptable acid in a pure solution or a suitable inert solvent. The pharmaceutically acceptable acid includes inorganic acids, including, but not limited to: hydrochloric acid, hydrobromic acid, hydroiodic acid, nitric acid, carbonic acid, phosphoric acid, phosphorous acid, and sulfuric acid. The pharmaceutically acceptable acids include organic acids, including, but not limited to: acetic acid, propionic acid, oxalic acid, isobutyric acid, maleic acid, malonic acid, benzoic acid, succinic acid, suberic acid, fumaric acid, lactic acid, mandelic acid, phthalic acid, benzenesulfonic acid, *p*-toluenesulfonic acid, citric acid, salicylic acid, tartaric acid, methanesulfonic acid, isonicotinic acid, acidic citric acid, oleic acid, tannic acid, pantothenic acid, bitartrate, ascorbic acid, gentisic acid, fumaric acid, gluconic acid, saccharic acid, formic acid, ethanesulfonic acid, pamoic acid (i.e., 4,4'-methylene-bis(3-hydroxyl-2-naphthoic acid)), and amino acids (e.g., glutamic acid and arginine). When a compound disclosed herein contains both relatively acidic functional group and relatively basic functional group, it can be converted to either base addition salt or acid addition salt. For details, see Berge et al., "Pharmaceutical Salts", Journal of Pharmaceutical Science 66:1-19 (1977), or Handbook of Pharmaceutical Salts: Properties, Selection, and Use (P. Heinrich Stahl and Camille G. Wermuth, ed., Wiley-VCH, 2002).

The term "stereoisomer" refers to isomers of a molecule having the same order of atoms or radicals but different spatial arrangement, such as cis-trans isomer, optical isomer or atropisomer. Such stereoisomers can be separated, purified and enriched by asymmetric synthesis or chiral resolution (including but not limited to thin layer chromatography, centrifugal partition chromatography, column chromatography, gas chromatography, and high pressure liquid chromatography), and can also be obtained by chiral resolution via bonding (chemical bonding, etc.) or salification (physical bonding, etc.) with other chiral compounds.

The term "tautomer" refers to functional isomers of a molecule where an atom rapidly migrates between two positions. For example, acetone and 1-propen-2-ol can be mutually converted when hydrogen atom rapidly migrates between oxygen and α-carbon atoms.

The term "isotopically labeled compound" refers to a compound where one or more atoms are replaced by one or more atoms having a specific atomic mass or mass number. Examples of isotope that can be incorporated into the compound disclosed herein include, but are not limited to, isotopes of hydrogen, carbon, nitrogen, oxygen, fluorine, sulfur, and chlorine (e.g., 2H, 3H, 13C, 14C, 15N, 180, 170, 18F, 35S, and 36Cl). An isotopically labeled compound of the present disclosure can generally be prepared by replacing a non-isotopically labeled reagent with an isotopically labeled reagent according to the methods described herein.

The term "halogen" refers to fluorine, chlorine, bromine or iodine.

The term "alkyl" refers to a linear or branched alkyl group having a specified number of carbon atoms. Examples of alkyl include methyl, ethyl, *n*-propyl, isopropyl, *n*-butyl, *tert*-butyl*,* isobutyl, *sec*-butyl, *n*-pentyl, *n*-hexyl, *n*-heptyl, *n*-octyl, and the like.

The term "alkylene" refers to a linking group between two other species, which may be linear or branched. Examples include, but are not limited to, -CH₂-, -CH₂CH₂-, -CH₂CH₂CH₂CH(CH₃)-and -CH₂CH(CH₂CH₃)CH₂-.

The term "alkoxy" refers to the group -O-R^{X}, wherein R^{X} is an alkyl as defined above.

The terms "cycloalkyl" and "carbocyclic ring" refer to a saturated cyclic group consisting only of carbon atoms having a specified number of carbon atoms (e.g., C₃-C₆), which is a monocyclic, bridged or spiro ring. The cycloalkyl includes, but is not limited to, cyclopropyl, cyclobutyl, cyclopentyl, and cyclohexyl.

The term "aryl" refers to an aromatic group consisting of carbon atoms, each ring having aromaticity. For example, phenyl or naphthyl.

The term "heteroaryl" refers to a cyclic group having a specified number of ring atoms (e.g., 5-12 members), a specified number of heteroatoms (e.g., 1, 2, or 3) and specified heteroatom species (one or more of N, O and S), which is monocyclic or polycyclic, and has at least one aromatic ring (according to the Hückel's rule). Heteroaryls are linked to other fragments of the molecule through aromatic or non-aromatic rings. Heteroaryls include, but are not limited to, furyl, pyrrolyl, thienyl, pyrazolyl, imidazolyl, oxazolyl, thiazolyl, pyridinyl, pyrimidinyl, and indolyl.

The terms "heterocyclyl", "heterocycle" or "heterocycloalkyl" refer to a cyclic group having a specified number of ring atoms (e.g., 3-8 members), a specified number of heteroatoms (e.g., 1, 2, or 3) and specified heteroatom species (one or more of N, O and S), which is monocyclic, bridged, or spiro, and where each ring is saturated. Heterocycloalkyls include, but are not limited to, azetidinyl, tetrahydropyrrolyl, tetrahydrofuryl, morpholinyl, piperidinyl, and the like.

The term "hydroxyl" refers to a -OH group.

The term "cyano" refers to a -CN group.

The term "oxo" refers to a =O group.

Substituted "Cₓ₁-C_{y1}" groups with specified numbers of carbon atoms (x1 and y1 are integers), for example, "Cₓ₁-C_{y1}" alkyl, "Cₓ₁-C_{y1}" cycloalkyl, "Cₓ₁-C_{y1}" cycloalkenyl, "Cₓ₁-C_{y1}" alkoxyl, "Cₓ₁-C_{y1}" alkenyl, "Cₓ₁-C_{y1}" alkynyl, "Cₓ₁-C_{y1}"aryl, "Cₓ₁-C_{y1}" heteroaryl, or "Cₓ₁-C_{y1}" heterocyclyl, all represent numbers of carbon atoms excluding substituents, e.g., a C₁-C₆ alkyl represents a C₁-C₆ alkyl excluding substituents.

The above preferred conditions may be combined arbitrarily to obtain preferred embodiments of the present disclosure without departing from the general knowledge in the art.

The reagents and starting materials used in the present disclosure are commercially available.

The positive/progressive effects of the present disclosure are as follows: the present disclosure provides a nitrogen-containing heterocyclic compound, a method for preparing the same and use thereof, wherein the nitrogen-containing heterocyclic compound has a favorable inhibiting effect on cells with various disease carrying KRAS G12C and/or KRAS G12D mutation, and is prospective for treating and/or preventing various diseases mediated by Ras.

### Detailed description of the embodiments

The present disclosure is further illustrated by the following examples, which are not intended to limit the present disclosure. Experimental procedures without specified conditions in the following examples were performed in accordance with conventional procedures and conditions, or in accordance with instructions.

In the present disclosure, room temperature refers to ambient temperature, or 10-35 °C. Overnight refers to 8-15 hours. Reflux refers to the reflux temperature of a solvent at atmospheric pressure.

The following is a list of abbreviations used in the examples:

| | |
|---|---|
| DMF | *N, N*-dimethylformamide |
| DMAC | *N, N*-dimethylacetamide |
| HATU | 2-(7-azobenzotriazol)-tetramethylurea hexafluorophosphate |
| EDCI | 1-(3-dimethylaminopropyl)-3-ethylcarbodiimide hydrochloride |
| DIPEA | diisopropylethylamine |
| Pd(PPh₃)₄ | tetrakis(triphenylphosphine)palladium |
| Pd(dppf)Cl₂ | [1,1'-bis(diphenylphosphino)ferrocene]palladium(II) dichloride dichloromethane complex |
| Pd₂(dba)₃ | tris(dibenzylideneacetone)dipalladium |
| LiHMDS | Lithium bis(trimethylsilyl)amide |
| *m*-CPBA | *m*-chloroperoxybenzoic acid |
| RuPhos | 2-dicyclohexylphosphino-2',6'-diisopropoxybiphenyl |
| XPhos | 2-dicyclohexylphosphino-2',4',6'-triisopropylbiphenyl |
| TIPS | Triisopropylsilyl |
| Tf | Trifluoromethanesulfonyl |
| NCS | *N*-chlorosuccinimide |
| DMF-DMA | *N, N*-Dimethylformamide-dimethyl acetal |
| THF | Tetrahydrofuran |
| DHP | 3,4-Dihydro-2H-pyran |
| THP | 2-Tetrahydropyran |
| TFA | Trifluoroacetic acid |
| TBAF | Tetrabutylammonium fluoride |
| MOM | Methoxymethyl |
| DMAP | 4-Dimethylaminopyridine |
| ADDP | Azodicarbonyl dipiperidine |
| DIAD | Diisopropyl azo-dicarboxylate |

### Synthetic route of intermediate I-1

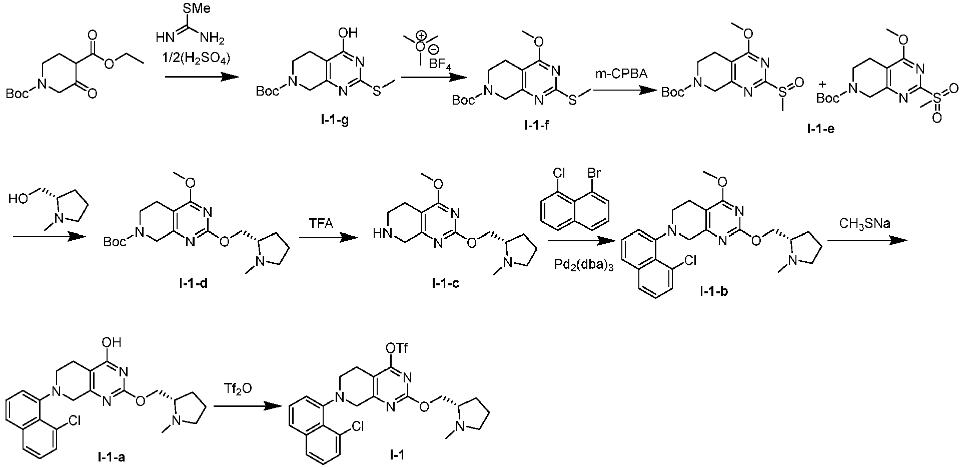

### Synthesis of compound I-1-g

Ethyl 1-*N*-*tert*-butoxycarbonyl-3-oxopiperidine-4-carboxylate (36 g, 132.69 mmol), methanol (500 mL) and 2-methyl-2-thiopseudourea sulfate (44.9 g, 239.11 mmol) were added to a reaction flask. In an ice-cold water bath, sodium methoxide (35.9 g, 664.2 mmol) was added to the above mixture and the mixture was stirred at room temperature overnight under nitrogen atmosphere. The next day, the mixture was adjusted to pH 6 with 1 M hydrochloric acid in an ice-cold water bath, and filtered. The filter cake was dried in air to obtain compound **I-1-g** (53 g) as a white solid, which was used in the next step without purification. LC-MS (ESI): m/z = 298.2 (M+H)⁺.

### Synthesis of compound I-1-f

The crude product of **I-1-g** (4.40 g, 14.8 mmol) was dissolved in dichloromethane (600 mL). Trimethyloxonium tetrafluoroborate (2.40 g, 16.30 mmol) was added at room temperature and the mixture was stirred for 2 hours under nitrogen atmosphere. The reaction was quenched by addition of saturated aqueous sodium bicarbonate (150 mL). Dichloromethane (150 mL × 2) was added for extraction, and the organic phase was washed with brine (100 mL × 2), and purified by column chromatography (mobile phase: ethyl acetate/petroleum ether = 1/5) to obtain compound **I-1-f** (2.10 g, 46%). LC-MS (ESI): m/z 312.1 (M+H)⁺.

### Synthesis of compound I-1-e

Compound **I-1-f** (2.10 g, 6.75 mmol) was dissolved in ethyl acetate (50 mL) and 85% pure m-CPBA (3.42 g, 16.88 mmol) was added at room temperature. After stirred at 0 °C for 3 hours, the mixture was added saturated sodium bicarbonate solution (180 mL) to quench the reaction. Ethyl acetate (500 mL × 2) was added for extraction, and the organic phase was concentrated and purified by column chromatography (mobile phase: ethyl acetate/petroleum ether = 1/3) to obtain compound **I-1-e** (2.10 g, 91%). LC-MS (ESI): m/z 328.1 (M+H)⁺, m/z 343.1 (M+H)⁺.

### Synthesis of compound I-1-d

In an ice-cold water bath, to a solution of compound **I-1-e** (2.10 g, 6.12 mmol) in toluene (60 mL) were serially added *N*-methyl-L-prolinol (0.85 g, 7.35 mmol) and sodium *tert*-butoxide (0.71 g, 7.35 mmol). After stirred in an ice-cold water bath and under nitrogen atmosphere for 0.5 hours, the mixture was added water (10 mL) to quench the reaction. Ethyl acetate (30 mL × 2) was added for extraction, and the organic phase was concentrated and purified by column chromatography (mobile phase: methanol/dichloromethane = 1/20) to obtain compound **I-1-d** (2.20 g, 95%). LC-MS (ESI): m/z 379.2 (M+H)⁺.

### Synthesis of compound I-1-c

Compound **I-1-d** (2.20 g, 5.81 mmol) was added to a mixed solution of dichloromethane (50 mL) and trifluoroacetic acid (5 mL) and the mixture was stirred at room temperature for 3 hours. The reaction mixture was concentrated, and was added saturated aqueous sodium bicarbonate (150 mL) to quench the reaction. Solid sodium sulfate was added to the aqueous solution, and a mixed solution (methanol/dichloromethane = 1120; 330 mL × 3) was added for extraction. The organic phase was dried over sodium sulfate, and concentrated to obtain compound **I-1-c** (1.83 g, 100%). LC-MS (ESI): m/z 279.1 (M+H)⁺.

### Synthesis of compound I-1-b

Compound **I-1-c** (1.60 g, 5.76 mmol), 1-bromo-8-chloronaphthalene (1.70 g, 7.08 mmol), RuPhos (0.50 g, 1.07 mmol), Pd₂(dba)₃ (0.50 g, 0.55 mmol), Cs₂CO₃ (6.80 g, 20.90 mmol) were added to toluene (100 mL), and the mixture was heated to 100 °C and stirred for 3 hours under nitrogen atmosphere. The reaction mixture was cooled to room temperature and was added water (100 mL) to quench the reaction and ethyl acetate (100 mL × 3) for extraction. The organic phase was concentrated and purified by column chromatography (mobile phase: methanol/dichloromethane = 1/20) to obtain compound **I-1-b** (1.23 g, 49%). LC-MS (ESI): m/z 439.2 (M+H)⁺.

### Synthesis of compound I-1-a

Compound **I-1-b** (1.23 g, 2.81 mmol) and sodium thiomethoxide (0.78 g, 11.23 mmol) were added to DMF (30 mL). The mixture was heated to 60 °C and stirred for 3 hours under nitrogen atmosphere. The reaction was quenched by adding water (120 mL) and diluted hydrochloric acid (6 mL, 12 mmol), and ethyl acetate (100 mL × 3) was added for extraction. The organic phase was concentrated and purified by column chromatography (mobile phase: methanol/dichloromethane = 1/10) to obtain compound **I-1-a** (0.89 g, 75%). LC-MS (ESI): m/z 425.1 (M+H)⁺.

### Synthesis of compound I-1

**I-1-a** (0.89 g, 2.10 mmol) and triethylamine (0.64 g, 6.3 mmol) were dissolved in dichloromethane (60 mL) and the mixture was reduced to -40 °C under nitrogen atmosphere, was slowly dropwise added trifluoromethanesulfonic anhydride (1.06 g, 3.80 mmol). After the addition, the mixture was stirred at -40 °C for 0.5 hours. The reaction was quenched by addition of saturated sodium bicarbonate solution (100 mL). Dichloromethane (100 mL × 2) was added for extraction, and the organic phase was concentrated and purified by column chromatography (mobile phase: methanol/dichloromethane = 1/20) to obtain compound **I-1** (0.76 g, 65%). LC-MS (ESI): m/z 557.1 (M+H)⁺.

### Synthetic route of intermediate I-2

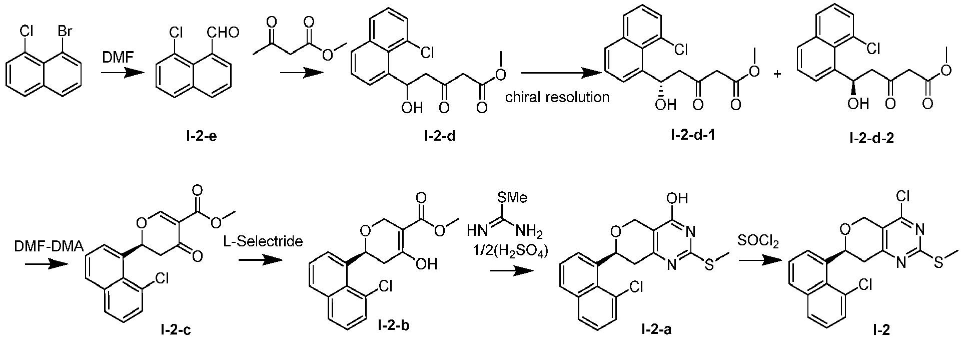

### Synthesis of compound I-2-e

Bromo-8-chloronaphthalene (500 mg, 2.07 mmol) was dissolved in THF (20 mL) and cooled to -78 °C. *n*-BuLi (2.5 M, 1.66 mL, 4.14 mmol) was added dropwise under nitrogen atmosphere. After the addition, the mixture was stirred at -78 °C for 10 minutes, and was added dropwise DMF (800 µL, 10.35 mmol) at -78 °C. After the addition, the reaction mixture was stirred at -78 °C for 30 minutes, warmed to room temperature and stirred for 2 hours. The reaction was quenched by adding 50 mL of saturated ammonium chloride solution, and ethyl acetate (50 mL × 2) was added for extraction. The organic phase was washed with brine (50 mL × 2), dried over anhydrous sodium sulfate, filtered and concentrated to obtain a crude product. The crude product was purified by a flash column chromatography (EA/PE = 1/10) to obtain compound **I-2-e** (330 mg, 84% yield) as a white solid. LC-MS (ESI): m/z = 191.0 [M+H]⁺; ¹H NMR (400 MHz, CDCl₃): *δ* 11.31 (s, 1H), 8.03 (dd, 1H, *J₁*=1.2Hz, *J₂*=8.4Hz), 7.92 (dd, 1H, *J₁*=1.2Hz, *J₂*=7.2Hz), 7.86 (1H, *J*=8.4Hz), 7.70 (dd, 1H, *J₁*=1.2Hz, *J₂*=7.6Hz), 7.59 (t, 1H, *J*=7.6Hz), 7.47 (t, 1H, *J*=8Hz).

### Synthesis of compound I-2-d

NaH (60%, 242 mg, 6.05 mmol) was added to 6 mL of THF at room temperature. Methyl acetoacetate (543 µL, 5.04 mmol) was then added at room temperature under nitrogen atmosphere . After stirred at room temperature for 30 minutes under nitrogen atmosphere, the mixture was added dropwise n -BuLi (2.5 M, 2.4 mL, 6.05 mmol) at -15 °C to -10 °C. After the addition, the mixture was kept at this temperature for 30 minutes, and was added a solution of compound **I-2-e** (320 mg, 1.68 mmol) in THF (10 mL) dropwise. After the addition, the mixture was stirred at a low temperature (-10 °C to 0 °C) for 2 hours, and the reaction was quenched by adding saturated ammonium chloride solution (50 mL) and the aqueous phase was extracted with ethyl acetate (50 mL × 2). The organic phase was washed with brine (50 mL × 2), dried over anhydrous sodium sulfate, filtered, and concentrated to obtain the crude product. The crude product was purified by a flash column chromatography (EA/DCM = 1/10) to obtain compound **I-2-d** (510 mg, 99% yield) as a white solid. LC-MS (ESI): m/z = 329.1 [M+Na]⁺; ¹H NMR (400 MHz, CDCl₃) : δ 8.06 (d, 1H, *J*=6.4Hz), 7.79 (d, 2H, *J*=8Hz), 7.58 (dd, 1H, *J₁*=7.6Hz, *J₂*=1.6Hz), 7.53 (t, 1H, *J*=7.6Hz), 7.34 (t, 1H, *J*=7.6Hz), 6.91 (dd, 1H, *J₁*=9.2Hz, *J₂*=2.4Hz), 3.74 (s, 3H), 3.54 (s, 2H), 3.36 (dd, 1H, *J₁*=18Hz, *J₂*=1.6Hz), 3.24 (d, 1H, *J*=3.6Hz), 2.85-2.75 (m, 1H).

### Synthesis of compounds I-2-d-1 and I-2-d-2

Compound **I-2-d** (8.5 g, 27.8 mmol) was prepared in a larger scale, and was separated by chiral resolution to obtain compound **I-2-d-1** (2.5 g, 29%) as a white solid and compound **I-2-d-2** (2.6 g, 31%) as a white solid.

| Conditions for chiral analysis | Conditions for chiral preparation |
|---|---|
| Instrument: SFC Method Station (Thar, Waters) | Instrument: SFC-150 (Thar, Waters) |
| Column: AD-H 4.6 × 100 mm, 5 µm (Daicel) | Column: AD 20 × 250 mm, 10 µm (Daicel) |
| Column temperature: 40 °C | Column temperature: 35 °C |
| Mobile phase: CO₂/ethanol (1% ammonium in methanol) = 75/25 | Mobile phase: CO₂/ethanol (0.2% ammonium in methanol) = 65/35 |
| Flow rate: 4.0 mL/min | Flow rate: 100 g/min |
| Wavelength: 254 nm | Pressure: 100 bar |
| Pressure: 120 bar | Wavelength: 214 nm |
| | Circulation: 5.0 min |
| | Sample solution: 8.5 g in 150 mL of methanol and dichloromethane |
| **I-2-d-1:** the retention time was 1.57 min; e.e% = 100.0%; | |
| **I-2-d-2:** the retention time was 2.33 min; e.e% = 99.12%. | |

| | |
|---|---|
| **I-2-d-1:** LC-MS (ESI): m/z = 329.1 [M+Na]⁺. **I-2-d-2:** LC-MS (ESI): m/z = 329.1 [M+Na]⁺. | |

### Synthesis of compound I-2-c

Compound **I-2-d-1** (2.3 g, 7.5 mmol) was dissolved in DCM (80 mL) at room temperature, and DMF-DMA (1.2 mL, 9.0 mmol) was added at room temperature under nitrogen atmosphere . After stirred at room temperature for 45 minutes, the reaction mixture was added BF₃·Et₂O (1.2 mL, 9.0 mmol). After the addition, the mixture was stirred at room temperature for 1 hour, and the reaction was quenched by adding saturated sodium bicarbonate solution. Dichloromethane (100 mL × 2) was added for extraction, and the organic phase was washed with brine (100 mL × 2), dried over anhydrous sodium sulfate, filtered, and concentrated to obtain a crude product of compound **I-2-c** (2.0 g, 84%), which was used directly in the next step. LC-MS (ESI): m/z = 317.1 [M+1]⁺ .

### Synthesis of compound I-2-b

Compound **I-2-c** (2.0 g, 6.31 mmol) was dissolved in THF (60 mL) at room temperature. Lithium tri-sec-butylborohydride (1 M in THF, 6.95 mL, 6.95 mmol) was added dropwise at - 78 °C under nitrogen atmosphere. After the addition, the mixture was stirred at -78 °C for 1 hour. The reaction was quenched by adding 1 M hydrochloric acid (20 mL), and ethyl acetate (100 mL × 2) was added for extraction. The organic phase was washed with brine (50 mL × 2), dried over anhydrous sodium sulfate, filtered, and concentrated to obtain a crude product, which was purified by a flash column chromatography (PE/EA = 0-15%) to obtain compound **I-2-b** (1.8 g, 89%) as a yellow oil. LC-MS (ESI): m/z = 319.0 [M+1]⁺ .

### Synthesis of compound I-2-a

Compound **I-2-b** (1.5 g, 4.71 mmol) was dissolved in methanol (30 mL) at room temperature. Sodium methoxide (1.27 g, 23.5 mmol) and 2-methyl-2-thiopseudourea sulfate (1.18 g, 4.24 mmol) were serially added at 0 °C under nitrogen atmosphere. After the addition, the mixture was warmed to room temperature and stirred for 20 hours. The reaction mixture was adjusted to pH 5 with 1 M diluted hydrochloric acid. A solid was precipitated, and the mixture was filtered. The filter cake was washed with a mixed solution of ethyl acetate (20 mL) and petroleum ether (20 mL), and the solid was collected and dried in vacuo to obtain a crude product of **I-2-a** (0.65 g, 39%) as a white solid. LC-MS (ESI): m/z = 359.1 [M+1]⁺.

### Synthesis of compound I-2

To a solution of **I-2-a** (4.0 g, 11.1 mmol) in DMF (40 mL) and DCM (20 mL) was added thionyl chloride (9.3 g, 78.0 mmol) dropwise in an ice-cold water bath. After the addition, the reaction nixture was stirred in an ice-cold water bath for 4 hours. The reaction mixture was slowly dropped into 60 mL of water with the internal temperature controlled at 0 -10 °C, and DCM was added for extraction. The organic phase was washed with saturated sodium bicarbonate and water, and concentrated. n-Heptane was added for slurrying, and the mixture was cooled to 0-10 °C and filtered. The residue was dried to obtain compound **I-2** (3.2 g, 76%). LC-MS (ESI): m/z = 377.0 [M+H]⁺. The structure of the compound was confirmed by monocrystal analysis.

### Synthetic route of intermediate I-3

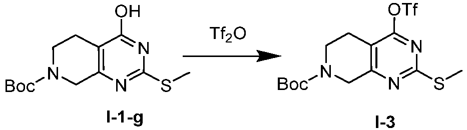

### Synthesis of compound I-3

Compound **I-1-g** (8.00 g, 26.90 mmol) was dissolved in DCM (100 mL) at room temperature, and DIPEA (22.23 mL, 134.50 mmol) and trifluoromethanesulfonic anhydride (11.30 mL, 67.30 mmol) were added serially to the above mixture in an ice-cold water bath under nitrogen atmosphere. After the addition, the reaction mixture was stirred for 2 hours in an ice-cold water bath and the reaction was quenched with saturated sodium bicarbonate solution, the organic phase was separated and the aqueous phase was extracted with dichloromethane. The organic phases were combined, dried over anhydrous sodium sulfate, filtered and concentrated to obtain a crude product which was purified by a flash column chromatography (mobile phase: ethyl acetate/petroleum ether: 0% to 10%) to obtain compound **I-3** (11.00 g, 95%) as a white solid. LC-MS (ESI): m/z 452.0 (M+H)⁺.

### Synthetic route of intermediate I-4

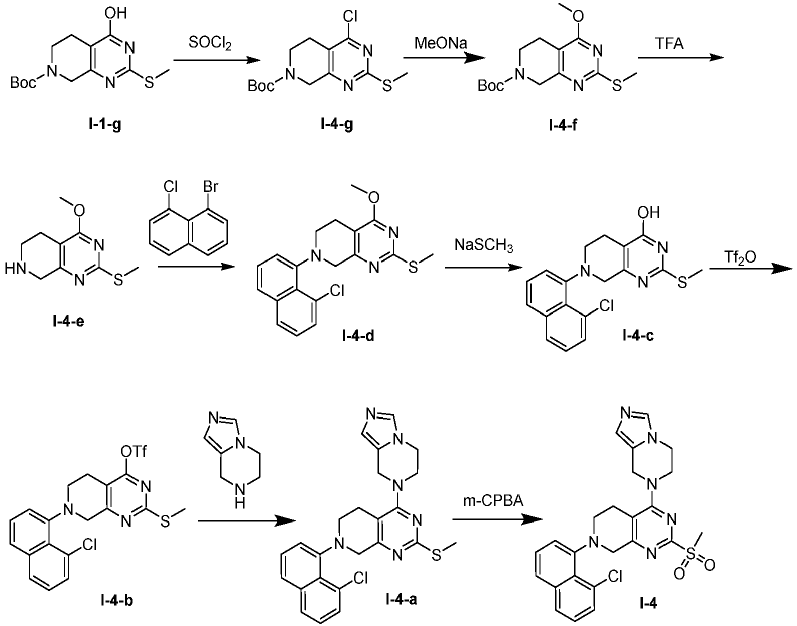

### Synthesis of compound I-4-g

**I-1-g** (29.00 g, 97.60 mmol) was dissolved in dichloromethane (200 mL) and DMF (100 mL). At 0 °C, thionyl chloride (14.30 g, 121.10 mmol) was added dropwise, and under nitrogen atmosphere the mixture was slowly warmed to room temperature and stirred for 4 hours. The reaction was quenched by addition of saturated aqueous sodium bicarbonate (800 mL). Dichloromethane (400 mL × 2) was added for extraction, and the organic phase was washed with brine (100 mL × 2), and purified by column chromatography (mobile phase: ethyl acetate/petroleum ether = 1/3) to obtain compound **I-4-g** (20.30 g, 67%). LC-MS (ESI): m/z 316.1 (M+H)⁺.

### Synthesis of compound I-4-f

Compound **I-4-g** (20.30 g, 64.40 mmol) was dissolved in methanol (200 mL), and sodium methoxide (13.90 g, 258.00 mmol) was added at room temperature. After the mixture was stirred at 60 °C for 2 hours, the reaction was quenched by adding water (180 mL). Dichloromethane (100 mL × 3) was added for extraction and the organic phase was concentrated to obtain compound **I-4-f** (18.30 g, 91%). LC-MS (ESI): m/z 312.1 (M+H)⁺.

### Synthesis of compound I-4-e

Compound **I-4-f** (18.30 g, 58.84 mmol) was added to a mixed solution of dichloromethane (80 mL) and trifluoroacetic acid (40 mL), and the mixture was stirred at room temperature for 3 hours. The reaction mixture was concentrated, and saturated aqueous sodium bicarbonate (200 mL) was added to quench the reaction. Solid sodium sulfate was added to the aqueous solution, and dichloromethane (300 mL × 3) was added for extraction. The organic phase was dried over sodium sulfate and concentrated to obtain compound **I-4-e** (14.00 g, 100%). LC-MS (ESI): m/z 212.1 (M+H)⁺.

### Synthesis of compound I-4-d

Compound **I-4-e** (12.40 g, 51.80 mmol), 1-bromo-8-chloronaphthalene (8.40 g, 39.80 mmol), RuPhos (3.70 g, 7.90 mmol), Pd₂(dba)₃ (3.64 g, 3.98 mmol) and Cs₂CO₃ (51.75 g, 159.20 mmol) were added to toluene (200 mL), and the mixture was heated to 100 °C and stirred for 3 hours under nitrogen atmosphere. The reaction mixture was cooled to room temperature, filtered and washed with dichloromethane (200 mL). The organic phases were concentrated and purified by column chromatography (mobile phase: petroleum ether/dichloromethane = 1/3) to obtain compound **I-4-d** (11.00 g, 75%). LC-MS (ESI): m/z 372.1 (M+H)⁺.

### Synthesis of compound I-4-c

To a solution of **I-4-d** (1.2 g, 3.23 mmol) in DMF (10 mL) at room temperature was added sodium thiomethoxide (905 mg, 12.91 mmol). The mixture was degassed and purged with nitrogen and stirred at 60 °C for 1 hour. The reaction mixture was cooled to room temperature and diluted slowly with water (20 mL) to precipitate a white solid. The mixture was filtered, and the filter cake was washed with water and dried to obtain compound **I-4-c** (1.4 g, 96%) as a white solid. LC-MS (ESI): m/z 358.2 (M+H) ⁺.

### Synthesis of compound I-4-b

To a solution of **I-4-c** (1.2 g, 3.35 mmol) in dichloromethane (30 mL) in an ice-cold water bath under nitrogen atmosphere were added DIPEA (2.77 mL, 16.77 mmol) and trifluoromethanesulfonic anhydride (1.41 mL, 8.38 mmol) serially. After the addition, the mixture was stirred at 0 °C for 2 hours. The reaction was quenched by adding saturated sodium bicarbonate solution. The organic phase was separated, and the aqueous phase was extracted with dichloromethane. The organic phases were combined, washed with water and brine, dried over anhydrous Na₂SO₄, filtered, and evaporated. The crude product was purified by a flash column chromatography (mobile phase: petroleum ether/ethyl acetate = 10/1 to 2/1) to obtain compound **I-4-b** (1.49 g, 90%) as an amber oil. LC-MS (ESI): m/z 490.0 (M+H) ⁺.

### Synthesis of compound I-4-a

Compound **I-4-b** (1.49 g, 3.04 mmol) was dissolved in DMF (15 mL) at room temperature, followed by serial addition of DIPEA (1.50 mL, 9.12 mmol) and 5,6,7,8-tetrahydroimidazo[1,5-*α*]pyrazine (487 mg, 3.95 mmol). After the addition, the reaction mixture was degassed and purged with nitrogen, and stirred at room temperature overnight. The next day, water (30 mL) was added to the reaction mixture, followed by extraction with ethyl acetate. The organic phase was washed with brine, dried over anhydrous sodium sulfate, filtered, and concentrated to obtain a crude product, which was purified by a flash column chromatography (mobile phase: methanol/dichloromethane = 0% to 10%) to obtain compound **I-4-a** (1.36 g, 96%) as a white solid. LC-MS (ESI): m/z 463.1 (M+H)⁺.

### Synthesis of compound I-4

Compound **I-4-a** (200 mg, 0.43 mmol) was dissolved in dichloromethane (10 mL) in an ice-cold water bath. *m*-Chloroperoxybenzoic acid (85%, 219 mg, 1.08 mmol) was added and the mixture was slowly warmed to room temperature and stirred for 2 hours. Upon completion, saturated aqueous sodium bicarbonate was added for neutralization. The organic phase was separated, and the aqueous phase was extracted with dichloromethane. The organic phases were combined, dried over anhydrous Na₂SO₄, filtered and evaporated. The crude product was purified by a flash column chromatography (mobile phase: dichloromethane/methanol = 1/0 to 10/1) to obtain **I-4** (200 mg, 93%) as an earthy yellow solid. LC-MS (ESI): m/z 495.1 (M+H)⁺.

### Example 1: Synthetic route of compound 1

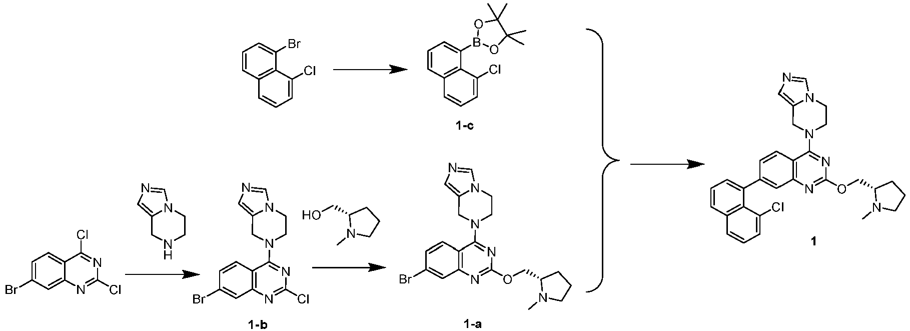

### Synthesis of compound 1-b

2,4-Dichloro-7-bromoquinazoline (500 mg, 1.81 mmol), DMF (10 mL), 5,6,7,8-tetrahydroimidazo [1,5-α]pyrazine (279 mg, 2.26 mmol) and DIPEA (1.49 mL, 9.06 mmol) were added to a reaction flask. The reaction mixture was stirred at room temperature overnight under nitrogen atmosphere. The next day, the reaction was quenched by adding 30 mL of water, and a precipitated solid was filtered and dried in vacuo to obtain compound **1-b** (621 mg, 94%) as an off-white solid, which was directly used in the next step without purification. LC-MS (ESI): m/z = 363.9 (M+H)⁺.

### Synthesis of compound 1-c

Bromo-8-chloronaphthalene (500 mg, 2.08 mmol), DMF (15 mL), bis(pinacolato)diboron (1.32 g, 5.21 mmol), potassium acetate (1.22 g, 12.5 mmol) and Pd(dppf)Cl₂ (152 mg, 0.21 mmol) were added to a reaction flask. The reaction mixture was degassed and purged with nitrogen for three times and stirred at 80 °C overnight. The next day, the mixture was cooled to room temperature, and separated by using 40 mL of water and 40 mL of ethyl acetate. The organic phase was washed with brine, concentrated by rotary evaporation, and purified by column chromatography (mobile phase: ethyl acetate/petroleum ether = 0/100 to 10/90) to obtain compound **1-c** (416 mg, 69%) as a white solid. LC-MS (ESI): m/z = 289.1 (M+H)⁺.

### Synthesis of compound 1-a

**1-b** (550 mg, 1.52 mmol), toluene (20 mL), *N*-methyl-L-prolinol (900 µL, 7.58 mmol) and sodium *tert*-butoxide (291 mg, 3.03 mmol) were added to a reaction flask. The reaction mixture was stirred at room temperature under nitrogen atmosphere for 1 hour. The reaction mixture was directly purified by column chromatography (mobile phase: methanol/dichloromethane = 0/100 to 10/90) to obtain compound **1-a** (614 mg, 92%) as a pale brown solid. LC-MS (ESI): m/z = 443.1 (M+H)⁺.

### Synthesis of compound 1

**1-c** (98 mg, 0.34 mmol), dioxane (20 mL), **1-a** (100 mg, 0.23 mmol), water (2 mL), cesium carbonate (221 mg, 0.68 mmol) and tetrakis(triphenylphosphine)palladium (26 mg, 0.023 mmol) were added to a reaction flask. The reaction mixture was degassed and purged with nitrogen for three times and stirred at 100 °C under nitrogen atmosphere overnight. The next day, the reaction mixture was concentrated by rotary evaporation and purified by column chromatography (mobile phase: methanol/dichloromethane = 0/100 to 10/90) to obtain compound **1** (63 mg, 35%) as a pale brown solid. LC-MS (ESI): m/z = 525.1 (M+H)⁺; ¹H NMR (400 MHz, CDCl₃) : *δ* 7.94(1H, d, *J* = 8Hz), 7.88(1H, d, *J* = 8Hz), 7.82(1H, d, *J* = 8Hz), 7.73(1H, s), 7.60-7.50(3H, m), 7.48-7.39(2H, m), 7.33(1H, d, *J* = 8.4Hz), 6.92(1H, s), 5.18-5.00(2H, m), 4.91-4.74(1H, m), 4.60-4.48(1H, m), 4.48-4.34(2H, m), 4.32-4.08(2H, m), 3.51-3.34(1H, m), 3.31-3.07(1H, m), 2.74(3H, s), 2.68-2.49(1H, m), 2.32-2.14(1H, m), 2.14-1.84(3H, m).

### Example 2: Synthetic route of compound 2

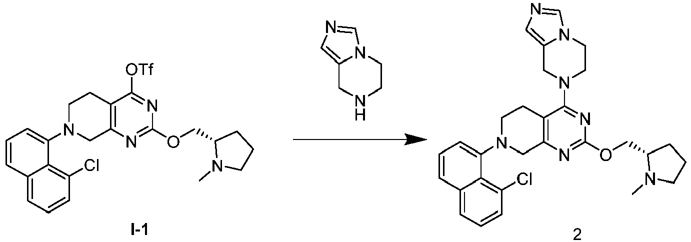

### Synthesis of compound 2

**I-1** (20 mg, 0.036 mmol) was dissolved in DMF (2 mL), and 5,6,7,8-tetrahydroimidazo[1,5-α]pyrazine (7 mg, 0.054 mmol) and DIPEA (30 µL, 0.18 mmol) were added. The mixture was stirred at room temperature under nitrogen atmosphere for 1 hour, directly subjected to pre-HPLC (ammonium bicarbonate), and lyophilized to obtain compound **2** (9 mg, 48%) as a white solid. LC-MS (ESI): m/z = 530.0 (M+H)⁺; ¹H NMR (400 MHz, CDCl₃) : *δ* 7.75(1H, d, *J*=7.6Hz), 7.61(1H, d, *J*=8.4Hz), 7.52(1H, d, *J*=8Hz), 7.48(1H, s), 7.44(1H, t, *J*=7.6Hz), 7.33(1H, t, *J*=8Hz), 7.22(1H, d, *J*=7.2Hz), 6.88(1H, s), 4.83-4.66(2H, m), 4.52-4.37(2H, m), 4.35-4.25(1H, m), 4.24-4.13(2H, m), 4.13-4.02(1H, m), 3.86(1H, d, *J*=17.6Hz), 3.78-3.66(1H, m), 3.63-3.49(1H, m), 3.27-3.06(3H, m), 2.83-2.66(1H, m), 2.64-2.56(1H, m), 2.51(3H, s), 2.40-2.25(1H, m), 2.14-2.02(1H, m), 1.93-1.71(3H, m).

### Example 3: Synthetic route of compound 3

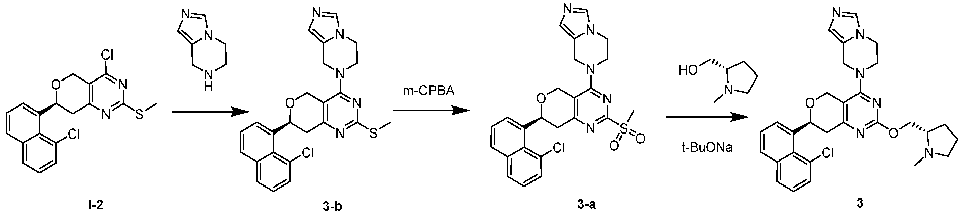

### Synthesis of compound 3-b

To a solution of **I-2** (1.00 g, 2.66 mmol) in DMF (10 mL) at room temperature were added *N*, *N*-diisopropylethylamine (1.03 g, 7.97 mmol) and 5,6,7,8-tetrahydroimidazo[1,5-*α*]pyrazine (426 mg, 3.45 mmol). After the addition, the reaction mixture was heated to 100 °C and stirred for two hours. Upon completion, ethyl acetate was added for dilution, and the mixture was serially washed with water and brine, dried over anhydrous Na₂SO₄, filtered and concentrated by rotary evaporation. The crude product was purified by a flash column chromatography (mobile phase: dichloromethane/methanol = 1/0 to 10/1) to obtain compound **3-b** (1.10 g, 89%) as a brown solid. LC-MS (ESI): m/z 464.1 (M+H)⁺.

### Synthesis of compound 3-a

Compound **3-b** (1.10 g, 2.37 mmol) was dissolved in dichloromethane (20 mL) in an ice-cold water bath. *m*-Chloroperoxybenzoic acid (85%, 1.20 g, 5.93 mmol) was added and the mixture was slowly warmed to room temperature and stirred for 2 hours. Upon completion, saturated aqueous sodium bicarbonate was added for neutralization. The organic phase was separated, and the aqueous phase was extracted with dichloromethane. The organic phases were combined, dried over anhydrous Na₂SO₄, filtered and evaporated. The crude product was purified by a flash column chromatography (mobile phase: dichloromethane/methanol = 1/0 to 10/1) to obtain **3-a** (1.00 g, 85%) as a white solid. LC-MS (ESI): m/z 495.9 (M+H) ⁺.

### Synthesis of compound 3

To a solution of **3-a** (1.00 g, 2.02 mmol) and *N*-methyl-L-prolinol (465 mg, 4.03 mmol) in toluene (20 mL) in an ice-cold water bath was added sodium *tert*-butoxide (388 mg, 4.03 mmol). After the addition, the reaction mixture was stirred in an ice-cold water bath for 10 minutes. Upon completion, the mixture was concentrated at reduced pressure, diluted with ethyl acetate, and serially washed with water and brine, dried over anhydrous Na₂SO₄, filtered and concentrated by rotary evaporation to obtain a brown oil. The crude product was purified by a flash column chromatography (mobile phase: dichloromethane/methanol = 1/0 to 10/1) to obtain compound **3** (400 mg, 37%) as a white solid. LC-MS (ESI): m/z 531.2 (M+H) ⁺; ¹H NMR (400MHz, CDCl₃): δ7.97 (1H, d, *J* = 6.8Hz), 7.81-7.86 (2H, m), 7.61 (1H, dd, *J* = 7.2, 1.2Hz), 7.56 (1H, t, *J* = 7.6Hz), 7.50 (1H, s), 7.37 (1H, t, *J* = 8.0Hz), 6.90 (1H, s), 6.50 (1H, dd, *J* = 10.8, 3.6Hz), 5.03 (1H, d, *J* = 13.2Hz), 4.85 (1H, d, *J* = 13.2Hz), 4.53-4.76 (3H, m), 4.26-4.42 (2H, m), 4.03-4.21 (2H, m), 3.62-3.72 (1H, m), 3.54-3.62 (1H, m), 2.95-3.50 (1H, m), 2.41-2.94 (5H, m), 1.72-2.32 (5H, m).

### Example 4: Synthetic route of compound 4

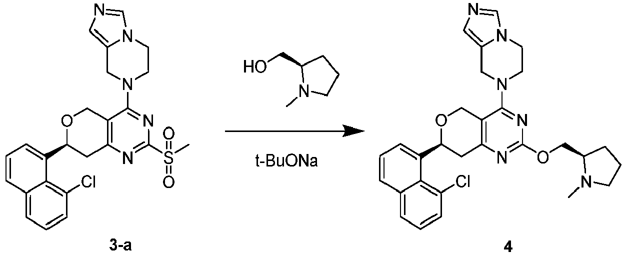

### Synthesis of compound 4

To a solution of **3-a** (50 mg, 0.10 mmol) and *N*-methyl-D-prolinol (23 mg, 0.20 mmol) in toluene (10 mL) in an ice-cold water bath was added sodium *tert*-butoxide (20 mg, 0.20 mmol). After the addition, the reaction mixture was stirred in an ice-cold water bath for 10 minutes. Upon completion, the mixture was concentrated at reduced pressure, diluted with ethyl acetate, and serially washed with water and brine, dried over anhydrous Na₂SO₄, filtered and concentrated by rotary evaporation to obtain a brown oil. The crude product was purified by a flash column chromatography (mobile phase: dichloromethane/methanol = 1/0 to 10/1) to obtain compound **4** (40 mg, 75%) as a white solid. LC-MS (ESI): m/z 531.2 (M+H) ⁺; ¹H NMR (400MHz, CDCl₃): *δ* 7.97 (1H, d, *J* = 6.8Hz), 7.81-7.85 (2H, m), 7.60 (1H, dd, *J* = 7.2, 1.2Hz), 7.56 (1H, t, *J* = 7.6Hz), 7.49 (1H, s), 7.37 (1H, d, *J* = 7.6Hz), 6.89 (1H, s), 6.50 (1H, dd, *J* = 11.2, 3.2Hz), 5.02 (1H, d, *J* = 13.6Hz), 4.85 (1H, d, *J* = 13.6Hz), 4.67 (2H, s), 4.25-4.40 (2H, m), 4.04-4.20 (2H, m), 3.62-3.73 (1H, m), 3.54-3.62 (1H, m), 3.34-3.50 (1H, m), 2.97-3.19 (1H, m), 2.83-2.91 (1H, m), 2.70 (3H, m), 2.49-2.81 (2H, m), 2.11-2.27 (1H, m), 1.97-2.11 (1H, m), 1.84-1.98 (2H, m).

### Example 5: Synthetic route of compound 5

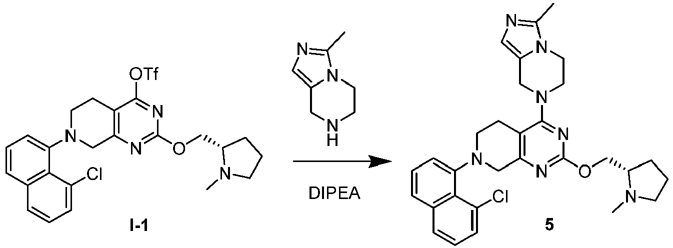

### Synthesis of compound 5

**I-1** (20 mg, 0.04 mmol), 3-methyl-5,6,7,8-tetrahydroimidazo[1,5-*α*]pyrazine (10 mg, 0.07 mmol), DMF (1.5 mL) and DIPEA (50 mg, 0.39 mmol) were added to a reaction flask. The reaction mixture was stirred at room temperature for 1 hour. The reaction mixture was directly subjected to pre-HPLC to obtain compound **5** (3 mg, 16%). LC-MS (ESI): m/z 544.2 (M+H) ⁺; ¹H NMR (400MHz, MeOD-*d₄*): *δ*8.34 (1H, d, *J* = 8.0Hz), 7.67 (1H, d, *J* = 8.0Hz), 7.52 (1H, d, *J* = 7.2Hz), 7.48 (1H, t, *J* = 8.0Hz), 7.38 (1H, d, *J* = 8.0Hz), 7.32 (1H, t, *J* = 8.0Hz), 6.70 (1H, s), 4.82 (1H, d, *J* = 16.0Hz), 4.73 (1H, d, *J* = 16.0Hz), 4.27-4.46(3H, m), 4.01-4.22 (3H, m), 3.63-3.82 (2H, m), 3.56-3.62 (1H, m), 3.08-3.32 (3H, m), 2.81-2.91 (1H, m), 2.69 (1H, d, *J* = 14.4Hz), 2.54 (3H, s), 2.33-2.49 (1H, m), 2.34 (3H, s), 2.05-2.18 (1H, m), 1.81-1.88 (2H, m), 1.66-1.80 (1H, m).

### Example 6: Synthetic route of compound 6

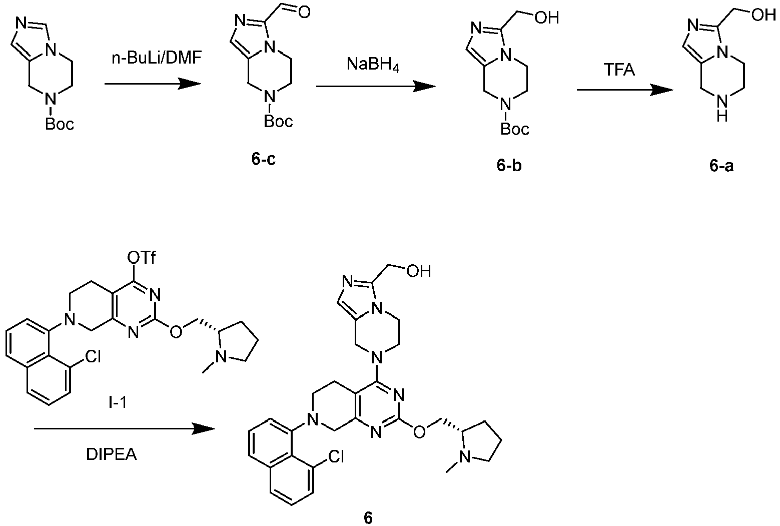

### Synthesis of compound 6-c

In a dry ice/acetone bath, *tert*-butyl 5,6-dihydroimidazo[1,5-*α*]pyrazine-7(8*H*)-carboxylate (500 mg, 2.24 mmol) was dissolved in anhydrous THF (20 mL) and the mixture was stirred at - 78 °C for 15 min. The mixture was added *n*-BuLi (2.5 M, 1.34 mL, 3.36 mmol), and was continuously stirred at this temperature for 30 min. Anhydrous DMF (0.35 mL, 4.48 mol) was added. After the addition, the mixture was slowly warmed to room temperature and continuously stirred for 2 hours. Upon completion, the reaction was quenched by adding water. The mixture was extracted with ethyl acetate, and the organic phases were combined, washed serially with water and brine, dried over anhydrous sodium sulfate, filtered, and concentrated to obtain a crude product, which was purified by a flash column chromatography (mobile phase: dichloromethane/methanol = 1/0 to 10/1) to obtain compound **6-c** (350 mg, 62%) as a colorless oil. LC-MS (ESI): m/z 252.2 (M+H) ⁺.

### Synthesis of compound 6-b

**6-c** (350 mg, 1.39 mol) was dissolved in methanol (10 mL) in an ice-cold water bath, and was added sodium borohydride (76 mg, 2.0 mmol) in portions. After the addition, the mixture was slowly warmed to room temperature and stirred for 1 hour. The reaction was quenched by adding water. The reaction mixture was poured into water and extracted with dichloromethane, and the organic phase was serially washed with water and brine, dried over anhydrous sodium sulfate, filtered, and concentrated to obtain a crude product, which was purified by a flash column chromatography (mobile phase: dichloromethane/methanol = 1/0 to 10/1) to obtain compound **6-b** (200 mg, 57%) as a pale yellowish solid. LC-MS (ESI): m/z 254.2 (M+H) ⁺.

### Synthesis of compound 6-a

To a solution of **6-b** (50 mg, 0.20 mmol) in dichloromethane (10 mL) was added trifluoroacetic acid (2 mL) at room temperature. The resulting mixture was stirred at room temperature for 4 hours. Upon completion, the reaction mixture was concentrated, carefully neutralized with a saturated solution of sodium bicarbonate to pH > 7 in an ice-cold water bath, and extracted with ethyl acetate. The organic phases were combined, washed with brine, dried over anhydrous sodium sulfate, filtered, and concentrated to obtain the product **6-a** (15 mg, 50%) as a brown oil. LC-MS (ESI): m/z 154.2 (M+H) ⁺.

### Synthesis of compound 6

Compound **I-1** (20 mg, 0.04 mmol) was dissolved in DMF (2 mL) at room temperature, followed by serial addition of DIPEA (23 mg, 0.18 mmol) and **6-a** (12 mg, 0.05 mmol). After the addition, the reaction mixture was degassed and purged with nitrogen, and stirred at room temperature overnight. The next day, the reaction mixture was filtered and directly purified by pre-HPLC to obtain compound **6** (10 mg, 50%) as a white solid. LC-MS (ESI): m/z 560.4 (M+H) ⁺; ¹H NMR (400MHz, CDCl₃): *δ* 7.75 (1H, d, *J* = 8.4Hz), 7.61 (1H, d, *J* = 7.6Hz), 7.52 (1H, d, *J* = 7.6Hz), 7.44 (1H, t, *J* = 8.0Hz), 7.33 (1H, t, *J* = 7.6Hz), 7.22 (1H, d, *J* = 7.6Hz), 6.79 (1H, s), 4.69 (2H, s), 4.65-4.75 (2H, m), 4.03-4.46 (6H, m), 3.85 (1H, d, *J* = 17.6Hz), 3.65-3.76 (1H, m), 3.51-3.61 (1H, m), 3.05-3.24 (3H, m), 2.54-2.74 (2H, m), 2.48 (3H, s), 2.22-2.35 (1H, m), 2.01-2.14 (1H, m), 1.69-1.88 (3H, m).

### Example 7: Synthetic route of compound 7

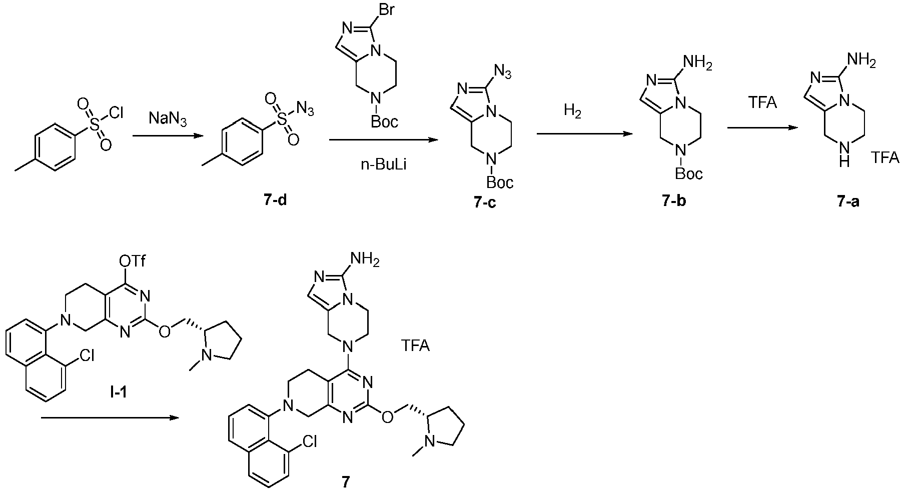

### Synthesis of compound 7-d

Sodium azide (286 mg, 4.4 mmol) was dissolved in water (3 mL) at room temperature, and acetone (5 mL) was added. The mixture was cooled to 0 °C before a solution of *p*-toluenesulfonyl chloride (762 mg, 4 mmol) in acetone (5 mL) was added dropwise. After the addition, the reaction mixture was warmed to room temperature and stirred for 4 hours. After concentration at reduced pressure, 10 mL of water was added, and the mixture was extracted with DCM (50 mL × 2). The organic phase was dried over anhydrous sodium sulfate, filtered, and concentrated at reduced pressure to obtain compound **7-d** (623 mg, 79%) as a colorless oil.

### Synthesis of compound 7-c

*Tert*-butyl 3-bromo-5,6-dihydroimidazo[1,5-α]pyrazine-7(8*H*)-carboxylate (100 mg, 0.33 mmol) was dissolved in 10 mL of THF at room temperature, and *n*-BuLi (158 µL, 0.40 mmol) was added dropwise under nitrogen atmosphere at -78 °C. After the addition, the reaction mixture was stirred for 30 minutes at -78 °C before a solution of compound **7-d** (130 mg, 0.66 mmol) in THF (5 mL) was added dropwise. The reaction mixture was stirred for another 30 minutes at -78 °C. The reaction was quenched by adding saturated ammonium chloride solution (20 mL) before 20 mL of water was added. The mixture was extracted with ethyl acetate (50 mL × 2), and the organic phase was dried over anhydrous sodium sulfate, filtered and concentrated at reduced pressure. The crude product was purified by a flash column chromatography (PE/EA = 1:1) to obtain compound **7-c** (70 mg, 80%) as a colorless oil. LC-MS (ESI): m/z = 265.2 [M+1]⁺.

### Synthesis of compound 7-b

Compound **7-c** (70 mg, 0.26 mmol) was dissolved in 30 mL of methanol at room temperature, and palladium on carbon (50 mg, 10% Pd, 50% wet) was added. The mixture was stirred at room temperature under hydrogen atmosphere for 16 hours. The reaction mixture was filtered and the filtrate was concentrated to obtain compound **7-b** (60 mg, 96%) as a colorless oil. LC-MS (ESI): m/z = 239.1[M+1]⁺.

### Synthesis of compound 7-a

Compound **7-b** (60 mg, 0.25 mmol) was dissolved in 10 mL of DCM at room temperature and was added 3 mL of trifluoroacetic acid, and the reaction mixture was stirred at room temperature for 3 hours. The reaction mixture was concentrated at reduced pressure to obtain a crude product, which was dried in vacuo for 1 hour to obtain a crude product of compound **7-a** (about 63 mg, 100%), which was used directly in the next step. LC-MS (ESI): m/z = 139.2[M+1]⁺.

### Synthesis of compound 7

The crude product of compound **7-a** (about 63 mg, 0.25 mmol) was dissolved in 5 mL of DMF at room temperature before DIPEA (206 µL, 1.25 mmol) and **I-1** (45 mg, 0.081 mmol) were added. The reaction mixture was stirred at room temperature under nitrogen atmosphere for 16 hours. The reaction mixture was purified by pre-HPLC (acidic, TFA) to obtain compound **7** (20 mg, 38%) as a yellow solid. LC-MS (ESI): m/z = 545.3 [M+1]⁺; ¹H NMR (400 MHz, Methanol*d₄*) : *δ* 7.84 (1H, dd, *J*₁=8Hz, *J*₂=0.8Hz), 7.70 (1H, d, *J*=7.6Hz), 7.56-7.46 (2H, m), 7.39 (1H, d, *J*=8Hz), 7.37-7.30 (1H, m), 6.76 (1H, s), 4.96-4.82 (1H, m), 4.80-4.68 (2H, m), 4.64-4.54 (1H, m), 4.40-4.20 (2H, m), 4.12-4.01 (1H, m), 4.00-3.82 (3H, m), 3.82-3.67 (2H, m), 3.66-3.56 (1H, m), 3.38-3.30 (1H, m), 3.27-3.16 (2H, m), 3.06 (3H, s), 2.78-2.67 (1H, m), 2.45-2.32 (1H, m), 2.28-1.94 (3H, m).

### Example 8: Synthetic route of compound 8

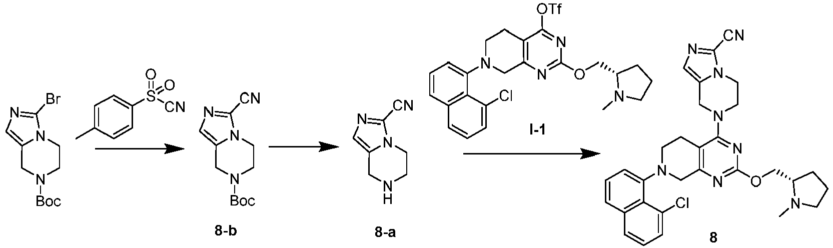

### Synthesis of compound 8-b

*Tert*-butyl 3-bromo-5,6-dihydroimidazo[1,5-*α*]pyrazine-7(8*H*)-carboxylate (100 mg, 0.33 mmol) and tetrahydrofuran (10 mL) were added to a reaction flask before 2.5 M *n*-butyllithium (159 µL, 0.40 mmol) was added dropwise in a dry ice/acetone bath. The reaction mixture was stirred at this temperature for 30 minutes, and was added a solution of *p*-tolylsulfonyl cyanide (120 mg, 0.66 mmol) in 5 mL of tetrahydrofuran dropwise. The reaction mixture was continuously stirred at this temperature for 30 minutes, and quenched by adding water. Ethyl acetate (30 mL × 2) was added for extraction, and the organic phase was washed with brine, dried over anhydrous sodium sulfate, concentrated by rotary evaporation, and purified by column chromatography (mobile phase: ethyl acetate/petroleum ether = 0/100 to 60/40) to obtain compound **8-b** (61 mg, 74%) as an off-white solid. LC-MS (ESI): m/z = 249.1 (M+H)⁺.

### Synthesis of compound 8-a

**8-b** (61 mg, 0.25 mmol), dichloromethane (10 mL) and trifluoroacetic acid (1 mL) were added to a reaction flask. The reaction mixture was stirred at room temperature overnight under nitrogen atmosphere. The next day, dichloromethane and trifluoroacetic acid were removed by rotary evaporation. 10 mL of dichloromethane and 1 mL of triethylamine were added and the mixture was concentrated by rotary evaporation to obtain a mixture of compound **8-a** (crude) as an oil which was used in the next step without purification. LC-MS (ESI): m/z = 149.0 (M+H)⁺.

### Synthesis of compound 8

**I-1** (20 mg, 0.036 mmol), DMF (2 mL), **8-a** (crude) and DIPEA (0.5 mL) were added to a reaction flask. The reaction mixture was stirred at room temperature under nitrogen atmosphere overnight. The next day, the reaction mixture was directly subjected to pre-HPLC (ammonium bicarbonate) and lyophilized to obtain compound **8** (4 mg, 20%) as a grey solid. LC-MS (ESI): m/z = 555.0 (M+H)⁺; ¹H NMR (400 MHz, CDCl₃) : *δ* 7.80-7.73(1H, m), 7.66-7.59(1H, m), 7.56-7.50(1H, m), 7.48-7.41(1H, m), 7.38-7.31(1H, m), 7.25-7.20(1H, m), 7.08(1H, s), 4.86-4.68(2H, m), 4.49-4.37(2H, m), 4.37-4.09(3H, m), 3.93-3.80(1H, m), 3.78-3.35(3H, m), 3.31-2.96(3H, m), 2.82-2.42(4H, m), 2.22-2.02(1H, m), 2.01-1.71(3H, m), 1.48-1.35(2H, m).

### Example 9: Synthetic route of compound 9

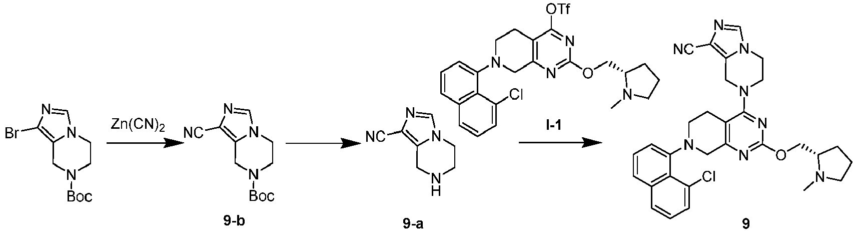

### Synthesis of compound 9-b

*Tert*-butyl 1-bromo-5,6-dihydroimidazo[1,5-*α*]pyrazine-7(8*H*)-carboxylate (200 mg, 0.66 mmol), DMA (15 mL), zinc cyanide (155 mg, 1.33 mmol), XPhos (63 mg, 0.13 mmol), Pd₂(dba)₃ (61 mg, 0.066 mmol) and zinc powder (20 mg) were added to a reaction flask. The mixture was degassed and purged with nitrogen for three times, and stirred at 100 °C for 2 days. Zinc cyanide (155 mg, 1.33 mmol), XPhos (63 mg, 0.13 mmol), Pd₂dba₃ (61 mg, 0.066 mmol) and zinc powder (20 mg) were added, and the mixture was stirred for 36 hours and cooled to room temperature. The reaction was quenched with water and extracted with ethyl acetate (50 mL × 2). The organic phase was washed with brine (50 mL × 3), concentrated by rotary evaporation, purified by column chromatography (mobile phase: ethyl acetate/petroleum ether = 0/100 to 60/40), subjected to preparative HPLC, and lyophilized to obtain compound **9-b** (12 mg, 7%) as a white solid. LC-MS (ESI): m/z = 249.1 (M+H)⁺.

### Synthesis of compound 9-a

**9-b** (12 mg, 0.048 mmol), dichloromethane (8 mL) and trifluoroacetic acid (1.5 mL) were added to a reaction flask. The reaction mixture was stirred at room temperature for 2 hours under nitrogen atmosphere. Dichloromethane and trifluoroacetic acid were removed by rotary evaporation. 10 mL of dichloromethane and 1 mL of triethylamine were added and the mixture was concentrated by rotary evaporation to obtain a mixture of compound **9-a** (crude) as a solid which was used in the next step without purification. LC-MS (ESI): m/z = 149.1 (M+H)⁺.

### Synthesis of compound 9

**I-1** (25 mg, 0.045 mmol), DMF (2 mL), **9-a** (crude) and DIPEA (0.5 mL) were added to a reaction flask. The reaction mixture was stirred at room temperature under nitrogen atmosphere overnight. The next day, the reaction mixture was directly subjected to pre-HPLC (ammonium bicarbonate) and lyophilized to obtain compound **9** (6 mg, 24%) as a pale brown solid. LC-MS (ESI): m/z = 555.0 (M+H)⁺; ¹H NMR (400 MHz, CDCl₃) : *δ* 7.76(1H, d, *J*=7.6Hz), 7.63(1H, d, *J*=8.4Hz), 7.56-7.49(2H, m), 7.45(1H, t, *J*=7.6Hz), 7.34(1H, t, *J*=8Hz), 7.23(1H, d, *J*=7.6Hz), 4.93-4.79(2H, m), 4.59(1H, bs), 4.43(1H, d, *J*=18Hz), 4.39-4.07(4H, m), 3.86(1H, d, *J*=18.8Hz), 3.78-3.65(1H, m), 3.63-3.53(1H, m), 3.43-3.09(3H, m), 2.96(1H, bs), 2.73-2.54(4H, m), 2.53-2.34(1H, m), 2.22-2.08(1H, m), 2.05-1.80(3H, m).

### Example 10: Synthetic route of compound 10

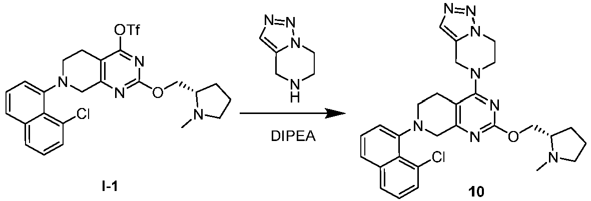

### Synthesis of compound 10

**I-1** (30 mg, 0.054 mmol), 4,5,6,7-tetrahydro-1,2,3-triazolo[1,5-*α*]pyrazine (10 mg, 0.081 mmol), *N, N*-diisopropylethylamine (14 mg, 0.108 mmol) and DMF (3 mL) were added to a reaction flask. The mixture was stirred at room temperature for 1 hour. The reaction mixture was directly purified by pre-HPLC to obtain compound **10** (14 mg, 49%) as a white solid. LC-MS (ESI): m/z 531.1 (M+H)⁺; ¹H NMR (400 MHz, CDCl₃) δ 7.77 (1H, d, *J*=8Hz), 7.64(1H, d, *J*=8Hz), 7.61(1H, s), 7.54(1H, d, *J*=6.8Hz), 7.46(1H, t, *J*=8Hz), 7.36(1H, t, *J*=8Hz), 7.24(1H, d, *J*=7.2Hz), 4.88-4.75 (2H, m), 4.70-4.58 (2H, m), 4.45(2H, d, *J*=18.4Hz), 4.26-4.13 (2H, m), 3.88(1H, d, *J*=17.6Hz), 3.79-3.70 (1H, m), 3.64-3.55 (1H, m), 3.29-3.09 (3H, m), 2.79-2.68(1H, m), 2.66-2.58(1H, m), 2.52(3H, s), 2.38-2.28 (1H, m), 2.14-2.01 (1H, m), 1.92-1.74(3H, m).

### Example 11: Synthetic route of compound 11

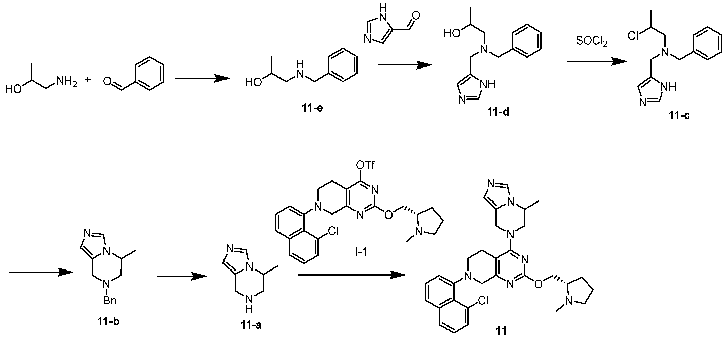

### Synthesis of compound 11-e

Benzaldehyde (1.06 g, 10 mmol), ethanol (30 mL), 1-amino-2-propanol (750 mg, 10 mmol) and sodium cyanoborohydride (1.26 g, 20 mmol) were added to a reaction flask. The reaction mixture was stirred at room temperature under nitrogen atmosphere overnight. The next day, the reaction was quenched with water, extracted with dichloromethane (100 mL × 3), concentrated by rotary evaporation, and the residue was purified by column chromatography (mobile phase: methanol/dichloromethane = 0/100 to 10/90) to obtain compound **11-e** (618 mg, 37%) as a colorless oil. LC-MS (ESI): m/z = 166.2 (M+H)⁺.

### Synthesis of compound 11-d

**11-e** (618 mg, 3.75 mmol), tetrahydrofuran (10 mL), methanol (10 mL), 4-imidazolecarboxaldehyde (431 mg, 4.49 mmol) and a 4A molecular sieve were added to a reaction flask. The reaction mixture was stirred at room temperature overnight under nitrogen atmosphere. The next day, the mixture was added sodium borohydride (171 mg, 4.49 mmol) and stirred for 1 hour, filtered and the filtrate was concentrated by rotary evaporation, and the residue was purified by column chromatography (mobile phase: ammonia in methanol/dichloromethane = 0/100 to 6/94) to obtain compound **11-d** (776 mg, 84%) as a white solid. LC-MS (ESI): m/z = 246.2 (M+H)⁺.

### Synthesis of compound 11-c

**11-d** (776 mg, 3.17 mmol), dichloroethane (50 mL) and thionyl chloride (919 µL, 12.7 mmol) were added to a reaction flask. The reaction mixture was stirred at 60 °C overnight under nitrogen atmosphere. The next day, the mixture was concentrated by rotary evaporation, treated with acetonitrile, and dried *in vacuo* to obtain compound **11-c** (833 mg, 100%) as a white solid. LC-MS (ESI): m/z = 264.0 (M+H)⁺.

### Synthesis of compound 11-b

Triethylamine (1.48 mL, 10.6 mmol) was added dropwise to a stirred solution of **11-c** (700 mg, 2.66 mmol) in acetonitrile (40 mL) in a reaction flask at room temperature. The reaction mixture was then stirred at 80 °C under nitrogen atmosphere overnight. The next day, the reaction mixture was concentrated by rotary evaporation, and the residue was purified by column chromatography (mobile phase: ammonia in methanol/dichloromethane = 0/100 to 5/95) to obtain compound **11-b** (250 mg, 41%) as a pale brown solid. LC-MS (ESI): m/z = 228.1 (M+H)⁺.

### Synthesis of compound 11-a

**11-b** (250 mg, 1.1 mmol), methanol (30 mL), hydrogen chloride in methanol (4 M, 1 mL) and 10% palladium on carbon (117 mg) were added to a reaction flask. The mixture was degassed and purged with hydrogen for three times and stirred at room temperature for 4 hours in hydrogen atmosphere. The mixture was filtered and concentrated by rotary evaporation. 10 mL of dichloromethane and 2 mL of triethylamine were added and the mixture was concentrated by rotary evaporation to obtain compound **11-a** (440 mg, crude) as a semi-solid which was directly used in the next step without purification. LC-MS (ESI): m/z = 138.1 (M+H)⁺.

### Synthesis of compound 11

**I-1** (25 mg, 0.045 mmol), DMF (2 mL), **11-a** (100 mg, crude) and DIPEA (0.5 mL) were added to a reaction flask. The reaction mixture was stirred at room temperature under nitrogen atmosphere overnight. The next day, the reaction mixture was directly subjected to pre-HPLC (ammonium bicarbonate) and lyophilized to obtain compound **11** (24 mg, 98%) as a white solid. LC-MS (ESI): m/z = 544.0 (M+H)⁺; ¹H NMR (400 MHz, CDCl₃) : *δ* 7.76(1H, d, *J*=8Hz), 7.66-7.56(2H, m), 7.56-7.50(1H, m), 7.49-7.39(1H, m), 7.34(1H, t, *J*=7.6Hz), 7.26-7.15(1H, m), 6.89(1H, s), 4.99-4.68(2H, m), 4.67-4.32(4H, m), 4.24-4.03(1H, m), 4.03-3.38(4H, m), 3.35-3.01(3H, m), 2.87(3H, s), 2.77-2.51(2H, m), 2.34-1.89(4H, m), 1.61(3H, t, *J*=6.4Hz).

### Example 12: Synthetic route of compound 12

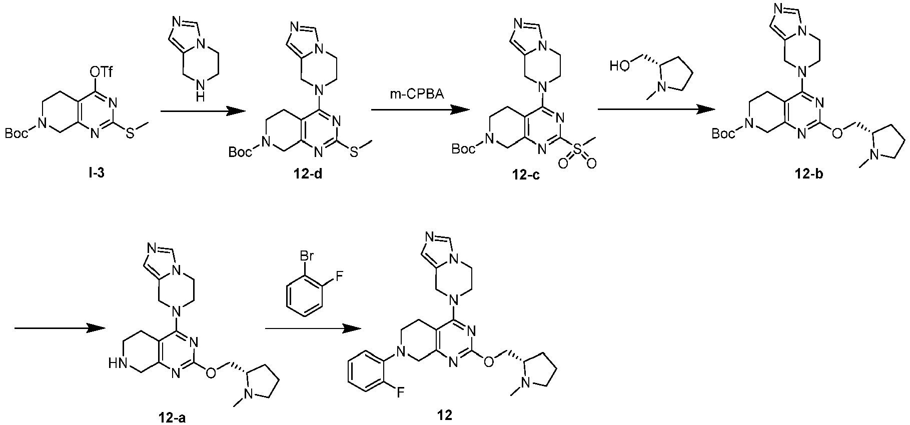

### Synthesis of compound 12-d

**I-3** (9 g, 20.98 mmol), DMF (100 mL), 5,6,7,8-tetrahydroimidazo[1,5-*α*]pyrazine (3.1 g, 25.17 mmol) and DIPEA (17.3 mL, 104.9 mmol) were added to a reaction flask. The reaction mixture was stirred at room temperature under nitrogen atmosphere for 1 hour. The reaction was quenched with saturated sodium bicarbonate solution, and the mixture was extracted with ethyl acetate (100 mL × 4), washed with brine (100 mL × 3), concentrated by rotary evaporation, and purified by column chromatography (mobile phase: methanol/dichloromethane = 0/100 to 7/93) to obtain compound **12-d** (8 g, 95%) as a white solid. LC-MS (ESI): m/z = 403.3 (M+H)⁺.

### Synthesis of compound 12-c

**12-d** (8 g, 19.9 mmol), ethyl acetate (150 mL) and *m*-chloroperoxybenzoic acid (10.07 g, 49.8 mmol) were added to a reaction flask. The reaction mixture was stirred at room temperature under nitrogen atmosphere for 2 hours. The reaction was quenched with saturated sodium sulfite solution before saturated sodium bicarbonate solution was added. The mixture was extracted with ethyl acetate (150 mL × 4), dried over anhydrous sodium sulfate, filtered, and the filtrate was concentrated by rotary evaporation to obtain compound **12-c** (9.8 g, 96%) as a pale brown solid which was directly used in the next step without purification. LC-MS (ESI): m/z = 435.0 (M+H)⁺.

### Synthesis of compound 12-b

**12-c** (9.8 g, 22.6 mmol), toluene (200 mL) and N-methyl-L-prolinol (4.7 mL, 39.5 mmol) were added to a reaction flask. In an ice-cold water bath, sodium *tert*-butoxide (4.3 g, 45.2 mmol) was slowly added, and the reaction mixture was stirred at this temperature for 30 minutes. The reaction was quenched with water, and the mixture was extracted with ethyl acetate (200 mL × 4), dried, concentrated by rotary evaporation, and purified by column chromatography (mobile phase: ammonia in methanol/dichloromethane = 0/100 to 5/95) to obtain compound **12-b** (6.3 g, 59%) as a white solid. LC-MS (ESI): m/z = 470.4 (M+H)⁺.

### Synthesis of compound 12-a

**12-b** (6.2 g, 13.2 mmol), dichloromethane (100 mL) and 4 M hydrogen chloride in methanol (40 mL) were added to a reaction flask. The reaction mixture was stirred at room temperature under nitrogen atmosphere overnight. The next day, the mixture was concentrated by rotary evaporation, and was added a mixed solvent of dichloromethane/methanol (10/1, 400 mL) and excess amounts of solid sodium bicarbonate and anhydrous sodium sulfate. The mixture was stirred for 10 minutes, filtered, and the filtrate was concentrated by rotary evaporation to obtain compound **12-a** (5.1 g, 104%) as a light brown solid which was directly used in the next step without purification. LC-MS (ESI): m/z = 370.2 (M+H)⁺.

### Synthesis of compound 12

**12-a** (200 mg, 0.54 mmol), toluene (30 mL), *o*-bromofluorobenzene (123 mg, 0.71 mmol), cesium carbonate (883 mg, 2.71 mmol), RuPhos (51 mg, 0.11 mmol) and Pd₂dba₃ (50 mg, 0.054 mmol) were added to a reaction flask. The reaction mixture was degassed and purged with nitrogen for three times and stirred at 100 °C overnight. The next day, the mixture was concentrated by rotary evaporation, purified by column chromatography (mobile phase: ammonia in methanol/dichloromethane = 0/100 to 10/90), subjected to pre-HPLC (ammonium bicarbonate) and lyophilized to obtain compound **12** (9 mg, 4%) as a white solid. LC-MS (ESI): m/z = 464.3 (M+H)⁺; ¹H NMR (400 MHz, CDCl₃) : *δ* 7.49(1H, s), 7.14-7.04(2H,m), 7.03-6.94(2H, m), 6.88(1H, s), 4.89-4.61(3H, m), 4.46-4.33(1H, m), 4.28-4.15(4H, m), 3.97-3.82(2H, m), 3.74-3.46(1H, m), 3.44-3.30(2H, m), 3.28-3.06(1H, m), 2.96-2.55(6H, m), 2.35-1.87(4H, m).

### Example 13: Synthetic route of compound 13

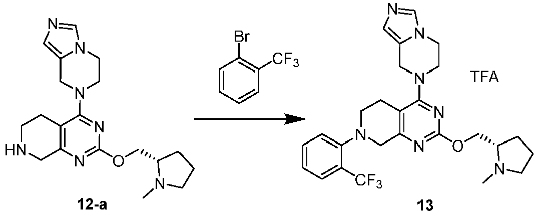

### Synthesis of compound 13

**12-a** (200 mg, 0.54 mmol), toluene (30 mL), o-bromo-trifluoromethylbenzene (158 mg, 0.71 mmol), cesium carbonate (883 mg, 2.71 mmol), RuPhos (51 mg, 0.11 mmol) and Pd₂dba₃ (50 mg, 0.054 mmol) were added to a reaction flask. The mixture was degassed and purged with nitrogen for three times and stirred at 100 °C overnight. The next day, the mixture was concentrated by rotary evaporation, and the residue was purified by column chromatography (mobile phase: ammonia in methanol/dichloromethane = 0/100 to 10/90), subjected to pre-HPLC (trifluoroacetic acid) and lyophilized to obtain compound **13** (7 mg, 2%) as a solid. LC-MS (ESI): m/z = 514.2 (M+H)⁺.

### Example 14: Synthetic route of compound 14

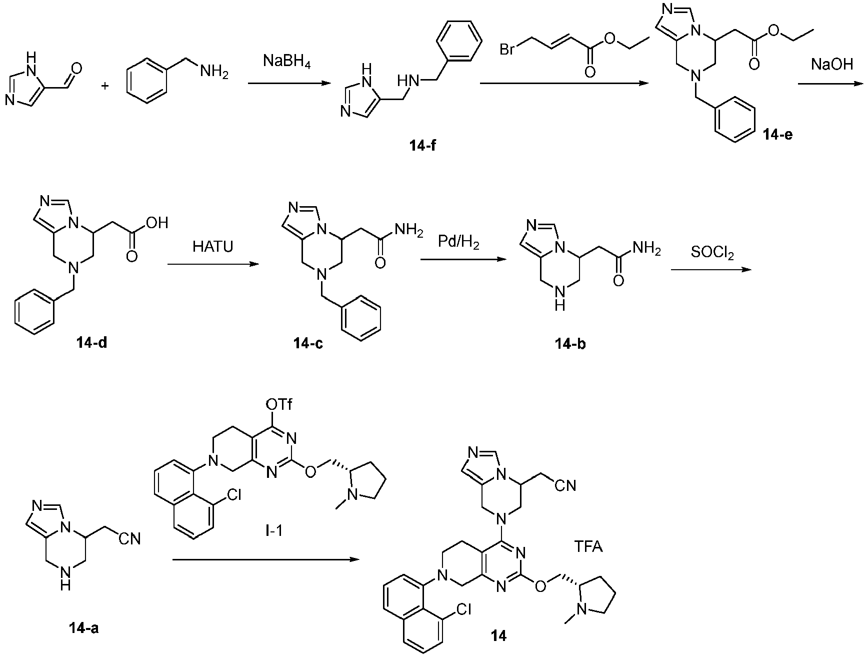

### Synthesis of compound 14-f

4-Imidazolecarboxaldehyde (961 mg, 10 mmol) was dissolved in 10 mL of methanol at room temperature, and was added THF (10 mL), benzylamine (1.07 g, 10 mmol) and a 4A molecular sieve (2 g) serially. The reaction mixture was stirred at room temperature under nitrogen atmosphere for 16 hours. The reaction mixture was cooled to 0 °C and was added sodium borohydride (456 mg, 12 mmol). The reaction mixture was warmed to room temperature and stirred for 1 hour. 5 mL of water was added to the reaction mixture, and the mixture was stirred for 10 minutes. The reaction mixture was filtered and the filtrate was concentrated at reduced pressure. The crude product was dissolved in 80 mL of ethyl acetate and washed with sodium hydroxide (2 M, 50 mL) solution. The organic phase was dried over anhydrous sodium sulfate, filtered, and concentrated at reduced pressure to obtain compound **14-f** (1.5 g, 80%) as a white solid. LC-MS (ESI): m/z = 188.2 [M+1]⁺.

### Synthesis of compound 14-e

Compound **14-f** (200 mg, 1.07 mmol) was dissolved in 20 mL of DCM at room temperature, DIPEA (530 µL, 3.21 mmol) and ethyl 4-bromocrotonate (206 mg, 1.07 mmol) were serially added. The reaction mixture was stirred at room temperature under nitrogen atmosphere for 16 hours. The reaction mixture was concentrated at reduced pressure and the crude product was purified by a flash column chromatography (DCM/MeOH = 10:1) to obtain compound **14-e** (280 mg, 87%) as a yellow oil. LC-MS (ESI): m/z = 300.2 [M+1]⁺.

### Synthesis of compound 14-d

Compound **14-e** (280 mg, 0.94 mmol) was dissolved in 10 mL of ethanol, and was added THF (10 mL), water (5 mL) and sodium hydroxide (187 mg, 4.68 mmol) serially. The reaction mixture was stirred at room temperature for 16 hours. The mixture was concentrated at reduced pressure, and the residue was dissolved in 10 mL of water. The solution was adjusted to pH 5-6 with 1 M hydrochloric acid and concentrated at reduced pressure. 10 mL of THF was added, and the mixture was concentrated at reduced pressure. The above procedures were repeated 3 times before a mixed solvent (DCM/MeOH = 10:1, 20 mL) was added. The mixture was filtered, and the filtrate was concentrated at reduced pressure to obtain a crude product of compound **14-d** (260 mg, 100%) as a white solid. LC-MS (ESI): m/z = 272.1 [M+1]⁺.

### Synthesis of compound 14-c

Compound **14-d** (262 mg, 0.97 mmol) was dissolved in 5 mL of DMF at room temperature. In an ice-cold water bath, HATU (551 mg, 1.45 mmol) and DIPEA (797 µL, 4.83 mmol) were added serially to the above mixture, and the reaction mixture was stirred at 0 °C for 30 minutes, then was added ammonium chloride (258 mg, 4.83 mmol), and the resulting mixture was warmed to room temperature and stirred for 16 hours. The reaction was quenched with 50 mL of water, and extracted with ethyl acetate (100 mL × 2). The organic phase was washed with brine (100 mL × 5), dried over anhydrous sodium sulfate, filtered and concentrated at reduced pressure. The crude product was purified by a flash column chromatography (DCM/MeOH (NH₃) =10:1) to obtain compound **14-c** (110 mg, 42%) as a yellow solid. LC-MS (ESI): m/z = 271.2 [M+1]⁺.

### Synthesis of compound 14-b

Compound **14-c** (110 mg, 0.41 mmol) was dissolved in 50 mL of methanol at room temperature. Palladium on carbon (50 mg, 10% Pd, 50% wet) and a solution of hydrogen chloride in methanol (4 M, 3 mL) were added under nitrogen atmosphere. The reaction mixture was stirred under hydrogen atmosphere at room temperature for 16 hours. The mixture was filtered and the filtrate was concentrated at reduced pressure to obtain a crude product of compound **14-b** (100 mg) as a yellow solid, which was used directly in the next step. LC-MS (ESI): m/z = 181.2 [M+1]⁺.

### Synthesis of compound 14-a

Compound **14-b** (100 mg, 0.46 mmol) was dissolved in 2 mL of pyridine at room temperature. The mixture was cooled to 0 °C, was added thionyl chloride (167 µL, 2.3 mmol) under nitrogen atmosphere. The reaction mixture was stirred at 0 °C for 30 minutes before quenched with 10 mL of water. The reaction mixture was lyophilized to obtain a crude product of compound **14-a** (200 mg) as a brown solid containing pyridine hydrochloride, which was directly used in the next step. LC-MS (ESI): m/z = 163.1 [M+1]⁺.

### Synthesis of compound 14

The crude product of compound **14-a** (about 200 mg) was dissolved in 5 mL of DMF at room temperature before DIPEA (297 µL, 1.79 mmol) and **I-1** (50 mg, 0.09 mmol) were added. The reaction mixture was stirred at room temperature under nitrogen atmosphere for 16 hours. The reaction was quenched with water (20 mL). Ethyl acetate (50 mL × 2) was added for extraction. The organic phase was washed with brine (50 mL × 3), dried over anhydrous sodium sulfate and filtered. The filtrate was concentrated at reduced pressure, and the crude product was purified by pre-HPLC (acidic, TFA) to obtain compound **14** (5.2 mg, 8.5%) as a white solid. LC-MS (ESI): m/z = 569.3 [M+1]⁺.

### Example 15: Synthetic route of compound 15

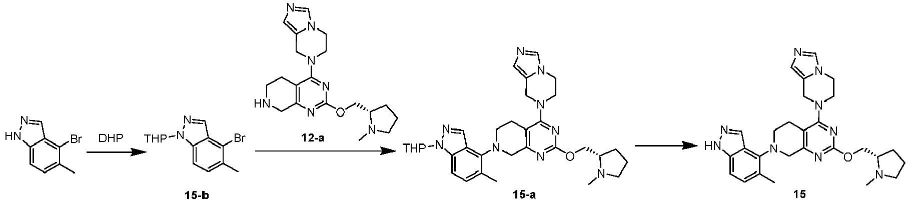

### Synthesis of compound 15-b

4-Bromo-5-methyl-1*H*-indazole (1 g, 4.76 mmol), dichloromethane (30 mL), 3,4-dihydro-2*H*-pyran (800 mg, 9.52 mmol) and *p*-toluenesulfonic acid (90 mL, 0.48 mmol) were added to a reaction flask. The mixture was stirred at room temperature under nitrogen atmosphere for 1.5 hours, concentrated by rotary evaporation and purified by column chromatography (mobile phase: ethyl acetate/petroleum ether = 0/100 to 10/90) to obtain compound **15-b** (1.4 g, 100%) as a white solid. LC-MS (ESI): m/z = 295.0 (M+H)⁺.

### Synthesis of compound 15-a

**12-a** (150 mg, 0.41 mmol), toluene (20 mL), **15-b** (239 mg, 0.81 mmol), cesium carbonate (662 mg, 2.03 mmol), RuPhos (38 mg, 0.081 mmol) and Pd₂dba₃ (37 mg, 0.041 mmol) were added to a reaction flask. The reaction mixture was degassed and purged with nitrogen for three times and stirred at 100 °C overnight. The next day, the mixture was concentrated by rotary evaporation and purified by column chromatography (mobile phase: ammonia in methanol/dichloromethane = 0/100 to 6/94) to obtain compound **15-a** (96 mg, 40%) as a pale brown solid. LC-MS (ESI): m/z = 584.3 (M+H)⁺.

### Synthesis of compound 15

**15-a** (96 mg, 0.17 mmol), dichloromethane (10 mL) and trifluoroacetic acid (1 mL) were added to a reaction flask. The reaction mixture was stirred at room temperature under nitrogen atmosphere for 1 hour, concentrated by rotary evaporation, subjected to pre-HPLC (ammonium bicarbonate), and lyophilized to obtain compound **15** (9 mg, 10%) as a white solid. LC-MS (ESI): m/z = 500.2 (M+H)⁺; ¹H NMR (400 MHz, CDCl₃) : *δ* 10.42(1H, bs), 8.05(1H,s), 7.60-7.48(1H, m), 7.27-7.19(2H, m), 6.96-6.84(1H, m), 5.06-4.88(1H, m), 4.84-4.69(2H, m), 4.59-4.44(1H, m), 4.37-4.16(4H, m), 4.03-3.75(3H, m), 3.60-3.32(3H, m), 2.98(3H, s), 2.88-2.77(2H, m), 2.42(3H, s), 2.37-1.99(5H, m).

### Example 16: Synthetic route of compound 16

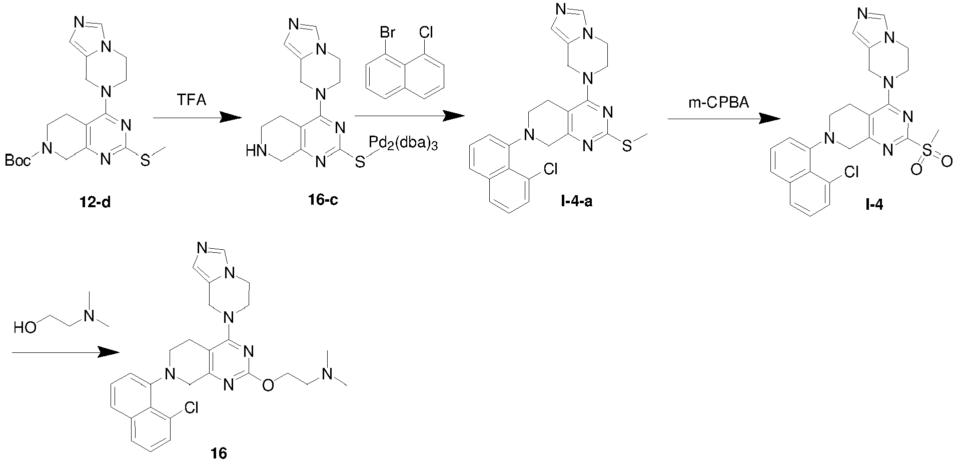

### Synthesis of compound 16-c

To a solution of **12-d** (1.75 g, 4.35 mmol) in dichloromethane (20 mL) was added trifluoroacetic acid (5 mL). The resulting reaction mixture was stirred at room temperature for 4 hours. Upon completion, the reaction mixture was concentrated, carefully neutralized with a saturated solution of sodium bicarbonate to pH > 7 in an ice-cold water bath, and extracted with ethyl acetate. The organic phases were combined, washed with brine, dried over anhydrous sodium sulfate, filtered, and concentrated to obtain the product **16-c** (1.00 g, 76%) as a brown oil. LC-MS (ESI): m/z 303.0 (M+H) ⁺.

### Synthesis of compound I-4-a

**16-c** (1.00 g, 3.97 mmol), 1-bromo-8-chloronaphthalene (1.10 g, 4.63 mmol), 2-dicyclohexylphosphino-2',6'-diisopropoxy-1,1'-biphenyl (309 mg, 0.66 mmol), cesium carbonate (3.23 g, 9.92 mmol), Pd₂(dba)₃ (302 mg, 0.33 mmol) and toluene (15 mL) were added to a reaction flask. The reaction mixture was degassed and purged with nitrogen and reacted at 100 °C overnight. Upon completion, the mixture was concentrated to obtain a crude product. The crude product was purified by a flash column chromatography (mobile phase: methanol/dichloromethane = 0% to 10%) and purified by pre-HPLC to obtain compound **I-4-a** (100 mg, 6.5%) as a white solid. LC-MS (ESI): m/z 463.2 (M+H)⁺; ¹H NMR (400MHz, CDCl₃): *δ* 7.75 (1H, d, *J* = 7.6Hz), 7.67 (1H, s), 7.62 (1H, d, *J* = 8.0Hz), 7.52 (1H, d, *J* = 7.2Hz), 7.44 (1H, t, *J* = 7.6Hz), 7.34 (1H, t, *J* = 7.6Hz), 7.22 (1H, d, *J* = 6.8Hz), 6.93 (1H, s), 4.69-4.78 (2H, m), 4.44 (1H, d, *J* = 17.2Hz), 4.30-4.37 (1H, m), 4.18-4.24 (1H, m), 4.04-4.10 (1H, m), 3.86 (1H, d, *J =* 17.6Hz), 3.69-3.76 (1H, m), 3.54-3.59 (1H, m), 3.11-3.25 (2H, m), 2.58-2.63 (1H, m), 2.52 (3H, s).

### Synthesis of compound I-4

Compound **I-4-a** (40 mg, 0.09 mmol) was dissolved in dichloromethane (10 mL) in an ice-cold water bath. *m*-Chloroperoxybenzoic acid (35 mg, 0.17 mmol) was added and the mixture was slowly warmed to room temperature and stirred for 2 hours. Upon completion, saturated aqueous sodium bicarbonate was added for neutralization. The organic phase was separated, and the aqueous phase was extracted with dichloromethane. The organic phases were combined, dried over anhydrous Na₂SO₄, filtered and evaporated. The crude product was purified by a flash column chromatography (mobile phase: dichloromethane/methanol = 0 to 10/1) to obtain **I-4** (15 mg, 35%) as an oil. LC-MS (ESI): m/z 495.1 (M+H) ⁺.

### Synthesis of compound 16

To a solution of **I-4** (15 mg, 0.03 mmol) and *N, N*-dimethylethanolamine (5 mg, 0.06 mmol) in toluene (10 mL) in an ice-cold water bath was added sodium *tert*-butoxide (6 mg, 0.06 mmol). After the addition, the reaction mixture was stirred in an ice-cold water bath for 10 minutes. Upon completion, the mixture was concentrated at reduced pressure, diluted with ethyl acetate, and serially washed with water and brine, dried over anhydrous Na₂SO₄, filtered and concentrated by rotary evaporation to obtain a brown oil. The crude product was purified by pre-HPLC to obtain compound **16** (5 mg, 33%) as a white solid. LC-MS (ESI): m/z 504.2 (M+H)⁺.

### Example 17: Synthetic route of compound 17

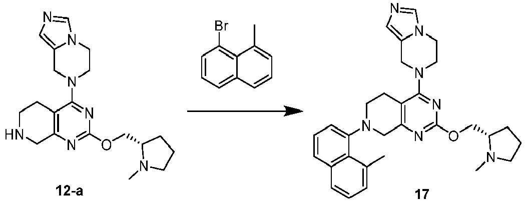

### Synthesis of compound 17

**12-a** (100 mg, 0.27 mmol), toluene (20 mL), 1-bromo-8-methylnaphthalene (119 mg, 0.54 mmol), cesium carbonate (441 mg, 1.36 mmol), RuPhos (25 mg, 0.054 mmol) and Pd₂dba₃ (25 mg, 0.027 mmol) were added to a reaction flask. The reaction mixture was degassed and purged with nitrogen for three times and stirred at 100 °C overnight. The next day, the mixture was concentrated by rotary evaporation. Toluene (20 mL), 1-bromo-8-methylnaphthalene (119 mg, 0.54 mmol), cesium carbonate (441 mg, 1.36 mmol), RuPhos (25 mg, 0.054 mmol) and Pd₂dba₃ (25 mg, 0.027 mmol) were added again. The reaction mixture was degassed and purged with nitrogen for three times and stirred at 100 °C overnight. The next day, the reaction mixture was concentrated by rotary evaporation, purified by column chromatography (mobile phase: ammonia in methanol/dichloromethane = 0/100 to 10/90), subjected to pre-HPLC (ammonium bicarbonate) and lyophilized to obtain compound **17** (7 mg, 5%) as a white solid. LC-MS (ESI): m/z = 510.3 (M+H)⁺; ¹H NMR (400 MHz, CDCl₃) : *δ* 7.70(1H, d, *J*=8.4Hz), 7.65(1H, d, *J*=8.4Hz), 7.50(1H, s), 7.43-7.31(2H, m), 7.26-7.18(2H, m), 6.89(1H, s), 4.84-4.65(2H, m), 4.53(1H, bs), 4.37-4.06(5H, m), 3.87-3.60(2H, m), 3.56-3.43(1H, m), 3.34-3.14(2H, m), 3.13-3.00(1H, m), 2.92(3H, s), 2.70-2.50(4H, m), 2.49-2.31(1H, m), 2.19-2.05(1H, m), 2.01-1.77(4H, m).

### Example 18: Synthetic route of compound 18

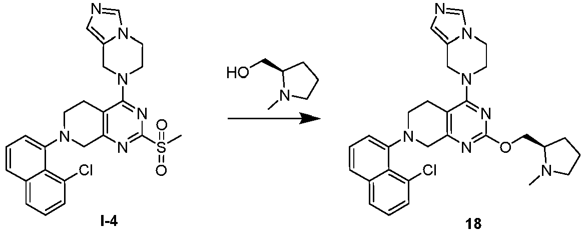

### Synthesis of compound 18

To a solution of **I-4** (25 mg, 0.05 mmol) and *N*-methyl-D-prolinol (12 mg, 0.10 mmol) in toluene (10 mL) in an ice-cold water bath was added sodium *tert*-butoxide (10 mg, 0.10 mmol). After the addition, the reaction mixture was stirred in an ice-cold water bath for 10 minutes. Upon completion, the mixture was concentrated at reduced pressure, diluted with ethyl acetate, and serially washed with water and brine, dried over anhydrous Na₂SO₄, filtered and concentrated by rotary evaporation to obtain a brown oil. The crude product was purified by pre-HPLC to obtain compound **18** (15 mg, 56%) as a white solid. LC-MS (ESI): m/z 530.2 (M+H) ⁺; ¹H NMR (400MHz, CDCl₃): *δ* 7.75 (1H, d, *J* = 8.0Hz), 7.61 (1H, d, *J* = 8.0Hz), 7.52 (1H, d, *J* = 7.6Hz), 7.49 (1H, s), 7.44 (1H, d, *J* = 8.0Hz), 7.33 (1H, t, *J* = 8.0Hz), 7.22 (1H, t, *J* = 7.6Hz), 6.88 (1H, s), 4.69-4.80 (2H, m), 4.41-4.50 (1H, m), 4.42 (1H, d, *J* = 10.0Hz), 4.25-4.35 (1H, m), 4.12-4.24 (2H, m), 4.02-4.12 (1H, m), 3.81-3.91 (1H, m), 3.67-3.77 (1H, m), 3.51-3.59 (1H, m), 3.10-3.24 (3H, m), 2.72-2.85 (1H, m), 2.55-2.65 (1H, m), 2.52 (3H, s), 2.30-2.41 (1H, m), 2.02-2.15 (1H, m), 1.73-1.96 (3H, m).

### Example 19: Synthetic route of compound 19

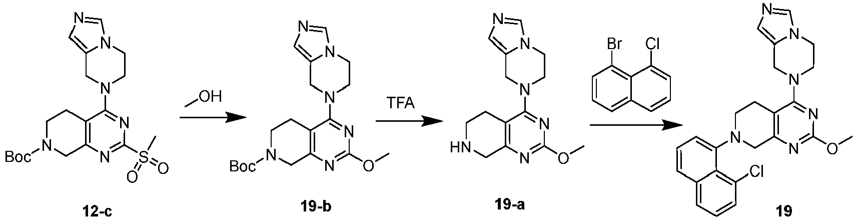

### Synthesis of compound 19-b

To a solution of **12-c** (400 mg, 0.92 mmol) and methanol (59 mg, 1.84 mmol) in toluene (10 mL) in an ice-cold water bath was added sodium tert-butoxide (177 mg, 1.84 mmol). After the addition, the reaction mixture was stirred in an ice-cold water bath for 10 minutes. Upon completion, the mixture was concentrated at reduced pressure, diluted with ethyl acetate, and serially washed with water and brine, dried over anhydrous Na₂SO₄, filtered and concentrated by rotary evaporation to obtain a crude product. The crude product was purified by a flash column chromatography (mobile phase: dichloromethane/methanol = 1/0 to 10/1) to obtain compound **19-b** (300 mg, 84%) as a brown oil. LC-MS (ESI): m/z 387.2 (M+H) ⁺.

### Synthesis of compound 19-a

To a solution of **19-b** (300 mg, 0.78 mmol) in dichloromethane (10 mL) was added trifluoroacetic acid (2 mL). The resulting reaction mixture was stirred at room temperature for 4 hours. Upon completion, the reaction mixture was concentrated, carefully neutralized with a saturated solution of sodium bicarbonate to pH > 7 in an ice-cold water bath, and extracted with ethyl acetate. The organic phases were combined, washed with brine, dried over anhydrous sodium sulfate, filtered, and concentrated to obtain the product **19-a** (150 mg, 67%) as a brown oil. LC-MS (ESI): m/z 287.1 (M+H) ⁺.

### Synthesis of compound 19

**19-a** (100 mg, 0.35 mmol), 1-bromo-8-chloronaphthalene (118 mg, 0.49 mmol), 2-dicyclohexylphosphino-2',6'-diisopropoxy-1,1'-biphenyl (33 mg, 0.07 mmol), cesium carbonate (341 mg, 1.05 mmol), Pd₂(dba)₃ (32 mg, 0.04 mmol) and toluene (10 mL) were added to a reaction flask. The reaction mixture was degassed and purged with nitrogen and reacted at 100 °C overnight. Upon completion, the mixture was concentrated to obtain a crude product. The crude product was purified by a flash column chromatography (mobile phase: methanol/dichloromethane = 0% to 10%) and purified by pre-HPLC to obtain compound **19** (30 mg, 19%) as a white solid. LC-MS (ESI): m/z 447.2 (M+H) ⁺; ¹H NMR (400MHz, CDCl₃): *δ* 7.75 (1H, d, *J* = 8.0Hz), 7.62 (1H, d, *J* = 7.2Hz), 7.54 (1H, s), 7.53 (1H, d, *J* = 7.6Hz), 7.44 (1H, t, *J* = 7.6Hz), 7.34 (1H, t, *J* = 7.6Hz), 7.23 (1H, d, *J* = 7.6Hz), 6.90 (1H, s), 4.70-4.81 (2H, m), 4.44 (1H, d, *J* = 18Hz), 4.29-4.36 (1H, m), 4.16-4.22 (1H, m), 4.06-4.12 (1H, m), 3.94 (3H, s), 3.86 (1H, d, *J* = 18Hz), 3.68-3.75 (1H, m), 3.55-3.59 (1H, m), 3.12-3.24 (2H, m), 2.56-2.63 (1H, m).

### Example 20: Synthetic route of compound 20

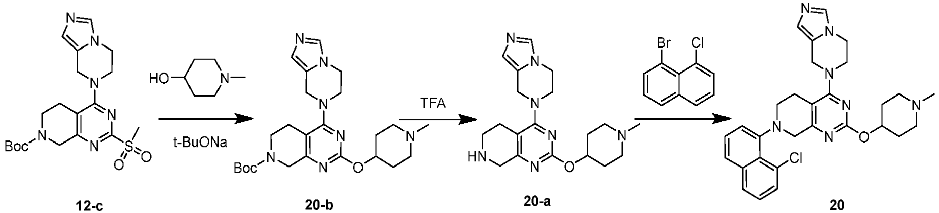

### Synthesis of compound 20-b

To a solution of **12-c** (400 mg, 0.92 mmol) and 1-methyl-4-piperidinol (212 mg, 1.84 mmol) in toluene (10 mL) in an ice-cold water bath was added sodium *tert*-butoxide (177 mg, 1.84 mmol). After the addition, the reaction mixture was stirred in an ice-cold water bath for 10 minutes. Upon completion, the mixture was concentrated at reduced pressure, diluted with ethyl acetate, and serially washed with water and brine, dried over anhydrous Na₂SO₄, filtered and concentrated by rotary evaporation. The crude product was purified by a flash column chromatography (mobile phase: dichloromethane/methanol = 1/0 to 10/1) to obtain **20-b** (0.2 g, 46%) as an oil. LC-MS (ESI): m/z 470.3 (M+H) ⁺.

### Synthesis of compound 20-a

To a solution of **20-b** (200 mg, 0.43 mmol) in dichloromethane (10 mL) was added trifluoroacetic acid (2 mL). The resulting reaction mixture was stirred at room temperature for 4 hours. Upon completion, the reaction mixture was concentrated, carefully neutralized with a saturated solution of sodium bicarbonate to pH > 7 in an ice-cold water bath, and extracted with ethyl acetate. The organic phases were combined, washed with brine, dried over anhydrous sodium sulfate, filtered, and concentrated to obtain the product **20-a** (120 mg, 76%) as a brown oil. LC-MS (ESI): m/z 370.1 (M+H)+.

### Synthesis of compound 20

**20-a** (120 mg, 0.41 mmol), 1-bromo-8-chloronaphthalene (137 mg, 0.57 mmol), 2-dicyclohexylphosphino-2',6'-diisopropoxy-1,1'-biphenyl (38 mg, 0.08 mmol), cesium carbonate (397 mg, 1.22 mmol), Pd₂(dba)₃ (37 mg, 0.04 mmol) and toluene (10 mL) were added to a reaction flask. The reaction mixture was degassed and purged with nitrogen and reacted at 100 °C overnight. Upon completion, the mixture was concentrated to obtain a crude product. The crude product was purified by a flash column chromatography (mobile phase: methanol/dichloromethane = 0% to 10%) and purified by pre-HPLC to obtain compound **20** (10 mg, 5%) as a white solid. LC-MS (ESI): m/z 530.3 (M+H) ⁺; ¹H NMR (400MHz, CDCl₃): *δ* 7.76 (1H, d, *J* = 7.2Hz), 7.63 (1H, d, *J* = 7.6Hz), 7.53 (1H, d, *J* = 7.6Hz), 7.51 (1H, s), 7.45 (1H, t, *J* = 8.0Hz), 7.35 (1H, t, *J* = 8.0Hz), 7.22 (1H, d, *J* = 7.6Hz), 6.91 (1H, s), 5.17-5.33 (1H, m), 4.69-4.81 (2H, m), 4.42 (1H, d, *J* = 18Hz), 4.26-4.32 (1H, m), 4.15-4.21 (1H, m), 4.05-4.11 (1H, m), 3.84 (1H, d, *J* = 18.4Hz), 3.70-3.77 (1H, m), 3.57-3.60 (1H, m), 2.98-3.29 (5H, m), 2.70 (3H, s), 2.53-2.64 (2H, m), 2.28-2.48 (2H, m), 2.09-2.23 (2H, m).

### Example 21: Synthetic route of compound 21

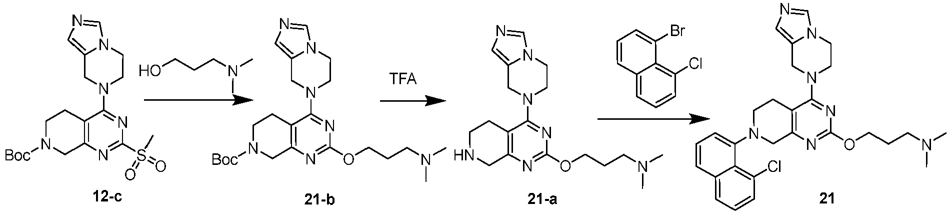

### Synthesis of compound 21-b

**12-c** (600 mg, 1.24 mmol), 3-dimethylamino-1-propanol (256 mg, 2.48 mmol), toluene (10 mL) and THF (2 mL) were added to a reaction flask. The mixture was degassed and purged with nitrogen and cooled to 0 °C, then was added sodium *tert*-butoxide (238 mg, 2.48 mmol). The reaction was stirred at room temperature for 6 hours. The reaction mixture was purified by column chromatography (mobile phase: DCM/MeOH = 10/0 to 10/2) to obtain compound **21-b** (360 mg, 50%). LC-MS (ESI): m/z 458.3 (M+H)⁺.

### Synthesis of compound 21-a

**21-b** (360 mg, 0.78 mmol), DCM (10 mL), and trifluoroacetic acid (3 mL) were added to a reaction flask. The reaction was stirred at room temperature for 5 hours. The reaction mixture was concentrated, adjusted to a basic pH by adding aqueous sodium bicarbonate and extracted with DCM/MeOH (10/2). The organic phases were combined, washed with brine, dried over anhydrous Na₂SO₄, filtered, evaporated and purified by column chromatography (mobile phase: DCM/MeOH = 10/0 to 10/2) to obtain compound **21-a** (200 mg, 64%). LC-MS (ESI): m/z 358.3 (M+H) ⁺.

### Synthesis of compound 21

**21-a** (100 mg, 0.28 mmol), 1-chloro-8-bromonaphthalene (94 mg, 0.39 mmol), toluene (10 mL), cesium carbonate (273 mg, 0.84 mmol), Pd₂(dba)₃ (26 mg, 0.03 mmol) and RuPhos (26 mg, 0.06 mmol) were added to a reaction flask. The reaction mixture was degassed and purged with nitrogen and reacted at 100 °C overnight. The reaction mixture was concentrated. The residue was dissolved in DMF. The mixture was filtered and purified by pre-HPLC to obtain compound **21** (11 mg, 8%) as a yellow solid. LC-MS (ESI): m/z 518.0 (M+H) ⁺; ¹H NMR (400MHz, DMSO): *δ* 7.92 (1H, d, *J* = 7.6Hz), 7.74 (1H, d, *J* = 7.6Hz), 7.16 (1H, s), 7.58 (1H, d, *J* = 6.0Hz), 7.53 (1H, t, *J* = 8.0Hz), 7.44 (1H, t, *J* = 8.0Hz), 7.34 (1H, d, *J* = 7.6Hz), 6.78 (1H, s), 4.72 (2H, q, *J* = 15.6Hz), 4.24-4.15 (5H, m), 4.03-3.99 (1H, q),3.76 (1H, d), 3.69-3.63 (1H, m), 3.50 (1H, d), 3.21-3.07 (2H, m) , 2.60 (1H, d, *J* = 14.8Hz), 2.32 (2H, t, *J* = 7.2Hz), 2.13 (6H, s), 1.80 (2H, m, *J* = 6.8Hz).

### Example 22: Synthetic route of compound 22

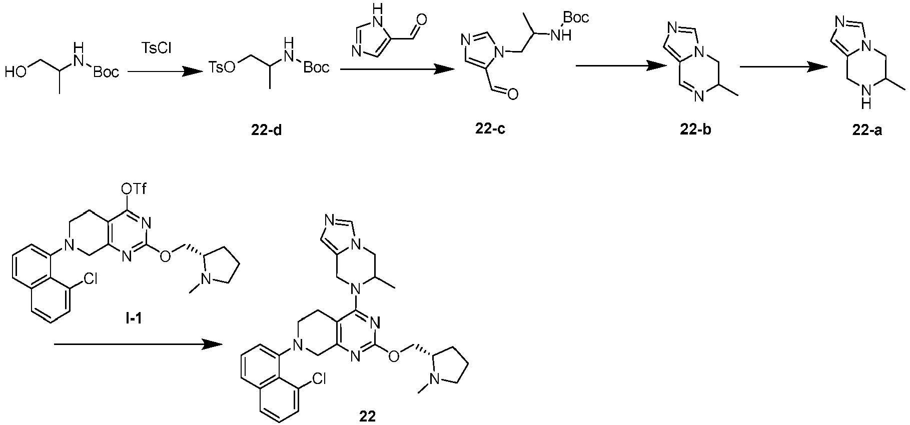

### Synthesis of compound 22-d

N-Boc-D/L-alaninol (1 g, 5.71 mmol), dichloromethane (20 mL) and pyridine (918 µL, 11.43 mmol) were added to a reaction flask. *p*-Toluenesulfonyl chloride (1.19 g, 6.29 mmol) was added in portions in an ice-cold water bath. The reaction mixture was stirred at room temperature overnight under nitrogen atmosphere. The next day, the mixture was concentrated by rotary evaporation and purified by column chromatography (mobile phase: ethyl acetate/petroleum ether = 0/100 to 30/70) to obtain compound **22-d** (524 mg, 28%) as a colorless oil. LC-MS (ESI): m/z = 352.2 (M+Na)⁺.

### Synthesis of compound 22-c

**22-d** (524 mg, 1.59 mmol), DMF (10 mL), 4-imidazolecarboxaldehyde (153 mg, 1.59 mmol) and potassium carbonate (439 mg, 3.18 mmol) were added to a reaction flask. The reaction mixture was stirred at 50 °C under nitrogen atmosphere overnight. The next day, the reaction was quenched with water. Ethyl acetate (30 mL × 4) was added for extraction. The organic phase was concentrated by rotary evaporation and purified by column chromatography (mobile phase: methanol/dichloromethane = 0/100 to 10/90) to obtain compound **22-c** (68 mg, 17%) as a white solid. LC-MS (ESI): m/z = 254.2 (M+H)⁺; ¹H NMR (400 MHz, CDCl₃) : *δ* 9.75(1H, s), 7.80(1H, s), 7.66(1H, s), 4.58(1H, d, *J*=8.4Hz), 4.52-4.43(1H, m), 4.37-422(1H, m), 4.06-3.92(1H, m), 1.36(9H, s), 1.20(3H, d, *J*=6.8Hz).

### Synthesis of compound 22-b

**22-c** (68 mg, 0.27 mmol), dichloromethane (10 mL) and trifluoroacetic acid (1 mL) were added to a reaction flask. The reaction mixture was stirred for 1 hour at room temperature under nitrogen atmosphere, and concentrated by rotary evaporation to obtain compound **22-b** (crude) as an oil, which was used in the next step without purification. LC-MS (ESI): m/z = 136.1 (M+H)⁺.

### Synthesis of compound 22-a

**22-b** (crude) and methanol (10 mL) were added to a reaction flask. Sodium cyanoborohydride (34 mg, 0.54 mmol) was slowly added in an ice-cold water bath. The mixture was stirred at this temperature for 30 minutes, concentrated by rotary evaporation, and purified by column chromatography (mobile phase: ammonia in methanol/dichloromethane = 0/100 to 10/90) to obtain compound **22-a** (26 mg, 70% in 2 steps) as a white solid. LC-MS (ESI): m/z = 138.1 (M+H)⁺.

### Synthesis of compound 22

**I-1** (30 mg, 0.054 mmol), DMF (2 mL), **22-a** (26 mg, 0.19 mmol) and DIPEA (0.5 mL) were added to a reaction flask and stirred at room temperature overnight under nitrogen atmosphere. The next day, the reaction mixture was directly subjected to pre-HPLC (ammonium bicarbonate) and lyophilized to obtain compound **22** (6 mg, 20%) as a grey solid. LC-MS (ESI): m/z = 544.0 (M+H)⁺; ¹H NMR (400 MHz, CDCl₃) : *δ* 7.80-7.71(1H, m), 7.66-7.56(1H, m), 7.53(1H, d, *J*=7.2Hz), 7.50-7.37(2H, m), 7.34(1H, t, *J*=8Hz), 7.26-7.13(1H, m), 6.89(1H, d, *J*=10.4Hz), 5.02-4.66(2H, m), 4.53-4.32(3H, m), 4.25-4.08(1H, m), 4.07-3.93(1H, m), 3.78(1H, d, *J*=17.2Hz), 3.64-3.44(1H, m), 3.39-3.20(1H, m), 3.14-3.02(2H, m), 2.98-2.83(1H, m), 2.72-2.62(1H, m), 2.61-2.52(1H, m), 2.48(3H, s), 2.33-2.21(1H, m), 2.12-1.98(1H, m), 1.89-1.79(1H, m), 1.41-1.21(5H, m).

### Example 23: Synthetic route of compound 23

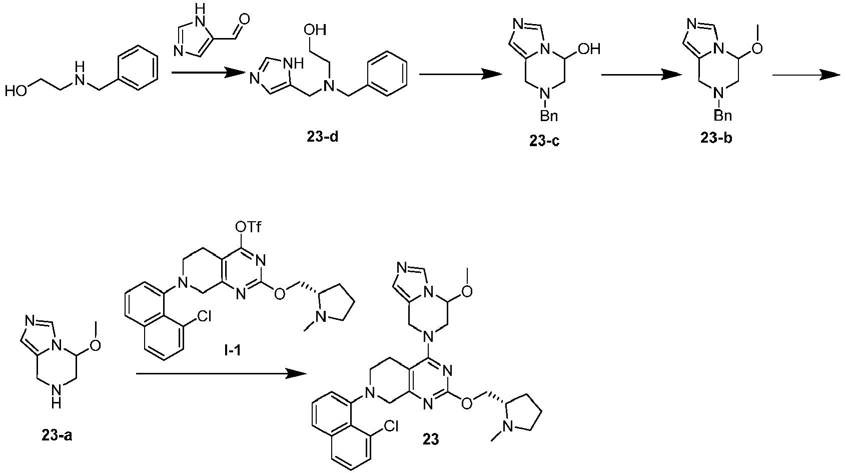

### Synthesis of compound 23-d

*N*-benzylethanolamine (1.32 g, 8.75 mmol), tetrahydrofuran (15 mL), methanol (15 mL), 4-imidazolecarboxaldehyde (700 mg, 7.29 mmol) and 4A molecular sieve were added to a reaction flask and stirred at room temperature overnight under nitrogen atmosphere. The next day, the mixture was added sodium borohydride (415 mg, 10.94 mmol) and stirred for 1 hour, filtered, concentrated by rotary evaporation, and the residue was purified by column chromatography (mobile phase: ammonia in methanol/dichloromethane = 0/100 to 6/94) to obtain compound **23-d** (1.5 g, 89%) as a colorless gum. LC-MS (ESI): m/z = 232.2 (M+H)⁺.

### Synthesis of compound 23-c

**23-d** (100 mg, 0.43 mmol), dichloromethane (20 mL) and Dess-Martin oxidant (183 mg, 0.43 mmol) were added to a reaction flask. The reaction mixture was stirred at room temperature overnight under nitrogen atmosphere. The next day, The mixture was added Dess-Martin oxidant (100 mg, 0.24 mmol) and stirred at room temperature for 30 minutes, then was added saturated sodium sulfite solution and saturated sodium bicarbonate solution. The mixture was stirred for another 10 minutes and extracted with dichloromethane (30 mL × 4). The organic phase was concentrated by rotary evaporation and purified by column chromatography (mobile phase: methanol/dichloromethane = 0/100 to 10/90) to obtain compound **23-c** (45 mg, 45%) as a white solid. LC-MS (ESI): m/z = 230.1 (M+H)⁺.

### Synthesis of compound 23-b

**23-c** (45 mg, 0.20 mmol) and tetrahydrofuran (10 mL) were added to a reaction flask. Sodium hydride (12 mg, 0.29 mmol) was added in an ice-cold water bath. The mixture was stirred at this temperature for 30 minutes. Iodomethane (48 µL, 0.77 mmol) was added dropwise. The reaction mixture was stirred in an ice-cold water bath for 1 hour before the reaction was quenched with water. The reaction mixture was extracted with dichloromethane (30 mL × 3), concentrated by rotary evaporation, and the residue was purified by column chromatography (mobile phase: methanol/dichloromethane = 0/100 to 3/97) to obtain compound **23-b** (10 mg, 21%) as a pale brown solid. LC-MS (ESI): m/z = 244.1 (M+H)⁺.

### Synthesis of compound 23-a

**23-b** (10 mg, 0.041 mmol), methanol (15 mL), 5 drops of hydrogen chloride in methanol (4 M ) and palladium on carbon (30 mg) were added to a reaction flask. The system was purged three times with hydrogen and stirred at room temperature for 3 hours. The mixture was filtered and concentrated by rotary evaporation at room temperature. 10 mL of dichloromethane and 1 mL of triethylamine were added and the mixture was concentrated by rotary evaporation to obtain compound **23-a** (crude) as a white solid which was directly used in the next step without purification. LC-MS (ESI): m/z = 154.2 (M+H)⁺.

### Synthesis of compound 23

**I-1** (20 mg, 0.036 mmol), DMF (2 mL), **23-a** (crude) and DIPEA (0.5 mL) were added to a reaction flask. The reaction mixture was stirred at room temperature overnight under nitrogen atmosphere. The next day, the reaction mixture was directly subjected to pre-HPLC (ammonium bicarbonate) and lyophilized to obtain compound **23** (2 mg, 10%) as a grey solid. LC-MS (ESI): m/z = 560.0 (M+H)⁺; ¹H NMR (400 MHz, CDCl₃) : *δ* 7.75(1H, d, *J*=8Hz), 7.68(1H, s), 7.61(1H, d, *J*=7.6Hz), 7.52(1H, d, *J*=7.6Hz), 7.44(1H, t, *J*=8Hz), 7.33(1H, t, *J*=8Hz), 7.25-7.18(1H, m), 6.93(1H, s), 5.47-5.32(1H, m), 4.98-4.87(1H, m), 4.57(1H, d, *J*=16Hz), 4.51-4.30(2H, m), 4.28-4.13(1H, m), 4.12-4.02(0.5H, m), 3.91-3.81(1H, m), 3.68-3.44(5H, m), 3.33-3.23(0.5H, m), 3.21-3.00(3H, m), 2.95-2.84(1H, m), 2.82-2.67(1H, m), 2.64-2.47(3H, m), 2.40-2.24(1H, m), 2.15-2.00(1H, m), 1.92-1.73(3H, m).

### Example 24: Synthetic route of compound 24

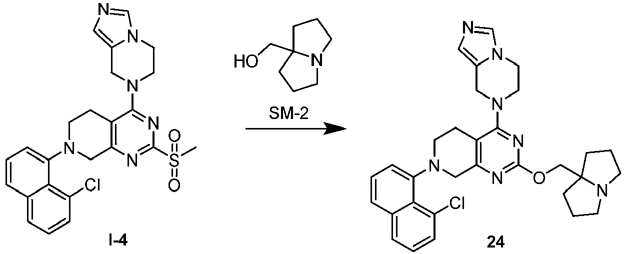

### Synthesis of compound 24

To a solution of **I-4** (50 mg, 0.1 mmol) and **SM-2** (29 mg, 0.20 mmol) in toluene (10 mL) in an ice-cold water bath was added sodium *tert*-butoxide (19 mg, 0.20 mmol). After the addition, the reaction mixture was stirred in an ice-cold water bath for 10 minutes. Upon completion, the mixture was concentrated at reduced pressure, diluted with ethyl acetate, and serially washed with water and brine, dried over anhydrous Na₂SO₄, filtered and concentrated by rotary evaporation to obtain a brown oil. The crude product was purified by a flash column chromatography (mobile phase: dichloromethane/ammonia in methanol (5%) = 1/0 to 10/1) to obtain compound **24** (25 mg, 45%) as a white solid. LC-MS (ESI): m/z 556.2 (M+H) ⁺; ¹H NMR (400MHz, CDCl₃): δ 7.75 (1H, d, *J* = 7.6Hz), 7.61 (1H, d, *J* = 8.4Hz), 7.52 (1H, d, *J* = 7.2Hz), 7.48 (1H, s), 7.43 (1H, t, *J* = 8.0Hz), 7.33 (1H, t, *J* = 7.6Hz), 7.21 (1H, d, *J* = 7.6Hz), 6.89 (1H, s), 4.69-4.80 (2H, m), 4.43 (1H, d, *J* = 18.0Hz), 4.25-4.35 (1H, m), 4.02-4.23 (4H, m), 3.88 (1H, d, *J* = 18.0Hz), 3.67-3.77 (1H, m), 3.51-3.61 (1H, m), 3.07-3.25 (4H, m), 2.52-2.74 (3H, m), 2.05-2.18 (2H, m), 1.81-1.96 (4H, m), 1.62-1.75 (2H, m).

### Example 25: Synthetic route of compound 25

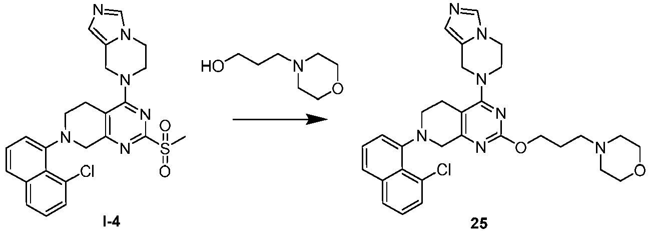

### Synthesis of compound 25

To a solution of **I-4** (50 mg, 0.1 mmol) and 3-(4-morpholinyl)-1-propanol (29 mg, 0.20 mmol) in toluene (10 mL) in an ice-cold water bath was added sodium *tert*-butoxide (19 mg, 0.20 mmol). After the addition, the reaction mixture was stirred in an ice-cold water bath for 10 minutes. Upon completion, the mixture was concentrated at reduced pressure, diluted with ethyl acetate, and serially washed with water and brine, dried over anhydrous Na₂SO₄, filtered and concentrated by rotary evaporation to obtain a brown oil. The crude product was purified by a flash column chromatography (mobile phase: dichloromethane/ammonia in methanol (5%) = 1/0 to 10/1) to obtain compound **25** (30 mg, 53%) as a white solid. LC-MS (ESI): m/z 560.3 (M+H)⁺; ¹H NMR (400MHz, CDCl₃): δ 7.76 (1H, d, *J* = 8.0Hz), 7.62 (1H, d, *J* = 7.6Hz), 7.53 (1H, dd, *J* = 7.2, 1.2Hz), 7.49 (1H, s), 7.44 (1H, t, *J* = 8.0Hz), 7.34 (1H, t, *J* = 8.0Hz), 7.23 (1H, d, *J* = 7.2Hz), 6.89 (1H, s), 4.68-4.79 (2H, m), 4.42 (1H, d, *J* = 17.6Hz), 4.35-4.38 (2H, m), 4.27-4.35 (1H, m), 4.14-4.20 (1H, m), 4.03-4.10 (1H, m), 3.79-3.87 (2H, m), 3.68-3.74 (6H, m), 3.54-3.59 (1H, m), 3.12-3.23 (1H, m), 2.54-2.65 (6H, m), 1.94-2.04 (1H, m), 1.71-1.77 (1H, m).

### Example 26: Synthetic route of compound 26

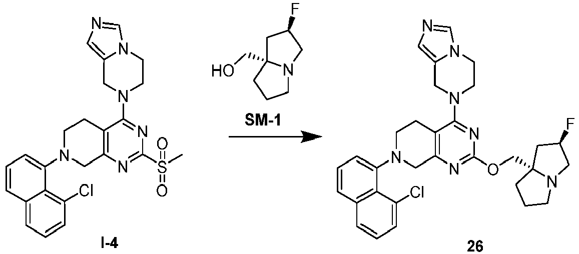

### Synthesis of compound 26

**I-4** (50 mg, 0.10 mmol), **SM-1** (32 mg, 0.20 mmol), sodium *tert*-butoxide (20 mg, 0.20 mmol) and toluene (15 mL) were added to a reaction flask. The reaction mixture was degassed and purged with nitrogen, and stirred at 0 °C for 3 hours. The reaction mixture was directly loaded onto a silica gel column for column chromatography (eluent: dichloromethane:ammonia in methanol = 100; 10:1) to obtain compound **26** (19 mg, 33%) as a white solid. LC-MS (ESI): m/z 574.3 (M+H) ⁺; ¹H NMR (400MHz, MeOD-*d₄*): *δ*7.82 (1H, dd, *J₁* = 1.2Hz, *J₂* = 8.0Hz), 7.68 (1H, d, *J* = 7.6Hz), 7.64 (1H, s), 7.53 (1H, dd, *J₁* = 1.2Hz, *J₂* = 7.2Hz), 7.49 (1H, t, *J* = 8.0Hz), 7.37 (1H, t, *J* = 7.6Hz), 7.32 (1H, d, *J* = 7.2Hz), 6.85 (1H, s), 5.27 (1H, d, *J* = 54.0Hz), 4.80 (1H, t, *J* = 15.2Hz), 4.01-4.40 (6H, m), 3.55-3.81 (3H, m), 3.10-3.31(5H, m), 2.90-3.03 (1H, m), 2.70 (1H, d, *J* = 14.4Hz), 1.80-2.38 (7H, m).

### Example 27: Synthetic route of compound 27

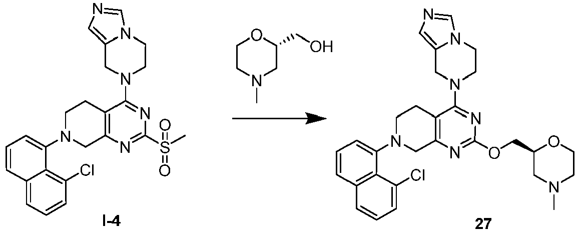

### Synthesis of compound 27

**I-4** (50 mg, 0.10 mmol), (S)-4-methyl-2-hydroxymethyl-morpholine (32 mg, 0.24 mmol), sodium *tert*-butoxide (20 mg, 0.20 mmol) and toluene (15 mL) were added to a reaction flask. The reaction mixture was degassed and purged with nitrogen, and stirred at 0 °C for 3 hours. The reaction mixture was directly loaded onto a silica gel column for column chromatography (eluent: dichloromethane: ammonia in methanol = 100; 10:1) to obtain compound **27** (25 mg, 46%) as a white solid. LC-MS (ESI): m/z 546.3 (M+H) ⁺; ¹H NMR (400MHz, MeOD-*d₄*): *δ* 7.83 (1H, dd, *J₁* = 1.2Hz, *J₂* = 8.0Hz), 7.67 (1H, d, *J* = 8.4Hz), 7.65 (1H, s), 7.53 (1H, dd, *J₁* = 0.8Hz, *J₂* = 7.6Hz), 7.48 (1H, t, *J* = 8.0Hz), 7.36 (1H, t, *J* = 7.6Hz), 7.31 (1H, d, *J* = 7.6Hz), 6.86 (1H, s), 4.79 (2H, t, *J* = 17.2Hz), 4.10-4.45(6H, m), 3.41-3.96 (6H, m), 3.11-3.33 (2H, m), 2.91 (1H, d, *J* = 11.6Hz), 2.73 (1H, d, *J* = 11.2Hz), 2.68 (1H, d, *J* = 15.2Hz), 2.33 (3H, s), 2.10-2.24 (1H, m), 2.02-2.16 (1H, m).

### Example 28: Synthetic route of compound 28

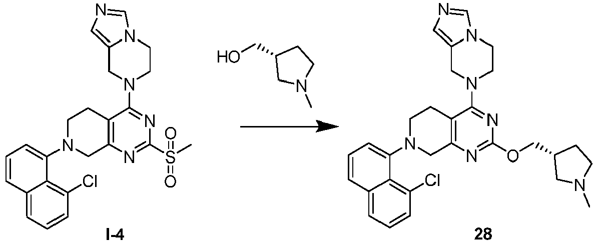

### Synthesis of compound 28

To a solution of **I-4** (50 mg, 0.1 mmol) and (3R)-1-methyl-3-pyrrolidinemethanol (23 mg, 0.20 mmol) in toluene (10 mL) in an ice-cold water bath was added sodium *tert*-butoxide (19 mg, 0.20 mmol). After the addition, the reaction mixture was stirred in an ice-cold water bath for 10 minutes. Upon completion, the mixture was concentrated at reduced pressure, diluted with ethyl acetate, and serially washed with water and brine, dried over anhydrous Na₂SO₄, filtered and concentrated by rotary evaporation to obtain a brown oil. The crude product was purified by a flash column chromatography (mobile phase: dichloromethane/ammonia in methanol (5%) = 1/0 to 10/1) to obtain compound **28** (30 mg, 56%) as a white solid. LC-MS (ESI): m/z 530.3 (M+H) ⁺; ¹H NMR (400MHz, CDCl₃): δ 7.76 (1H, d, *J* = 8.0Hz), 7.62 (1H, d, *J* = 8.0Hz), 7.52 (1H, d, *J* = 7.2Hz), 7.49 (1H, s), 7.44 (1H, t, *J* = 8.0Hz), 7.34 (1H, t, *J* = 7.2Hz), 7.22 (1H, d, *J* = 7.6Hz), 6.89 (1H, s), 4.69-4.79 (2H, m), 4.42 (1H, d, *J* = 18.0Hz), 4.26-4.34 (3H, m), 4.15-4.21 (1H, m), 4.04-4.10 (1H, m), 3.85 (1H, d, *J* = 17.6Hz), 3.70-3.76 (1H, m), 3.54-3.59 (1H, m), 3.12-3.23 (2H, m), 2.65-3.08 (5H, m), 2.57-2.64 (1H, m), 2.51 (3H, s), 2.14-2.21 (1H, m), 1.71-1.84 (1H, m).

### Example 29: Synthetic route of compound 29

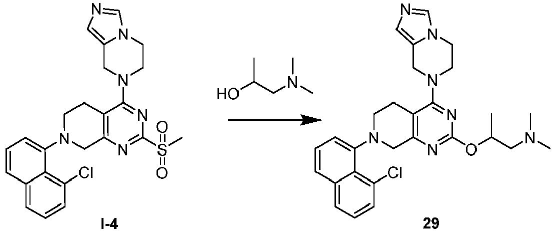

### Synthesis of compound 29

To a solution of **I-4** (50 mg, 0.1 mmol) and 1-dimethylamino-2-propanol (21 mg, 0.20 mmol) in toluene (10 mL) in an ice-cold water bath was added sodium *tert*-butoxide (19 mg, 0.20 mmol). After the addition, the reaction mixture was stirred in an ice-cold water bath for 10 minutes. Upon completion, the mixture was concentrated at reduced pressure, diluted with ethyl acetate, and serially washed with water and brine, dried over anhydrous Na₂SO₄, filtered and concentrated by rotary evaporation to obtain a brown oil. The crude product was purified by a flash column chromatography (mobile phase: dichloromethane/ammonia in methanol (5%) = 1/0 to 10/1) to obtain compound **29** (35 mg, 67%) as a white solid. LC-MS (ESI): m/z 518.3 (M+H)⁺; ¹H NMR (400MHz, CDCl₃): δ 7.76 (1H, d, *J* = 8.0Hz), 7.61 (1H, d, *J* = 8.0Hz), 7.53 (1H, d, *J* = 7.6Hz), 7.48 (1H, s), 7.44 (1H, t, *J* = 8.0Hz), 7.34 (1H, t, *J* = 8.0Hz), 7.23 (1H, d, *J* = 7.2Hz), 6.88 (1H, s), 5.28-5.41 (1H, m), 4.68-4.78 (2H, m), 4.41 (1H, d, *J* = 17.6Hz), 4.25-4.32 (1H, m), 4.14-4.19 (1H, m), 4.01-4.09 (1H, m), 3.84 (1H, dd, *J* = 18.0, 8.4Hz), 3.67-3.75 (1H, m), 3.54-3.60 (1H, m), 3.12-3.22 (2H, m), 2.69-2.76 (1H, m), 2.54-2.61 (1H, m), 2.41-2.47 (1H, m), 2.33 (6H, d, *J* = 6.8Hz), 1.36 (3H, t, *J* = 6.0Hz).

### Example 30: Synthetic route of compound 30

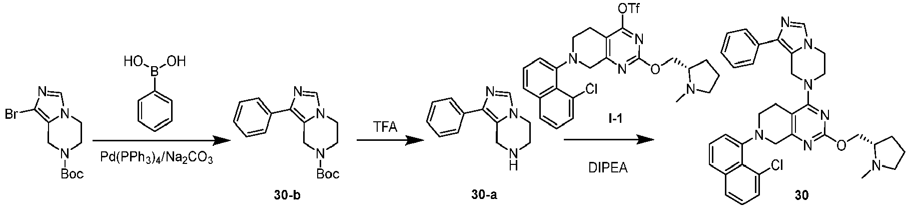

### Synthesis of compound 30-b

*Tert***-**butyl 1-bromo-5,6-dihydroimidazo[1,5-*α*]pyrazine-7(8*H*)-carboxylate (100 mg, 0.33 mmol), phenylboronic acid (81 mg, 0.66 mmol), tetrakis(triphenylphosphine)palladium (19 mg, 0.0165 mmol), sodium carbonate (70 mg, 0.66 mmol), 1,4-dioxane (10 mL) and water (2 mL) were added to a reaction flask. The mixture was degassed and purged with nitrogen and stirred at 90 °C overnight. The next day, the reaction mixture was cooled to room temperature and diluted with ethyl acetate and water. The organic phase was separated, dried over anhydrous Na₂SO₄, filtered, concentrated by rotary evaporation, and the residue was purified by column chromatography (mobile phase: (dichloromethane:methanol = 10:1)/dichloromethane, 0-100%) to obtain compound **30-b** (78 mg, 79%) as a yellow solid. LC-MS (ESI): m/z 300.2 (M+H) ⁺.

### Synthesis of compound 30-a

To a solution of **30-b** (78 mg, 0.26 mmol) in dichloromethane (6 mL) was added trifluoroacetic acid (2 mL) at room temperature. The mixture was stirred at room temperature overnight, concentrated by rotary evaporation, adjusted to a basic pH by adding aqueous sodium bicarbonate, and extracted with ethyl acetate. The organic phase was dried over anhydrous Na₂SO₄, filtered, and the filtrate was concentrated by rotary evaporation to obtain a crude product of compound **30-a** (48 mg, 92%) as a yellow solid. LC-MS (ESI): m/z 200.1(M+H) ⁺.

### Synthesis of compound 30

**I-1** (20 mg, 0.036 mmol), **30-a** (14 mg, 0.072 mmol), *N, N*-diisopropylethylamine (14 mg, 0.108 mmol) and DMF (3 mL) were added to a reaction flask. The mixture was stirred at room temperature for 1 hour. The reaction mixture was directly purified by pre-HPLC to obtain compound **30** (15.8 mg, 73%) as a white solid. LC-MS (ESI): m/z 606.3 (M+H) ⁺; ¹H NMR (400 MHz, CDCl₃) δ7.76(1H, d, *J*=7.6Hz), 7.66(2H, d, *J*=7.6Hz), 7.63(1H, d, *J*=4.8Hz), 7.58(1H, s), 7.53(1H, d, *J*=7.2Hz), 7.47-7.40 (3H, m), 7.34(1H, t, *J*=8Hz), 7.27-7.21 (2H, m), 4.97(2H, s), 4.47-4.40 (2H, m), 4.38-4.32 (1H, m), 4.26-4.16 (2H, m), 4.15-4.09 (1H, m), 3.90-3.84 (1H, m), 3.82-3.72 (1H, m), 3.61-3.54 (1H, m), 3.43-3.19 (2H, m), 3.18-3.10 (2H, m), 2.72-2.57 (3H, m), 2.50(3H, s), 2.31(2H, q, *J*=8.4Hz), 2.10-2.04(1H,m).

### Example 31: Synthetic route of compound 31

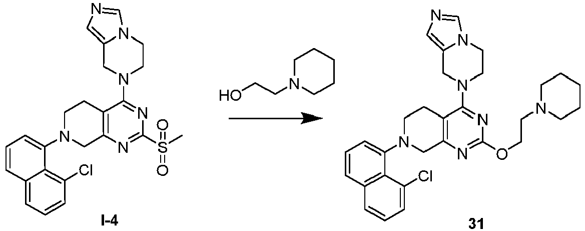

### Synthesis of compound 31

**I-4** (50 mg, 11.65 mmol), *N*-hydroxyethylpiperidine (32 mg, 0.25 mmol), sodium *tert-*butoxide (20 mg, 0.20 mmol) and toluene (15 mL) were added to a reaction flask. The reaction mixture was degassed and purged with nitrogen, and stirred at 0 °C for 3 hours. The reaction mixture was directly loaded onto a silica gel column for column chromatography (eluent: dichloromethane: ammonia in methanol = 100:1 to 10:1) to obtain compound **31** (14 mg, 26%) as a white solid. LC-MS (ESI): m/z 544.2 (M+H) ⁺; ¹H NMR (400MHz, MeOD-*d₄*): *δ* 8.93 (1H, s), 7.84 (1H, d, *J* = 8.0Hz), 7.71 (1H, d, *J* = 8.0Hz), 7.45-7.60 (3H, m), 7.30-7.43 (2H, m), 5.08 (1H, d, *J*= 16.4Hz), 4.94 (1H, d, *J* = 16.8Hz), 4.66-4.80 (2H, m), 4.40-4.65 (2H, m), 4.37 (2H, d, *J* = 17.6Hz), 3.90-4.00 (1H, m), 3.78 (1H, d, *J* = 18.0Hz), 3.50-3.78(5H, m), 2.93-3.40 (4H, m), 2.76 (1H, d, *J* = 14.8Hz), 1.80-2.04 (5H, m), 1.42-1.76 (1H, m).

### Example 32: Synthetic route of compound 32

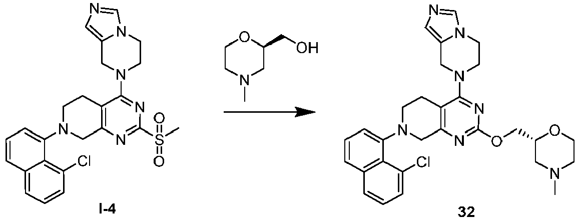

### Synthesis of compound 32

**I-4** (50 mg, 11.65 mmol), (*R*)-4-methyl-2-hydroxymethyl-morpholine (32 mg, 0.24 mmol), sodium *tert*-butoxide (20 mg, 0.20 mmol) and toluene (15 mL) were added to a reaction flask. The reaction mixture was degassed and purged with nitrogen, and stirred at 0 °C for 3 hours. The reaction mixture was directly loaded onto a silica gel column for column chromatography (eluent: dichloromethane: ammonia in methanol = 100 to 10:1) to obtain compound **32** (25 mg, 46%) as a white solid. LC-MS (ESI): m/z 546.3 (M+H)⁺; ¹H NMR (400MHz, MeOD-*d₄*): *δ* 7.81 (1H, d, *J* = 8.0Hz), 7.67 (1H, d, *J* = 7.2Hz), 7.64 (1H, s), 7.52 (1H, d, *J* = 7.2Hz), 7.48 (1H, t, *J* = 8.0Hz), 7.36 (1H, t, *J* = 8.0Hz), 7.31 (1H, d, *J* = 7.6Hz), 6.85 (1H, s), 4.79 (2H, t, *J* = 19.6Hz), 4.10-4.45(6H, m), 3.41-3.96 (6H, m), 3.11-3.33 (2H, m), 2.89 (1H, d, *J* = 11.6Hz), 2.71 (1H, d, *J* = 10.0Hz), 2.67 (1H, d, *J* = 15.6Hz), 2.31 (3H, s), 2.10-2.24 (1H, m), 2.00-2.10 (1H, m).

### Example 33: Synthetic route of compound 33

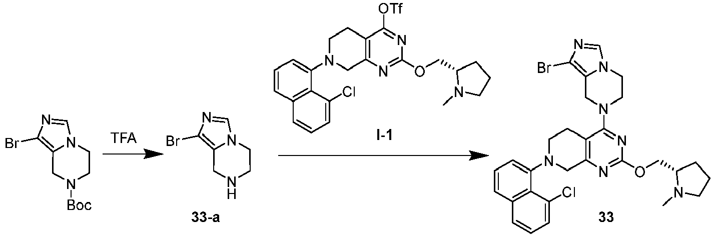

### Synthesis of compound 33-a

To a solution of *tert*-butyl 1-bromo-5,6-dihydroimidazo[1,5-*α*]pyrazine-7(8*H*)-carboxylate (30 mg, 0.1 mmol) in dichloromethane (5 mL) was added trifluoroacetic acid (1 mL) at room temperature. The mixture was stirred at room temperature overnight, concentrated by rotary evaporation, adjusted to a basic pH by adding aqueous sodium bicarbonate, and extracted with ethyl acetate. The organic phase was dried over anhydrous Na₂SO₄, filtered, and the filtrate was concentrated by rotary evaporation to obtain a crude product of compound **33-a** (16 mg, 80%) as a yellow solid. LC-MS (ESI): m/z 202.0(M+H) ⁺.

### Synthesis of compound 33

**I-1** (30 mg, 0.054 mmol), **33-a** (16 mg, 0.081 mmol), *N, N*-diisopropylethylamine (21 mg, 0.162 mmol) and DMF (3 mL) were added to a reaction flask. The mixture was stirred at room temperature for 1 hour. The reaction mixture was directly purified by pre-HPLC to obtain compound **33** (17.5 mg, 53%) as a white solid. LC-MS (ESI): m/z 610.1 (M+H)⁺; ¹H NMR (400 MHz, CDCl₃) *δ* 7.76(1H, dd, *J*=8.4Hz, 1.2Hz), 7.62(1H, d, *J*=7.6Hz), 7.53(1H, dd, *J*=7.6Hz, 1.2Hz), 7.46(1H, d, *J*=8Hz), 7.13(1H, s), 7.34(1H, t, *J*=8Hz), 7.23(1H, d, *J*=7.2Hz), 4.69-4.57 (2H, m), 4.49-4.41 (2H, m), 4.33-4.25 (1H, m), 4.22-4.06 (3H, m), 3.87 (1H, dd, *J*=18Hz, 2.8Hz), 3.73-3.64 (1H, m), 3.62-3.54 (1H, m), 3.28-3.10 (3H, m), 2.78-2.69 (1H, m), 2.61(1H, d, *J*=14Hz), 2.52(3H, d, *J*=0.8Hz), 2.32 (1H, dd, *J*=16.4Hz, 8.8Hz), 2.14 - 2.07 (1H, m), 1.92-1.73 (3H, m).

### Example 34: Synthetic route of compound 34

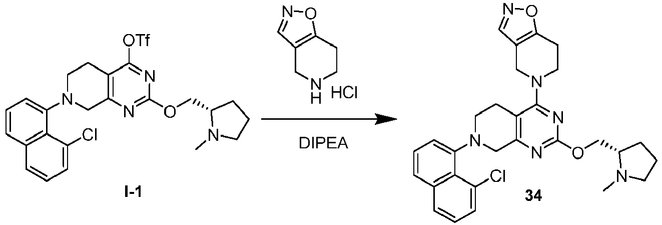

### Synthesis of compound 34

**I-1** (30 mg, 0.054 mmol), 4,5,6,7-tetrahydroisooxazolo[4,5-c]piperidine hydrochloride (13 mg, 0.081 mmol), N, N-diisopropylethylamine (21 mg, 0.162 mmol) and DMF (3 mL) were added to a reaction flask. The mixture was stirred at room temperature for 1 hour. The reaction mixture was directly purified by pre-HPLC to obtain compound **34** (21.6 mg, 76%) as a white solid. LC-MS (ESI): m/z 531.2 (M+H)⁺; ¹H NMR (400 MHz, CDCl₃) : δ 8.17(1H, s), 7.76(1H, dd, *J*=8Hz, 0.8Hz), 7.62(1H, dd, *J*=8Hz, 0.8Hz), 7.53(1H, dd, *J*=8Hz, 1.6Hz), 7.45(1H, t, *J*=8Hz), 7.34(1H, t, *J*=8Hz), 7.23(1H, dd, *J*=7.6Hz, 0.4Hz), 4.57-4.39 (4H, m), 4.22-4.15 (1H, m), 4.11-4.03 (1H, m), 3.87(1H, d, *J*=17.6Hz), 3.61-3.41 (2H, m), 3.23-3.10 (4H, m), 3.00-2.91 (1H, m), 2.77-2.67 (1H, m), 2.61- 2.54 (1H, m), 2.51(3H, d, *J*=1.2Hz), 2.31 (1H, dd, *J*=16.8Hz, 8.8Hz), 2.14-2.07 (1H, m), 1.90-1.72 (3H, m).

### Example 35: Synthetic route of compound 35

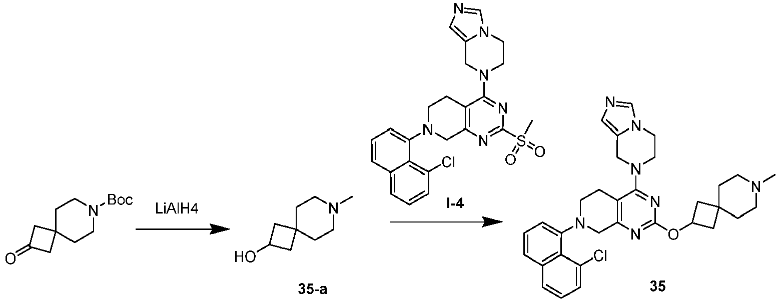

### Synthesis of compound 35-a

*Tert*-butyl 2-oxo-7-azaspiro[3.5]nonane-7-carboxylate (280 mg, 1.17 mmol) was dissolved in THF (10 mL) in an ice-cold water bath, then was added LiAlH₄ (2.5 M, 0.7 mL, 1.75 mmol). The mixture was slowly warmed to room temperature and stirred overnight. The reaction was quenched by carefully adding Na₂SO₄·10H₂O. The mixture was filtered, and the filtrate was concentrated to obtain **35-a** (150 mg, 70%) as a white waxy solid. LC-MS (ESI): m/z 156.3 (M+H) ⁺.

### Synthesis of compound 35

To a solution of **I-4** (50 mg, 0.1 mmol) and **35-a** (31 mg, 0.20 mmol) in toluene (10 mL) in an ice-cold water bath was added sodium *tert*-butoxide (19 mg, 0.20 mmol). After the addition, the reaction mixture was stirred in an ice-cold water bath for 10 minutes. Upon completion, the mixture was concentrated at reduced pressure, diluted with ethyl acetate, and serially washed with water and brine, dried over anhydrous Na₂SO₄, filtered and concentrated by rotary evaporation to obtain a brown oil. The crude product was purified by a flash column chromatography (mobile phase: dichloromethane/ammonia in methanol (5%) = 1/0 to 10/1) to obtain compound **35** (30 mg, 52%) as a white solid. LC-MS (ESI): m/z 570.3 (M+H) ⁺; ¹H NMR (400MHz, CDCl₃): δ 7.76 (1H, d, *J* = 8.0Hz), 7.62 (1H, d, *J* = 7.8Hz), 7.53 (1H, d, *J* = 7.6Hz), 7.49 (1H, s), 7.43 (1H, t, *J* = 8.4Hz), 7.34 (1H, t, *J* = 8.4Hz), 7.23 (1H, d, *J* = 7.6Hz), 6.89 (1H, s), 5.08-5.15 (1H, m), 4.72 (2H, s), 4.40 (1H, d, *J* = 18.0Hz), 4.25-4.32 (1H, m), 4.12-4.18 (1H, m), 4.00-4.08 (1H, m), 3.82 (1H, d, *J* = 17.6Hz), 3.68-3.75 (1H, m), 3.53-3.60 (1H, m), 3.10-3.26 (2H, m), 2.64-3.05 (3H, m), 2.53-2.66 (2H, m), 2.58 (3H, s), 2.36-2.45 (2H, m), 1.99-2.07 (2H, m), 1.71-1.93 (2H, m), 1.25-1.37 (2H, m).

### Example 36: Synthetic route of compound 36

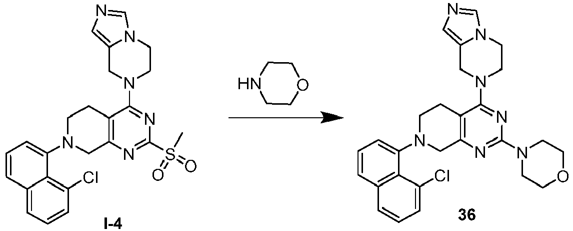

### Synthesis of compound 36

To a solution of **I-4** (50 mg, 0.1 mmol) in 1,4-dioxane (10 mL) was added morpholine (88 mg, 1.0 mmol) at room temperature. After the addition, the reaction mixture was heated at reflux overnight. Upon completion, the mixture was concentrated at reduced pressure to obtain a crude product. The crude product was purified by pre-HPLC to obtain compound **36** (20 mg, 40%) as a white solid. LC-MS (ESI): m/z 502.2 (M+H) ⁺; ¹H NMR (400MHz, CDCl₃): δ 7.75 (1H, d, *J* = 8.0Hz), 7.61 (1H, s), 7.60 (1H, d, *J* = 8.0Hz), 7.52 (1H, d, *J* = 7.2Hz), 7.43 (1H, t, *J* = 8.0Hz), 7.33 (1H, t, *J* = 8.0Hz), 7.22 (1H, d, *J* = 7.6Hz), 6.91 (1H, s), 4.58-4.73 (2H, m), 4.35 (1H, d, *J* = 17.2Hz), 4.24-4.32 (1H, m), 4.13-4.22 (1H, m), 3.92-4.02 (1H, m), 3.68-3.82 (9H, m), 3.60-3.69 (1H, m), 3.49-3.58 (1H, m), 3.06-3.18 (2H, m), 2.50-2.62 (1H, m).

### Example 37: Synthetic route of compound 37

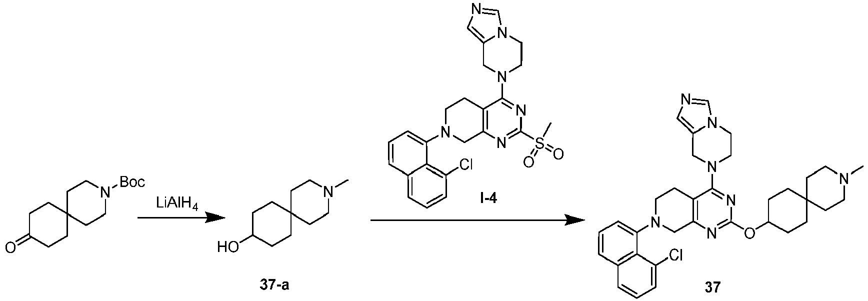

### Synthesis of compound 37-a

Boc-9-oxo-3-azaspiro[5.5]undecane (450 mg, 1.68 mmol) was dissolved in THF (10 mL) in an ice-cold water bath. LiAlH₄ (2.5 M, 1.0 mL, 2.52 mmol) was added. The mixture was slowly warmed to room temperature and stirred overnight. The reaction was quenched by carefully adding Na₂SO₄·10H₂O. The mixture was filtered, and the filtrate was concentrated to obtain **37-a** (250 mg, 81%) as a white waxy solid. LC-MS (ESI): m/z 184.2 (M+H)⁺.

### Synthesis of compound 37

To a solution of **I-4** (50 mg, 0.1 mmol) and **37-a** (37 mg, 0.20 mmol) in toluene (10 mL) in an ice-cold water bath was added sodium *tert*-butoxide (19 mg, 0.20 mmol). After the addition, the reaction mixture was stirred in an ice-cold water bath for 10 minutes. Upon completion, the mixture was concentrated at reduced pressure, diluted with ethyl acetate, and serially washed with water and brine, dried over anhydrous Na₂SO₄, filtered and concentrated by rotary evaporation to obtain a brown oil. The crude product was purified by pre-HPLC (acidic) to obtain compound **37** (20 mg, 33%) as a white solid. LC-MS (ESI): m/z 598.3 (M+H)⁺; ¹H NMR (400MHz, MeOD-*d₄*): δ 8.94 (1H, s), 7.85 (1H, d, *J* = 8.0Hz), 7.72 (1H, d, *J* = 7.6Hz), 7.49-7.56 (3H, m), 7.35-7.41 (2H, m), 5.16-5.23 (1H, m), 5.15 (1H, d, *J* = 16.4Hz), 5.01 (1H, d, *J* = 16.4Hz), 4.37-4.62 (3H, m), 4.35 (1H, d, *J* = 17.6Hz), 3.98-4.06 (1H, m), 3.80 (1H, d, *J* = 18.0Hz), 3.59-3.67 (1H, m), 3.29-3.42 (3H, m), 3.21-3.29 (1H, m), 3.07-3.19 (2H, m), 2.87 (3H, s), 2.74-2.83 (1H, m), 1.87-2.09 (5H, m), 1.74-1.87 (2H, m), 1.53-1.70 (4H, m), 1.39-1.50 (1H, m).

### Example 38: Synthetic route of compound 38

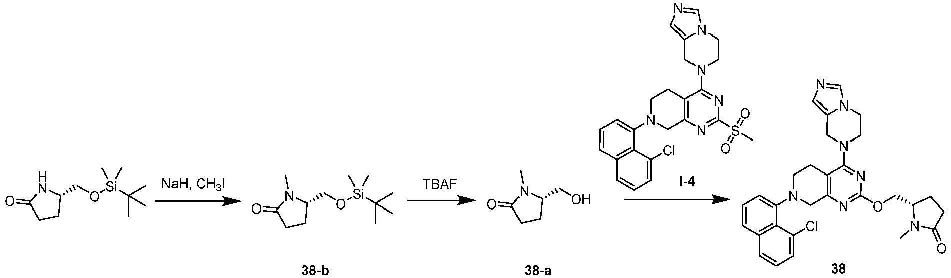

### Synthesis of compound 38-b

(*S*)-5-((*tert*-butyldimethylsilyloxy) methyl)pyrrolidin-2-one (1.0 g, 4.36 mmol) was added into sodium hydride (60%, 349 mg, 8.72 mmol) in THF (20 mL) at 0 °C. The mixture was warmed to room temperature and reacted for 0.5 hours before iodomethane (0.41 mL, 6.54 mmol) was added. The reaction mixture was warmed to room temperature and stirred for 5 hours. The reaction was quenched by adding saturated aqueous ammonium chloride in an ice-cold water bath. The reaction mixture was extracted with ethyl acetate, and the organic phases were combined, dried over anhydrous sodium sulfate, filtered, and the filtrate was concentrated by rotary evaporation to obtain compound **38-b** (0.9 g, 85%) as an oil. LC-MS (ESI): m/z 244.2 (M+H)⁺.

### Synthesis of compound 38-a

To a solution of **38-b** (0.9 g, 3.70 mmol) in THF (10 mL) was added tetrabutylammonium fluoride (1 M in THF, 4.44 mL, 4.44 mmol) at room temperature. The resulting reaction mixture was stirred at room temperature overnight. Upon completion, the mixture was concentrated. The crude product was purified by a flash column chromatography (mobile phase: dichloromethane/methanol = 1/0 to 10/1) to obtain compound **38-a** (0.4 g, 84%) as a white waxy solid. LC-MS (ESI): m/z 130.2 (M+H)⁺.

### Synthesis of compound 38

To a solution of **I-4** (50 mg, 0.1 mmol) and **38-a** (26 mg, 0.20 mmol) in toluene (10 mL) in an ice-cold water bath was added sodium *tert*-butoxide (19 mg, 0.20 mmol). After the addition, the reaction mixture was stirred in an ice-cold water bath for 10 minutes. Upon completion, the mixture was concentrated at reduced pressure, diluted with ethyl acetate, and serially washed with water and brine, dried over anhydrous Na₂SO₄, filtered and concentrated by rotary evaporation to obtain a brown oil. The crude product was purified by a flash column chromatography (mobile phase: dichloromethane/ammonia in methanol (5%) = 1/0 to 10/1) to obtain compound **38** (20 mg, 36%) as a white solid. LC-MS (ESI): m/z 544.2 (M+H) ⁺; ¹H NMR (400MHz, CDCl₃): δ 7.76 (1H, d, *J* = 7.6Hz), 7.63 (1H, d, *J* = 7.6Hz), 7.56 (1H, s), 7.53 (1H, d, *J* = 7.6Hz), 7.45 (1H, t, *J* = 7.6Hz), 7.34 (1H, t, *J* = 7.6Hz), 7.23 (1H, d, *J* = 7.6Hz), 6.91 (1H, s), 4.69-4.80 (2H, m), 4.26-4.47 (4H, m), 4.15-4.24 (1H, m), 4.04-4.13 (1H, m), 3.80-3.92 (2H, m), 3.69-3.76 (1H, m), 3.53-3.62 (1H, m), 3.10-3.27 (2H, m), 2.92 (3H, d, *J =* 2.4Hz), 2.46-2.67 (2H, m), 2.31-2.40 (1H, m), 2.17-2.27 (1H, m), 1.94-2.07 (1H, m).

### Example 39: Synthetic route of compound 39

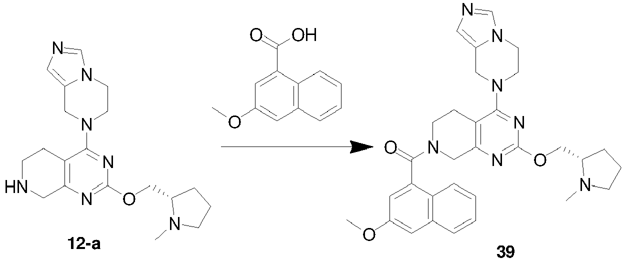

### Synthesis of compound 39

**12-a** (50 mg, 0.14 mmol), DMF (2 mL), 3-methoxy-1-naphthoic acid (68 mg, 0.34 mmol), HATU (129 mg, 0.34 mmol) and DIPEA (115 µL, 0.70 mmol) were added to a reaction flask. The reaction mixture was then stirred at room temperature overnight under nitrogen atmosphere. The reaction mixture was directly subjected to pre-HPLC (ammonium bicarbonate) and lyophilized to obtain compound **39** (5 mg, 7%) as a white solid. LC-MS (ESI): m/z = 554.0 (M+H)⁺; ¹H NMR (400 MHz, CDCl₃): *δ* 7.84-7.68 (2H, m), 7.54-7.31(3H, m), 7.24-7.18(1H, m), 7.18-7.11(1H, m), 6.88(1H, d, *J*=22Hz), 5.05-4.85(1H, m), 4.69(2H, d, *J*=43.2Hz), 4.41-4.04(5H, m), 4.00-3.88(4H, m), 3.88-3.79(1H, m), 3.45-3.32(1H, m), 2.94-2.85(1H, m), 2.81-2.37(5H, m), 2.28-1.75(5H, m), 1.50-1.39(2H, m).

### Example 40: Synthetic route of compound 40

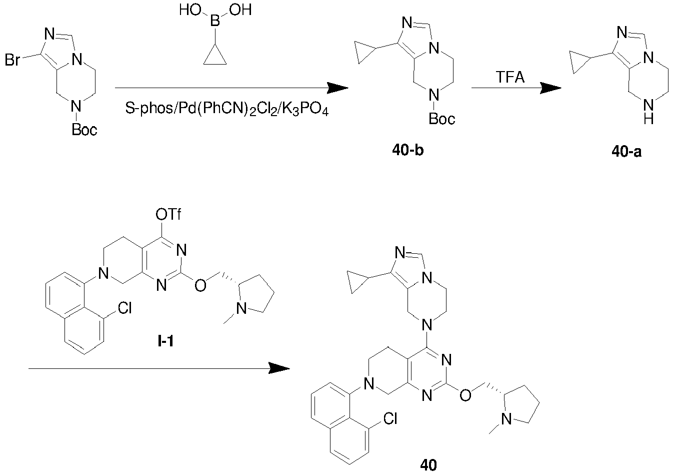

### Synthesis of compound 40-b

*Tert*-butyl 1-bromo-5,6-dihydroimidazo[1,5-α]pyrazine-7(8*H*)-carboxylate (200 mg, 0.66 mmol), cyclopropylboronic acid (114 mg, 1.32 mmol), bis(cyanophenyl)palladium dichloride (13 mg, 0.033 mmol), 2-dicyclohexylphosphino-2',6'-dimethoxy-1,1'-biphenyl (27 mg, 0.066 mmol), potassium phosphate (420 mg, 1.98 mmol) and toluene (15 mL) were added to a reaction flask. The mixture was degassed and purged with nitrogen and stirred at 120 °C overnight. The next day, the reaction mixture was cooled to room temperature and diluted with ethyl acetate and water. The organic phase was separated, dried over anhydrous Na₂SO₄, filtered, concentrated by rotary evaporation, and the residue was purified by column chromatography (mobile phase: dichloromethane : methanol = 10:1/dichloromethane, 0-100%) to obtain compound **40-b** (120 mg, 69%) as a yellow solid. LC-MS (ESI): m/z 264.1 (M+H)⁺.

### Synthesis of compound 40-a

To a solution of **40-b** (120 mg, 0.456 mmol) in dichloromethane (6 mL) was added trifluoroacetic acid (2 mL) at room temperature. The mixture was stirred at room temperature overnight and concentrated by rotary evaporation to obtain a crude product of compound **40-a** (130 mg) as a yellow solid, which was directly used in the next step without purification. LC-MS (ESI): m/z 164.1(M+H) ⁺.

### Synthesis of compound 40

**I-1** (40 mg, 0.072 mmol), **40-a** (18 mg, 0.108 mmol), *N*, *N*-diisopropylethylamine (47 mg, 0.36 mmol) and DMF (2 mL) were added to a reaction flask. The mixture was stirred at room temperature for 1 hour. The reaction mixture was directly purified by pre-HPLC to obtain compound **40** (5.2 mg, 13%) as a white solid. LC-MS (ESI): m/z 570.3 (M+H) ⁺; ¹H NMR (400 MHz, CDCl₃) δ7.77(1H, d, *J*=7.6Hz), 7.63(1H, d, *J*=8Hz), 7.54(1H, d, *J*=7.6Hz), 7.46(1H, t, *J*=8Hz), 7.38-7.33 (2H, m), 7.24(1H, t, *J*=7.6Hz), 4.84- 4.68 (2H, m), 4.50- 4.41 (2H, m), 4.31-4.03 (4H, m), 3.87(1H, d, *J*=17.6Hz), 3.74- 3.65 (1H, m), 3.62- 3.55 (1H, m), 3.28- 3.11 (3H, m), 2.80- 2.67 (1H, m), 2.62(1H, d, *J*=14Hz), 2.52(3H, s), 2.37- 2.28 (1H, m), 2.14- 2.03 (1H, m), 1.92- 1.74 (4H, m), 0.85(4H, d, *J*=8Hz).

### Example 41: Synthetic route of compound 41

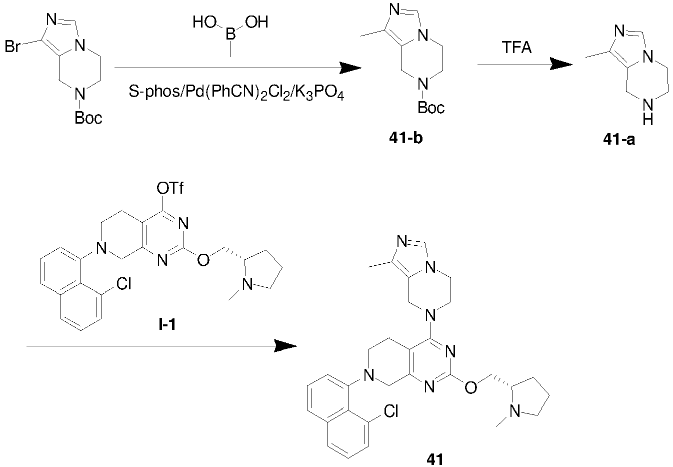

### Synthesis of compound 41-b

Tert-butyl 1-bromo-5,6-dihydroimidazo[1,5-α]pyrazine-7(8*H*)-carboxylate (200 mg, 0.66 mmol), methylboronic acid (79 mg, 1.32 mmol), bis(cyanophenyl)palladium dichloride (13 mg, 0.033 mmol), 2-dicyclohexylphosphino-2',6'-dimethoxy-1,1'-biphenyl (27 mg, 0.066 mmol), potassium phosphate (420 mg, 1.98 mmol) and toluene (10 mL) were added to a reaction flask. The mixture was degassed and purged with nitrogen and stirred at 120 °C overnight. The next day, the reaction mixture was cooled to room temperature and diluted with ethyl acetate and water. The organic phase was separated, dried over anhydrous Na₂SO₄, filtered, concentrated by rotary evaporation, and the residue was purified by column chromatography (mobile phase: dichloromethane: methanol = 10:1/dichloromethane, 0-100%) to obtain compound **41-b** (190 mg) as a yellow solid. LC-MS (ESI): m/z 238.1 (M+H) ⁺.

### Synthesis of compound 41-a

To a solution of **41-b** (190 mg, 0.8 mmol) in dichloromethane (6 mL) was added trifluoroacetic acid (2 mL) at room temperature. The mixture was stirred at room temperature overnight. Water was added. The mixture was extracted with ethyl acetate, and the aqueous phase was basified to pH 7-8 with aqueous sodium bicarbonate solution and lyophilized to obtain a crude product of compound **41-a** (110 mg) as a yellow solid, which was directly used in the next step without purification. LC-MS (ESI): m/z 138.1(M+H) ⁺.

### Synthesis of compound 41

**I-1** (50 mg, 0.09 mmol), **41-a** (37 mg, 0.27 mmol), *N*, *N*-diisopropylethylamine (58 mg, 0.45 mmol) and DMF (3 mL) were added to a reaction flask. The mixture was stirred at room temperature for 1 hour. Upon completion, water was added and the mixture was extracted with ethyl acetate. The organic phase was separated, dried over anhydrous Na₂SO₄, filtered, and the filtrate was concentrated by rotary evaporation. The crude product was purified by pre-HPLC to obtain compound **41** (12.2 mg, 25%) as a white solid. LC-MS (ESI): m/z 544.3 (M+H) ⁺; ¹H NMR (400 MHz, CDCl₃) δ7.76(1H, d, *J* =8Hz), 7.62(1H, d, *J*=7.6Hz), 7.53(1H, d, *J*=8Hz), 7.51- 7.40 (2H, m), 7.34(1H, t, *J*=8Hz), 7.23(1H, dd, *J*=7.2Hz,0.8Hz), 4.81- 4.58 (2H, m), 4.50- 4.40 (2H, m), 4.32- 4.15 (3H, m), 4.09- 4.00 (1H, m), 3.86(1H, d, *J*=18Hz), 3.76- 3.61 (1H, m), 3.61- 3.53 (1H, m), 3.25- 3.12 (3H, m), 2.79- 2.70 (1H, m), 2.64- 2.57 (1H, m), 2.52(3H, d, *J*=1.2Hz), 2.49-2.21 (2H, m), 2.19 (2H, s), 2.13- 2.07 (1H, m), 1.90- 1.73 (3H, m).

### Example 42: Synthetic route of compound 46

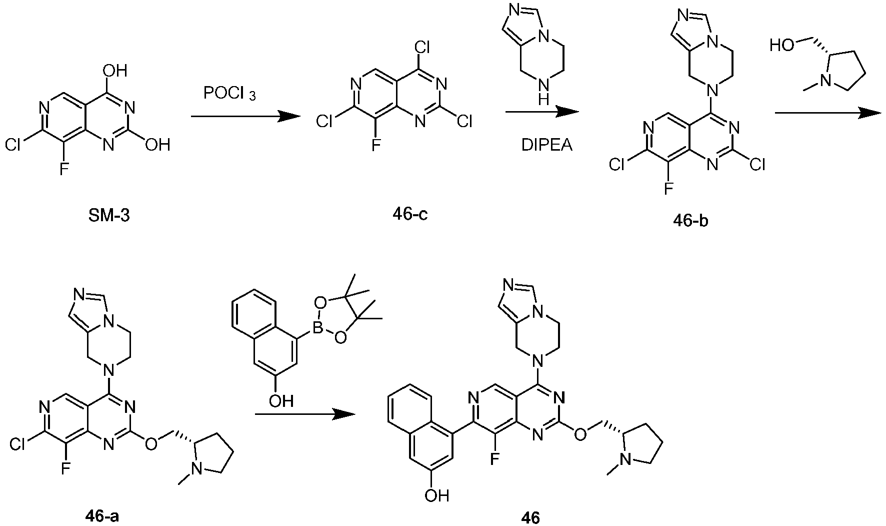

### Synthesis of compound 46-c

**SM-3** (1.10 g, 5.12 mmol), phosphorus oxychloride (11 mL) and *N, N-*diisopropylethylamine (4.2 mL) were added to a reaction flask. The mixture was degassed and purged with nitrogen and stirred at 100 °C for 1 hour. The reaction mixture was cooled to room temperature, evaporated and directly used in the next step without purification.

### Synthesis of compound 46-b

Dichloromethane (100 mL) was added to a reaction flask containing **46-c** (1.28 g, 5.12 mmol). The mixture was stirred at -40 °C for 10 minutes before *N*, *N*-diisopropylethylamine (20 mL) was slowly added. 5,6,7,8-tetrahydro-imidazo[1,5-α]pyrazine (0.63 g, 5.12 mmol) was added. The mixture was stirred at -40 °C for 1 hour. Upon completion, the reaction mixture was added saturated aqueous sodium bicarbonate (200 mL) and the aqueous phase was extracted with dichloromethane (200 mL × 2) and the organic phase was concentrated. The residue was dried in nitrogen to obtain compound **46-b** (3.00 g, 200%) as a yellow solid. LC-MS (ESI): m/z 339.3 (M+H) ⁺.

### Synthesis of compound 46-a

Dioxane (10 mL) was added to a reaction flask containing **46-b** (500 mg, 1.48 mmol), the mixture was added *N*-methyl-L-prolinol (345 mg, 3.00 mmol) and *N*, *N*-diisopropylethylamine (390 mg, 3.00 mmol). The mixture was stirred at 85 °C for 3 hours. The reaction mixture was loaded onto a silica gel column for column chromatography (mobile phase: dichloromethane/methanol (amine) = 25/1 to 10/1) to obtain compound **46-a** (65 mg, 11%). LC-MS (ESI): m/z 418.2 (M+H) ⁺.

### Synthesis of compound 46

Dioxane (10 mL) and water (1 mL) were added to a reaction flask containing **46-a** (65 mg, 0.16 mmol). 4-(4,4,5,5-Tetramethyl-1,2,3-dioxaborolan-2-yl) naphthalen-2-ol (42 mg, 0.16 mmol), potassium carbonate (70 mg, 0.50 mmol) and tetrakis(triphenylphosphine)palladium (11 mg, 0.01 mmol) were added to the reaction mixture. The mixture was stirred at 100 °C for 3 hours under argon atmosphere. The reaction mixture was concentrated and subjected to pre-HPLC to obtain compound **46** (34 mg, 33%, TFA salt). LC-MS (ESI): m/z 526.2 (M+H) ⁺.¹H NMR (400MHz, MeOD-*d₄*): *δ*9.30 (1H, s), 8.94-9.03 (1H, m), 7.76 (1H, d, *J =* 8.0Hz), 7.15-7.60 (6H, m), 5.42 (2H, s), 4.50-4.80 (4H, m), 3.70-3.96 (3H, m), 3.24-3.30 (4H, m), 3.11 (2H, s), 1.96-2.50 (4H, m).

### Example 43: Synthetic route of compound 47

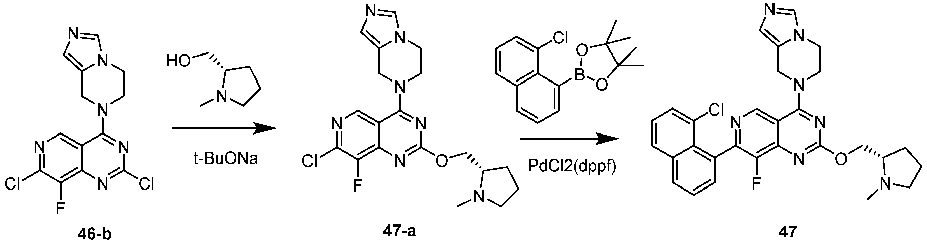

### Synthesis of compound 47-a

Toluene (10 mL) and tetrahydrofuran (10 mL) were added to a reaction flask containing **46-b** (0.113 g, 0.33 mmol). The mixture was stirred at 0 °C for 10 minutes, then was added *N*-Methyl-L-prolinol (78 mg, 0.68 mmol) and sodium *tert*-butoxide (96 mg, 1.00 mmol). The mixture was stirred at 0 °C for 1 hour. The reaction mixture was loaded onto a silica gel column for column chromatography (mobile phase: dichloromethane/methanol (amine) = 25/1 to 10/1) to obtain compound **47-a** (100 mg, 73%). LC-MS (ESI): m/z 418.1 (M+H) ⁺.

### Synthesis of compound 47

Dioxane (12 mL) and water (3 mL) were added to a reaction flask containing **47-a** (100 mg, 0.24 mmol), and the mixture was added 1-(4,4,5,5-Tetramethyl-1,2,3-dioxaborolan-2-yl) naphthalen-8-yl chloride (100 mg, 0.35 mmol), sodium carbonate (110 mg, 1.00 mmol) and PdCl₂(dppf) (30 mg, 0.04 mmol). The resulting mixture was stirred at 100 °C for 3 hours under argon atmosphere. The reaction mixture was concentrated and subjected to pre-HPLC to obtain compound **47** (5.6 mg, 4%). LC-MS (ESI): m/z 544.2(M+H) ⁺. ¹H NMR (400MHz, MeOD-*d₄*): *δ* 9.18 (1H, s), 8.14 (1H, d, *J* = 8.0Hz), 7.99 (1H, d, *J* = 8.8Hz), 7.45-7.80 (5H, m), 6.94 (1H, s), 5.30 (2H, s), 4.56-4.90 (2H, m), 4.40-4.46 (4H, m), 3.40-3.53 (1H, m), 3.10-3.30 (1H, m), 2.95-3.16 (1H, m), 2.62 (3H, s), 2.05-2.26 (1H, m), 1.76-1.96 (3H, m).

### Example 44: Synthetic route of compound 48

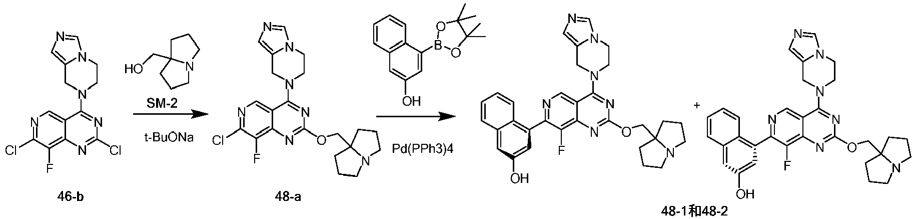

### Synthesis of compound 48-a

Toluene (10 mL) and tetrahydrofuran (30 mL) were added to a reaction flask containing **46-b** (0.55 g, 1.64 mmol). The mixture was stirred at 0 °C for 10 minutes, then was added **SM-2** (42 mg, 0.30 mmol) and sodium tert-butoxide (40 mg, 0.41 mmol). The mixture was stirred at 0 °C for 1 hour. The reaction mixture was loaded onto a silica gel column for column chromatography (mobile phase: dichloromethane/methanol (amine) = 25/1 to 10/1) to obtain compound **48-a** (140 mg, 100%). LC-MS (ESI): m/z 444.1 (M+H) ⁺.

### Synthesis of compounds 48-1 and 48-2

Dioxane (10 mL) and water (1 mL) were added to a reaction flask containing **48-a** (140 mg, 0.32 mmol). 4-(4,4,5,5-Tetramethyl-1,2,3-dioxaborolan-2-yl) naphthalen-2-ol (128 mg, 0.47 mmol), potassium carbonate (138 mg, 1.00 mmol) and tetrakis(triphenylphosphine)palladium (31 mg, 0.03 mmol) were added to the reaction mixture. The mixture was stirred at 85 °C for 18 hours under argon atmosphere. The reaction mixture was concentrated and the residue was purified by column chromatography (mobile phase: dichloromethane/methanol (amine) = 25/1 to 10/1) to obtain a mixture. The mixture was then subjected to pre-HPLC to obtain compounds **48-1** (9 mg, 4%, TFA salt) and **48-2** (6.8 mg, 4%, TFA salt). LC-MS (ESI): m/z 552.0 (M+H) ⁺. **48-1:** ¹H NMR (400MHz, MeOD-*d₄*): *δ*9.06 (1H, s), 8.98 (1H, s), 7.93 (1H, d, *J* = 8.0Hz), 7.65 (1H, d, *J* = 8.0Hz), 7.31-7.60 (6H, m), 5.34 (2H, s), 4.53-4.70 (2H, m), 4.34-4.46 (2H, m), 4.42 (2H, s), 3.60-3.73 (2H, m), 3.30-3.43 (2H, m), 2.10-2.43 (8H, m). **48-2:** ¹H NMR (400MHz, MeOD-*d₄*): δ9.04 (1H, s), 7.90 (1H, d, *J* = 8.0Hz), 7.72 (1H, s), 7.70 (1H, d, *J* = 10.8Hz), 7.62 (1H, s), 7.52 (1H, t, *J* = 8.0Hz), 7.40 (1H, t, *J* = 8.0Hz), 7.34 (1H, s), 6.90 (1H, s), 5.26 (2H, s), 4.38-4.50 (2H, m), 4.30-4.36 (2H, m), 2.90-3.03 (5H, m), 2.20-2.33 (6H, m), 1.65-1.76 (2H, m).

### Example 45: Synthetic route of compound 49

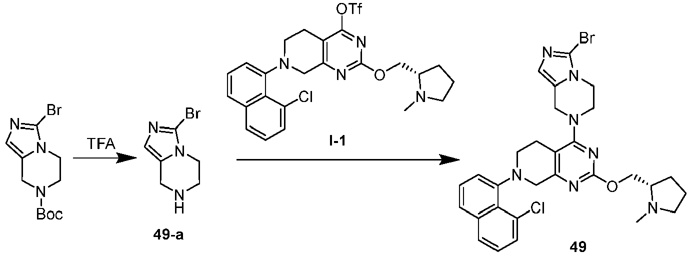

### Synthesis of compound 49-a

To a solution of *tert*-butyl 3-bromo-5,6-dihydroimidazo[1,5-*α*]pyrazine-7(8*H*)-carboxylate (30 mg, 0.1 mmol) in dichloromethane (5 mL) was added trifluoroacetic acid (1 mL) at room temperature. The mixture was stirred at room temperature overnight and concentrated by rotary evaporation to obtain a crude product of compound **49-a** (20 mg) as a white solid, which was directly used in the next step without purification. LC-MS (ESI): m/z 202.1(M+H)⁺.

### Synthesis of compound 49

**I-1** (30 mg, 0.054 mmol), **49-a** (16 mg, 0.08 mmol), *N*, *N*-diisopropylethylamine (21 mg, 0.162 mmol) and DMF (3 mL) were added to a reaction flask. The mixture was stirred at room temperature for 1 hour. After the reaction was completed, water was added and the mixture was extracted with ethyl acetate. The organic phase was dried over anhydrous Na₂SO₄, filtered, and the filtrate was concentrated by rotary evaporation. The crude product was purified by pre-HPLC to obtain compound **49** (7.3 mg, 22%) as a white solid. LC-MS (ESI): m/z 608.1(M+H) ⁺; ¹H NMR (400 MHz, CDCl₃): δ 7.77(1H, dd, *J*=8.4Hz, 0.8Hz), 7.63(1H, dd, *J*=8Hz, 0.4Hz), 7.54(1H, dd, *J*=7.6Hz, 1.2Hz), 7.46(1H, t, *J*=8Hz), 7.35(1H, t, *J*=8Hz), 7.23(1H, d, *J*=6.8Hz), 6.88(1H, s), 4.75(1H, dd, *J*=16Hz, 4.8Hz), 4.66(1H, d, *J*=15.6Hz), 4.48-4.38 (2H, m), 4.23-4.00 (4H, m), 3.87(1H, d, *J*=18Hz), 3.77-3.67 (1H, m), 3.63-3.55 (1H, m), 3.25-3.09 (3H, m), 2.77-2.65 (1H, m), 2.64-2.57 (1H, m), 2.51 (3H, s), 2.37-2.25 (1H, m), 2.14-2.02 (1H, m), 1.88-1.76 (3H, m).

### Example 46: Synthetic route of compound 50

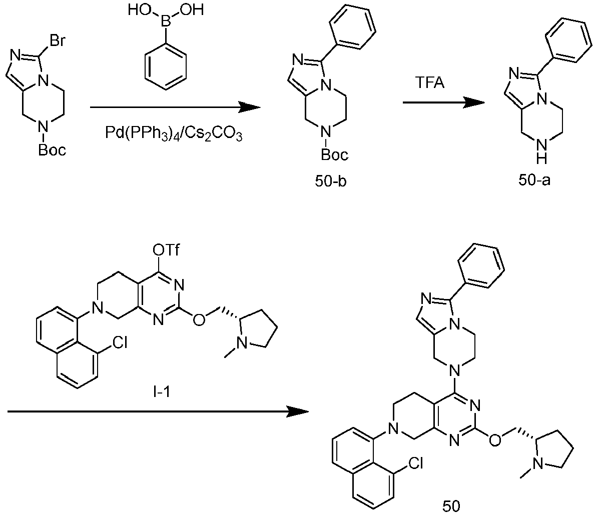

### Synthesis of compound 50-b

*Tert*-butyl 3-bromo-5,6-dihydroimidazo[1,5-*α*]pyrazine-7(8*H*)-carboxylate (100 mg, 0.33 mmol), phenylboronic acid (81 mg, 0.66 mmol), tetrakis(triphenylphosphine)palladium (19 mg, 0.0165 mmol), cesium carbonate (215 mg, 0.66 mmol), 1,4-dioxane (15 mL) and water (3 mL) were added to a reaction flask. The mixture was degassed and purged with nitrogen and stirred at 90 °C overnight. The next day, the reaction mixture was cooled to room temperature and diluted with ethyl acetate and water. The organic phase was separated, dried over anhydrous Na₂SO₄, filtered, and the filtrate was concentrated by rotary evaporation to obtain a crude product of compound **50-b** (105 mg) as a yellow solid. LC-MS (ESI): m/z 300.1 (M+H) ⁺.

### Synthesis of compound 50-a

To a solution of **50-b** (105 mg, 0.42 mmol) in dichloromethane (8 mL) was added trifluoroacetic acid (2 mL) at room temperature. The mixture was stirred at room temperature overnight and concentrated by rotary evaporation to obtain a crude product of compound **50-a** (110 mg) as a yellow solid, which was directly used in the next step. LC-MS (ESI): m/z 200.1(M+H) ⁺.

### Synthesis of compound 50

**I-1** (30 mg, 0.054 mmol), **50-a** (21 mg, 0.108 mmol), *N*, *N*-diisopropylethylamine (35 mg, 0.27 mmol) and DMF (3 mL) were added to a reaction flask. The mixture was stirred at room temperature for 1 hour. After the reaction was completed, water was added and the mixture was extracted with ethyl acetate. The organic phase was dried over anhydrous Na₂SO₄, filtered, and the filtrate was concentrated by rotary evaporation. The crude product was purified by pre-HPLC to obtain compound **50** (3 mg, 9%) as a white solid. LC-MS (ESI): m/z 606.3 (M+H) ⁺; ¹H NMR (400 MHz, CDCl₃): δ 7.77(1H, dd, *J*=8Hz, 0.8Hz), 7.72-7.67 (2H, m), 7.63(1H, d, *J*=7.6Hz), 7.54(1H, dd, *J*=7.6Hz, 1.2Hz), 7.51-7.40 (4H, m), 7.35(1H, t, *J*=7.6Hz), 7.25(1H, d, *J*=7.6Hz), 7.02(1H, s), 4.93-4.81 (2H, m), 4.50-4.37 (3H, m), 4.31-4.15 (2H, m), 4.14-4.06 (1H, m), 3.88(1H, d, J=17.6Hz), 3.75-3.65 (1H, m), 3.62-3.55 (1H, m), 3.29-3.09 (3H, m), 2.77-2.61 (2H, m), 2.51(3H, s), 2.36-2.27 (1H, m), 2.14-2.01 (1H, m), 1.87-1.74 (3H, m).

### Example 47: Synthetic route of compound 51

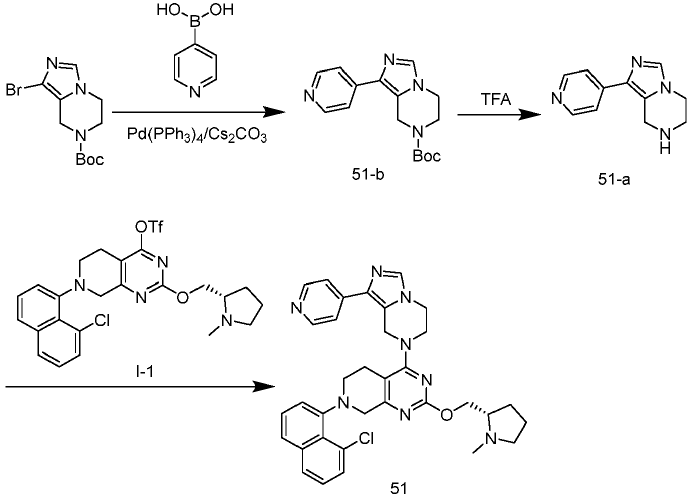

### Synthesis of compound 51-b

Tert-butyl 1-bromo-5,6-dihydroimidazo[1,5-α]pyrazine-7(8*H*)-carboxylate (200 mg, 0.66 mmol), pyridin-4-ylboronic acid (163 mg, 1.32 mmol), tetrakis(triphenylphosphine)palladium (38 mg, 0.033 mmol), cesium carbonate (430 mg, 1.32 mmol), 1,4-dioxane (15 mL) and water (3 mL) were added to a reaction flask. The mixture was degassed and purged with nitrogen and stirred at 90 °C overnight. The next day, the reaction mixture was cooled to room temperature and diluted with ethyl acetate and water. The organic phase was separated, dried over anhydrous Na₂SO₄, filtered, and the filtrate was concentrated by rotary evaporation. The crude product was purified by pre-HPLC to obtain compound **51-b** (35 mg, 18%) as a white solid. LC-MS (ESI): m/z 301.1 (M+H) ⁺.

### Synthesis of compound 51-a

To a solution of **51-b** (35 mg, 0.12 mmol) in dichloromethane (5 mL) was added trifluoroacetic acid (1 mL) at room temperature. The mixture was stirred at room temperature overnight and concentrated by rotary evaporation to obtain a crude product of compound **51-a** (25 mg) as a white solid, which was directly used in the next step. LC-MS (ESI): m/z 201.1(M+H) ⁺.

### Synthesis of compound 51

**I-1** (30 mg, 0.054 mmol), **51-a** (22 mg, 0.108 mmol), N, N-diisopropylethylamine (35 mg, 0.27 mmol) and DMF (3 mL) were added to a reaction flask. The mixture was stirred at room temperature for 1 hour. Upon completion, water was added and the mixture was extracted with ethyl acetate. The organic phase was dried over anhydrous Na₂SO₄, filtered, and the filtrate was concentrated by rotary evaporation. The crude product was purified by pre-HPLC to obtain compound **51** (10.4 mg, 32%) as a white solid. LC-MS (ESI): m/z 607.2 (M+H) ⁺; ¹H NMR (400 MHz, CDCl₃): δ 8.63(2H, dd, *J*=4.8Hz, 1.2Hz), 7.77(1H, dd, *J*=8Hz, 0.8Hz), 7.66-7.60 (2H, m), 7.59- 7.50 (3H, m), 7.48-7.42 (1H, m), 7.35(1H, t, *J*=8Hz), 7.25(1H, dd, *J*=7.6Hz, 1.2Hz), 5.10-4.93 (2H, m), 4.50-4.31 (3H, m), 4.29-4.09 (3H, m), 3.93-3.84 (1H, m), 3.83-3.73 (1H, m), 3.64-3.57 (1H, m), 3.53-3.38 (1H, m), 3.31-3.08 (3H, m), 2.89-2.70 (1H, m), 2.67-2.59 (1H, m), 2.53(3H, s), 2.40-2.27 (1H, m), 2.18-2.00 (3H, m).

### Example 48: Synthetic route of compound 52

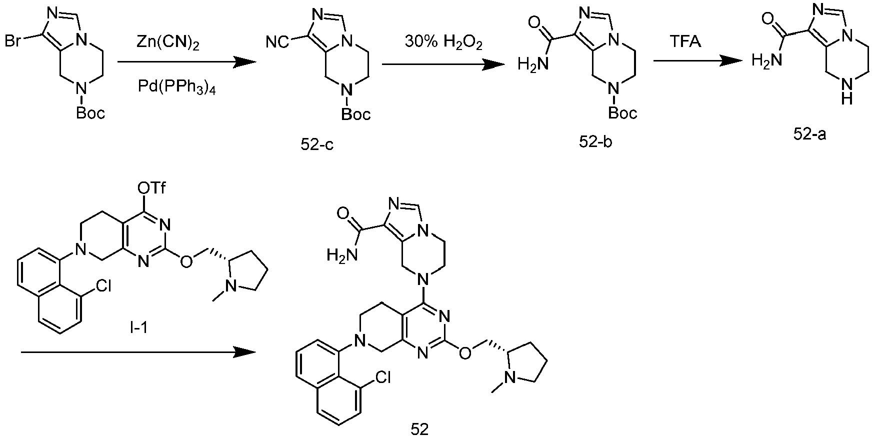

### Synthesis of compound 52-c

*Tert*-butyl 1-bromo-5,6-dihydroimidazo[1,5-*α*]pyrazine-7(8*H*)-carboxylate (400 mg, 1.32 mmol), zinc cyanide (311 mg, 2.65 mmol), tetrakis(triphenylphosphine)palladium (76 mg, 0.066 mmol), and DMF (3 mL) were added to a 10-mL microwave tube. The microwave tube was degassed and purged with nitrogen, and was stirred at 120 °C for 3 hours in a microwave condition. After that, the reaction mixture was cooled to room temperature, and diluted with ethyl acetate and water. The organic phase was separated, dried over anhydrous Na₂SO₄, filtered and concentrated by rotary evaporation. The crude product was purified by column chromatography (mobile phase: dichloromethane: methanol = 10:1/dichloromethane, 0-100%) to obtain compound **52-c** (200 mg, 61%) as a yellow solid. LC-MS (ESI): m/z 249.1 (M+H) ⁺.

### Synthesis of compound 52-b

To a solution of **52-c** (0.2 g, 0.8 mmol) in methanol (5 mL) were added 30% hydrogen peroxide (1 mL) and 3 M aqueous sodium hydroxide (1.5 mL) at room temperature. The mixture was stirred at room temperature for 1 hour, and concentrated by rotary evaporation. Aqueous sodium sulfite was added. The mixture was extracted with ethyl acetate, and the organic phase was dried over anhydrous Na₂SO₄, filtered, and the filtrate was concentrated by rotary evaporation. A small amount of ethyl acetate was added to the concentrate, and an insoluble solid was observed. The mixture was filtered to obtain a filter cake of compound **52-b** (42 mg, 20%) as a white solid. LC-MS (ESI): m/z 267.1(M+H) ⁺.

### Synthesis of compound 52-a

To a solution of **52-b** (42 mg, 0.16 mmol) in dichloromethane (5 mL) was added trifluoroacetic acid (1 mL) at room temperature. The mixture was stirred at room temperature overnight and concentrated by rotary evaporation to obtain a crude product of compound **52-a** (26 mg) as a white solid, which was directly used in the next step. LC-MS (ESI): m/z 167.1(M+H) ⁺.

### Synthesis of compound 52

**I-1** (40 mg, 0.072 mmol), **52-a** (24 mg, 0.14 mmol), *N*, *N*-diisopropylethylamine (47 mg, 0.36 mmol) and DMF (3 mL) were added to a reaction flask. The mixture was stirred at room temperature for 1 hour. Upon completion, water was added and the mixture was extracted with ethyl acetate. The organic phase was dried over anhydrous Na₂SO₄, filtered, and the filtrate was concentrated by rotary evaporation. The crude product was purified by pre-HPLC to obtain compound **52** (3.2 mg, 8%) as a white solid. LC-MS (ESI): m/z 573.3(M+H) ⁺; ¹H NMR (400 MHz, CDCl₃): δ 7.76(1H, dd, J=8Hz, 0.8Hz), 7.61(1H, d, *J*=7.6Hz), 7.54-7.50 (1H, m), 7.47-7.42 (2H, m), 7.34(1H, t, *J*=8Hz), 7.22(1H, d, *J*=7.6Hz), 6.86(1H, s), 5.29(1H, s), 5.21(1H, dd, J=17.6Hz, 1.2Hz), 5.07(1H, dd, *J*=17.6Hz, 1.2Hz), 4.55-4.35 (3H, m), 4.31-4.16 (3H, m), 3.87-3.74 (2H, m), 3.64-3.56 (1H, m), 3.38-3.21 (2H, m), 3.20-3.11(1H, m), 2.92-2.82 (1H, m), 2.74-2.63 (1H, m), 2.59(3H, s), 2.48- 2.38 (1H, m), 2.22- 1.93 (4H, m).

### Example 49: Synthetic route of compound 53

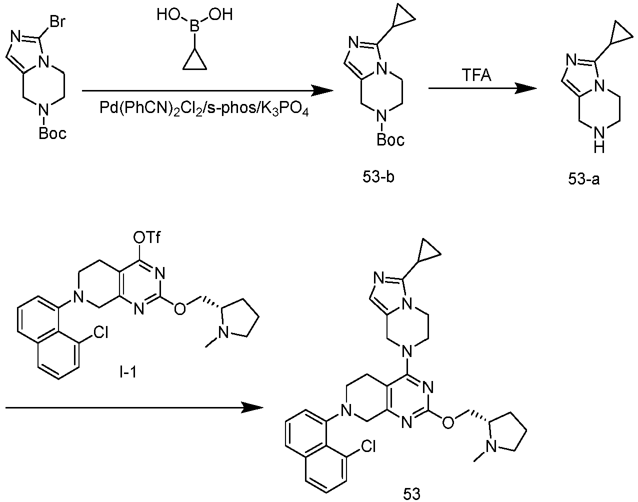

### Synthesis of compound 53-b

*Tert*-butyl 3-bromo-5,6-dihydroimidazo[1,5-*α*]pyrazine-7(8*H*)-carboxylate (200 mg, 0.66 mmol), cyclopropylboronic acid (114 mg, 1.32 mmol), bis(cyanophenyl)palladium dichloride (25 mg, 0.066 mmol), 2-dicyclohexylphosphino-2',6'-dimethoxy-1,1'-biphenyl (27 mg, 0.066 mmol), potassium phosphate (420 mg, 1.98 mmol) and toluene (10 mL) were added to a reaction flask. The mixture was degassed and purged with nitrogen and stirred at 120 °C overnight. The next day, the reaction mixture was cooled to room temperature and diluted with ethyl acetate and water. The organic phase was separated, dried over anhydrous Na₂SO₄, filtered, concentrated by rotary evaporation, and the residue was purified by column chromatography (mobile phase: dichloromethane: methanol = 10:1/dichloromethane, 0-100%) to obtain compound **53-b** (167 mg, 96%) as a yellow solid. LC-MS (ESI): m/z 264.1 (M+H) ⁺.

### Synthesis of compound 53-a

To a solution of **53-b** (167 mg, 0.63 mmol) in dichloromethane (5 mL) was added trifluoroacetic acid (1 mL) at room temperature. The mixture was stirred at room temperature overnight and concentrated by rotary evaporation to obtain a crude product of compound **53-a** (105 mg) as a yellow solid, which was directly used in the next step. LC-MS (ESI): m/z 164.1(M+H) ⁺.

### Synthesis of compound 53

**I-1** (35 mg, 0.063 mmol), **53-a** (21 mg, 0.126 mmol), *N*, *N*-diisopropylethylamine (41 mg, 0.315 mmol) and DMF (3 mL) were added to a reaction flask. The mixture was stirred at room temperature for 1 hour. Upon completion, water was added and the mixture was extracted with ethyl acetate. The organic phase was dried over anhydrous Na₂SO₄, filtered, and the filtrate was concentrated by rotary evaporation. The crude product was purified by pre-HPLC to obtain compound **53** (12.5 mg, 35%) as a white solid. LC-MS (ESI): m/z 570.3(M+H) ⁺; ¹H NMR (400 MHz, CDCl₃): *δ* 7.76(1H, d, *J*=7.6Hz), 7.63(1H, d, *J*=8Hz), 7.53(1H, d, *J*=7.6Hz), 7.46(1H, t, *J*=8Hz), 7.35(1H, t, *J*=8Hz), 7.23(1H, d, *J*=7.6Hz), 6.75(1H, s), 4.80-4.60 (3H, m), 4.48- 4.32 (3H, m), 4.29-4.22 (2H, m), 4.21-4.09 (3H, m), 3.86(1H, dd, *J*=17.6Hz, 3.2Hz), 3.81-3.73 (1H, m), 3.61- 3.54 (1H, m), 3.50-3.41 (1H, m), 3.26-3.06 (3H, m), 2.71(3H, d, *J*=1.2Hz), 2.67-2.55 (2H, m), 2.26-2.15 (1H, m), 1.83-1.71 (1H, m), 1.08-0.93 (4H, m).

### Example 50: Synthetic route of compound 54

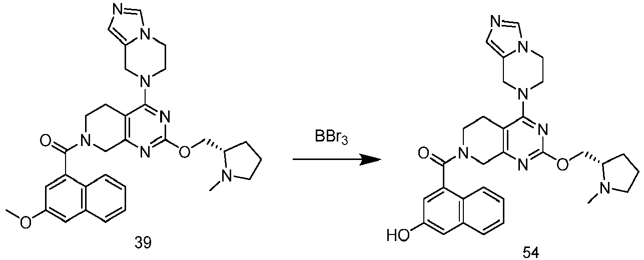

### Synthesis of compound 54

**39** (25 mg, 0.045 mmol), DCM (3 mL) and a solution of boron tribromide in dichloromethane (17%, 1 mL) were added to a reaction flask. The reaction mixture was stirred at room temperature under nitrogen atmosphere for 1 hour and concentrated by rotary evaporation at room temperature. The residue was dissolved in methanol, subjected to pre-HPLC (ammonium bicarbonate), and lyophilized to obtain compound **54** (4 mg, 18%) as a white solid. LC-MS (ESI): m/z = 540.1 (M+H)⁺.

### Example 51: Synthetic route of compound 55

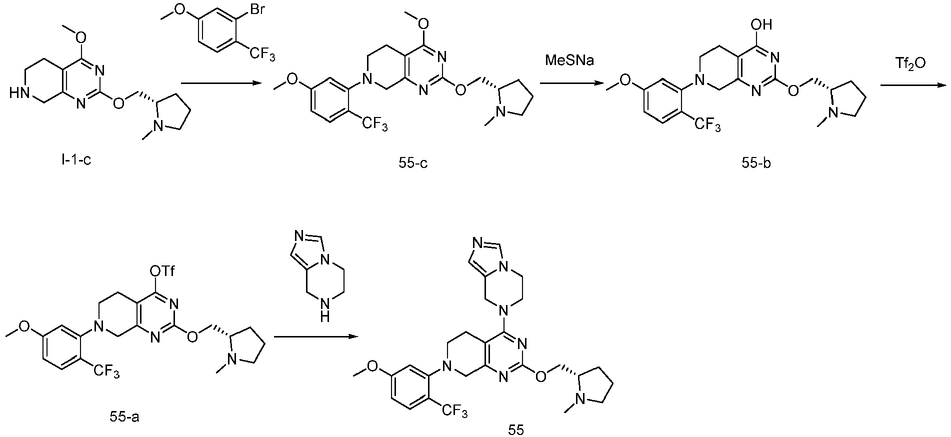

### Synthesis of compound 55-c

**I-1-c** (200 mg, 0.72 mmol), toluene (30 mL), 3-bromo-4-trifluoromethylanisole (274 mg, 1.08 mmol), cesium carbonate (1.18 g, 3.60 mmol), RuPhos (67 mg, 0.14 mmol) and Pd₂dba₃ (66 mg, 0.072 mmol) were added to a reaction flask. The reaction mixture was degassed and purged with nitrogen for three times and stirred at 100 °C overnight. The next day, the reaction mixture was concentrated by rotary evaporation, purified by column chromatography (mobile phase: methanol/dichloromethane = 0/100 to 10/90) to obtain compound **55-c** (102 mg, 31%) as a colorless gum. LC-MS (ESI): m/z = 453.1 (M+H)⁺.

### Synthesis of compound 55-b

**55-c** (102 mg, 0.23 mmol), DMF (10 mL) and sodium thiomethoxide (63 mg, 0.90 mmol) were added to a reaction flask. The mixture was stirred at 60 °C under nitrogen atmosphere for 1 hour before sodium thiomethoxide (150 mg, 2.14 mmol) was added. The reaction mixture was stirred for another 1 hour and cooled to room temperature, and the reaction was quenched with water. The reaction mixture was adjusted to pH 6 with 1 N hydrochloric acid and extracted with dichloromethane/methanol = 10/1 (30 mL × 3). The organic phase was concentrated by rotary evaporation and the residue was purified by column chromatography (mobile phase: methanol/dichloromethane = 0/100 to 10/90) to obtain compound **55-b** (64 mg, 65%) as a pale brown solid. LC-MS (ESI): m/z = 439.1 (M+H)⁺;

### Synthesis of compound 55-a

**55-b** (64 mg, 0.15 mmol), dichloromethane (20 mL) and triethylamine (61 µL, 0.44 mmol) were added to a reaction flask. In a dry ice bath, trifluoromethanesulfonic anhydride (62 mg, 0.22 mmol) was added dropwise. The reaction mixture was stirred at this temperature for 30 minutes, and the reaction was quenched with saturated sodium bicarbonate solution. The mixture was extracted with dichloromethane (30 mL × 2), dried over anhydrous sodium sulfate, filtered, concentrated by rotary evaporation, and the residue was purified by column chromatography (mobile phase: methanol/dichloromethane = 0/100 to 5/95) to obtain compound **55-a** (47 mg, 56%) as a pale brown solid. LC-MS (ESI): m/z = 571.2 (M+H)⁺.

### Synthesis of compound 55

**55-a** (47 mg, 0.082 mmol), DMF (5 mL), 5,6,7,8-tetrahydroimidazo[1,5-*α*]pyrazine (20 mg, 0.16 mmol) and DIPEA (68 µL, 0.41 mmol) were added to a reaction flask. The reaction mixture was stirred at room temperature under nitrogen atmosphere for 1 hour, and the reaction was quenched with saturated sodium bicarbonate solution. The mixture was extracted with ethyl acetate (30 mL × 3) and washed with brine (20 mL × 3). The organic phase was concentrated by rotary evaporation, and the residue was purified by column chromatography (mobile phase: ammonia in methanol/dichloromethane = 0/100 to 10/90) to obtain compound **55** (41 mg, 92%) as a pale brown solid. LC-MS (ESI): m/z = 544.2 (M+H)⁺.

### Example 52: Synthesis of comparative compound 1'

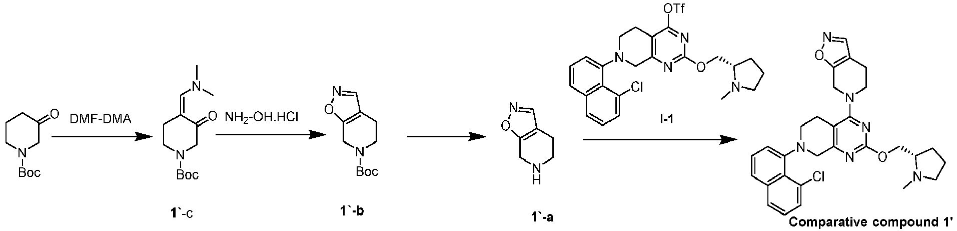

### Synthesis of compound 1'-c

*N*-*tert*-butoxycarbonyl-3-piperidone (2.5 g, 12.56 mmol) and DMF-DMA (21 mL) were added to a reaction flask. The reaction mixture was stirred at 100 °C for 2 hours, and the reaction mixture was concentrated by rotary evaporation and the residue was purified by column chromatography (mobile phase: methanol/dichloromethane = 0/100 to 5/95) to obtain compound **1'-c** (2.2 g, 69%) as a yellow oil. LC-MS (ESI): m/z = 255.2 (M+H)⁺.

### Synthesis of compound 1'-b

Compound **1'-c** (2.1 g, 8.27 mmol), methanol (100 mL) and hydroxylamine hydrochloride (0.71 g, 10 mmol) were added to a reaction flask. The reaction mixture was stirred at room temperature under nitrogen atmosphere overnight before 1 g of hydroxylamine hydrochloride was added. The reaction mixture was continuously stirred overnight, and the reaction was quenched with saturated sodium bicarbonate solution. The mixture was extracted with ethyl acetate (50 mL × 2), washed with brine, dried over anhydrous sodium sulfate, concentrated by rotary evaporation, and the residue was purified by column chromatography (mobile phase: ethyl acetate/petroleum ether = 0/100 to 30/70) to obtain a mixture of **1'-b** (63 mg, 3%) as a white solid. LC-MS (ESI): m/z = 225.1 (M+H)⁺; ¹H NMR (400 MHz, CDCl₃): *δ* 8.18(1H, s), 4.70(2H, s), 3.72-3.54(2H, m), 2.74-2.60(2H, m), 1.48(9H, s).

### Synthesis of compound 1'-a

**1'-b** (60 mg, 0.27 mmol), dichloromethane (10 mL) and trifluoroacetic acid (1 mL) were added to a reaction flask. The reaction mixture was stirred at room temperature for 1 hour under nitrogen atmosphere. Dichloromethane and trifluoroacetic acid were removed by rotary evaporation. 10 mL of dichloromethane and 1 mL of triethylamine were added and the mixture was concentrated by rotary evaporation to obtain a mixture of compound **1'-a** (crude) as a pale brown oil, which was used in the next step without purification. LC-MS (ESI): m/z = 125.1 (M+H)⁺.

### Synthesis of comparative compound 1'

**I-1** (20 mg, 0.036 mmol), DMF (2 mL), **1'-a** (crude) and DIPEA (0.5 mL) were added to a reaction flask. The reaction mixture was stirred at room temperature under nitrogen atmosphere for 1 hour. The reaction mixture was directly subjected to pre-HPLC (ammonium bicarbonate) and lyophilized to obtain comparative compound **1'** (1.2 mg, 6%) as a white solid. LC-MS (ESI): m/z = 531.0 (M+H)⁺.

### Example 53: Synthetic route of compound 42

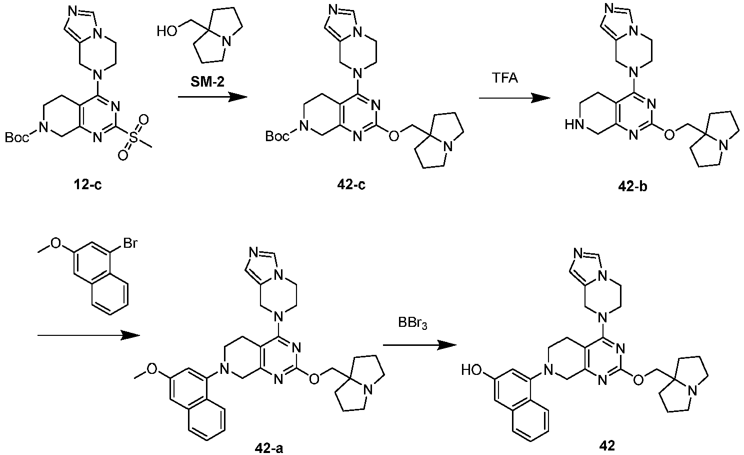

### Synthesis of compound 42-c

**12-c** (1.20 g, 2.76 mmol), **SM-2** (507 mg, 3.59 mmol) and THF (10 mL) were added to a reaction flask. The mixture was degassed and purged with nitrogen and cooled to 0 °C, then was added sodium *tert*-butoxide (530 mg, 5.52 mmol). The reaction was stirred at room temperature for 4 hours. The reaction mixture was concentrated and the residue was purified by column chromatography (mobile phase: DCM/MeOH = 10/0 to 10/1) to obtain compound **42-c** (800 mg, 58%). LC-MS (ESI): m/z 496.1 (M+H) ⁺.

### Synthesis of compound 42-b

**42-c** (800 mg, 1.61 mmol), DCM (4 mL), and trifluoroacetic acid (2 mL) were added to a reaction flask. The reaction was stirred at room temperature for 4 hours. The reaction mixture was concentrated, adjusted to a basic pH by adding aqueous sodium bicarbonate and extracted with DCM/MeOH (10/2). The organic phases were combined, washed with brine, dried over anhydrous Na₂SO₄, filtered, evaporated and the residue was purified by column chromatography (mobile phase: DCM/MeOH = 10/0 to 10/1) to obtain compound **42-b** (730 mg, 97%). LC-MS (ESI): m/z 396.1 (M+H) ⁺.

### Synthesis of compound 42-a

**42-b** (400 mg, 1.00 mmol), 1-bromo-3-methoxynaphthalene (284 mg, 1.20 mmol), toluene (15 mL), cesium carbonate (978 mg, 3.00 mmol), Pd₂(dba)₃ (92 mg, 0.10 mmol) and RuPhos (94 mg, 0.20 mmol) were added to a reaction flask. The reaction mixture was degassed and purged with nitrogen and reacted at 100 °C overnight. The reaction mixture was concentrated and the residue was purified by column chromatography (mobile phase: DCM/MeOH = 10/0 to 10/1) to obtain compound **42-a** (200 mg, 36%). LC-MS (ESI): m/z 552.3 (M+H) ⁺.

### Synthesis of compound 42

**42-a** (100 mg, 0.18 mmol) and DCM (3 mL) were added to a reaction flask. Boron tribromide (1 mL) was added dropwise. The reaction was stirred at room temperature overnight. The reaction mixture was concentrated. The residue was dissolved in methanol. The mixture was purified by pre-HPLC to obtain compound **42** (14 mg, 14%) as a yellow solid. LC-MS (ESI): m/z 538.2 (M+H) ⁺; ¹H NMR (400MHz, CDCl₃): δ 8.01 (1H, d, *J* = 8.4Hz), 7.59 (1H, d, *J* = 7.6Hz), 7.47 (1H, s), 7.34 (1H, t, *J* = 6.8Hz), 7.25 (1H, t, *J* = 7.2Hz), 6.91-6.87 (2H, m), 6.76 (1H, s), 4.69 (2H, s), 4.34 (2H, s), 4.24-4.13 (4H, m), 3.81 (2H, s),3.54 (2H, t, *J* = 4.0Hz), 2.85-2.73 (4H, m), 2.26-2.19 (2H, m), 2.10-1.94 (6H, m), 1.84-1.78 (2H, m).

### Example 54: Synthetic route of compound 43

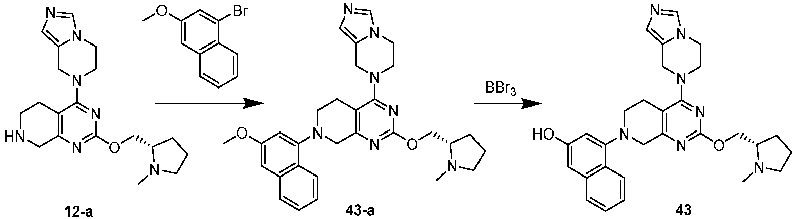

### Synthesis of compound 43-a

**12-a** (1.00 g, 2.70 mmol), 1-bromo-3-methoxynaphthalene (770 mg, 3.25 mmol), toluene (20 mL), cesium carbonate (2.64 g, 8.10 mmol), Pd₂(dba)₃ (247 mg, 0.27 mmol) and RuPhos (255 mg, 0.54 mmol) were added to a reaction flask at room temperature. The reaction mixture was degassed and purged with nitrogen and reacted at 100 °C overnight. The reaction mixture was concentrated and the residue was purified by column chromatography (mobile phase: DCM/MeOH = 10/0 to 10/1) to obtain compound **43-a** (520 mg, 37%). LC-MS (ESI): m/z 526.3 (M+H) ⁺.

### Synthesis of compound 43

At room temperature, **43-a** (50 mg, 0.09 mmol) and DCM (3 mL) were added to a reaction flask, and boron tribromide (1 mL) was added dropwise. The reaction was stirred at room temperature overnight. The next day, the reaction mixture was concentrated. The residue was dissolved in methanol. The mixture was purified by pre-HPLC to obtain compound **43** (6 mg, 12%) as a yellow solid. LC-MS (ESI): m/z 512.3 (M+H) ⁺.

### Example 55: Synthetic route of compound 44

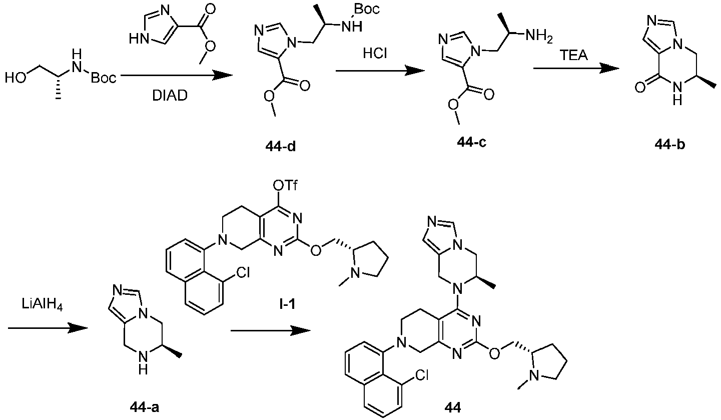

### Synthesis of compound 44-d

*Tert*-butyl (*R*)-(1-hydroxypropan-2-yl) carbamate (1.75 g, 10 mmol), methyl imidazole-4-carboxylate (1.51 g, 12 mmol) and triphenylphosphine (4.45 g, 17 mmol) were added to a three-necked flask. The mixture was degassed and purged with nitrogen for three times, then was added tetrahydrofuran (100 mL) and DIAD (3.34 mL, 17 mmol) dropwise in a dry ice bath. After the addition, the mixture was warmed to room temperature and stirred overnight. The next day, the reaction mixture was concentrated by rotary evaporation. Water was added. The mixture was adjusted to pH 1 with 1 M hydrochloric acid, and extracted twice with ethyl acetate. An excess amount of sodium bicarbonate was added to the aqueous phase, and ethyl acetate was added for extraction (80 mL × 2). The organic phase was washed with brine, dried over anhydrous sodium sulfate, concentrated by rotary evaporation, and the residue was purified by column chromatography (mobile phase: ethyl acetate/dichloromethane = 0/100 to 100/0) to obtain compound **44-d** (1.48 g, 52%) as a white solid. LC-MS (ESI): m/z = 284.2 (M+H)⁺.

### Synthesis of compound 44-c

**44-d** (1.48 g, 5.23 mmol) and hydrochloric acid in methanol (4 M, 20 mL) were added to a reaction flask. The reaction mixture was stirred at room temperature overnight under nitrogen atmosphere. The next day, the reaction mixture was concentrated by rotary evaporation and treated with methanol to obtain compound **44-c** (crude) as a colorless gum. LC-MS (ESI): m/z = 184.1 (M+H)⁺.

### Synthesis of compound 44-b

**44-c** (crude), methanol (30 mL) and triethylamine (6 mL) were added to a reaction flask. The mixture was stirred at 50 °C overnight under nitrogen atmosphere. The next day, the reaction mixture was concentrated by rotary evaporation and the residue was purified by column chromatography (mobile phase: ammonia in methanol/dichloromethane = 0/100 to 5/95) to obtain compound **44-b** (668 mg, 85% in 2 steps) as a white solid. LC-MS (ESI): m/z = 152.1 (M+H)⁺.

### Synthesis of compound 44-a

**44-b** (640 mg, 4.24 mmol), tetrahydrofuran (40 mL) and 2.5 M LiAlH₄ (3.4 mL, 8.48 mmol) were added to a reaction flask. The mixture was heated at reflux at 80 °C overnight under nitrogen atmosphere. The next day, cooled in an ice-cold water bath, the reaction mixture was added saturated aqueous sodium sulfate until no bubbles were formed. The mixture was dried over anhydrous sodium sulfate and filtered. The filtrate was washed twice with tetrahydrofuran, concentrated by rotary evaporation and the residue was purified by column chromatography (mobile phase: ammonia in methanol/dichloromethane = 0/100 to 5/95) to obtain compound **44-a** (370 mg, 64%) as a white solid. LC-MS (ESI): m/z = 138.1 (M+H)⁺.

### Synthesis of compound 44

**I-1** (60 mg, 0.11 mmol), DMF (2 mL), **44-a** (30 mg, 0.22 mmol) and DIPEA (89 µL, 0.54 mmol) were added to a reaction flask. The mixture was stirred at room temperature overnight under nitrogen atmosphere. The reaction mixture was filtered, subjected to pre-HPLC (ammonium bicarbonate), lyophilized and the residue was purified by column chromatography (mobile phase: ammonia in methanol/dichloromethane = 0/100 to 5/95) to obtain compound **44** (7 mg, 12%) as a pale brown solid. LC-MS (ESI): m/z = 544.0 (M+H)⁺.

### Example 56: Synthetic route of compound 45

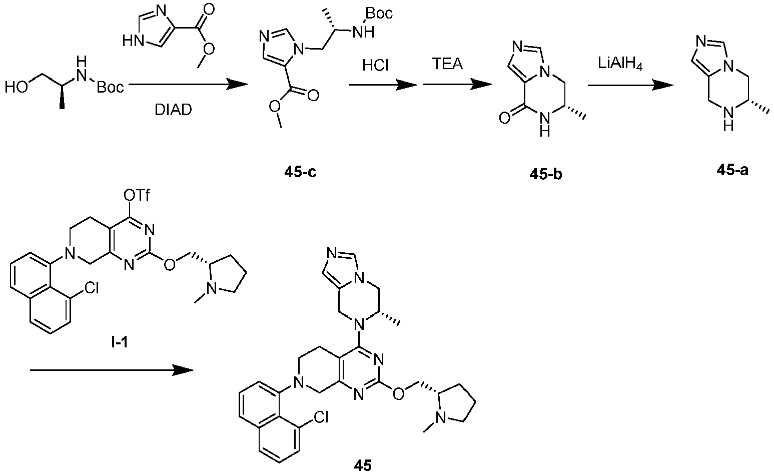

### Synthesis of compound 45-c

*Tert*-butyl (*S*)-(1-hydroxypropan-2-yl)carbamate (605 mg, 3.45 mmol), methyl imidazole-4-carboxylate (535 mg, 4.24 mmol) and triphenylphosphine (1.5 g, 5.76 mmol) were added to a three-necked flask. The mixture was degassed and purged with nitrogen for three times, then in a dry ice bath, was added tetrahydrofuran (40 mL), and DIAD (1.13 mL, 5.76 mmol) dropwise. After the addition, the mixture was warmed to room temperature and stirred overnight. The next day, the reaction mixture was concentrated by rotary evaporation. The crude product was purified by column chromatography (mobile phase: ethyl acetate/dichloromethane = 0/100 to 100/0) to obtain compound **45-c** (500 mg, 51%) as a white solid. LC-MS (ESI): m/z = 284.1 (M+H)⁺.

### Synthesis of compound 45-b

**45-c** (450 mg, 1.59 mmol) and hydrochloric acid in methanol (4 M, 8 mL) were added to a reaction flask. The reaction mixture was stirred at room temperature overnight under nitrogen atmosphere. The next day, the reaction mixture was concentrated by rotary evaporation and treated with methanol. The resulting colorless gum was dissolved in methanol (20 mL), was added triethylamine (2 mL). The mixture was stirred at 50 °C under nitrogen atmosphere overnight. The reaction mixture was concentrated by rotary evaporation and the residue was purified by column chromatography (mobile phase: ammonia in methanol/dichloromethane = 0/100 to 5/95) to obtain compound **45-b** (200 mg, 83%) as a white solid. LC-MS (ESI): m/z = 152.1 (M+H)⁺.

### Synthesis of compound 45-a

**45-b** (100 mg, 0.66 mmol), tetrahydrofuran (10 mL) and 2.5 M LiAlH₄ (0.99 mL, 1.98 mmol) were added to a reaction flask. The mixture was heated at reflux at 80 °C overnight under nitrogen atmosphere. The next day, in an ice-cold water bath, the mixture was added saturated aqueous sodium sulfate until no bubbles were formed. The mixture was dried over anhydrous sodium sulfate, filtered and washed twice with tetrahydrofuran. The filtrate was concentrated by rotary evaporation and the residue was purified by column chromatography (mobile phase: ammonia in methanol/dichloromethane = 0/100 to 5/95) to obtain compound **45-a** (50 mg, 55%) as a white solid. LC-MS (ESI): m/z = 138.2 (M+H)⁺.

### Synthesis of compound 45

**I-1** (60 mg, 0.11 mmol), DMF (2 mL), **45-a** (20 mg, 0.15 mmol) and DIPEA (70 mg, 0.54 mmol) were added to a reaction flask. The reaction mixture was stirred at room temperature overnight under nitrogen atmosphere. The reaction mixture was filtered, subjected to pre-HPLC (ammonium bicarbonate), lyophilized and the residue was purified by column chromatography (mobile phase: ammonia in methanol/dichloromethane = 0/100 to 5/95) to obtain compound **45** (30 mg, 51%) as a pale brown solid. LC-MS (ESI): m/z = 544.3 (M+H)⁺; ¹H NMR (400 MHz, CDCl₃): δ 7.72-7.81 (1H, m), 7.55-7.66 (1H, m), 7.38-7.55 (3H, m), 7.30-7.37 (1H, m), 7.12-7.26 (1H, m), 6.85-6.93 (1H, m), 4.65-5.01 (2H, m), 4.32-4.55 (2H, m), 4.13-4.26 (1H, m), 3.94-4.08 (1H, m), 3.72-3.90 (1H, m), 3.45-3.66 (1H, m), 3.02-3.39 (3H, m), 2.74-3.00 (1H, m), 2.54 (3H, s), 2.45-2.63 (1H, m), 2.26-2.44 (1H, m), 2.02-2.16 (1H, m), 1.59-1.94 (5H, m), 1.29-1.37 (3H, m).

### Example 57: Synthetic route of compound 56

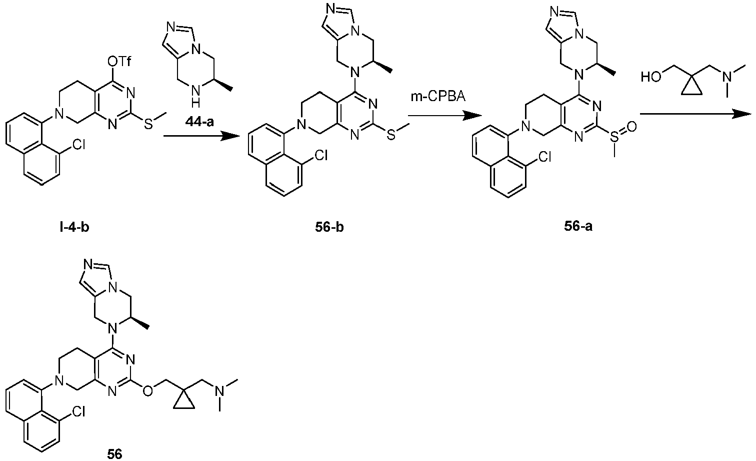

### Synthesis of compound 56-b

**I-4-b** (1300 mg, 2.65 mmol), **44-a** (436 mg, 3.18 mmol), DIPEA (684 mg, 5.30 mmol) and DMF (10 mL) were added to a reaction flask. The mixture was degassed and purged with nitrogen and reacted at 90 °C for 3 hours. The reaction mixture was cooled and was added water and a solid was precipitated. The mixture was filtered and the filter cake was evaporated and the residue was purified by column chromatography (mobile phase: DCM/MeOH = 10/0 to 10/2) to obtain compound **56-b** (300 mg, 24%). LC-MS (ESI): m/z 477.1 (M+H) ⁺.

### Synthesis of compound 56-a

**56-b** (20 mg, 0.04 mmol) and DCM (10 mL) were added to a reaction flask. The mixture was degassed and purged with nitrogen. m-Chloroperoxybenzoic acid (11 mg, 0.06 mmol) was added. The reaction was stirred at room temperature for 1 hour. The reaction was quenched with aqueous sodium bicarbonate. The mixture was extracted with DCM, and the organic phases were combined, dried and concentrated to obtain a crude product compound **56-a** (30 mg). LC-MS (ESI): m/z 493.1 (M+H) ⁺.

### Synthesis of compound 56

The crude product of **56-a** (30 mg, 0.04 mmol), { 1-[(dimethylamino)methyl]cyclopropyl}methanol (16 mg, 0.12 mmol) and DCM (3 mL) were added to a reaction flask. The mixture was degassed and purged with nitrogen, was added sodium *tert-*butoxide (12 mg, 0.12 mmol). The reaction was stirred at room temperature for 1 hour. The reaction was stirred at room temperature for 0.5 hours. The reaction mixture was concentrated and the residue was purified by pre-HPLC (acidic, FA) to obtain compound **56** (6 mg, 27%). LC-MS (ESI): m/z 558.2 (M+H) ⁺; ¹H NMR (400MHz, CD₃OD): *δ*7.85 (1H, d, *J* = 7.2Hz), 7.72-7.64(2H, m), 7.56-7.47(2H, m), 7.41-7.43(2H, m), 6.89(1H, d, *J =* 5.6Hz), 5.20-4.76 (2H, m), 4.58-415 (5H, m), 3.85-3.37 (3H, m), 3.27-3.13 (2H, m), 2.86-2.55 (9H, m), 1.39-1.28 (3H, m), 0.82-0.64 (4H, m).

### Example 58: Synthetic route of compound 57

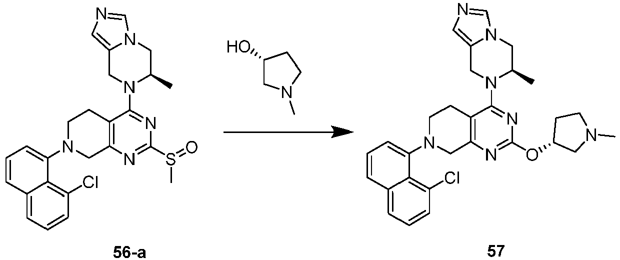

### Synthesis of compound 57

The crude product of **56-a** (30 mg, 0.04 mmol), (*R*)-(-)-1-methyl-3-hydroxypyrrolidine (12 mg, 0.12 mmol) and DCM (3 mL) were added to a reaction flask. The mixture was degassed and purged with nitrogen. Sodium *tert*-butoxide (12 mg, 0.12 mmol) was added. The mixture was stirred at room temperature for 0.5 hours. The reaction mixture was concentrated and the residue was purified by pre-HPLC (acidic, formic acid) to obtain compound **57** (11 mg, 52%). LC-MS (ESI): m/z 530.3 (M+H) ⁺; ¹H NMR (400MHz, CD₃OD): *δ*7.85 (1H, d, *J =* 8.0Hz), 7.71-7.64(2H, m), 7.56-7.47(2H, m), 7.41-7.30(2H, m), 6.90(1H, s), 5.48-5.37 (1H, m), 4.80-4.73 (2H, m), 4.56-4.10 (3H, m), 3.81-3.37 (3H, m), 3.26-2.68 (7H, m), 2.51-2.41 (4H, m), 2.14-2.07 (1H, m), 1.41-1.28(3H, m).

### Example 59: Synthetic route of compound 58

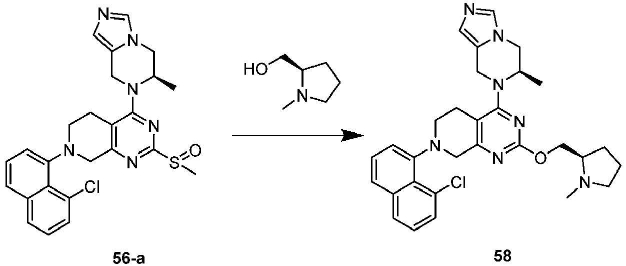

### Synthesis of compound 58

**56-a** (30 mg, 0.04 mmol), *N*-methyl-D-prolinol (14 mg, 0.12 mmol) and DCM (3 mL) were added to a reaction flask. The mixture was degassed and purged with nitrogen, was added sodium tert-butoxide (12 mg, 0.12 mmol). The mixture was stirred at room temperature for 0.5 hours. The reaction mixture was concentrated and the residue was purified by pre-HPLC (acidic, formic acid) to obtain compound **58** (9 mg, 41%). LC-MS (ESI): m/z 544.2 (M+H) ⁺.

### Example 60: Synthetic route of compound 59

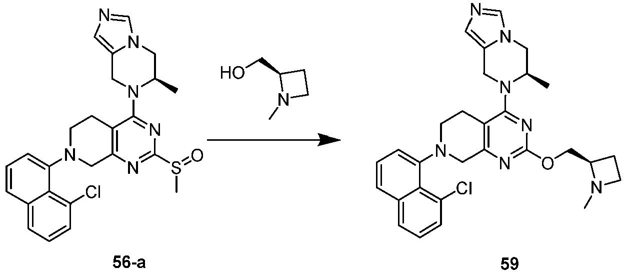

### Synthesis of compound 59

The crude product of **56-a** (30 mg, 0.04 mmol), 1-methyl-2-azetidinemethanol (12 mg, 0.12 mmol) and DCM (3 mL) were added to a reaction flask. The mixture was degassed and purged with nitrogen, was added sodium *tert*-butoxide (12 mg, 0.12 mmol). The mixture was stirred at room temperature for 0.5 hours. The reaction mixture was concentrated and the residue was purified by pre-HPLC (acidic, formic acid) to obtain compound **59** (11 mg, 51%). LC-MS (ESI): m/z 530.3 (M+H) ⁺.

### Example 61: Synthetic route of compound 60

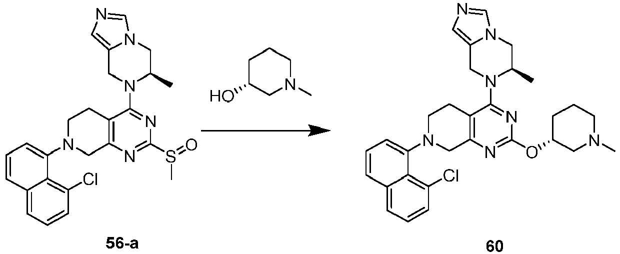

### Synthesis of compound 60

**56-a** (30 mg, 0.04 mmol), (*R*)-1-methyl-3-hydroxypiperidine (14 mg, 0.12 mmol) and DCM (3 mL) were added to a reaction flask. The mixture was degassed and purged with nitrogen, was added sodium *tert*-butoxide (12 mg, 0.12 mmol). The mixture was stirred at room temperature for 0.5 hours. The reaction mixture was concentrated and the residue was purified by pre-HPLC (acidic, formic acid) to obtain compound **60** (12 mg, 55%). LC-MS (ESI): m/z 544.2 (M+H) ⁺.

### Example 62: Synthetic route of compound 61

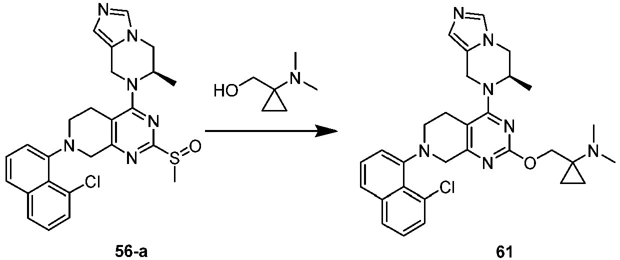

### Synthesis of compound 61

**56-a** (30 mg, 0.04 mmol), [1-(dimethylamino)cyclopropyl]methanol (14 mg, 0.12 mmol) and DCM (3 mL) were added to a reaction flask. The mixture was degassed and purged with nitrogen, was added sodium *tert*-butoxide (12 mg, 0.12 mmol). The mixture was stirred at room temperature for 0.5 hours. The reaction mixture was concentrated and the residue was purified by pre-HPLC (acidic, formic acid) to obtain compound **61** (8 mg, 37%). LC-MS (ESI): m/z 544.3 (M+H) ⁺.

### Example 63: Synthetic route of compound 62

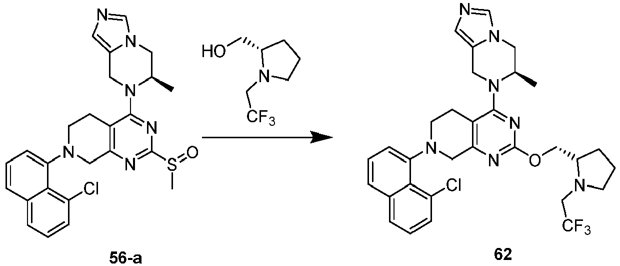

### Synthesis of compound 62

**56-a** (30 mg, 0.04 mmol), *N*-trifluoroethyl-L-prolinol (22 mg, 0.12 mmol) and DCM (3 mL) were added to a reaction flask. The mixture was degassed and purged with nitrogen, was added sodium tert-butoxide (12 mg, 0.12 mmol). The mixture was stirred at room temperature for 0.5 hours. The reaction mixture was concentrated and the residue was purified by pre-HPLC (acidic, formic acid) to obtain compound **62** (13 mg, 53%). LC-MS (ESI): m/z 612.3 (M+H) ⁺; ¹H NMR (400MHz, CD₃OD): *δ*7.84 (1H, d, *J* = 8.4Hz), 7.76 (1H, d, *J* = 7.6Hz), 7.70 (1H, t, *J* = 6.8Hz), 7.55-7.46 (2H, m), 7.43-7.29 (2H, m), 6.94 (1H, d, *J* = 4.4Hz), 5.20-4.76 (2H, m), 4.57-4.15 (5H, m), 4.84-3.35 (4H, m), 3.30-3.11 (4H, m), 2.71-2.55 (2H, m), 2.06-1.69 (4H, m) ,1.40-1.28 (4H,m).

### Example 64: Synthetic route of compound 63

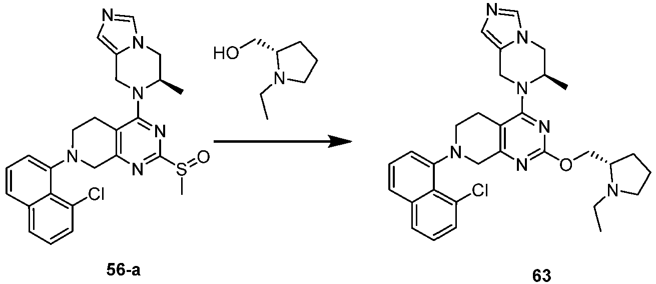

### Synthesis of compound 63

**56-a** (30 mg, 0.04 mmol), *N*-ethyl-L-prolinol (16 mg, 0.12 mmol) and DCM (3 mL) were added to a reaction flask. The mixture was degassed and purged with nitrogen, was added sodium *tert-*butoxide (12 mg, 0.12 mmol). The mixture was stirred at room temperature for 0.5 hours. The reaction mixture was concentrated and the residue was purified by pre-HPLC (acidic, formic acid) to obtain compound 63 (8 mg, 36%). LC-MS (ESI): m/z 558.3 (M+H) ⁺.

### Example 65: Synthetic route of compound 64

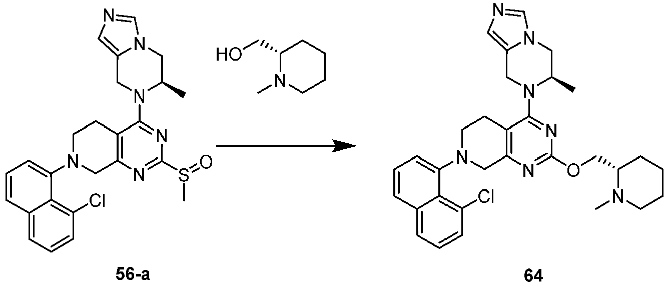

### Synthesis of compound 64

56-a (30 mg, 0.04 mmol), (2*S*)-*N*-methyl-2-piperidinemethanol (12 mg, 0.12 mmol) and DCM (3 mL) were added to a reaction flask. The mixture was degassed and purged with nitrogen, was added sodium tert-butoxide (12 mg, 0.12 mmol). The mixture was stirred at room temperature for 0.5 hours. The reaction mixture was concentrated and the residue was purified by pre-HPLC (acidic, formic acid) to obtain compound **64** (6 mg, 27%). LC-MS (ESI): m/z 558.3 (M+H) ⁺.

### Example 66: Synthetic route of compound 65

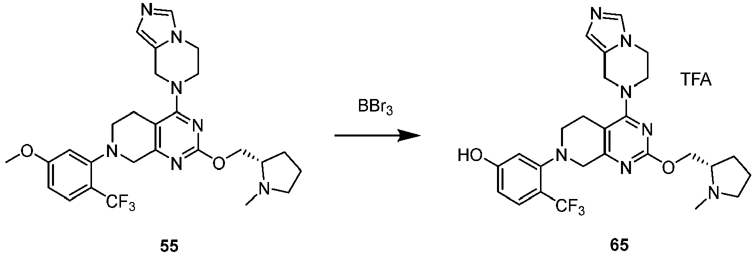

### Synthesis of compound 65

55 (30 mg, 0.055 mmol), DCM (6 mL) and 17% boron tribromide (2 mL) were added to a reaction flask. The reaction mixture was stirred at room temperature under nitrogen atmosphere overnight. The next day, the mixture was concentrated by rotary evaporation at room temperature. The residue was dissolved in methanol, subjected to pre-HPLC (TFA), and lyophilized to obtain compound **65** (15.3 mg, 43%) as an off-white solid. LC-MS (ESI): m/z = 530.3 (M+H)⁺; ¹H NMR (400 MHz, CD₃OD) : *δ* 8.92(1H, d, *J*=1.2Hz), 7.51(1H, d, *J*=8.4Hz), 7.47(1H, d, *J*=0.8Hz), 6.91(1H, d, *J*=2.4Hz), 6.73(1H, dd, *J*=8.4Hz, *J*=2Hz), 4.99(2H, s), 4.76(1H, dd, *J*=12.4Hz, *J*=3.2Hz), 4.58(1H, dd, *J*=12.4Hz, *J*=7.2Hz), 4.52(2H, t, *J*=4.8Hz), 4.13(2H, t, *J*=5.6Hz), 4.02(2H, s), 3.93-3.83(1H, m), 3.79-3.68(1H, m), 3.27-3.20(1H, m), 3.17(2H, t, *J*=5.2Hz), 3.07(3H, s), 2.94(2H, t, *J*=5.2Hz), 2.47-2.32(1H, m), 2.28-1.95(3H, m).

### Example 67: Synthetic route of compound 66

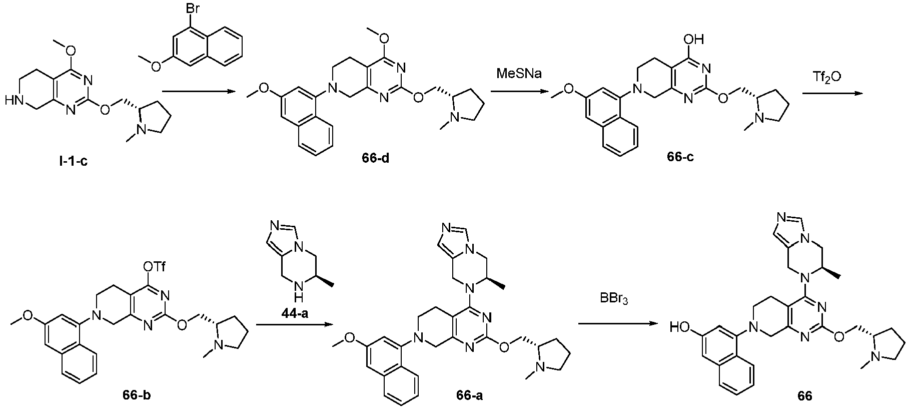

### Synthesis of compound 66-d

**I-1-c** (200 mg, 0.72 mmol), dioxane (30 mL), 1-bromo-3-methoxynaphthalene (255 mg, 1.08 mmol), cesium carbonate (1.18 g, 3.60 mmol), RuPhos (67 mg, 0.14 mmol) and Pd₂(dba)₃ (66 mg, 0.072 mmol) were added to a reaction flask. The reaction mixture was degassed and purged with nitrogen for three times and stirred at 100 °C overnight. The next day, the reaction mixture was concentrated by rotary evaporation and the residue was purified by column chromatography (mobile phase: methanol/dichloromethane = 0/100 to 10/90) to obtain compound **66-d** (116 mg, 37%) as a pale brown solid. LC-MS (ESI): m/z = 435.1 (M+H)⁺.

### Synthesis of compound 66-c

**66-d** (116 mg, 0.27 mmol), DMF (3 mL) and sodium thiomethoxide (200 mg, 2.86 mmol) were added to a reaction flask. The reaction mixture was stirred at 60 °C under nitrogen atmosphere for 2 hours. The reaction mixture was cooled to room temperature, and the reaction was quenched with water. The mixture was adjusted to pH 6 with 1 M hydrochloric acid, and extracted with dichloromethane/methanol = 10/1 (30 mL × 4). The organic phase was concentrated by rotary evaporation and the residue was purified by column chromatography (mobile phase: methanol/dichloromethane = 0/100 to 10/90) to obtain compound **66-c** (80 mg, 71%) as an off-white solid. LC-MS (ESI): m/z = 421.3 (M+H)⁺.

### Synthesis of compound 66-b

**66-c** (80 mg, 0.19 mmol), dichloromethane (20 mL) and triethylamine (79 µL, 0.57 mmol) were added to a reaction flask. In a dry ice bath, trifluoromethanesulfonic anhydride (48 µL, 0.29 mmol) was added dropwise to the above mixture. The reaction mixture was stirred at this temperature for 30 minutes, and the reaction was quenched with saturated sodium bicarbonate solution. The mixture was extracted with dichloromethane (30 mL × 2), dried over anhydrous sodium sulfate, filtered, concentrated by rotary evaporation, and the residue was purified by column chromatography (mobile phase: methanol/dichloromethane = 0/100 to 10/90) to obtain compound **66-b** (84 mg, 80%) as a white solid. LC-MS (ESI): m/z = 552.8 (M+H)⁺.

### Synthesis of compound 66-a

**66-b** (84 mg, 0.15 mmol), DMF (6 mL), **44-a** (42 mg, 0.30 mmol) and DIPEA (125 µL, 0.76 mmol) were added to a reaction flask. The reaction mixture was stirred at room temperature under nitrogen atmosphere overnight. The next day, the reaction was quenched with saturated sodium bicarbonate solution. The mixture was extracted with ethyl acetate (30 mL × 2) and washed with brine (20 mL × 3). The organic phase was concentrated by rotary evaporation, and the residue was purified by column chromatography (mobile phase: ammonia in methanol/dichloromethane = 0/100 to 6/94) to obtain compound **66-a** (45 mg, 55%) as a pale brown solid. LC-MS (ESI): m/z = 540.3 (M+H)⁺.

### Synthesis of compound 66

**66-a** (45 mg, 0.083 mmol), DCM (6 mL) and 17% boron tribromide (3 mL) were added to a reaction flask. At room temperature, the reaction mixture was stirred under nitrogen atmosphere for 4 hours and concentrated by rotary evaporation. The residue was dissolved in methanol, subjected to pre-HPLC (NH₄HCO₃) and lyophilized to obtain compound **66** (4.1 mg, 9%) as a white solid. LC-MS (ESI): m/z = 526.3 (M+H)⁺.

### Example 68: Synthetic route of compound 67

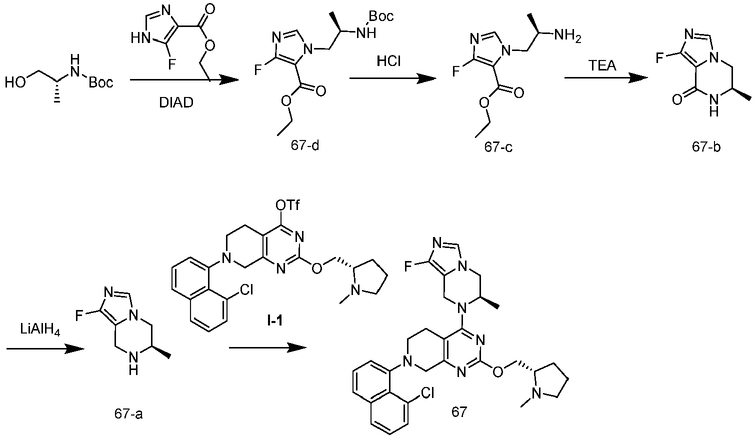

### Synthesis of compound 67-d

*Tert*-butyl (*R*)-(1-hydroxypropan-2-yl)carbamate (100 mg, 0.57 mmol), ethyl 5-fluoro-1*H-*imidazole-4-carboxylate (99 mg, 0.69 mmol) and triphenylphosphine (254 mg, 0.97 mmol) were added to a three-necked flask. The mixture was degassed and purged with nitrogen for three times before tetrahydrofuran (20 mL) was added. In a dry ice bath, the above mixture was added DIAD (191 µL, 0.97 mmol) dropwise. After the addition, the mixture was stirred at room temperature overnight. The next day, the reaction mixture was concentrated by rotary evaporation and the residue was purified by column chromatography (mobile phase: ethyl acetate/petroleum ether = 0/100 to 50/50) to obtain compound **67-d** (140 mg, 78%) as a white solid. LC-MS (ESI): m/z = 316.0 (M+H)⁺.

### Synthesis of compound 67-c

**67-d** (140 mg, 0.45 mmol) and 4 M hydrochloric acid in methanol (10 mL) were added to a reaction flask. The reaction mixture was stirred at room temperature overnight under nitrogen atmosphere. The next day, the reaction mixture was concentrated by rotary evaporation to obtain compound **67-c** (crude) as a colorless gum. LC-MS (ESI): m/z = 215.9 (M+H)⁺.

### Synthesis of compound 67-b

**67-c** (crude), methanol (10 mL) and triethylamine (1.5 mL, 10.81 mmol) were added to a reaction flask. The reaction mixture was then stirred at 70 °C under nitrogen atmosphere overnight. The next day, the reaction mixture was concentrated by rotary evaporation and the residue was purified by column chromatography (mobile phase: ammonia in methanol/dichloromethane = 0/100 to 5/95) to obtain compound **67-b** (64 mg, 85%) as a white solid. LC-MS (ESI): m/z = 170.1 (M+H)⁺.

### Synthesis of compound 67-a

**67-b** (64 mg, 0.38 mmol), tetrahydrofuran (15 mL) and 2.5 M lithium aluminum tetrahydrate in tetrahydrofuran (0.3mL, 0.76 mmol) were added to a reaction flask. The reaction mixture was stirred at 80 °C under nitrogen atmosphere overnight. The next day, the reaction mixture was cooled to room temperature, was added saturated aqueous sodium sulfate to quench the reaction. The mixture was dried over anhydrous sodium sulfate, filtered and washed twice with tetrahydrofuran. The filtrates were combined, concentrated by rotary evaporation and the residue was purified by column chromatography (mobile phase: ammonia in methanol/dichloromethane = 0/100 to 5/95) to obtain compound **67-a** (14 mg, 24%) as a white solid. LC-MS (ESI): m/z = 156.0 (M+H)⁺.

### Synthesis of compound 67

**67-a** (14 mg, 0.090 mmol), N-methylpyrrolidinone (3 mL), I-1 (50 mg, 0.090 mmol) and DIPEA (74 µL, 0.45 mmol) were added to a reaction flask. The reaction mixture was stirred for 3 hours at 100 °C under nitrogen atmosphere. The reaction mixture was cooled to room temperature. The reaction was quenched with saturated sodium bicarbonate solution. The resulting reaction mixture was extracted with ethyl acetate (30 mL × 2). The organic phase was washed with brine (30 mL × 3), concentrated by rotary evaporation, purified by column chromatography (mobile phase: ammonia in methanol/dichloromethane = 0/100 to 5/95), and purified by thin layer chromatography (dichloromethane/ammonia in methanol = 10/1) to obtain compound **67** (5 mg, 10%) as a white solid. LC-MS (ESI): m/z = 562.2 (M+H)⁺; ¹H NMR (400 MHz, CDCl₃) : *δ* 7.76(1H, d, *J*=8Hz), 7.62(1H, t, *J*=8Hz), 7.55-7.51(1H, m), 7.49-7.39(1H, m), 7.34(1H, t, *J*=8Hz), 7.17(1H, d, *J*=7.6Hz), 7.09(1H, d, *J*=7.2Hz), 4.96-4.44(1H, m), 4.77-4.58(2H, m), 4.51-4.26(3H, m), 4.24-4.15(1H, m), 4.04-3.90(1H, m), 3.77(1H, d, *J*=18Hz), 3.66-3.47(1H, m), 3.39-3.22(1H, m), 3.16-3.04(1H, m), 3.00-2.87(1H, m), 2.81-2.51(4H, m), 2.27-2.12(1H, m), 2.09-1.83(3H, m), 1.41-1.27(5H, m).

### Example 69: Synthetic route of compound 68

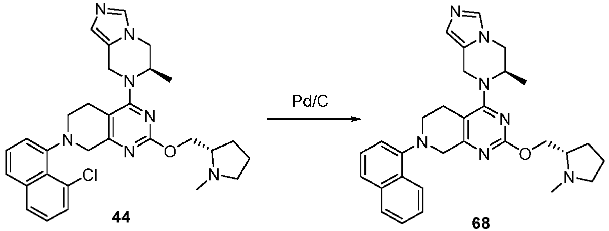

### Synthesis of compound 68

**44** (30 mg, 0.055 mmol), methanol (30 mL), triethylamine (0.5 mL) and 10% Pd/C (50 mg) were added to a reaction flask. The reaction mixture was purged with hydrogen for three times, and stirred at room temperature for 48 hours, filtered, subjected to pre-HPLC (ammonium bicarbonate) and lyophilized to obtain compound **68** (5.2 mg, 18%) as a white solid. LC-MS (ESI): m/z = 510.0 (M+H)⁺; ¹H NMR (400 MHz, CDCl₃) : *δ* 8.27-8.19(1H, m), 7.90-7.83(1H, m), 7.61(1H, d, *J*=8Hz), 7.56-7.47(3H, m), 7.43(1H, t, *J*=8Hz), 7.14(1H, d, *J*=6.8Hz), 6.92(1H, bs), 4.91(1H, d, *J*=16Hz), 4.86-4.77(1H, m), 4.72-4.54(2H, m), 4.44-4.16(4H, m), 4.02(1H, d, *J*=11.6Hz), 3.63-3.49(1H, m), 3.46-3.29(1H, m), 3.26-2.92(3H, m), 2.82-2.60(4H, m), 2.59-2.41(1H, m), 2.24-2.10(1H, m), 2.08-1.97(1H, m), 1.96-1.83(2H, m), 1.34(3H, d, *J*=6.8Hz).

### Example 70: Synthetic route of compound 69

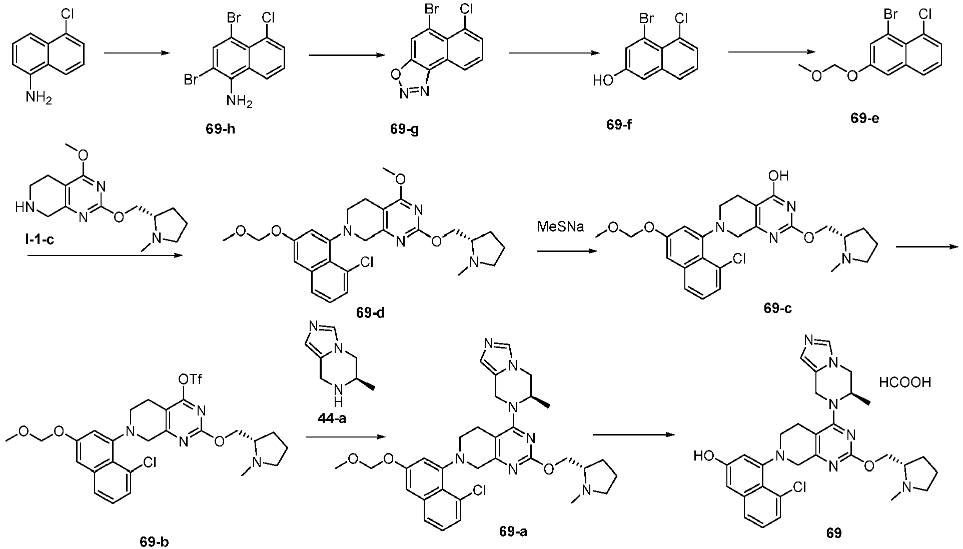

### Synthesis of compound 69-h

amino-5-chloronaphthalene (10 g, 56.5 mmol) and glacial acetic acid (480 mL) were added to a reaction flask. Bromine (6.4 mL, 123.2 mmol) was slowly added to the above mixture in an ice-cold water bath. The reaction was stirred at 70 °C for 1 hour. The reaction mixture was cooled in an ice-cold water bath, filtered and washed with acetic acid. The filter cake was adjusted to pH 7 by adding 15% aqueous sodium hydroxide (about 100 mL), extracted with dichloromethane (300 mL × 2), dried over anhydrous sodium sulfate, filtered and concentrated by rotary evaporation to obtain compound **69-h** (12.4 g, 63%) as a dark solid, which was directly used in the next step without purification. ¹H NMR (400 MHz, CDCl₃): *δ* 7.94 (1H, s), 7.77 (1H, dd, *J*=8.4Hz, 1.2Hz), 7.65 (1H, dd, *J*=7.6Hz, *J*=1.2Hz), 7.36 (1H, dd, *J*=8.4Hz, 7.6Hz), 4.65 (2H, brs).

### Synthesis of compound 69-g

**69-h** (12.4 g, 37.23 mmol), glacial acetic acid (236 mL) and propionic acid (30 mL) were added to a reaction flask. In an ice-cold water bath, the above mixture was added sodium nitrite (3.85 g, 55.86 mmol). After the addition, the reaction was stirred at this temperature for 30 minutes and at room temperature for 1 hour. The reaction was quenched with water (300 mL) and the reaction mixture was filtered. The filter cake was washed with water and dissolved in ethyl acetate. The resulting mixture was dried over anhydrous sodium sulfate, filtered, and the filtrate was concentrated by rotary evaporation to obtain compound **69-g** (9.2 g, 88%) as a dark solid. LC-MS (ESI): m/z = 282.7 (M+H)⁺.

### Synthesis of compound 69-f

**69-g** (9.2 g, 32.62 mmol), ethanol (200 mL) and tetrahydrofuran (100 mL) were added to a reaction flask. Sodium borohydride (2.48 g, 65.25 mmol) was slowly added in an ice-cold water bath. After the addition, the reaction was stirred at room temperature for 2 hours. The reaction was quenched with 10% aqueous potassium hydrogen sulfate. Ethanol and tetrahydrofuran were removed by rotary evaporation, and the residue was extracted with ethyl acetate (500 mL × 2), dried, filtered, concentrated by rotary evaporation, and the residue was purified by column chromatography (mobile phase: ethyl acetate/petroleum ether = 0/100 to 30/70) to obtain compound **69-f** (2.5 g, 30%) as a dark solid. LC-MS (ESI): m/z = 254.7 (M-H)⁻.

### Synthesis of compound 69-e

**69-f** (1.5 g, 5.86 mmol), dichloromethane (50 mL) and DIPEA (2.4 mL, 14.65 mmol) were added to a reaction flask. Bromo(methoxy)methane (0.86 mL, 10.55 mmol) was added dropwise to the above mixture in an ice-cold water bath. After the addition, the reaction was stirred at this temperature for 30 minutes. The reaction was quenched with water. The mixture was extracted with dichloromethane (50 mL × 3), concentrated by rotary evaporation and the residue was purified by column chromatography (mobile phase: ethyl acetate/petroleum ether = 0/100 to 5/95) to obtain compound **69-e** (1.5 g, 85%) as a brown solid. ¹H NMR (400 MHz, CDCl₃): *δ* 7.68 (1H, d, *J*=2.4Hz), 7.65 (1H, dd, *J*=8Hz, *J*=0.8Hz), 7.50 (1H, dd, *J*=7.6Hz, *J*=1.2Hz), 7.37 (1H, d, *J*=2.4Hz), 7.30 (1H, t, *J*=8Hz), 5.26 (2H, s), 3.51 (3H, s).

### Synthesis of compound 69-d

**69-e** (3.24 g, 10.8 mmol), **I-1-c** (2 g, 7.2 mmol), dioxane (80 mL), cesium carbonate (11.7 g, 36 mmol), RuPhos (0.67 g, 1.44 mmol) and Pd₂dba₃ (0.66 g, 0.72 mmol) were added to a reaction flask. The reaction mixture was degassed and purged with nitrogen for three times and stirred at 100 °C overnight under nitrogen atmosphere. The next day, the reaction mixture was concentrated by rotary evaporation and the residue was purified by column chromatography (mobile phase: ammonia in methanol/dichloromethane = 0/100 to 5/95) to obtain compound **69-d** (2.1 g, 47%) as a brown solid. LC-MS (ESI): m/z = 499.2 (M+H)⁺.

### Synthesis of compound 69-c

**69-d** (2.1 g, 4.22 mmol), DMF (20 mL) and sodium thiomethoxide (1.5 g, 21.08 mmol) were added to a reaction flask. The reaction mixture was stirred at 60 °C under nitrogen atmosphere for 1 hour. The reaction mixture was cooled to room temperature before the reaction was quenched with water. The mixture was adjusted to pH 6 with 1 M hydrochloric acid and extracted with ethyl acetate (100 mL × 5). The organic phase was dried, and ethyl acetate and DMF were removed. The resulting mixture was purified by column chromatography (mobile phase: ammonia in methanol/dichloromethane = 0/100 to 5/95) to obtain compound **69-c** (1.5 g, 73%) as a pale brown solid. LC-MS (ESI): m/z = 485.1 (M+H)⁺.

### Synthesis of compound 69-b

**69-c** (500 mg, 1.03 mmol), dichloromethane (50 mL) and triethylamine (430 µL, 3.09 mmol) were added to a reaction flask. In a dry ice bath, the mixture was added trifluoromethanesulfonic anhydride (260 µL, 1.55 mmol) dropwise. After the addition, the reaction mixture was stirred at this temperature for 30 minutes, and the reaction was quenched with saturated sodium bicarbonate solution. The mixture was extracted with dichloromethane (50 mL × 2), dried over anhydrous sodium sulfate, filtered, concentrated by rotary evaporation, and the residue was purified by column chromatography (mobile phase: methanol/dichloromethane = 0/100 to 3/97) to obtain compound **69-b** (550 mg, 86%) as a pale brown solid. LC-MS (ESI): m/z = 617.1 (M+H)⁺.

### Synthesis of compound 69-a

**69-b** (550 mg, 0.89 mmol), DMF (8 mL), **44-a** (159 mg, 1.16 mmol) and DIPEA (736 µL, 4.46 mmol) were added to a reaction flask. The reaction mixture was stirred under argon atmosphere at 100 °C for 2 hours. The reaction was quenched with saturated sodium bicarbonate solution. The mixture was extracted with ethyl acetate (50 mL × 2) and washed with brine (30 mL × 3). The organic phase was concentrated by rotary evaporation, and the residue was purified by column chromatography (mobile phase: ammonia in methanol/dichloromethane = 0/100 to 7/93) to obtain compound **69-a** (217 mg, 40%) as a pale brown solid. LC-MS (ESI): m/z = 603.8 (M+H)⁺.

### Synthesis of compound 69

**69-a** (20 mg, 0.033 mmol) and acetonitrile (2 mL) were added to a reaction flask. 4 M 1,4-dioxane solution of hydrogen chloride (2 mL) was added to the above mixture in an ice-cold water bath. The reaction was stirred at this temperature for 30 minutes before the reaction was quenched with 7 M ammonia in methanol (3 mL). The mixture was concentrated by rotary evaporation. 2 mL of methanol was added. The mixture was filtered, subjected to pre-HPLC (formic acid), and lyophilize to obtain compound **69** (5.3 mg, 28%) as a white solid. LC-MS (ESI): m/z = 560.3 (M+H)⁺.

### Example 71: Synthetic route of compound 70

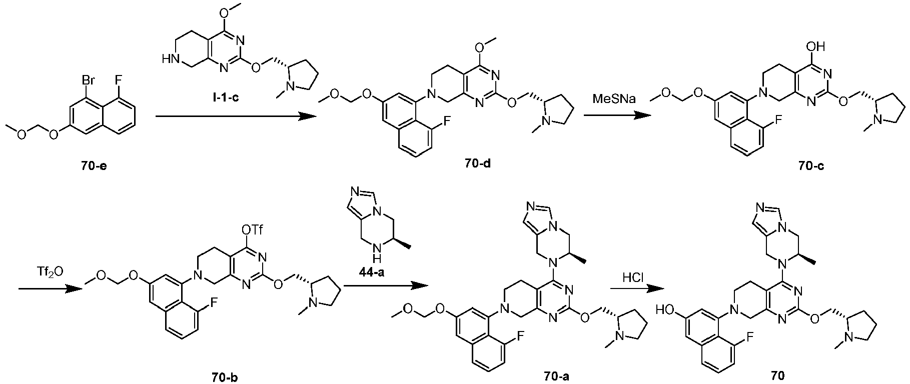

### Synthesis of compound 70-e

**70-e** was synthesized with reference to the method described in Patent No. WO2021041671A1.

### Synthesis of compound 70-d

Compound **I-1-c** (1.40 g, 4.92 mmol), **70-e** (1.60 g, 5.76 mmol), RuPhos (0.36 g, 0.77 mmol), Pd₂(dba)₃ (0.36 g, 0.63 mmol) and Cs₂CO₃ (5.60 g, 17.23 mmol) were added to toluene (100 mL). The mixture was heated to 100 °C under nitrogen atmosphere and stirred for 12 hours. The reaction mixture was cooled to room temperature, filtered and washed with ethyl acetate (100 mL × 3). The organic phase was concentrated and the residue was purified by column chromatography (mobile phase: methanol/dichloromethane = 1/20) to obtain compound **70-d** (1.79 g, 76%). LC-MS (ESI): m/z 483.3 (M+H) ⁺.

### Synthesis of compound 70-c

Compound **70-d** (1.75 g, 3.63 mmol) and sodium thiomethoxide (1.27 g, 18.15 mmol) were added to DMF (20 mL). The mixture was heated to 60 °C under nitrogen atmosphere and stirred for 2 hours. Water (120 mL) and diluted hydrochloric acid (6.80 mL, 3.40 mmol) were added to the reaction mixture to quench the reaction, and the mixture was filtered. The solid was purified by column chromatography (mobile phase: methanol/dichloromethane = 1/10) to obtain compound **70-c** (1.4 g, 88%). LC-MS (ESI): m/z 469.2 (M+H) ⁺.

### Synthesis of compound 70-b

**70-c** (0.33 g, 0.70 mmol) and triethylamine (0.21 g, 2.1 mmol) were dissolved in dichloromethane (25 mL) and the temperature was reduced to -40 °C under nitrogen atmosphere. Trifluoromethanesulfonic anhydride (0.36 g, 1.27 mmol) was added slowly dropwise to the above mixture. After the addition, the mixture was stirred at -40 °C for 1 hour. The reaction was quenched by addition of saturated sodium bicarbonate solution (40 mL), was added dichloromethane (40 mL × 2) for extraction, and the organic phase was concentrated and the residue was purified by column chromatography (mobile phase: methanol/dichloromethane = 1/10) to obtain compound **70-b** (0.38 g, 91%). LC-MS (ESI): m/z 600.7 (M+H) ⁺.

### Synthesis of compound 70-a

**70-b** (380 mg, 0.63 mmol), **44-a** (112 mg, 0.82 mmol), DMAC (5 mL) and DIPEA (245 mg, 1.90 mmol) were added to a reaction flask. The reaction was warmed from room temperature to 100 °C and stirred for 1.5 hours. Ice-cold water (100 mL) was added to quench the reaction. The mixture was extracted with dichloromethane (40 mL × 2), concentrated, and the residue was purified by column chromatography (mobile phase: methanol/dichloromethane = 1/20) to obtain compound **70-a** (80 mg, 22%). LC-MS (ESI): m/z 588.3 (M+H) ⁺.

### Synthesis of compound 70

**70-a** (20 mg, 0.03 mmol) was dissolved in acetonitrile (2 mL). The mixture was cooled to 0 °C under argon atmosphere, was added a solution of hydrogen chloride in 1,4-dioxane (2 mL, 8.00 mmol) slowly dropwise and the mixture was stirred at 0 °C for 0.5 hours. The reaction mixture was concentrated, subjected to pre-HPLC and lyophilized to obtain compound **70** (5 mg, 27%, formic acid). LC-MS (ESI): m/z 544.3 (M+H) ⁺. ¹H NMR (400MHz, CD₃OD): δ8.48 (1H, s), 7.68 (1H, s), 7.44 (1H, d, *J*= 8.4Hz), 7.25-7.36 (1H, m), 6.83-6.97 (3H, m), 6.80 (1H, s), 4.68-4.80 (2H, m), 4.33-4.66 (2H, m), 3.90-4.36(4H, m), 3.55-3.78 (3H, m), 3.33-3.52 (1H, m), 2.56-3.32 (7H, m), 2.28-2.42 (1H, m), 1.91-2.22 (3H, m), 1.23-1.49 (3H, m).

### Synthetic methods for similar compounds:

| compound | structure | Synthetic method |
|---|---|---|
| **70A** | 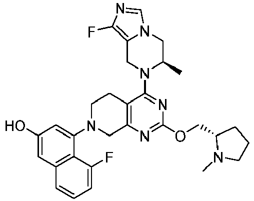 | Synthesized according to synthesis of compound 70, compound 67-a was used instead of compound 44-a. |
| **70B** | 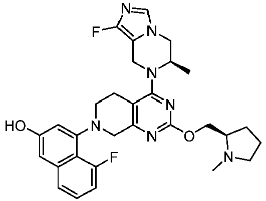 | Synthesized according to synthesis of compound 70, *N-*methyl-D-prolinol was used instead of *N*-methyl-L-prolinol, and compound 67-a was used instead of compound 44-a. |
| **70C** | 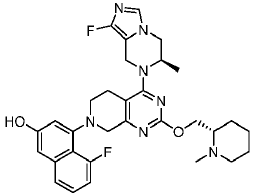 | Synthesized according to synthesis of compound 70, (*S*)-1-Methylpiperidine-2-methanol was used instead of *N-*methyl-L-prolinol, and compound 67-a was used instead of compound 44-a. |
| 70D | 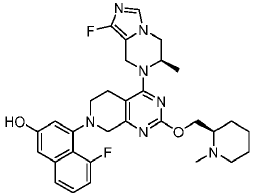 | Synthesized according to synthesis of compound 70, (*R*)-1-Methylpiperidine-2-methanol was used instead of *N-*methyl-L-prolinol, and compound 67-a was used instead of compound 44-a. |

### Example 72: Synthetic route of compound 71

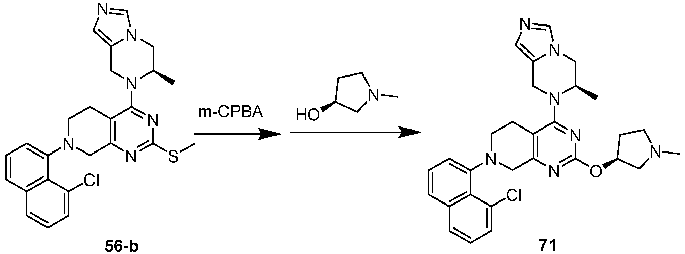

### Synthesis of compound 71

Compound **56-b** (20 mg, 0.042 mmol) was dissolved in dichloromethane (10 mL). After degassed and purged with nitrogen for three times, the mixture was added *m*-chloroperoxybenzoic acid (8.39 mg, 0.083 mmol), then stirred at room temperature for 1 hour. Saturated sodium bicarbonate solution (5 mL) was added. The reaction mixture was extracted with dichloromethane (50 mL) and dried over sodium sulfate. The sodium sulfate was removed by filtration. The resulting mixture was concentrated by rotary evaporation at reduced pressure to obtain a crude product. Dichloromethane (6 mL) and (*S*)-(+)-1-methyl-3-hydroxypyrrolidine (8 mg, 0.08 mmol) were added to the crude product. After degassed and purged with nitrogen for three times, the mixture was added sodium *tert*-butoxide (8 mg, 0.08 mmol), and the reaction mixture was stirred at room temperature for 30 min. The reaction mixture was concentrated by rotary evaporation at reduced pressure to obtain a crude product, which was purified by pre-HPLC (formic acid) to obtain compound 71 (5 mg, 23%) as a yellow solid. LC-MS (ESI): m/z 530.3 (M+H) ⁺.

### Example 73: Synthetic route of compound 72

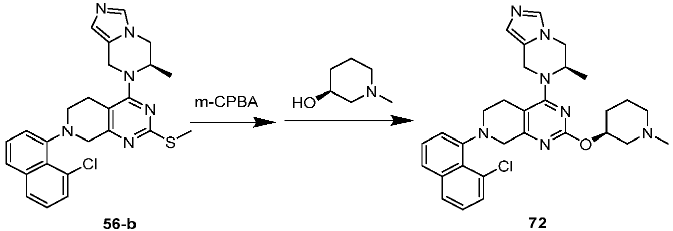

### Synthesis of compound 72

Compound **56-b** (20 mg, 0.04 mmol) was dissolved in dichloromethane (10 mL). After degassed and purged with nitrogen for three times, the reaction mixture was added *m-*chloroperoxybenzoic acid (17 mg, 0.08 mmol), then stirred at room temperature for 1 h. Saturated sodium bicarbonate solution (5 mL) was added. The reaction mixture was extracted with dichloromethane (50 mL) and dried over sodium sulfate. The drying agent was removed by filtration. The resulting mixture was concentrated by rotary evaporation at reduced pressure to obtain a crude product. Dichloromethane (6 mL) and (*S*)-1-methyl-3-hydroxypiperidine (10 mg, 0.08 mmol) were added to the crude product. After degassed and purged with nitrogen for three times, the reaction mixture was added sodium *tert*-butoxide (8 mg, 0.08 mmol), and stirred at room temperature for 30 min. The reaction mixture was concentrated by rotary evaporation at reduced pressure to obtain a crude product, which was purified by pre-HPLC (formic acid) to obtain compound **72** (5 mg, 20%) as a yellow solid. LC-MS (ESI): m/z 544.2 (M+H)⁺.

### Example 74: Synthetic route of compound 73

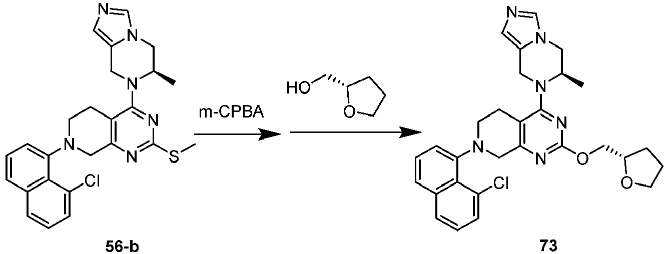

### Synthesis of compound 73

Compound **56-b** (20 mg, 0.04 mmol) was dissolved in dichloromethane (10 mL). After degassed and purged with nitrogen for three times, the reaction mixture was added *m-*chloroperoxybenzoic acid (17 mg, 0.08 mmol), then stirred at room temperature for 1 h, and was added saturated sodium bicarbonate solution (5 mL). The reaction mixture was extracted with dichloromethane (50 mL) and dried over sodium sulfate. The drying agent was removed by filtration. The resulting mixture was concentrated by rotary evaporation at reduced pressure to obtain a crude product. Dichloromethane (6 mL) and (*S*)-(tetrahydrofuran-2-yl) methanol (8 mg, 0.08 mmol) were added to the crude product. After degassed and purged with nitrogen for three times, the reaction mixture was added sodium *tert*-butoxide (8 mg, 0.08 mmol) and stirred at room temperature for 30 min. The reaction mixture was concentrated by rotary evaporation at reduced pressure to obtain a crude product, which was purified by pre-HPLC (formic acid) to obtain compound **73** (8 mg, 36%) as a yellow solid. LC-MS (ESI): m/z 531.2 (M+H)⁺.

### Example 75: Synthetic route of compound 74

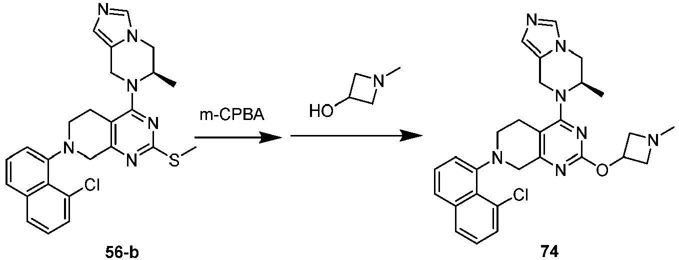

### Synthesis of compound 74

Compound **56-b** (20 mg, 0.04 mmol) was dissolved in dichloromethane (10 mL). After degassed and purged with nitrogen for three times, the reaction mixture was added *m-*chloroperoxybenzoic acid (17 mg, 0.08 mmol), then stirred at room temperature for 1 h. Saturated sodium bicarbonate solution (5 mL) was added. The reaction mixture was extracted with dichloromethane (50 mL) and dried over sodium sulfate. The sodium sulfate was removed by filtration. The resulting mixture was concentrated by rotary evaporation at reduced pressure to obtain a crude product. Dichloromethane (6 mL) and 1-methyl-3-hydroxyazetidine (7 mg, 0.08 mmol) were added to the crude product. After degassed and purged with nitrogen for three times, the reaction mixture was was added sodium *tert*-butoxide (7.97 mg, 0.08 mmol), and stirred at room temperature for 30 min. The reaction mixture was concentrated by rotary evaporation at reduced pressure to obtain a crude product, which was purified by pre-HPLC (formic acid) to obtain compound **74** (8 mg, 36%) as a yellow solid. LC-MS (ESI): m/z 516.2 (M+H)⁺.

### Example 76: Synthetic route of compound 75

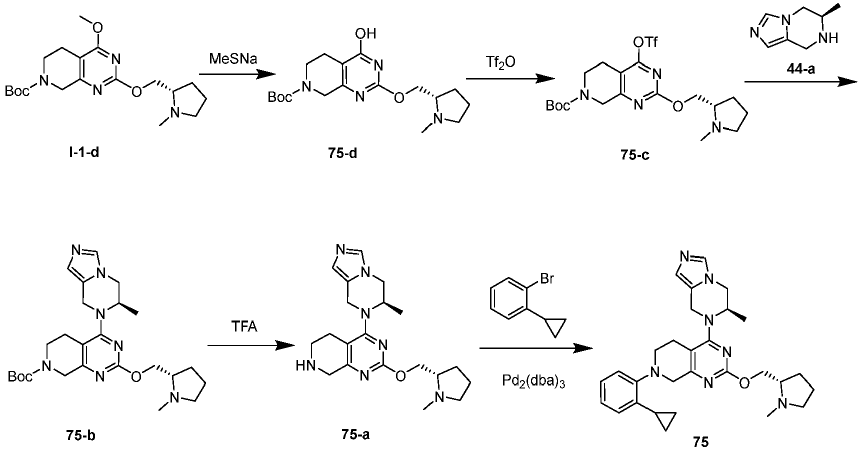

### Synthesis of compound 75-d

Under nitrogen atmosphere, compound **I-1-d** (200 mg, 0.53 mmol) was dissolved in DMF (10 mL), and sodium thiomethoxide (148 mg, 2.11 mmol) was added to the solution. After the addition, the reaction mixture was heated to 60 °C and stirred for 2 hours. Upon completion, the reaction mixture was neutralized with diluted hydrochloric acid to about pH 7. The precipitated solid was collected by filtration, washed with water and dried to obtain compound **75-d** (150 mg, 78%) as an earthy yellow solid. LC-MS (ESI): m/z 365.2 (M+H) ⁺.

### Synthesis of compound 75-c

Compound **75-d** (150 mg, 0.41 mmol) was dissolved in DCM (20 mL) at room temperature, and was added DIPEA (0.34 mL, 2.06 mmol) and trifluoromethanesulfonic anhydride (0.17 mL, 1.03 mmol) serially in an ice-cold water bath under nitrogen atmosphere. After the addition, the reaction mixture was stirred for 2 hours in an ice-cold water bath and the reaction was quenched with saturated sodium bicarbonate solution, the organic phase was separated and the aqueous phase was extracted with dichloromethane. The organic phases were combined, dried over anhydrous sodium sulfate, filtered, and concentrated to obtain a crude product, which was purified by a flash column chromatography (mobile phase: methanol/dichloromethane = 0% to 10%) to obtain compound **75-c** (200 mg, 98%) as a white solid. LC-MS (ESI): m/z 497.1 (M+H) ⁺.

### Synthesis of compound 75-b

Compound **75-c** (200 mg, 0.40 mmol) was dissolved in DMF (5 mL) at room temperature, followed by serial addition of DIPEA (0.33 mL, 2.01 mmol) and **44-a** (72 mg, 0.52 mmol). After the addition, the reaction mixture was degassed and purged with nitrogen, and stirred at 100 °C for 2 h. Water (10 mL) was added to the reaction mixture, followed by extraction with ethyl acetate. The organic phase was washed with brine, dried over anhydrous sodium sulfate, filtered, and concentrated to obtain a crude product, which was purified by a flash column chromatography (mobile phase: methanol/dichloromethane = 0% to 10%) to obtain compound **75-b** (80 mg, 41%) as a white solid. LC-MS (ESI): m/z 484.3 (M+H) ⁺.

### Synthesis of compound 75-a

A solution of **75-b** (80 mg, 0.17 mmol) in dichloromethane (10 mL) was added trifluoroacetic acid (2 mL). The resulting mixture was stirred at room temperature for 4 hours. Upon completion, the mixture was concentrated, carefully neutralized with a saturated solution of sodium bicarbonate to pH > 7 in an ice-cold water bath, and extracted with ethyl acetate. The organic phases were combined, washed with brine, dried over anhydrous sodium sulfate, filtered, and concentrated to obtain the product **75-a** (40 mg, 80%) as a brown oil. LC-MS (ESI): m/z 384.3 (M+H) ⁺.

### Synthesis of compound 75

**75-a** (40 mg, 0.10 mmol), 1-bromo-2-cyclopropylbenzene (29 mg, 0.15 mmol), 2-dicyclohexylphosphino-2',6'-diisopropoxy-1,1'-biphenyl (10 mg, 0.02 mmol), cesium carbonate (102 mg, 0.31 mmol), Pd₂(dba)₃ (10 mg, 0.01 mmol) and toluene (10 mL) were added to a reaction flask. The reaction mixture was degassed and purged with nitrogen and reacted at 100 °C overnight. Upon completion, the mixture was concentrated to obtain a crude product. The crude product was purified by a flash column chromatography (mobile phase: methanol/dichloromethane = 0% to 10%) to obtain compound 75 (10 mg, 19%) as a white solid. LC-MS (ESI): m/z 499.9 (M+H) ⁺; ¹H NMR (400 MHz, CDCl₃): *δ* 7.47 (1H, s), 7.02-7.16 (3H, m), 6.90 (1H, s), 6.83 (1H, d, *J =* 7.6Hz), 4.84 (1H, d, *J* = 16.4Hz), 4.71-4.79 (1H, m), 4.62 (1H, d, *J* = 15.6Hz), 4.41-4.53 (1H, m), 4.36 (1H, dd, *J* = 12.0, 4.4Hz), 4.26 (1H, d, *J* = 18.0Hz), 4.14-4.23 (1H, m), 4.07 (1H, d, *J =* 18.0Hz), 4.00 (1H, d, *J* = 11.6Hz), 3.44-3.51 (1H, m), 3.06-3.28 (2H, m), 2.89-3.00 (1H, m), 2.72-2.86 (1H, m), 2.61-2.73 (1H, m), 2.55 (3H, s), 2.28-2.43 (2H, m), 2.06-2.15 (1H, m), 1.76-1.85 (3H, m), 1.30 (3H, d, *J* = 6.4Hz), 0.99-1.06 (2H, m), 0.72-0.80 (2H, m).

### Example 77: Synthetic route of compound 76

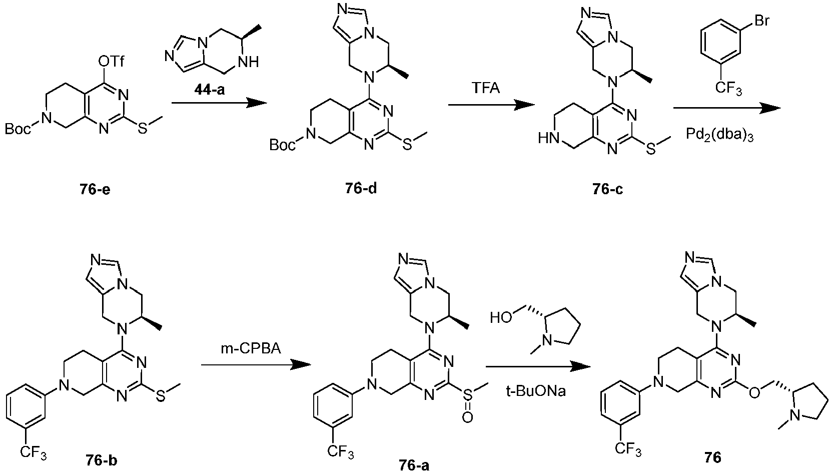

### Synthesis of compound 76-d

Compound **76-e** (1.00 g, 2.33 mmol) was dissolved in DMF (10 mL) at room temperature, followed by serial addition of DIPEA (1.15 mL, 6.99 mmol) and **44-a** (415 mg, 3.03 mmol). After the addition, the reaction mixture was degassed and purged with nitrogen, and stirred at 100 °C for 4 h. The reaction mixture water (20 mL) was added to, followed by extraction with ethyl acetate. The organic phase was washed with brine, dried over anhydrous sodium sulfate, filtered, and concentrated to obtain a crude product, which was purified by a flash column chromatography (mobile phase: methanol/dichloromethane = 0% to 10%) to obtain compound **76-d** (400 mg, 41%) as a white solid. LC-MS (ESI): m/z 417.0 (M+H) ⁺.

### Synthesis of compound 76-c

To a solution of **76-d** (400 mg, 0.96 mmol) in dichloromethane (10 mL) was added trifluoroacetic acid (2 mL). The resulting reaction mixture was stirred at room temperature for 4 hours. Upon completion, the reaction mixture was concentrated, carefully neutralized with a saturated solution of sodium bicarbonate to pH > 7 in an ice-cold water bath, and extracted with ethyl acetate. The organic phases were combined, washed with brine, dried over anhydrous sodium sulfate, filtered, and concentrated to obtain the product **76-c** (250 mg, 82%) as a brown oil. LC-MS (ESI): m/z 317.2 (M+H)+.

### Synthesis of compound 76-b

**76-c** (250 mg, 0.79 mmol), m-bromo-trifluorotoluene (249 mg, 1.11 mmol), 2-dicyclohexylphosphino-2',6'-diisopropoxy-1,1'-biphenyl (74 mg, 0.16 mmol), cesium carbonate (773 mg, 2.37 mmol), Pd₂(dba)₃ (72.4 mg, 0.08 mmol) and toluene (15 mL) were added to a reaction flask. The reaction mixture was degassed and purged with nitrogen and reacted at 100 °C overnight. Upon completion, the mixture was concentrated to obtain a crude product. The crude product was purified by a flash column chromatography (mobile phase: methanol/dichloromethane = 0% to 10%) to obtain compound **76-b** (30 mg, 8%) as a white solid. LC-MS (ESI): m/z 461.1 (M+H) ⁺.

### Synthesis of compound 76-a

Compound **76-b** (30 mg, 0.07 mmol) was dissolved in dichloromethane (10 mL) in an ice-cold water bath, then was added *m*-chloroperoxybenzoic acid (16 mg, 0.08 mmol) and the mixture was slowly warmed to room temperature and stirred for 2 hours. Upon completion, the mixture was added saturated aqueous sodium bicarbonate for neutralization. The organic phase was separated, and the aqueous phase was extracted with dichloromethane. The organic phases were combined, dried over anhydrous Na₂SO₄, filtered and evaporated. The crude product was purified by a flash column chromatography (mobile phase: dichloromethane/methanol = 0 to 10/1) to obtain **76-a** (15 mg, 48%) as a white solid. LC-MS (ESI): m/z 477.1 (M+H) ⁺.

### Synthesis of compound 76

To a solution of **76-a** (15 mg, 0.03 mmol) and *N*-methyl-L-prolinol (7.3 mg, 0.06 mmol) in toluene (10 mL) in an ice-cold water bath was added sodium tert-butoxide (6 mg, 0.06 mmol). After the addition, the reaction mixture was stirred in an ice-cold water bath for 10 minutes. Upon completion, the mixture was concentrated at reduced pressure, diluted with ethyl acetate, and serially washed with water and brine, dried over anhydrous Na₂SO₄, filtered and concentrated by rotary evaporation to obtain a brown oil. The crude product was purified by pre-HPLC to obtain compound **76** (1.5 mg, 9%) as a white solid. LC-MS (ESI): m/z 528.3 (M+H) ⁺.

### Example 78: Synthetic route of compound 77

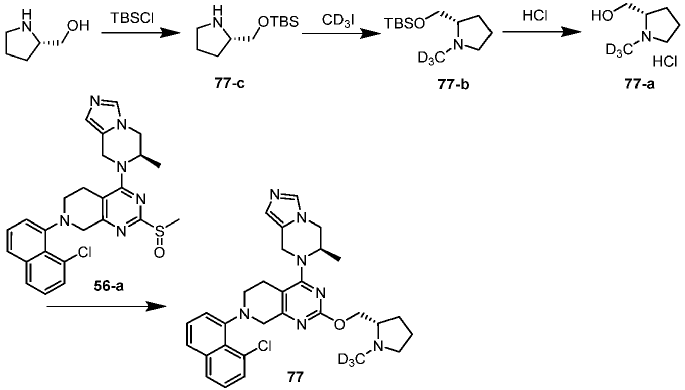

### Synthesis of compound 77-c

(*S*)-2-hydroxymethylpyrrolidine (5 g, 49.45 mmol) was dissolved in 80 mL of THF. The mixture was cooled to 0 °C, and was added diethyl ether (80 mL), triethylamine (9.15 mL, 65.77 mmol) and TBSCl (9.7 g, 64.28 mmol) serially. The reaction mixture was stirred at 0 °C for 1 hour, and warmed to room temperature and stirred overnight. The reaction was quenched with 50% potassium carbonate solution (80 mL). The mixture was extracted with 50 mL of ethyl acetate. The organic phase was washed with brine (50 mL × 2), dried over anhydrous sodium sulfate, filtered, and concentrated. The resulting solid was added DCM (100 mL). The mixture was washed with saturated sodium carbonate (100 mL × 3), and the organic phase was dried over anhydrous sodium sulfate, filtered, and concentrated to obtain a crude product. The crude product was purified by a flash column chromatography (DCM/MeOH = 10:1) to obtain compound **77-c** (5.88 g, 55%) as a pale yellow oil. LC-MS (ESI): m/z = 216.2 [M+H]⁺.

### Synthesis of compound 77-b

Compound **77-c** (4.8 g, 22.28 mmol) was dissolved in 120 mL of THF, and cooled to 0 °C, was added NaH (60%, 1.07 g, 26.76 mmol) under nitrogen atmosphere. The reaction mixture was stirred at 0 °C for 30 minutes before a solution of CD₃I (1.67 mL, 26.76 mmol) in THF (10 mL) was added slowly dropwise. After the addition, the reaction mixture was warmed to room temperature and stirred overnight. At 0 °C, 100 mL of saturated ammonium chloride solution was added, followed by extraction with ethyl acetate (100 mL × 2). The organic phases were combined, washed with brine (100 mL × 3), dried over anhydrous sodium sulfate, filtered, and concentrated to obtain a crude product. The crude product was purified by a flash column chromatography (DCM/MeOH = 10:1) to obtain compound **77-b** (2.8 g, 54%) as a pale yellow semi-solid. LC-MS (ESI): m/z = 233.3 [M+H]⁺.

### Synthesis of compound 77-a

Compound **77-b** (200 mg, 0.86 mmol) was dissolved in 1 mL of methanol at room temperature, then was added a solution of hydrogen chloride in methanol (4 M, 10 mL) at 0 °C. The reaction mixture was stirred at room temperature under nitrogen atmosphere for 18 hours. The reaction mixture was concentrated at reduced pressure to obtain a crude product of compound **77-**a as a brown oil. LC-MS (ESI): m/z = 119.3 [M+1]⁺.

### Synthesis of compound 77

Compound **77-a** (100 mg, crude) was suspended in 5 mL of toluene at room temperature, was added sodium *tert*-butoxide (115 mg). The reaction mixture was stirred at room temperature for 10 minutes. Compound **56-a** (30 mg, 0.055 mmol) was dissolved in 5 mL of toluene and was added to the above mixture. The reaction mixture was stirred at room temperature under nitrogen atmosphere for 1 hour. The reaction was quenched with 20 mL of water. Ethyl acetate (50 mL × 2) was added for extraction. The organic phase was dried over anhydrous sodium sulfate, filtered and concentrated at reduced pressure. The crude product was purified by pre-HPLC to obtain compound **77** (12.5 mg, 38%) as a white solid. LC-MS (ESI): m/z = 547.2 [M+1]⁺; ¹H NMR (400 MHz, CD₃OD) : *δ* 8.53 (1H, s), 7.83 (1H, d, *J*=6.8Hz), 7.74-7.59 (2H, m), 7.58-7.44 (2H, m), 7.72-7.21 (2H, m), 6.88 (1H, s), 4.68-4.29 (4H, m), 4.26-4.09 (2H, m), 3.87-3.35 (5H, m), 3.24-3.07 (2H, m), 3.03-2.82 (1H, m), 2.77-2.63 (1H, m), 2.37-1.80 (5H, m), 1.45-1.17 (3H, m).

### Example 79: Synthetic route of compound 78

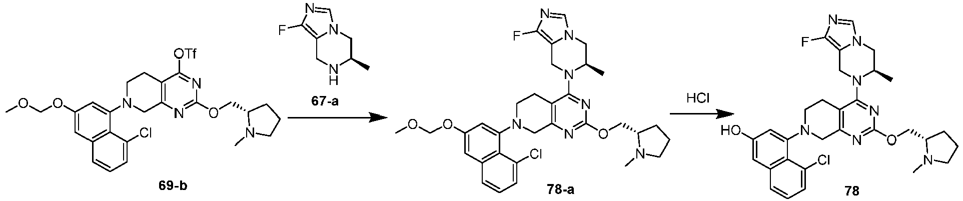

### Synthesis of compound 78-a

**69-b** (160 mg, 0.26 mmol), DIPEA (112 mg, 0.87 mmol), **67-a** (59 mg, 0.38 mmol) and DMSO (2 mL) were added to a reaction flask. The mixture was degassed and purged with nitrogen and stirred at 100 °C for 3 hours. The reaction mixture was added to 10 mL of brine solution and a solid was precipitated. The mixture was filtered and the filter cake was concentrated by rotary evaporation and the residue was purified by column chromatography (mobile phase: DCM/MeOH = 10/0 to 10/1) to obtain compound **78-a** (70 mg, 43%). LC-MS (ESI): m/z 623.1 (M+H) ⁺.

### Synthesis of compound 78

**78-a** (70 mg, 0.11 mmol) and acetonitrile (2 mL) were added to a reaction flask. The mixture was degassed and purged with nitrogen and cooled to 0 °C, was added a solution of hydrogen chloride in 1,4-dioxane (0.5 mL). The reaction was stirred at 0 °C for 10 minutes. The reaction was quenched with ammonia in methanol, and the reaction mixture was concentrated by rotary evaporation and the residue was purified by pre-HPLC to obtain compound **78** (9 mg, 14%). LC-MS (ESI): m/z 578.3 (M+H) ⁺.

### Synthetic methods for similar compounds

| Compound | Structure | Synthetic method |
|---|---|---|
| **78A** | 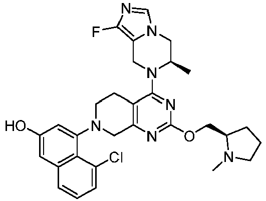 | Synthesized according to synthesis of compound 78, *N*-methyl-D-prolinol was used instead of *N-*methyl-L-prolinol. |
| **78B** | 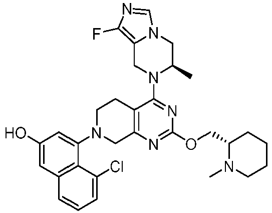 | Synthesized according to synthesis of compound 78, (*S*)-1-Methylpiperidine-2-methanol was used instead of *N*-methyl-L-prolinol. |
| **78C** | 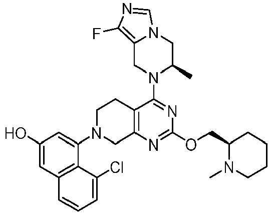 | Synthesized according to synthesis of compound 78, (*R*)-1-Methylpiperidine-2-methanol was used instead of *N*-methyl-L-prolinol. |

### Example 80: Synthetic route of compound 79

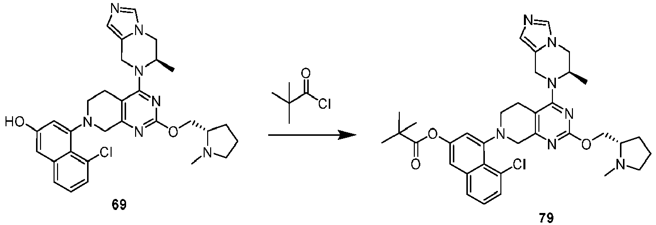

### Synthesis of compound 79

tetrahydrofuran (3 mL) and triethylamine (100 mg, 1.00 mmol) were added to a reaction flask containing **69** (26 mg, 0.046 mmol), respectively, and after stirred for 10 min at room temperature, the mixture was added pivaloyl chloride (8.3 mg, 0.07 mmol) slowly. The reaction mixture was stirred for 0.5 h at room temperature. The reaction mixture was concentrated and prepared by Pre-HPLC to obtain compound **79** (12mg, 40%) . LC-MS (ESI): m/z 644.0 (M+H) ⁺; ¹H NMR (400MHz, CD₃OD): *δ*7.79 (1H, d, *J* = 8.4Hz), 7.63 (1H, d, *J =* 10.0Hz), 7.53 (1H, d, *J* = 7.6Hz), 7.35-7.49 (2H, m), 6.80-7.10 (2H, m), 4.310-4.60 (5H, m), 3.3-3.93 (2H, m), 3.08-3.28 (3H, m), 2.60-2.76 (2H, m), 2.47 (3H, d, *J*= 10.8Hz), 2.30-2.43 (1H, m), 2.00-2.14 (1H, m), 1.50-1.94 (3H, m), 1.18-1.41 (14H, m),0.80-0.94 (1H, m).

### Example 81: Synthetic route of compound 80

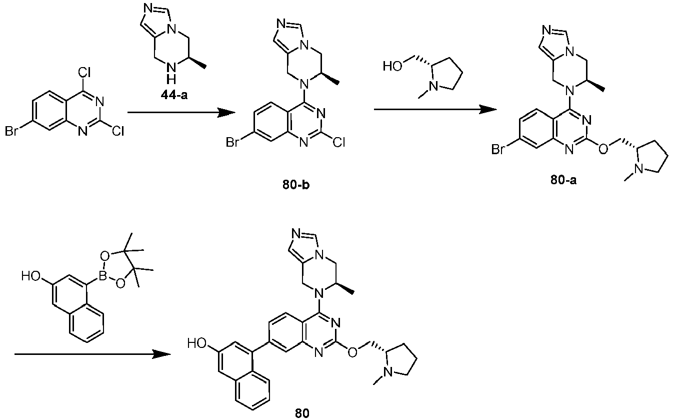

### Synthesis of compound 80-b

2,4-dichloro-7-bromoquinazoline (300 mg, 1.08 mmol), **44-a** (178 mg, 1.30 mmol), *N,N-*diisopropylethylamine (279 mg, 2.16 mmol) and DMF (10 mL) were added to a reaction flask, and the mixture was stirred at room temperature overnight. Upon completion, the reaction was added water and a solid was precipitated, stirred for 10 min, filtered and the filter cake was dried to get compound **80-b** (260 mg, 64%) as a yellow solid. LC-MS (ESI): m/z 378.0(M+H) ⁺.

### Synthesis of compound 80-a

*N*-methyl-L-prolinol (159 mg, 1.38 mmol) and sodium *tert*-butoxide (132 mg, 1.38 mmol) were slowly added to a solution of **80-b** (260 mg, 0.69 mmol) in toluene (10 mL) in an ice-cold water bath. The mixture was warmed to room temperature slowly and stirred at room temperature overnight. The next day, the reaction mixture was added water and extracted with ethyl acetate (50 mL*2). The combined organic phases were dried over anhydrous Na₂SO₄, filtered and the reaction mixture was concentrated by rotary evaporation, and the crude product was purified by column chromatography (mobile phase: (dichloromethane : methanol = 10:1)&dichloromethane, 0-100%) to get compound 80-a ( 180mg, 57% ) as a yellow solid. LC-MS (ESI): m/z 457.1 (M+H) ⁺.

### Synthesis of compound 80

**80-a**(75mg,0.164mmol), 4-(4,4,5,5-tetramethyl-1,3,2-dioxaborolan-2-yl)naphthalen-2-ol (44 mg, 0.164 mmol), tetrakis(triphenylphosphine)palladium (19 mg, 0.0164 mmol), cesium carbonate (107 mg, 0.328 mmol), 1,4-dioxane (20 mL) and water (4 mL) were combined in a reaction flask. The mixture was degassed and purged with nitrogen for three times and stirred at 90°C overnight. The next day, the reaction mixture was cooled to room temperature, concentrated by rotary evaporation, was added water, and extracted with ethyl acetate (50 mL*2). The combined organic phases were dried over anhydrous Na₂SO₄, filtered, concentrated by rotary evaporation, and the crude product was purified by column chromatography (mobile phase: (dichloromethane: methanol = 10:1)/dichloromethane, 0-100%) and preparative HPLC to obtain compound **80** (26 mg, 31%) as a white solid. LC-MS (ESI): m/z 521.3 (M+H)⁺; ¹H NMR (400 MHz, CDCl₃) δ 7.78 (d, *J* = 8.8 Hz, 1H), 7.68 (t, *J* = 9.6 Hz, 2H), 7.60 (s, 1H), 7.39-7.34(m, 3H), 7.27-7.24(m, 2H), 7.20-7.15(m, 1H), 6.88 (s, 1H), 5.19(d, *J =* 16.4 Hz, 1H), 5.14-5.07(m, 1H), 4.96(d, *J* = 16 Hz, 1H), 4.57(dd, *J* = 11.2Hz, 5.2Hz, 1H), 4.33(dd, *J =* 10.8Hz, 5.2Hz, 2H), 3.93 (d, *J =* 12.0Hz,1H), 3.25-3.19 (m, 1H), 2.84-2.78 (m, 1H), 2.60 (s, 3H), 2.41-2.33 (m, 1H), 2.13-2.06 (m, 1H), 1.99-1.80 (m, 4H), 1.31 (d, *J* = 7.2 Hz, 3H).

### Example 82: Synthetic route of compound 81

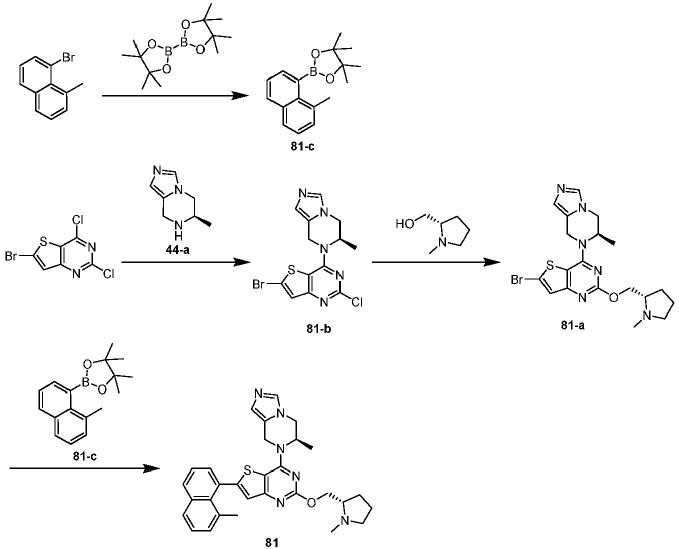

### Synthesis of compound 81-c

To a reaction flask was added 1-bromo-8-methylnaphthalene (1 g, 4.52 mmol), biboronic acid pinacol ester (1.26 g, 4.98 mmol), [1,1'-bis(diphenylphosphino)ferrocene]palladium dichloride (165 mg, 0.226 mmol), potassium acetate (886 mg, 9.04 mmol) and 1,4-dioxane (30 mL), respectively. The mixture was degassed and purged with nitrogen and stirred at 90°C overnight. The next day, the reaction mixture was cooled to room temperature, removed the solvent by rotary evaporation, added water and extracted with ethyl acetate (50 mL*2). The combined organic phases were dried over anhydrous Na₂SO₄, filtered, removed the solvent by rotary evaporation, and the crude product was purified by column chromatography (mobile phase: ethyl acetate/petroleum ether, 0-1%) to give compound **81-c** (755 mg, 62%) as a white solid. LC-MS (ESI): m/z 269.1(M+H) ⁺.

### Synthesis of compound 81-b

6-bromo-2,4-dichlorothieno[3,2-d]pyrimidine (300 mg, 1.06 mmol), **44-a** (174 mg, 1.27 mmol), *N,N*-diisopropylethylamine (274 mg, 2.12 mmol) and DMF (10 mL) were added to a reaction flask. The mixture was stirred at room temperature overnight. Upon completion, the reaction was added water and a solid was precipitated, stirred for 10 min, filtered, and the filter cake was dried to get compound **81-b** (200 mg, 49%) as a yellow solid. LC-MS (ESI): m/z 384.0(M+H) ⁺.

### Synthesis of compound 81-a

Sodium hydrogen (60% in oil, 42 mg, 1.04 mmol) was added slowly to a solution of *N-*methyl-L-prolinol (120 mg, 1.04 mmol) in tetrahydrofuran (15 mL) in an ice-cold water bath. After addition, the reaction mixture was warmed slowly to room temperature and stirred at room temperature for 3 h. Then was added **81-b** (200 mg, 0.52 mmol) and stirred at 60°C overnight. The reaction was concentrated to remove the solvent by rotary evaporation, was added water and extracted by ethyl acetate (50 mL). The organic phase was dried over anhydrous Na₂SO₄, filtered, removed the solvent by rotary evaporation and the crude product was purified by column chromatography (mobile phase: dichloromethane: methanol = 10:1/dichloromethane, 0-100%) to give compound **81-a** (120 mg, 50%) as a yellow solid. LC-MS (ESI): m/z 463.1 (M+H) ⁺.

### Synthesis of compound 81

To a reaction flask were added **81-a** (120 mg, 0.26 mmol), **81-c** (83 mg, 0.31 mmol), tetrakis(triphenylphosphine)palladium (30 mg, 0.026 mmol), cesium carbonate (170 mg, 0.52 mmol), 1,4-dioxane (15 mL) and water (3 mL). The mixture was degassed and purged with nitrogen and stirred at 90°C overnight. The next day, the reaction mixture was cooled to room temperature, concentrated to remove the solvent by rotary evaporation, added water, and extracted with ethyl acetate (50 mL*2). The combined organic phase was dried over anhydrous Na₂SO₄, filtered, removed the solvent by rotary evaporation, and the crude product was purified by column chromatography (mobile phase: dichloromethane: methanol = 10:1/dichloromethane, 0-100%) and pre-HPLC to obtain compound **81** (16 mg, 12%) as a white solid. LC-MS (ESI): m/z 525.3 (M+H) ⁺; ¹H NMR (400 MHz, CDCl₃) δ 7.96 (dd, *J* = 8.0, 0.8Hz, 1H), 7.81 (d, *J* = 8Hz, 1H), 7.56-7.52(m, 2H), 7.50-7.42(m, 2H), 7.34 (d, *J* = 7.2Hz, 1H), 7.22 (s, 1H), 6.99 (s, 1H), 5.54-5.41 (m, 2H), 4.99 (d, *J =* 15.6Hz, 1H), 4.52 (dd, *J =* 10.8, 5.2 Hz, 1H), 4.33-4.28 (m, 2H), 4.12 (dd, *J =* 12.4, 1.2 Hz, 1H), 3.19-3.12 (m, 1H), 2.82-2.75 (m, 1H), 2.55 (s, 3H), 2.36 (s, 3H), 2.34-2.30 (m, 1H), 2.14-2.10 (m, 1H), 1.91-1.78 (m, 3H), 1.28 (d, *J* = 6.8 Hz, 3H).

### Example 83: Synthetic route of compound 82

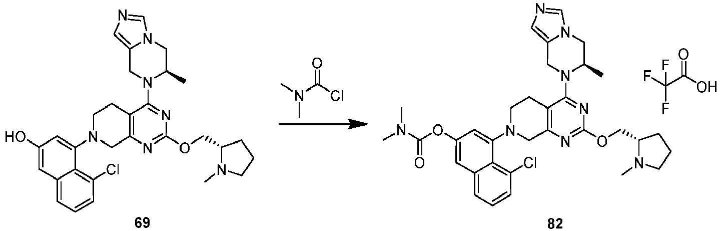

### Synthesis of compound 82

To a reaction flask containing **69** (26 mg, 0.046 mmol) was added DMF (5 mL) and potassium carbonate (70 mg, 0.50 mmol) respectively, and after stirred at room temperature for 10 min, the reaction mixture was slowly added dimethylcarbamoyl chloride (16 mg, 0.15 mmol). The mixture was stirred at room temperature for 12 hours. Filtered, the compound **82** (3 mg, 10%) was obtained by pre-HPLC (mobile phase containing trifluoroacetic acid). LC-MS (ESI): m/z 631.0 (M+H) ⁺.

### Example 84: Synthetic route of compound 83

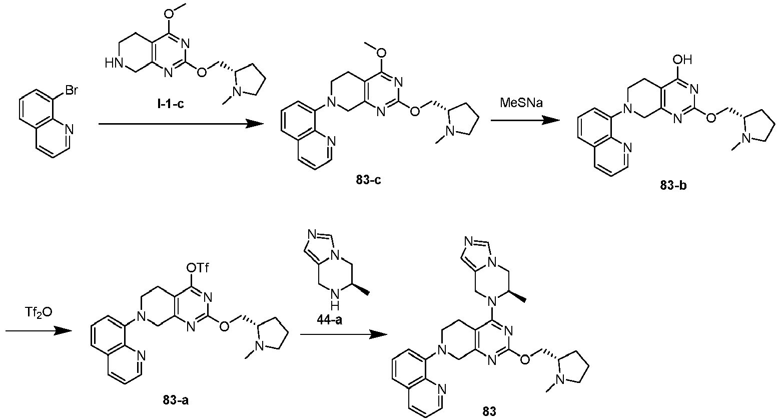

### Synthesis of compound 83-c

To a reaction flask were added 8-bromoquinoline (372 mg, 1.80 mmol), **1-1-c** (500 mg, 1.80 mmol), dioxane (50 mL), cesium carbonate (2.93 g, 9.00 mmol), RuPhos (168 mg, 0.36 mmol) and Pd₂(dba)₃ (165 mg, 0.18 mmol). After addition, the mixture was degassed and purged with nitrogen for three times and stirred at 100°C overnight. The next day, the reaction mixture was concentrated to dryness by rotary evaporation and the residue was purified by column chromatography (mobile phase: methanol/dichloromethane 0/100 to 10/90) to obtain compound **83-c** (273 mg, 37%) as a light brown solid. LC-MS (ESI): m/z = 406.2 (M+H)⁺.

### Synthesis of compound 83-b

To a reaction flask, **83-c** (273 mg, 0.67 mmol), DMF (5 mL) and sodium thiomethoxide (236 mg, 3.37 mmol) were added respectively and the mixture was stirred at 60°C for 2 h under protection of N₂. The reaction mixture was cooled to room temperature, quenched by water, adjusted pH to 7-8 with 1M hydrochloric acid, extracted with ethyl acetate (40mL* 10), the organic phase was dried over anhydrous sodium sulfate, filtered, concentrated to remove ethyl acetate and DMF, and the residue was purified by column chromatography(mobile phase: ammonia methanol/dichloromethane 0/100 to 10/90) to give compound **83-b** (116mg, 44%) as a light brown solid. LC-MS (ESI): m/z = 392.2 (M+H)⁺.

### Synthesis of compound 83-a

To a reaction flask was added **83-b** (116 mg, 0.3 mmol), dichloromethane (20 mL) and triethylamine (124 µL, 0.9 mmol). The mixture was added trifluoromethanesulfonic anhydride (75 µL, 0.45 mmol) dropwise under dry ice cooling. After addition, the reaction mixture was stirred cooling in dry ice for 30 min, quenched by saturated sodium bicarbonate solution, extracted with dichloromethane (50 mL*2), dried over anhydrous sodium sulfate, filtered, concentrated to dryness by rotary evaporation and the residue was purified by column chromatograpy (mobile phase: methanol/dichloromethane 0/100 to 7/93) to give compound **83-a** (84 mg, 54%) as a light brown solid. LC-MS (ESI): m/z = 524.1 (M+H)⁺.

### Synthesis of compound 83

**83-a** (84 mg, 0.16 mmol), DMSO (5 mL), **44-a** (66 mg, 0.48 mmol) were added respectively to a reaction flask, and the mixture was stirred at room temperature overnight under protection of nitrogen. The next day, the reaction was quenched by adding saturated sodium bicarbonate solution and extracted with ethyl acetate (30 mL*2). The organic phase was washed with brine (30 mL*3), concentrated to dryness by rotary evaporation and the residue was purified by column chromatography (mobile phase: ammonia-methanol/dichloromethane 0/100 to 8/92) to give compound **83** (32 mg, 39%) as a light brown solid. LC-MS (ESI): m/z = 511.2 (M+H)⁺; ¹H NMR (400 MHz, CDCl₃) : *δ* 8.93 (1H, s), 8.16 (1H, d, *J =* 7.6Hz), 7.64-7.37 (4H, m), 7.21 (1H, s), 6.89 (1H, s), 4.89 (1H, d, *J* = 16.0Hz), 4.78 (1H, s), 4.71-4.56 (2H, m), 4.47 (1H, s), 4.41-4.29 (2H, m), 4.27-4.08 (2H, m), 4.01 (1H, d, *J =* 11.6Hz), 3.42-3.08 (3H, m), 2.91-2.69 (2H, m), 2.56 (3H, s), 2.44-2.27 (1H, m), 2.21-2.20 (1H, m), 1.98-1.76 (3H, m), 1.35-1.19 (3H, m).

### Synthetic methods for similar compounds

| Compound | Structure | Synthetic method |
|---|---|---|
| **83A** | 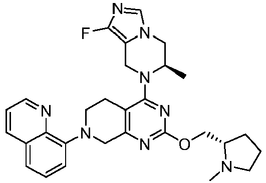 | Synthesized according to synthesis of compound 83, compound 67-a was used instead of compound 44-a. |
| **83B** | 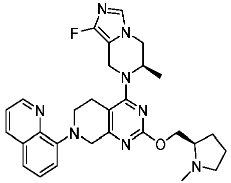 | Synthesized according to synthesis of compound 83, *N-*methyl-D-prolinol was used instead of *N*-methyl-L-prolinol, and compound 67-a was used instead of compound 44-a. |
| **83C** | 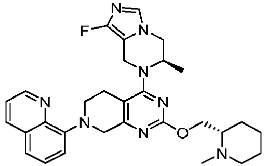 | Synthesized according to synthesis of compound 83, (*S*)-1-Methylpiperidine-2-methanol was used instead of *N*-methyl-L-prolinol, and compound 67-a was used instead of compound 44-a. |
| **83D** | 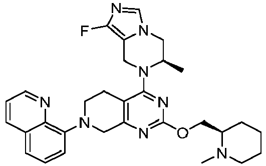 | Synthesized according to synthesis of compound 83, (*R*)-1-Methylpiperidine-2-methanol was used instead of *N*-methyl-L-prolinol, and compound 67-a was used instead of compound 44-a. |

### Example 85: Synthetic route of compound 84

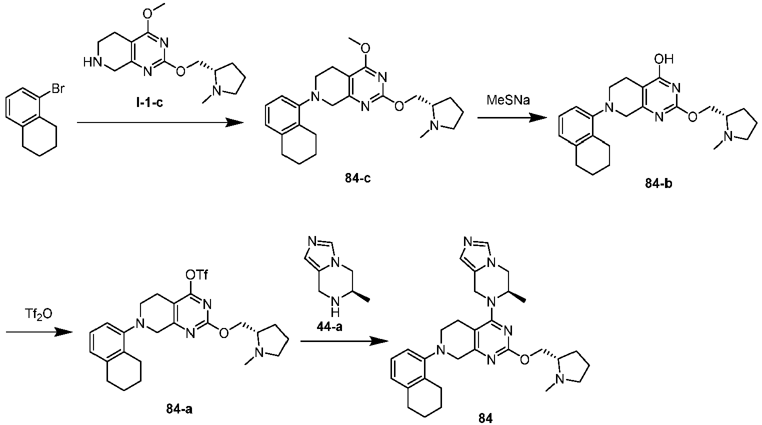

### Synthesis of compound 84-c

To a reaction flask were added 5-bromo-1,2,3,4-tetrahydronaphthalene (378 mg, 1.80 mmol), **I-1-c** (500 mg, 1.80 mmol), dioxane (50 mL), cesium carbonate (2.93 g, 9.00 mmol), RuPhos (168 mg, 0.36 mmol) and Pd₂(dba)₃ ( 165 mg, 0.18 mmol). The mixture was degassed and purged with nitrogen for three times and stirred at 100°C overnight. The next day, the reaction mixture was concentrated to dryness by rotary evoporation and the residue was purified by column chromatography (mobile phase: ammonia-methanol/dichloromethane 0/100 to 5/95) to give compound **84-c** (130 mg) as a light brown solid. LC-MS (ESI): m/z = 408.8 (M+H)⁺.

### Synthesis of compound 84-b

To a reaction flask were added **84-c** (130 mg, 0.32 mmol), DMF (2 mL) and sodium thiomethoxide (112 mg, 1.59 mmol) respectively and the mixture was stirred at 60°C for 1 h under the protection of nitrogen. The reaction mixture was cooled to room temperature, quenched by water, adjusted pH to 7-8 with 1M hydrochloric acid, and extracted with ethyl acetate (50mL*2). The organic phase was dried over anhydrous sodium sulfate, concentrated to remove ethyl acetate and DMF, and the residue was purified by column chromatography (mobile phase: ammonia-methanol/dichloromethane 0/100 to 8/92) to give compound **84-b** (40 mg, 32%) as a light brown solid. LC-MS (ESI): m/z = 395.2 (M+H)⁺.

### Synthesis of compound 84-a

To a reaction flask were added **84-b** (40 mg, 0.1 mmol), methylene chloride (10 mL) and triethylamine (42 µL, 0.3 mmol), then trifluoromethanesulfonic anhydride (26 µL, 0.15 mmol) was added dropwise to the mixture under cooling of dry ice. After addition, the reaction mixture was stirred while cooling in dry ice for 30 min. The reaction mixture was added 2 00 µL of triethylamine and 30 µL of trifluoromethanesulfonic anhydride and stirred for another 30 min. The reaction was quenched by adding saturated sodium bicarbonate solution, extracted with dichloromethane (30 mL*2), dried over anhydrous sodium sulfate, filtered and concentrated to dryness by rotary evaporation to give compound **84-a** (45 mg, 84%) as a light brown solid. LC-MS (ESI): m/z = 527.1 (M+H)⁺.

### Synthesis of compound 84

To a reaction flask were added **84-a** (45 mg, 0.086 mmol), DMSO (4 mL) and **44-a** (35 mg, 0.26 mmol) respectively and the mixture was stirred at room temperature overnight under the protection of nitrogen. The next day, the reaction was quenched by saturated sodium bicarbonate solution and extracted with ethyl acetate (30 mL*2). The organic phase was washed with brine (30 mL*3), concentrated to dryness by rotary evaportaion and the residue was purified by column chromatography (mobile phase: ammonia-methanol/dichloromethane 0/100 to 5/95) to give compound **84** (32 mg, 39%) as a white solid. LC-MS (ESI): m/z = 514.3 (M+H)⁺; ¹H NMR (400 MHz, CDCl₃) : *δ* 7.47 (1H, s), 7.12 (1H, t, *J* = 7.6Hz), 6.94-6.86 (3H, m), 4.85 (1H, d, *J* = 16.4Hz), 4.80-4.71 (1H, m), 4.62 (1H, d, *J* = 16.0Hz), 4.54-4.43 (1H, m), 4.35 (1H, dd, *J* = 12.0Hz, *J =* 4.4Hz), 4.26-4.17 (1H, m), 4.13-3.94 (3H, m), 3.31-3.16 (2H, m), 3.06-2.88 (2H, m), 2.87-2.71 (5H, m), 2.68-2.59 (1H, m), 2.56 (3H, s), 2.45-2.33 (1H, m), 2.17-2.05 (1H, m), 1.98-1.75 (8H, m), 1.31 (3H, d, *J*=7.2Hz).

### Example 86: Synthetic route of compound 85

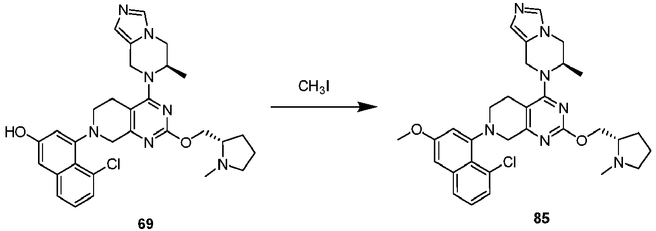

### Synthesis of coumpound 85

To a reaction flask containing **69** (26 mg, 0.046 mmol) were added DMF (3 mL) and potassium carbonate (19.3 mg, 0.14 mmol) respectively, and after stirring at room temperature, the reaction mixture was slowly added iodomethane (8 mg, 0.056 mmol). The mixture was stirred at room temperature for 2 hours. Filtered, the compound 85 (3.4 mg, 13%) was obtained by Pre-HPLC. LC-MS (ESI): m/z 574.2 (M+H) ⁺.

### Example 87: Synthetic route of compound 86

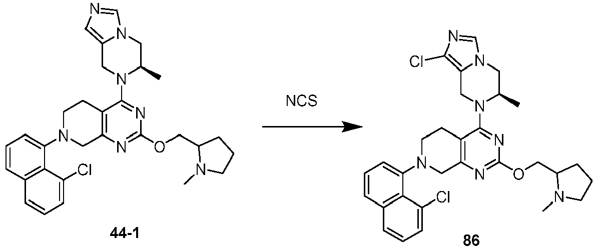

### Synthesis of compound 44-1

Synthesized according to synthesis of compound 44, 1-Methyl-2-pyrrolidinemethanol was used instead of N-methyl-L-prolinol.

### Synthesis of compound 86

To a reaction flask were added compound **44-1** (100 mg, 0.18 mmol), ethanol (5 mL) and *N-*chlorosuccinimide (NCS) (27 mg, 0.20 mmol), respectively, and the mixture was stirred at room temperature under the protection of nitrogen for three days. The reaction mixture was filtered and the filtrate was purified by Pre-HPLC (NH₄HCO₃) and the sample was lyophilized to give compound **86** (5.2 mg, 5%) as a white solid. LC-MS (ESI): m/z = 578.2 (M+H)⁺; ¹H NMR (400 MHz, CDCl₃) : *δ* 7.76 (1H, d, *J* = 8Hz), 7.65-7.57 (1H, m), 7.53 (1H, d, *J* = 7.6Hz), 7.48 (1H, t, *J* = 7.6Hz), 7.39-7.31 (2H, m), 7.29-7.13 (1H, m), 4.92-4.76 (1H, m), 4.74-4.13 (6H, m), 4.07-3.73 (2H, m), 3.65-3.37 (1H, m), 3.42-3.02 (3H, m), 2.84-2.70 (1H, m), 2.63-2.48 (4H, m), 2.41-2.29 (1H, m), 2.15-2.06 (1H, m), 1.97-1.85 (3H, m), 1.39-1.27 (3H, m).

### Synthetic methods for similar compounds

| Compound | Structure | Synthetic method |
|---|---|---|
| **86A** | 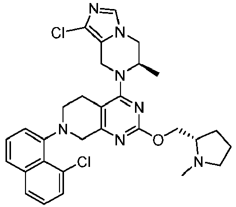 | Synthesized according to synthesis of compound **86**,compound **44** was used instead of **44-I.** |
| **86B** | 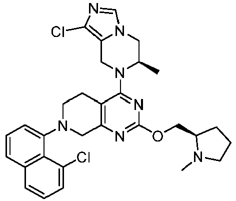 | Synthesized according to synthesis of compound **86**,compound **58** was used instead of **44-I.** |
| **86C** | 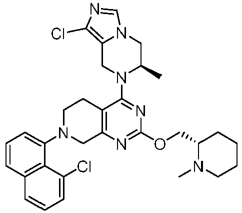 | Synthesized according to synthesis of compound **44** and **86,** compound **99-a** was used instead of compound **1-1.** |
| **86D** | 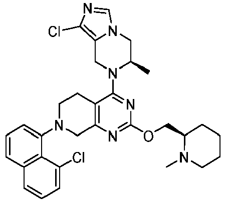 | Synthesized according to synthesis of compound **99-a, 44** and **86,** (*R*)-1-Methylpiperidine-2-methanol was used instead of (*S*)-1-methylpiperidine-2-methanol. |
| **86E** | 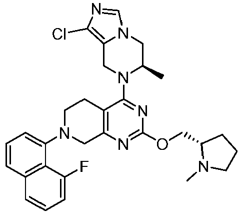 | Synthesized according to synthesis of compound **44** and **86,** compound **106-a** was used instead of compound **I-1.** |
| **86F** | 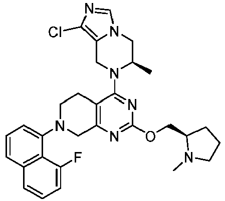 | Synthesized according to synthesis of compound **106-a, 44** and **86,** *N-*methyl-D-prolinol was used instead of *N*-methyl-L-prolinol. |
| **86G** | 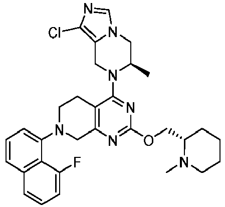 | Synthesized according to synthesis of compound **44** and **86,** compound **113-a** was used instead of **1-1.** |
| **86H** | 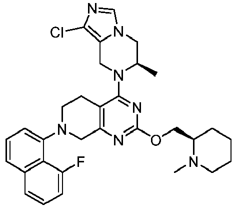 | Synthesized according to synthesis of compound **113-a, 44** and **86,** (*R*)-1-methylpiperidine-2-methanol was used instead of (*S*)-1-methylpiperidine-2-methanol. |

### Example 88: Synthetic route of compound 87 and 89

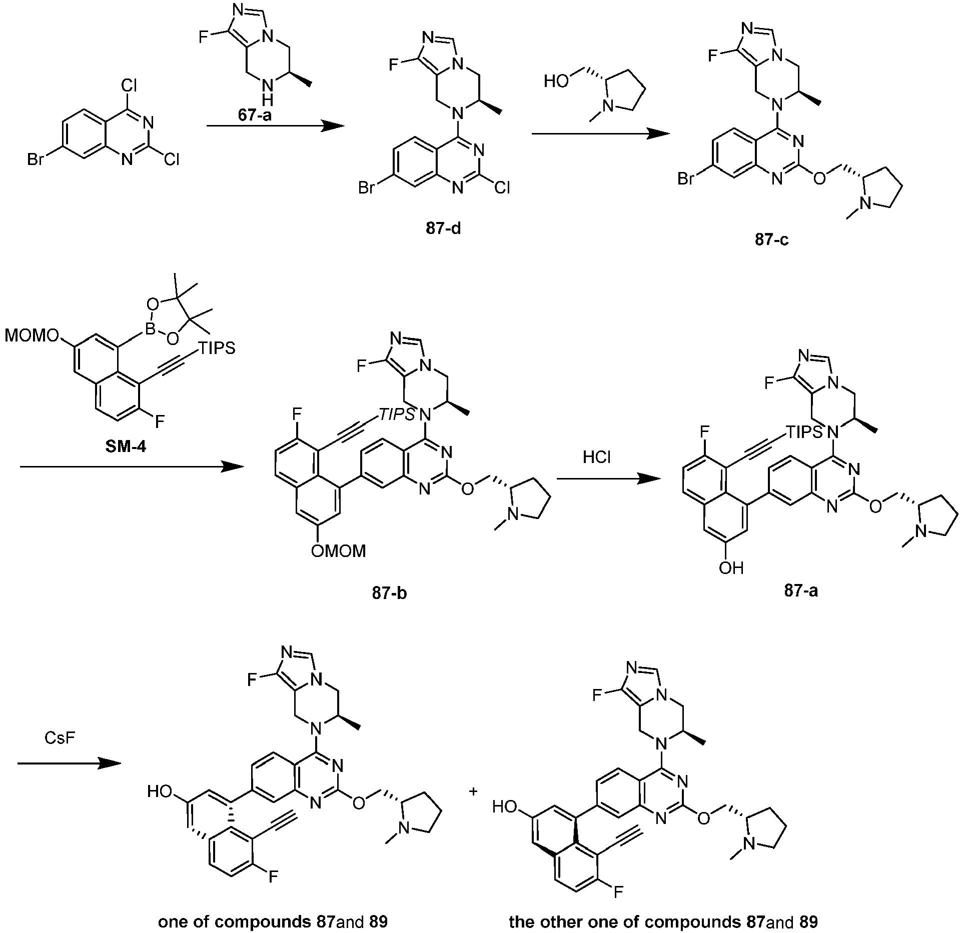

### Synthesis of compound 87-d

To a reaction flask were added 2,4-dichloro-7-bromoquinazoline (100 mg, 0.36 mmol), **67-a** (67 mg, 0.43 mmol), tetrahydrofuran (10 mL) and DIPEA (299 µL, 1.81 mmol), respectively, and the mixture was stirred at room temperature overnight under the protection of nitrogen. The next day, the reaction was quenched by water and extracted with ethyl acetate (30 mL*2). The organic phase was washed with brine three times, dried over anhydrous sodium sulfate and concentrated to dryness to give compound **87-d** (137 mg, 96%) as a light yellow solid, which was used directly in the next step without purification. LC-MS (ESI): m/z = 395.9 (M+H)⁺.

### Synthesis of compound 87-c

To a reaction vial were added **87-d** (137 mg, 0.35 mmol), toluene (10 mL), N-methyl-L-prolinol (206 µL, 1.73 mmol) and sodium tert-butoxide (67 mg, 0.70 mmol), respectively, and the mixture was stirred at room temperature for 1 h under the protection of nitrogen. The reaction was purified directly by column chromatography (mobile phase: methanol/dichloromethane 0/100 to 10/90) to give compound **87-c** (117 mg, 71%) as an off-white solid. LC-MS (ESI): m/z = 475.1 (M+H)⁺.

### Synthesis of compound 87-b

**87-c** (117 mg, 0.30 mmol), 1,4-dioxane (20 mL), **SM-4** (182 mg, 0.36 mmol), water (2 mL), cesium carbonate (290 mg, 0.90 mmol) and tetrakis(triphenylphosphine)palladium (34 mg, 0.03 mmol) were added to a reaction flask, respectively. After degassed and purged with nitrogen for three times, the mixture was stirred at 100°C overnight. The next day, the mixture was concentrated to dryness and the residue was purified by column chromatography (mobile phase: ammonia-methanol/dichloromethane 0/100 to 10/90) to give compound **87-b** (81 mg, 53%) as a light brown solid.

### Synthesis of compound 87-a

To a reaction flask were added **87-b** (40 mg, 0.064 mmol) and acetonitrile (3 mL) respectivelyrespectively. The flask was cooled to -10°C, and was added 4 M 1,4-dioxane solution of hydrogen chloride (1 mL). The reaction mixture was stirred at this temperature for 30 min, quenched by adding 7M ammonia-methanol, concentrated to dryness, added ammonia-methanol/dichloromethane (1:10) solution, filtered, and the filtrate was concentrated to dryness to obtain compound **87-a** (crude) as a light brown solid, which was used directly in the next step without purification. LC-MS (ESI): m/z = 581.2 (M+H)⁺, 737 (M+H)⁺.

### Synthesis of compounds 87 and 89

**87-a** (crude, 0.064 mmol), DMF (2 mL), and cesium fluoride (97 mg, 0.64 mmol) were added respectively to a reaction flask, and the mixture was stirred at room temperature overnight under the protection of nitrogen. The next day, the reaction mixture was filtered and the filtrate was purified by pre-HPLC (NH₄HCO₃), and the sample was lyophilized to give compound **87** (4.2 mg, 11%) as an off-white solid and **89** (7 mg, 19%) as a light brown solid.

Conditions for LC-MS:
Mobile phase: A:water(10mM NH₄HCO₃); B:acetonitrile
Gradient: 5%B increases to 95%B in 1.3 minutes, 95%B lasts for 1.7 minutes
Flow rate: 1.8mL/min
Column: Xbridge C18,3.5µm,4.6*50mm
Column temperature: 45°C
87: LC-MS (ESI): m/z = 581.3 (M+H)⁺, retention time: 1.781 (UV254); ¹H NMR (400 MHz, CDCl₃) : *δ* 7.75-7.65(2H, m), 7.64-7.58(1H, m), 7.32-7.28(1H, m), 7.25-7.16(2H, m), 7.14-7.01(1H, m), 5.24-5.03(2H, m), 5.00-4.80(2H, m), 4.67-4.38(2H, m), 4.04-3.93(1H, m), 3.69-3.50(1H, m), 3.48-3.29(1H, m), 2.93-2.70(5H, m), 2.32-2.11(2H, m), 2.09-1.96(2H, m), 1.43-1.34(3H, m).
**89:** LC-MS (ESI): m/z = 581.2 (M+H)⁺ , retention time: 1.961 (UV254); ¹H NMR (400 MHz, CDCl₃) : *δ* 8.10(1H, s), 7.96-7.91(1H, m), 7.87-7.81(1H, m), 7.72-7.64(1H, m), 7.61-7.53(1H, m), 7.51-7.40(2H, m), 7.16-6.98(2H, m), 4.95-4.79(3H, m), 4.73-4.63(2H, m), 4.46-4.36(1H, m), 4.31-4.21(1H, m), 3.88-3.79(1H, m), 3.45-3.32(1H, m), 3.17-3.03(1H, m), 2.82(3H, s), 2.67-2.48(3H, m), 2.26-2.15(2H, m), 1.26(3H, d, *J*=6.4Hz).

### Example 89: Synthetic route of compound 88

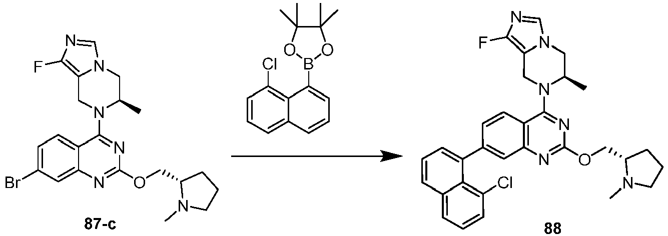

### Synthesis of compound 88

To a reaction flask were added **87-c** (100 mg, 0.21 mmol), 1,4-dioxane (20 mL), 1-(4,4,5,5-tetramethyl-1,2,3-dioxaborolan-2-yl)-8-chloro-naphthalene (73 mg, 0.25 mmol), water (2 mL), cesium carbonate (206 mg, 0.63 mmol) and Pd( PPh₃)₄ (24 mg, 0.021 mmol), and the mixture was degassed and purged with nitrogen for three times and stirred at 100°C overnight. The next day, the reaction mixture was concentrated to dryness and the residue was purified by column chromatography (mobile phase: ammonia-methanol/dichloromethane 0/100 to 5/95), then purified by pre-HPLC (NH₄HCO₃), and the sample was lyophilized to give compound **88** (29 mg, 25%) as a light brown solid. LC-MS (ESI): m/z = 557.3 (M+H)⁺; ¹H NMR (400 MHz, CDCl₃) : δ 7.94 (1H, d, *J =* 8Hz), 7.88 (1H, d, *J =* 8Hz), 7.82-7.75 (1H, m), 7.75-7.72 (1H, m), 7.59-7.50 (2H, m), 7.47-7.38 (2H, m), 7.35-7.28 (1H, m), 7.12 (1H, d, *J =* 3.2Hz), 5.25-5.08 (2H, m), 4.96-4.83 (1H, m), 4.63-4.49 (2H, m), 4.40-4.28 (1H, m), 4.08-3.96 (1H, m), 3.15 (1H, t, *J =* 8Hz), 2.87-2.72 (1H, m), 2.54(3H, s), 2.39-2.26(1H, m), 2.16-2.02 (1H, m), 1.95-1.69 (3H, m), 1.42 (3H, d, *J =* 6.8Hz).

### Synthetic methods for similar compounds

| Compounds | Structure | Synthetic methods |
|---|---|---|
| **88A** | 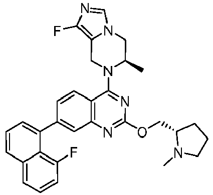 | Synthesized according to synthesis of compound **88,** 1-(4,4,5,5-Tetramethyl-1,2,3-dioxaborolan-2-yl)-8-fluoro-naphthalene was used instead of 1-(4,4,5,5-tetramethyl-1,2,3-dioxaborolan-2-yl)-8-chloronaphthalene. |
| **88B** | 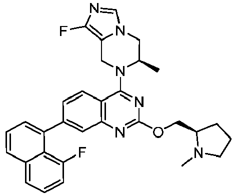 | Synthesized according to synthesis of compound **88,** *N*-methyl-D-prolinol instead of *N*-methyl-L-prolinol and 1-(4,4,5,5-tetramethyl-1,2,3-dioxaborolan-2-yl)-8-fluoronaphthalene was used instead of 1-(4,4,5,5-tetramethyl-1,2,3-dioxaborolan-2-yl)-8-chloronaphthalene. |
| **88C** | 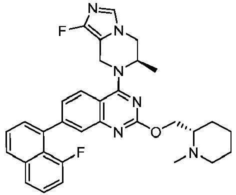 | Synthesized according to synthesis of compound **88,** (*S*)-1-methylpiperidine-2-methanol was used instead of *N*-methyl-L-prolinol, and 1-(4,4,5,5-tetramethyl-1,2,3-dioxaborolan-2-yl)-8-fluoro-naphthalene was used instead of 1-(4,4,5,5-tetramethyl-1,2,3-dioxaborolan-2-yl)-8-chloronaphthalene. |
| **88D** | 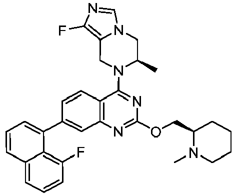 | Synthesized according to synthesis of compound **88,** (*R*)-1-methylpiperidine-2-methanol was used instead of *N*-methyl-L-prolinol, and 1-(4,4,5,5-tetramethyl-1,2,3-dioxaborolan-2-yl)-8-fluoro-naphthalene was used instead of 1-(4,4,5,5-tetramethyl-1,2,3-dioxaborolan-2-yl)-8-chloronaphthalene. |
| **88E** | 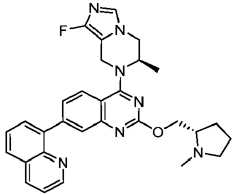 | Synthesized according to synthesis of compound **88,** quinoline-8-boronic acid was used instead of 1-(4,4,5,5-tetramethyl-1,2,3-dioxaborolan-2-yl)-8-chloronaphthalene. |
| **88F** | 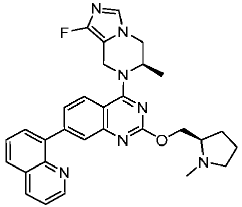 | Synthesized according to synthesis of compound **88,** *N*-methyl-D-prolinol was used instead of *N-*methyl-L-prolinol, and quinoline-8-boronic acid was used instead of 1-(4,4,5,5-tetramethyl-1,2,3-dioxaborolan-2-yl)-8-chloronaphthalene. |
| **88G** | 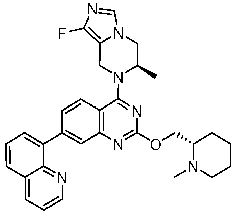 | Synthesized according to synthesis of compound **88,** (*S*)-1-methylpiperidine-2-methanol was used instead of *N*-methyl-L-prolinol, and quinoline-8-boronic acid was used instead of 1-(4,4,5,5-tetramethyl-1,2,3-dioxaborolan-2-yl)-8-chloro-naphthalene. |
| **88H** | 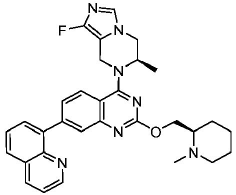 | Synthesized according to synthesis of compound **88,** (*R*)-1-methylpiperidine-2-methanol was used instead of *N*-methyl-L-prolinol, and quinoline-8-boronic acid was used instead of 1-(4,4,5,5-tetramethyl-1,2,3-dioxaborolan-2-yl)-8-chloro-naphthalene. |

### Example 90: Synthetic route of compound 90

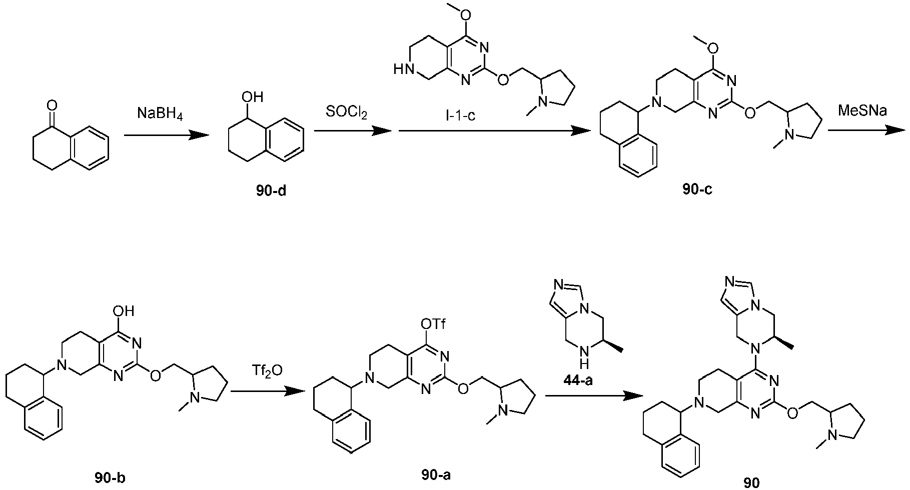

### Synthesis of compound 90-d

To a reaction flask were added 1-tetralone (1.5 g, 10.27 mmol) and methanol (30 mL), and sodium borohydride (586 mg, 15.41 mmol) was added slowly to the above mixture while stirring. After addition, the reaction mixture was stirred at room temperature under the protection of nitrogen for 30 min. The reaction mixture was concentrated to dryness and the residue was purified by column chromatography (mobile phase: ethyl acetate/petroleum ether (0/100 to 30/70) to give compound **90-d** (1.5 g, 99%) as a colorless oil. ¹H NMR (400 MHz, CDCl₃) : δ 7.45-7.38 (1H, m), 7.23-7.16 (2H, m), 7.13-7.06(1H, m), 4.77 (1H, t, *J =* 5.2Hz), 2.88-2.66 (2H, m), 2.06-1.86 (3H, m), 1.84-1.71 (2H, m).

### Synthesis of compound 90-c

To a reaction flask were added **90-d** (400 mg, 2.70 mmol), chloroform (50 mL), and sulfoxide chloride (1.6 mL, 21.6 mmol) respectively, and the mixture was refluxed at 60°C for 2 hours under the protection of nitrogen. The reaction mixture was cooled to room temperature, quenched with water, and extracted with dichloromethane (50 mL*2). The organic phase was dried over anhydrous sodium sulfate and removed the solvent by rotary evaporation to give an oil, to which acetonitrile (30 mL), compound **I-1-c** (225 mg, 0.81 mmol), potassium carbonate (1.9 g, 13.5 mmol) and potassium iodide (90 mg, 0.54 mmol) were added and the mixture was stirred at 90°C overnight under the protection of nitrogen. The reaction mixture was filtered, concentrated by rotary evaporation and the residue was purified by column chromatography (mobile phase: methanol/dichloromethane 0/100 to 10/90) to give compound **90-c** (141 mg, 43%) as a white solid. LC-MS (ESI): m/z = 409.7 (M+H)⁺.

### Synthesis of compound 90-b

To a reaction flask were added **90-c** (141 mg, 0.35 mmol), DMF (2 mL), and sodium thiomethoxide (121 mg, 1.73 mmol) respectively and the mixture was stirred at 60°C for 1 h under the protection of nitrogen. The reaction mixture was cooled to room temperature, quenched with water, adjusted to pH 7 by adding 1 M hydrochloric acid, added a small amount of anhydrous sodium sulfate, and extracted with ethyl acetate (30 mL*3). The organic phase was dried, concentrated to dryness by rotary evaporation and the residue was purified by column chromatography (mobile phase: ammonia-methanol/dichloromethane 0/100 to 10/90) to give compound **90-b** (81 mg, 59%) as a white solid. LC-MS (ESI): m/z = 395.7 (M+H)⁺.

### Synthesis of compound 90-a

**90-b** (81 mg, 0.21 mmol), dichloromethane (20 mL) and triethylamine (87 µL, 0.63 mmol) were added to a reaction flask, and trifluoromethanesulfonic anhydride (53 uL, 0.32 mmol) was added dropwise to the above mixture while stirring and cooling in dry ice. After addition, the reaction mixture was stirred under dry ice cooling for 1 hour. The reaction was quenched with water and extracted with dichloromethane (30 mL*3). The organic phase was dried, concentrated to dryness and the residue was purified by column chromatography (mobile phase: methanol/dichloromethane 0/100 to 5/95) to give compound **90-a** (110 mg, 100%) as a white solid. LC-MS (ESI): m/z = 527.7 (M+H)⁺.

### Synthesis of compound 90

To a reaction vial were added **90-a** (110 mg, 0.21 mmol), DMSO (5 mL) and **44-a** (86 mg, 0.63 mmol) respectively and the mixture was stirred at room temperature overnight under the protection of nitrogen. The next day, the reaction was quenched with water and extracted with ethyl acetate (30 mL*2). The organic phase was washed with brine (30 mL*3), concentrated to dryness and the residue was purified by column chromatography (mobile phase: ammonia-methanol/dichloromethane 0/100 to 5/95) to give compound **90** (63 mg, 59%) as a white solid. LC-MS (ESI): m/z = 514.3 (M+H)⁺; ¹H NMR (400 MHz, CDCl₃) : *δ* 7.78-7.62 (1H, m), 7.45(1H, s), 7.22-7.05 (3H, m), 6.89 (1H, s), 4.93-4.65 (2H, m), 4.64-4.50 (1H, m), 4.48-4.24 (2H, m), 4.22-4.10 (1H, m), 4.06-3.78 (3H, m), 3.78-3.53 (1H, m), 3.15 (1H, bs), 2.87-2.66 (5H, m), 2.63-2.25 (6H, m), 2.13-1.93 (3H, m), 1.85-1.61 (5H, m), 1.32-1.25 (3H, m).

### Example 91: Synthetic route of compounds 91 and 92

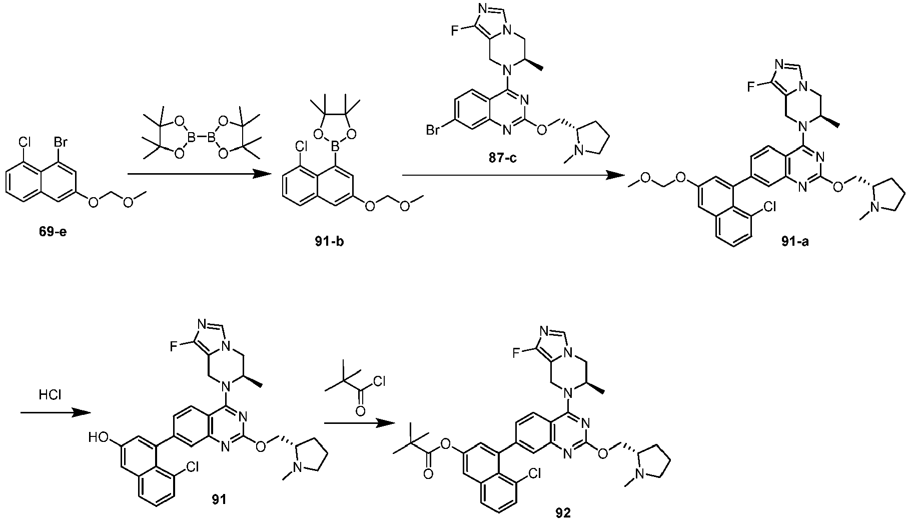

### Synthesis of compound 91-b

**69-e** (1 g, 3.33 mmol), biboronic acid pinacol ester (2.54 g, 10 mmol), 1,4-dioxane (60 mL), potassium acetate (1.96 g, 20 mmol) and PdCl₂(dppf) (244 mg, 0.33 mmol) were added to a reaction flask and the mixture was degassed and purged with nitrogen for three times and stirred at 100°C overnight. The next day, the reaction was quenched with water and extracted with ethyl acetate (50 mL*3). The organic phase was washed with brine, concentrated to dryness and the residue was purified by column chromatography (mobile phase: ethyl acetate/petroleum ether 0/100 to 10/90) to give compound **91-b** (468 mg, 40%) as a white solid. LC-MS (ESI): m/z = 349 (M+H)⁺.

### Synthesis of compound 91-a

To a reaction flask were added **87-c** (50 mg, 0.11 mmol), 1,4-dioxane (10 mL), **91-b** (44 mg, 0.13 mmol), water (1 mL), cesium carbonate (103 mg, 0.32 mmol) and Pd(PPh₃)₄(12 mg, 0.011 mmol), and the mixture was degassed and purged with nitrogen for three times and stirred at 100°C overnight. The next day, the reaction mixture was concentrated to dryness and the residue was purified by column chromatography (mobile phase: ammonia-methanol/dichloromethane 0/100 to 5/95) to give compound **91-a** (51 mg, 78%) as an off-white solid. LC-MS (ESI): m/z = 617.7 (M+H)⁺.

### Synthesis of compound 91

To a reaction vial were added **91-a** (51 mg, 0.083 mmol) and acetonitrile (3 mL). Cooled at -10°C, the mixture was added a solution of hydrogen chloride in1,4-dioxane (4 M, 1 mL) and stirred at the same temperature for 1.5 h. The reaction was quenched with 7M ammonia-methanol solution, concentrated to dryness, added dichloromethane/ammonia-methanol solution (10/1), filtered, the filtrate was concentrated to dryness and the residue was purified by column chromatography (mobile phase: ammonia-methanol/dichloromethane 0/100 to 5/95) to give compound **91** (35 mg, 74%) as an off-white solid. LC-MS (ESI): m/z = 573.1 (M+H)⁺.

### Synthesis of compound 92

To a reaction flask were added **91** (29 mg, 0.051 mmol), dichloromethane (10 mL) and triethylamine (35 µL, 0.25 mmol), and pivaloyl chloride (10 uL, 0.076 mmol) was added to the above mixture in an ice-cold water bath. After addition, the reaction was stirred for 30 min, quenched by adding saturated sodium bicarbonate solution and extracted by dichloromethane (30 mL*2). The organic phase was dried, concentrated to dryness and the residue was purified by column chromatography (mobile phase: ammonia-methanol/dichloromethane 0/100 to 4/96) to give compound **92** (20 mg, 60%) as a white solid. LC-MS (ESI): m/z = 657.1 (M+H)⁺; ¹H NMR (400 MHz, CDCl₃) : δ 7.85-7.72 (3H, m), 7.66 (1H, d, *J =* 2.4Hz), 7.53-7.48 (1H, m), 7.43 (1H, t, *J =* 8Hz), 7.37-7.30 (1H, m), 7.19-7.16 (1H, m), 7.12 (1H, d, *J =* 3.2Hz), 5.24-5.10 (2H, m), 4.96-4.86 (1H, m), 4.72-4.61 (1H, m), 4.60-4.51 (1H, m), 4.48-4.36 (1H, m), 4.07-3.98 (1H, m), 3.32-3.18 (1H, m), 3.00-2.84 (1H, m), 2.61 (3H, s), 2.50-2.34 (1H, m), 2.20-2.06 (1H, m), 1.99-1.78 (3H, m), 1.45-1.40 (3H, m), 1.39 (9H, d, *J =* 1.6Hz).

### Example 92: Synthetic route of compounds 93 and 94

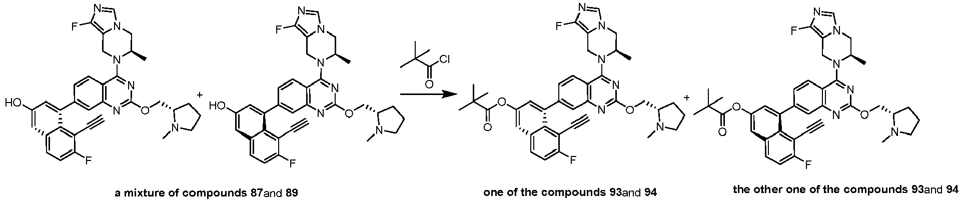

### Synthesis of compounds 93 and 94

To a reaction flask were added a mixture of compounds **87** and **89** (76 mg, 0.13 mmol), dichloromethane (10 mL), triethylamine (90 µL, 0.65 mmol), then pivaloyl chloride (24 uL, 0.2 mmol) was added to the above mixture in an ice-cold water bath. After addition, the reaction mixture was stirred for 30 min, quenched by adding saturated sodium bicarbonate solution and extracted by dichloromethane (30 mL*2). The organic phase was dried over anhydrous sodium sulfate, concentrated to dryness by rotary evaporation, purified by pre-HPLC (NH₄HCO₃), and the sample was lyophilized to give compound **93** (17.6 mg, 20%) as an off-white solid and **94** (16.5 mg, 19%) as a light yellow solid.

Conditions for LC-MS:
Mobile phase: A:water(10mM NH₄HCO₃); B:acetonitrile
Gradient: 5%B increases to 95%B in 1.3 minutes, 95%B lasts for 1.7 minutes
Flow rate: 1.8mL/min
Column: Xbridge C18,3.5µm,4.6*50mm
Column temperature: 45°C
Compound **93:** LC-MS (ESI): m/z = 665.2 (M+H)⁺, retention time 2.378 (UV254); ¹H NMR (400 MHz, CDCl₃) : *δ* 7.92-7.85(1H, m), 7.80-7.74(2H, m), 7.64(1H, d, *J*=2.4Hz), 7.41-7.33(2H, m), 7.25-7.19(1H, m), 7.13(1H, d, *J*=4Hz), 5.25-5.08(2H, m), 4.96-4.86(1H, m), 4.79-4.64(1H, m), 4.62-4.54(1H, m), 4.51-4.39(1H, m), 4.08-3.99(1H, m), 3.42-3.20(1H, m), 3.10-2.88(1H, m), 2.90(1H, d, *J*=14Hz), 2.66(3H, s), 2.55-2.38(1H, m), 2.23-2.09(1H, m), 2.06-1.82(3H, m), 1.44(3H, d, *J*=6Hz), 1.39(9H, d, *J*=2.8Hz).
Compound **94:** LC-MS (ESI): m/z = 665.2 (M+H)⁺ , retention time 2.676 (UV254); ¹H NMR (400 MHz, CDCl₃) : *δ* 8.12-8.06(1H, m), 7.95(1H, d, *J*=8.8Hz), 7.84(1H, dd, *J*=8.8Hz, *J*=4Hz), 7.80(1H, s), 7.76-7.71(1H, m), 7.58(1H, d, *J*=1.6Hz), 7.50(1H, s), 7.32(1H, t, *J*=9.2Hz), 7.15(1H, s), 5.31-5.14(2H, m), 4.99-4.89(1H, m), 4.77-4.65(1H, m), 4.61-4.52(1H, m), 4.12-4.03(1H, m), 3.94-3.73(1H, m), 3.64-3.43(1H, m), 3.08-2.71(4H, m), 2.44-1.96(5H, m), 1.47(3H, d, *J*=6.4Hz), 1.44(9H, s).

### Example 93: Synthetic route of compound 95

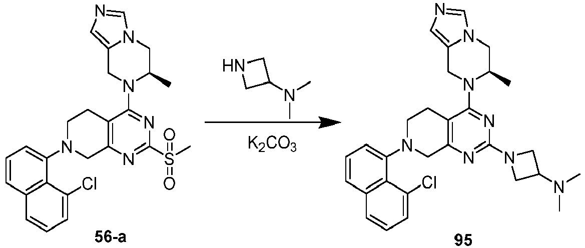

### Synthesis of compound 95

To a solution of **56-a** (160 mg, 0.314 mmol) in DMF (15 mL) were added 3-(dimethylamino)azetidine dihydrochloride (272 mg, 1.572 mmol) and potassium carbonate (434.4 mg, 3.143 mmol) at room temperature, respectively, and the mixture was stirred at 80°C overnight. The next day, the mixture was added water and extracted with ethyl acetate (50 mL*2). The combined organic layers were washed with brine, dried over anhydrous sodium sulfate, filtered and concentrated to dryness, and the crude product was purified by pre-HPLC to give compound **95** (11 mg, 6.61%) as a white solid. LC-MS (ESI): m/z = 529.2 (M+H)⁺.

### Example 94: Synthetic route of compound 96

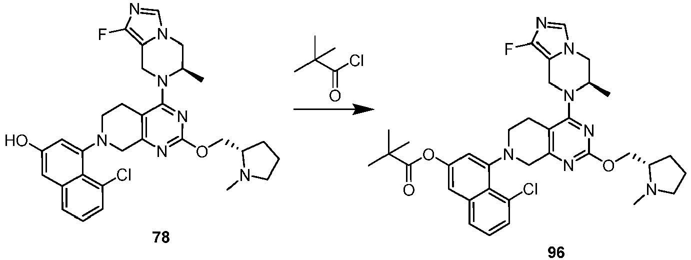

### Synthesis of compound 96

To a reaction flask were added **78** (50 mg, 0.086 mmol), dichloromethane (10 mL) and triethylamine (60 µL, 0.432 mmol), then the mixture was added pivaloyl chloride (16 uL, 0.13 mol) in an ice-cold water bath. After addition, the reaction was stirred for 30 min, quenched by adding saturated sodium bicarbonate solution and extracted by dichloromethane (30 mL*2). The organic phase was dried over anhydrous sodium sulfate, concentrated to dryness and the residue was purified by column chromatography (mobile phase: ammonia-methanol/dichloromethane 0/100 to 6/94) to give compound **96** (44 mg, 77%) as a white solid. LC-MS (ESI): m/z = 662.2 (M+H)⁺; ¹H NMR (400 MHz, CDCl₃) : δ 7.73-7.65 (m, 1H), 7.52-7.45 (m, 1H), 7.37-7.28 (m, 2H), 7.08 (d, 1H, *J* = 8Hz), 6.90 (dd, 1H, *J* = 35.6Hz, *J* = 2.4Hz), 4.97-4.58 (m, 3H), 4.56-4.34 (m, 3H), 4.28-4.11 (m, 1H), 4.05-3.74 (m, 2H), 3.67-3.47 (m, 1H), 3.41-3.14 (m, 2H), 3.08-2.74 (m, 2H), 2.66-2.48 (m, 4H), 2.46-2.32 (m, 1H), 2.17-2.03 (m, 1H), 1.97-1.74 (m, 3H), 1.43-1.28 (m, 12H).

### Example 95: Synthetic route of compound 97

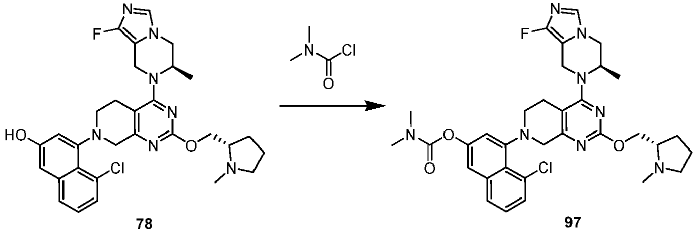

### Synthesis of compound 97

To a reaction flask were added **78** (100 mg, 0.173 mmol), DMF (10 mL), potassium carbonate (72 mg, 0.52 mmol), dimethylcarbamoyl chloride (28 mg, 0.26 mmol), respectively, and the mixture was stirred at room temperature under the protection of nitrogen for 2 hours. The reaction was quenched with water and extracted with ethyl acetate (30 mL*2). The organic phase was washed with brine (30 mL*3), concentrated to dryness and the residue was purified by column chromatography (mobile phase: ammonia-methanol/dichloromethane 0/100 to 5/95) to give compound **97** (82 mg, 73%) as a white solid. LC-MS (ESI): m/z = 649.3 (M+H)⁺; ¹H NMR (400 MHz, CDCl₃): δ 7.72-7.64 (m, 1H), 7.50-7.44 (m, 1H), 7.40-7.29 (m, 2H), 7.08 (d, 1H, *J =* 9.2Hz), 6.98 (dd, 1H, *J* = 45.2Hz, *J* = 2.4Hz), 4.93-4.56 (m, 3H), 4.53-4.35 (m, 3H), 4.26-4.13 (m, 1H), 4.08-3.73 (m, 2H), 3.67-3.43 (m, 1H), 3.36-2.94 (m, 9H), 2.86-2.73 (m, 1H), 2.67-2.48 (m, 4H), 2.44-2.31 (m, 1H), 2.15-2.03 (m, 1H), 1.94-1.74 (m, 3H), 1.32 (dd, 3H, *J* = 15.6Hz, *J* = 6.8Hz).

### Example 96: Synthetic route of compound 98

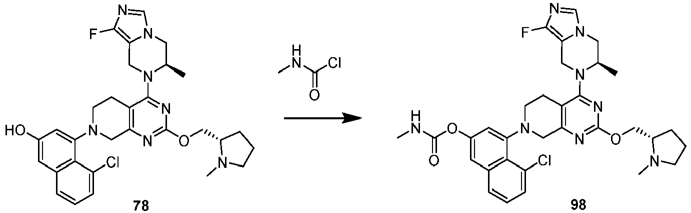

### Synthesisi of compound 98

To a reaction vial were added 78 (2 g, 3.46 mmol), dichloromethane (50 mL) and DIPEA (3.01 mL, 17.3 mmol), and the mixture was added a solution of methylcarbamic chloride in dichloromethane while cooling in ice-cold water. After addition, the mixture was stirred at room temperature under nitrogen atmosphere for 2 hours. The organic phase was dried over anhydrous sodium sulfate, concentrated to dryness and the residue was purified by column chromatography (mobile phase: ammonia-methanol/dichloromethane 0/100 to 4/96) to give compound 98 (637 mg, 29%) as a white solid. LC-MS (ESI): m/z = 635.2 (M+H)⁺.

### Example 97: Synthetic route of compound 99

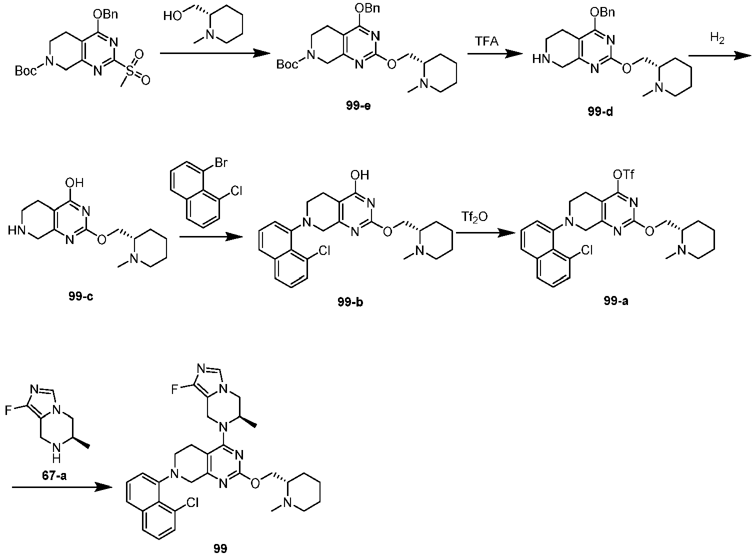

### Synthesis of compound 99-e

Toluene(9 mL) and solid sodium *tert*-butoxide (0.82 g, 8.53 mmol) were combined in a reaction flask at room temperature. Cooled to 0 °C in an ice-cold water bath, the mixture was added ( *S*)-1-methylpiperidine-2-methanol (1.02 g, 7.89 mmol) dropwise. The reaction was stirred in the ice-cold water bath for half an hour after the dropwise addition. The reaction was added *tert-*butyl 4-benzyloxy-2-methylsulfonyl-5,8-piperidino[3,4-d]-pyrimidine-7(6H)-carboxylate (3.00 g, 7.15 mmol) in toluene (24 mL) dropwise in an ice-cold water bath and stirred in the ice-cold water bath until the reaction was complete as monitored by LCMS. The reaction was quenched by adding brine (40 mL), extracted with ethyl acetate(40 mL), partitioned, and the organic phase was dried over anhydrous magnesium sulfate and concentrated to give compound 99-e (3.35 g) which was used directly in the next step. LC-MS (ESI): m/z = 469.7 (M+H)⁺.

### Synthesis of compound 99-d

99-e (3.35 g, 7.15 mmol) and toluene (18 mL) were combined in a reaction vial, and the resulting mixture was added TFA (8.15 g, 71.48 mmol) and stirred at room temperature for 1 h. The reaction was completed as monitored by LCMS, quenched by adding 5% aqueous sodium hydroxide, adjusted pH to 10, and extracted with ethyl acetate (50 mL). The organic phase was dried over Na₂SO₄ and concentrated to obtain compound 99-d (2.1 g), which was used directly in the next step. LC-MS (ESI): m/z = 369.3 (M+H)⁺.

### Synthesis of compound 99-c

99-d (2.1 g, 5.70 mmol) was dissolved in isopropanol (20 mL), was added purified water (6 mL) and 10% Pd/C (100 mg) after degassed and purged with nitrogen. Then purged with hydrogen, the reaction was stirred at room temperature under atmosphere of hydrogen. The reaction was completed as monitored by LCMS. The reaction mixture was filtered and the filtrate was concentrated to dryness. The resulting solid was slurried with a mixture of isopropanol (1 mL) and methyltetrahydrofuran (10 mL). Filtered and dried in vacuum to give compound 99-c (1.1 g, 55% total yield in three steps). LC-MS (ESI): m/z = 279.2 (M+H)⁺; ¹H NMR (400 MHz, D₂O) δ 4.32 (dd, *J* = 3.5 Hz, 12.1 Hz, 1H), 4.12(dd, *J* = 4.0 Hz, 12.3 Hz, 1H), 3.58 (s, 2H), 3.13-3.09 (m, 1H), 2.98 (t, *J* = 6.0 Hz, 2H), 2.96-2.92 (m, 1H), 2.72-2.65 (m, 1H), 2.55 (s, 3H), 2.33 (t, *J* = 5.9 Hz, 2H), 1.75-1.65 (m, 3H), 1.65-1.50 (m, 2H), 1.40-1.30 (m, 1H).

### Synthesis of compound 99-b

To a reaction flask were added 99-c (278 mg, 1.00 mmol), toluene (20 mL), BINAP (124 mg, 0.20 mmol), Pd₂(dba)₃ (91 mg, 0.10 mmol), sodium *tert*-butoxide (479 mg, 5.99 mmol) and 1-bromo-8-chloronaphthalene (289 mg, 1.20 mmol), the resluting mixture was stirred at 110°C under nitrogen atmosphere for 3 hours. The reaction mixtuere was filtered, and the organic phase was concentrated to dryness and the residue was purified by column chromatography (mobile phase: methanol (with NH₃)/dichloromethane 1/20 to 1/10) to give compound 99-b (250 mg, 57%). LC-MS (ESI): m/z 439.6 (M+H) ⁺.

### Synthesis of compound 99-a

To a reaction vial were added 99-b (250 mg, 0.57 mmol), DIPEA (589 mg, 4.56 mmol) and dichloromethane (50 mL). The resulting mixture was stirred in a dry ice bath (about -40°C) under nitrogen atmosphere for 10 min, then was added Tf₂O (289 mg, 1.03 mmol) dropwise slowly and stirred for 60 min. The reaction was quenched with saturated sodium bicarbonate (100 mL), extracted with dichloromethane (100 mL*3) and the organic phases was concentrated to dryness to give compound 99-a (300 mg, 92%). LC-MS (ESI): m/z 571.6 (M+H) ⁺.

### Synthesis of compound 99

99-a (150 mg, 0.26 mmol), DMAC (3 mL), 67-a (60 mg, 0.39 mmol) and DIPEA (170 mg, 1.32 mmol) were combined in a reaction vial and stirred at 100°C for 1 h under nitrogen atmosphere. The reaction was quenched with brine (20 mL), filtered the solid, and the crude product was purified by column chromatography (mobile phase: methanol (with NH₃)/dichloromethane 1/20 to 1/10) and compound 99 was concentrated (28 mg, 19%). LC-MS (ESI): m/z 576.3 (M+H) ⁺; ¹H NMR (400MHz, DMSO-d6): *δ*7.88-7.98 (1H, m), 7.75 (1H, t, *J =* 7.2Hz), 7.21-7.56 (5H, m), 4.52-4.77 (2H, m), 3.65-4.43 (6H, m), 3.34-3.60 (1H, m), 2.83-3.33 (2H, m), 2.72-2.82 (1H, m), 2.53-2.72 (1H, m), 2.11-2.40 (4H, m), 1.91-2.11 (1H, m), 1.62-1.78 (2H, m), 1.01-1.61 (8H, m).

### Synthetic method for similar compound

| Compound | Structure | Synthetic methods |
|---|---|---|
| **100** | 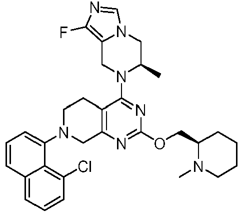 | Synthesized according to synthesis of compound 99, (*R*)-1-methylpiperidine-2-methanol was used instead of (*S*)-1-methylpiperidine-2-methanol. |

### Example 98: Synthetic route of compound 101

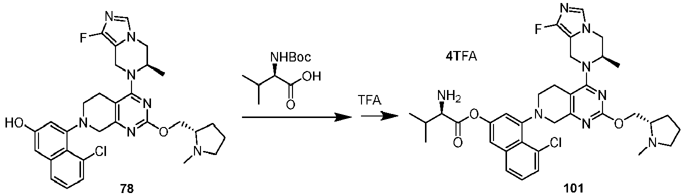

### Synthesis of compound 101

EDCI (25 mg, 0.13 mmol) and DMAP (2 mg, 0.017 mmol) were added sequentially to a solution of compound Boc-D-valine (24 mg, 0.11 mmoL) and compound 78 (50 mg, 0.086 mmol) in dichloromethane(10 mL) at room temperature. The resulting mixture was stirred at room temperature under nitrogen atmosphere for 60 min, and then concentrated at reduced pressure to obtain the crude intermediate. The crude intermediate was dissolved in dichloromethane (3 mL), cooled to 0°C, added TFA (1 mL) dropwise, and the reaction mixture was warmed to room temperature and stirred for 1 hour. The reaction mixture was concentrated at reduced pressure to obtain the crude compound. The crude product was dissolved in acetonitrile (2 mL), purified by reversed phase flash chromatography (C18, 1% TFA in water/acetonitrile 5% to 50%) and lyophilized to give compound 101 (80 mg, 82%). LC-MS (ESI): m/z = 677.3 [M+1]⁺; ¹H NMR (400 MHz, DMSO-*d*6): *δ* 9.90 (1H, brs), 8.67 (3H, s), 7.98 (1H, d, *J*=8.4Hz), 7.67- 7.58 (2H, m), 7.52 (1H, t, *J*=8.0Hz), 7.39 (1H, d, *J*=9.2Hz), 7.11 (1H, dd, *J*1=1.6Hz, J2=35.2Hz), 4.82-3.98 (10H, m), 3.58-3.30 (3H, m), 3.20-3.05 (2H, m), 3.05-2.72 (5H, m), 2.63 (1H, d, *J*=14.8Hz), 2.45-2.49 (1H, m), 2.49-2.15 (1H, m), 2.10-1.97 (1H, m), 1.98-1.75 (2H, m), 1.27-1.04 (10H, m).

### Example 99: Synthetic route of compound 102

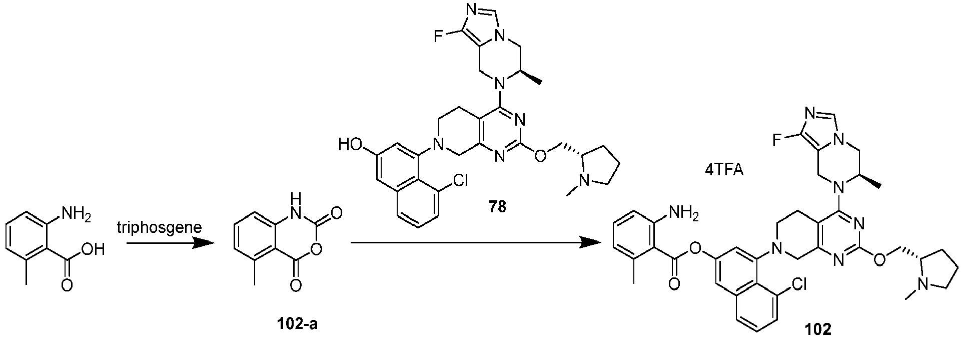

### Synthesis of compound 102-a

To a reaction vial were added 2-amino-6-methylbenzoic acid (876 mg, 5.80 mmol), 1,4-dioxane (10 mL) and triphosgene (570 mg, 1.92 mmol), and the resulting mixture was refluxed at 110 °C under nitrogen atmosphere for 2 hours. The reaction mixture was cooled with ice-cold water, filtered, and the filter cake was washed with petroleum ether to give compound 102-a (916 mg, 89%).

### Synthesis of compound 102

To a reaction vial were added 78 (50 mg, 0.086 mmol), dichloromethane (25 mL), 102-a (31 mg, 0.17 mmol) and DMAP (32 mg, 0.26 mmol), and the resulting mixture was refluxed at 40 °C under atmosphere of nitrogen overnight. The reaction mixture was concentrated to dryness, and the residue was dissolved in acetonitrile(1 mL) and 1 M hydrochloric acid(1 mL), purified by pre-HPLC (TFA), and lyophilized to give compound 102 (20 mg, 20%). LC-MS (ESI): m/z = 711.4 (M+H)⁺; ¹H NMR (400 MHz, DMSO-d6) : *δ* 9.67(1H, brs), 7.99-7.91(1H, m), 7.70(1H, dd, *J*=10.8Hz, J=2.4Hz), 7.60(1H, d, *J*=7.6Hz), 7.50(1H, t, *J*=8.0Hz), 7.39(1H, d, *J*=11.6Hz), 7.35-7.09(2H, m), 6.69(1H, t, *J*=7.2Hz), 6.49(1H, t, *J*=7.2Hz), 4.99-4.37(6H, m), 4.35-3.91(6H, m), 3.87-3.75(3H, m), 3.27-2.99(3H, m), 2.97-2.74(3H, m), 2.71-2.58(1H, m), 2.35-2.15(1H, m), 2.12-1.75(3H, m), 1.20(3H, dd, J=24.8Hz, *J*=6.4Hz); 19F NMR(400M, DMSO-*d*6) : *δ* -69.75(12F, s), - 134.81(1F, d, *J*=112Hz).

### Example 100: Synthetic route of compound 103

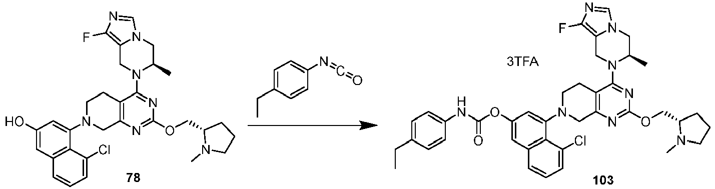

### Synthesis of compound 103

To a reaction flask were added 78 (50 mg, 0.086 mmol), DCM (5 mL), acetonitrile (5 mL), and DMAP (10 mg, 0.08 mmol). The resulting mixture was degassed and purged with nitrogen and stirred under atmosphere of nitrogen for 10 min, then was added 4-ethylphenyl isocyanate (38 mg, 0.24 mmol) and stirred at room temperature overnight. The reaction mixture was concentrated and the residue was purified by pre-HPLC to give compound 103 (15 mg, 16%) as a yellow solid. LC-MS (ESI): m/z 725.4 (M+H) ⁺.

### Synthetic methods for similar compounds

| Compounds | Structure | Synthetic methods |
|---|---|---|
| **103A** | 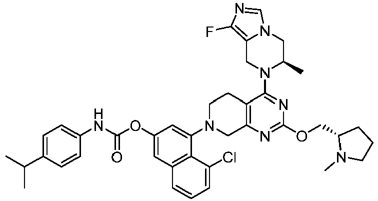 | Synthesized according to synthesis of compound **103, 4-**isopropylphenyl isocyanate was used instead of 4-ethylphenyl isocyanate. |
| **103B** | 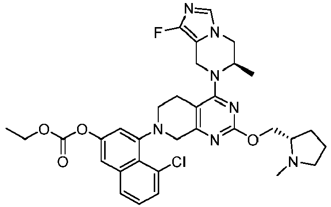 | Synthesized according to synthesis of compound 98, ethyl chloroformate was used instead of methylaminocarbonyl chloride. |

### Example 101: Synthetic route of compound 104

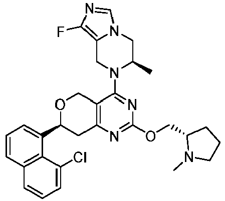

Synthesized according to synthesis of compound 3, 67-a was used instead of 5,6,7,8-tetrahydroimidazo[1,5-α]pyrazine, LC-MS (ESI): m/z 563.2 (M+H) ⁺.

### Example 102: Synthetic route of compound 105

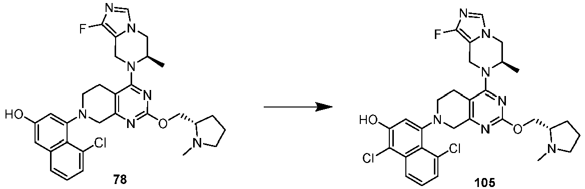

### Synthesis of compound 105

To a reaction vial were added 78 (100 mg, 0.17 mmol), dichloromethane (10 mL) and NCS (35 mg, 0.26 mmol) and the resulting mixture was stirred at room temperature under nitrogen atmosphere for 1 hour. The reaction mixture was purified directly by column chromatography (mobile phase: ammonia-methanol/dichloromethane=0/100 to 8/92), then pre-HPLC (NH₄HCO₃) and lyophilized to give compound 105 (14 mg, 13%). LC-MS (ESI): m/z = 612.2 (M+H)⁺; ¹H NMR (400 MHz, CDCl₃) : *δ* 8.12-8.04(1H, m), 7.46-7.37(2H, m), 7.12-7.01(2H, m), 4.94-4.32 (6H, m), 4.23-3.03(7H, m), 2.97-2.48(5H, m), 2.35-1.92 (5H, m), 1.35(3H, dd, *J*=18.4Hz, *J*=6.8Hz).

### Example 103: Synthetic route of compound 106

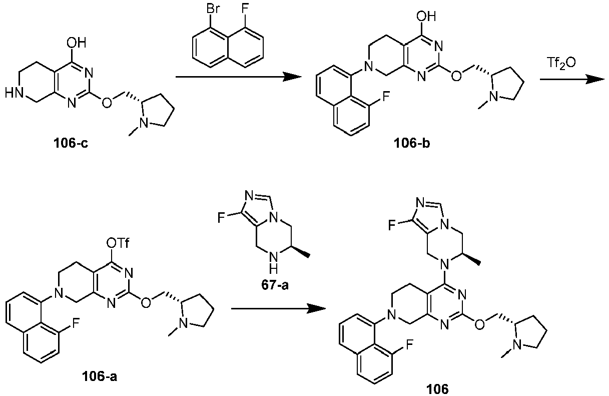

### Synthesis of compound 106-c

Synthesized according to synthesis of compound 99-c, (*S*)-1-Methylpyrrolidine-2-methanol was used instead of (*S*)-1-methylpiperidine-2-methanol.

### Synthesis of compound 106-b

In a flask were combined 106-c (200 mg, 0.76 mmol), toluene (40 mL), BINAP (94 mg, 0.15 mmol), Pd₂(dba)₃ (69 mg, 0.08 mmol), sodium *tert*-butoxide (363 mg, 3.78 mmol) and 1-bromo-8-fluoronaphthalene (200 mg, 0.89 mmol), and the resulting mixture was stirred at 110°C under nitrogen atmosphere for 3 hours. The reaction mixture was filtered, the filtrate was concentrated to dryness and the residue was purified by column chromatography (mobile phase: methanol (containing NH₃)/dichloromethane 1/20 to 1/10) and concentrated to give compound 106-b (190 mg, 61%). LC-MS (ESI): m/z 409.2 (M+H)⁺.

### Synthesis of compound 106-a

To a reaction flask was added 106-b (150 mg, 0.33 mmol), DBU (75 mg, 0.49 mmol), DMAP (1.2 mg, 0.01 mmol) and dichloromethane (15 mL), and the resulting mixture was stirred at room temperature under nitrogen atmosphere for 10 min. The above mixture was added *N* - phenylbis(trifluoromethanesulfonyl)imide (262 mg, 0.73 mmol) and continued stirring for 3 hours. The reaction was quenched with water (20 mL), extracted with dichloromethane (100 mL*3), and the organic phase was concentrated and the residue was purified by column chromatography (mobile phase:methanol/dichloromethane 1/30 to 1/10) to give compound 106-a (120 mg, 45%) . LC-MS (ESI): m/z 541.6 (M+H)⁺.

### Synthesis of compound 106

To a vial was added 106-a (60 mg, 0.11 mmol), DMAC (3 mL), 67-a (21 mg, 0.13 mmol) and DIPEA (14 mg, 0.11 mmol), and the resulting mixture was stirred at 60°C under nitrogen atmosphere for 6 hours. The reaction was quenched with brine (20 mL). Extracted with ethyl acetate, the organic phase was concentrated, and the crude product was purified by column chromatography (mobile phase: methanol (with NH₃)/dichloromethane 1/30 to 1/10), and concentrated to afford compound 106 (22 mg, 36%). LC-MS (ESI): m/z 546.3(M+H) ⁺; ¹H NMR (400MHz, DMSO-*d*6): *δ*7.67 (1H, d, J = 8.0Hz), 7.59 (1H, d, J = 8.4Hz), 735-7.50 (2H, m), 7.32 (1H, s), 7.12-7.25 (2H, m), 4.52-4.70 (2H, m), 4.19-4.41 (4H, m), 3.89-4.18 (3H, m), 3.55-3.77 (1H, m), 3. 32-3.45 (1H, m), 2.86-3.23 (2H, m), 2.61-2.85 (2H, m), 2.50 (3H, s), 2.29-3.41 (1H, m), 2.03-2.19 (1H, m),1.19-1.83 (3H, m), 1.14-1.41 (3H, m).

### Example 104: Synthetic route of compound 107

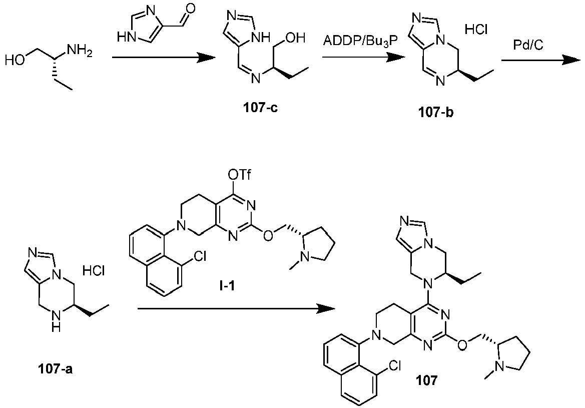

### Synthesis of compound 107-c

To a reaction vial were added (R)-2-amino-1-butanol (0.93 g, 10.41 mmol), 4-imidazolecarboxaldehyde (1 g, 10.41 mmol) and tetrahydrofuran (20 mL). The resulting mixture was stirred at room temperature for 2 hours under nitrogen atmosphere. The reaction solvent was removed to give compound 107-c (crude) as a colorless gel. LC-MS (ESI): m/z = 168.1 (M+H)⁺.

### Synthesis of compound 107-b

107-c (crude), tetrahydrofuran (50 mL), ADDP (4.46 g, 17.69 mmol) and Bu₃P (3.58 g, 17.69 mmol) were combined in a reaction flask, and the resulting mixture was stirred at 80°C under nitrogen atmosphere overnight. The next day, the reaction mixture was cooled to room temperature, filtered, and the filtrate was added 4 M hydrochloric acid/1,4-dioxane (3.37 mL, 13.49 mmol) dropwise while stirring, filtered, and the filter cake was washed with acetonitrile to give compound 107-b (1.49 g, 77%). LC-MS (ESI): m/z = 150.2 (M+H)⁺.

### Synthesis of compound 107-a

To a reaction vial were added 107-b (1.49 g, 8.03 mmol), methanol (50 mL) and 10% palladium carbon (150 mg), the resulting mixture was degassed and purged with hydrogen gas three times, then stirred at room temperature under hydrogen atmosphere overnight. The next day, the mixture was filtered and the filtrate was concentrated to dryness to give compound 107-a (1.5 g, 100%). LC-MS (ESI): m/z = 152.2 (M+H) ⁺.

### Synthesis of compound 107

To a reaction vial were added 107-a (25 mg, 0.13 mmol), DMSO (3 mL), I-1 (50 mg, 0.090 mmol) and DIPEA (78 µL, 0.449 mmol), and the mixture was stirred at 100°C under nitrogen atmosphere for 3 hours. The reaction was quenched by adding saturated sodium bicarbonate solution, extracted with ethyl acetate (30 mL*2), the organic layers were washed with brine (30 mL*3), concentrated to dryness, and purified first by column (mobile phase: ammonia-methanol/dichloromethane 0/100 to 5/95), and then prepared by TLC purification (unfolding agent: ammonia-methanol/dichloromethane 10/90) to give compound 107 (3 mg, 6%). LC-MS (ESI): m/z = 558.4 (M+H)⁺; ¹H NMR (400 MHz, CDCl₃) : *δ* 7.79-7.71(1H, m), 7.66-7.57(1H, m), 7.46(1H, d, J=7.2Hz), 7.49-7.38(2H, m), 7.27(1H, t, J=8Hz), 7.25-6.74(2H, m), 5.39-4.54(3H, m), 4.52-3.99(4H, m), 3.65(2H, q, J=7.2Hz), 3.66- 3.55(1H, m), 3.14-3.03(1H, m), 2.86-2.71(2H, m), 2.60-2.52(1H, m), 2.26-2.18(1H, m), 2.04-1.92(2H, m), 1.85-1.74(3H, m), 1.65-1.54(3H, m), 1.24-1.18(2H, m), 1.00-0.90(3H, m).

### Example 105: Synthetic route of compound 108

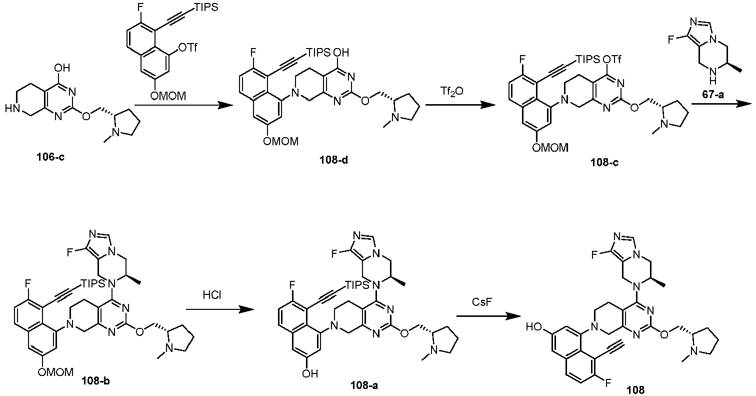

### Synthesis of compound 108-d

To a reaction vial were added 106-c (400 mg, 1.51 mmol), 7-fluoro-3-(methoxymethoxy)-8-((triisopropylmethylsilyl)ethynyl)naphthalen-1-yl trifluoromethanesulfonate (889 mg, 1.66 mmol), toluene (30 mL), sodium tert-butoxide (432 mg, 4.50 mmol), Pd₂( dba)₃ (137 mg, 0.15 mmol), and BINAP (186 mg, 0.30 mmol). The resulting mixture was degassed and purged with nitrogen and stirred at 110°C under nitrogen atmosphere for 3 hours. The reaction mixture was concentrated and the residue was purified by column chromatography (mobile phase: DCM/MeOH 10/0 to 10/1) to give compound 108-d (400 mg, 41%). LC-MS (ESI): m/z 649.5 (M+H) ⁺.

### Synthesis of compound 108-c

108-d (200 mg, 0.31 mmol), DIPEA (116 mg, 0.90 mmol) and DCM (20 mL) were combined in a reaction flask. After degassed and purged with nitrogen, the resulting mixture was added Tf₂O (152 mg, 0.54 mmol) dropwise at -10°C and stirred at -10°C under nitrogen atmosphere for 1 hour. The reaction mixture was quenched with water, extracted with DCM, the organic phases were combined, dried and concentrated, and the residue was purified by column chromatography (mobile phase: PE/EA 10/0 to 10/10) to give compound 108-c (120 mg, 50%). LC-MS (ESI): m/z 781.5 (M+H) ⁺.

### Synthesis of compound 108-b

108-c (120 mg, 0.15 mmol), 67-a (47 mg, 0.30 mmol), DIPEA (58 mg, 0.45 mmol), and DMSO (3 mL) were combined in a reaction flask. The reaction was degassed and purged with nitrogen and stirred at 90°C under nitrogen atmosphere for 3 hours. The reaction mixture was cooled, added water and extracted with DCM/MeOH(10:1). The organic phases were combined, dried, concentrated and the residue was purified by column chromatography (mobile phase: DCM/MeOH 10/0 to 10/1) to give compound 108-b (100 mg, 83%). LC-MS (ESI): m/z 787.1 (M+H) ⁺.

### Synthesis of compound 108-a

To a reaction flask were added 108-b (100 mg, 0.13 mmol) and acetonitrile (4 mL). After degassed and purged with nitrogen, the mixture was added hydrogen chloride/1,4-dioxane solution (1 mL) at 0 °C and stirred at 0 °C for 30 min. The reaction solvent was concentrated to dryness to give compound the crude 108-a (100 mg). LC-MS (ESI): m/z 743.1 (M+H) ⁺.

### Synthesis of compound 108

To a reaction flask were added crude 108-a (100 mg, 0.12 mmol), DMF (2 mL), and cesium fluoride (182 mg, 1.20 mmol). After degassed and purged with nitrogen, the reaction was stirred at room temperature under nitrogen atmosphere overnight. The reaction mixture was filtered and the residue was purified by pre-HPLC to give compound 108 (2 mg, 3%) as a yellow solid. LC-MS (ESI): m/z 586.5 (M+H) ⁺.

### Example 106: Synthetic route of compound 109

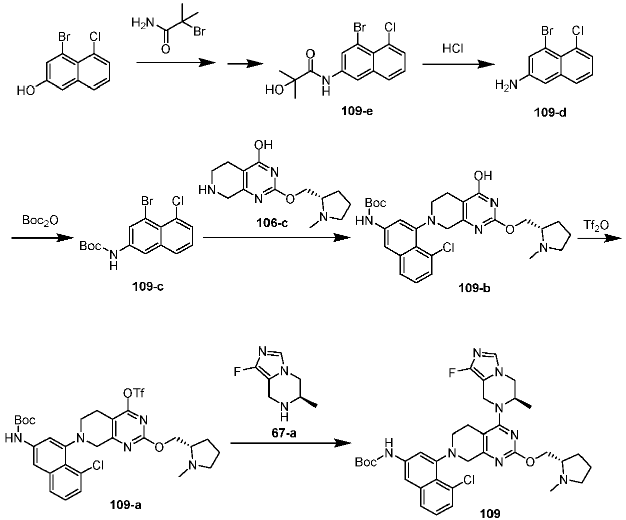

### Synthesis of compound 109-e

To a reaction flask were added 4-bromo-5-chloro-2-naphthol (15 g, 58.25 mmol), DMF (100 mL) and sodium hydroxide (6.99 g, 174.75 mmol) sequentially. The resulting mixture was stirred at room temperature for 1 hour, then was added 2-Bromoisobutyramide (29 g, 174.75 mmol) and stirred at room temperature under nitrogen atmosphere overnight. The next day, the mixture was added sodium hydroxide (20.97 g, 524.25 mmol) and stirred at 60°C for 2 hours. Cooled to room temperature, the reaction was quenched with water (1000 mL), filtered, and the filter cake was washed with water to give compound 109-e (crude), which was used directly in the next step without purification. LC-MS (ESI): m/z = 342.0 (M+H)⁺.

### Synthesis of compound 109-d

109-e (crude), ethanol (120 mL) and 6 M hydrochloric acid (120 mL) were combined in a reaction flask, and the resulting mixture was refluxed at 90°C for 24 hours. The next day, the reaction was removed ethanol by rotary evaporation, added water (200 mL) and ethyl acetate (300 mL*2). The aqueous phase was neutralized to pH 9 with diluted sodium hydroxide solution, extracted with ethyl acetate (300 mL*2). The organic phase was dried over anhydrous sodium sulfate and concentrated to dryness to give compound 109-d (8.6 g, 58%) as a brown solid, which was used directly in the next step without purification. LC-MS (ESI): m/z = 257.9 (M+H)⁺.

### Synthesis of compound 109-c

A solution of NaOH (545 mg, 13.65 mmol) in H₂O (10 mL) in a reaction flask was added THF (50 mL), 1-bromo-3-amino-8-chloronaphthalene (700 mg, 2.73 mmol) and di*-tert-*butyl dicarbonate (2975 mg, 13.65 mmol). The reaction was refluxed at 60°C for 48 hours. The mixture was concentrated to remove THF, extracted with ethyl acetate, the organic phase was dried and concentrated, and the residue was purified by column chromatography (mobile phase: DCM/EA=10/0 to 10/1) to give compound 109-c (720 mg, 73%). LC-MS (ESI): m/z 357.5 (M+H) ⁺.

### Synthesis of compound 109-b

106-c (1034 mg, 3.91 mmol), 109-c (700 mg, 1.96 mmol), toluene (50 mL), sodium tert-butoxide (940 mg, 9.80 mmol), Pd₂(dba)₃ (274 mg, 0.30 mmol) and BINAP (373 mg, 0.60 mmol) were combined in a reaction flask. After degassed and purged with nitrogen, the reaction was stirred at 100°C under nitrogen atmosphere for 30 min. The reaction mixture was concentrated and the residue was purified by column chromatography (mobile phase: DCM/MeOH 10/0 to 10/1) to give compound 109-b (490 mg, 46%). LC-MS (ESI): m/z 541.1 (M+H) ⁺.

### Synthesis of compound 109-a

109-b (490 mg, 0.91 mmol), DIPEA (348 mg, 2.70 mmol) and DCM (40 mL) were combined in a reaction flask. After degassed and purged with nitrogen, the reaction was added Tf₂O (507 mg, 1.80 mmol) dropwise at -10 °C under nitrogen atmosphere and stirred at -10 °C for 1 hour. The reaction was quenched with water, extracted with DCM, the organic phases were combined, dried and concentrated, and the residue was purified by column chromatography (mobile phase: DCM/MeOH = 10/0 to 10/1) to give compound 109-a (270 mg, 44%). LC-MS (ESI): m/z 673.1 (M+H) ⁺.

### Synthesis of compound 109

109-a (270 mg, 0.40 mmol), 67-a (124 mg, 0.80 mmol), DIPEA (155 mg, 1.20 mmol) and DMSO (5 mL) were combined in a reaction flask. After degassed and purged with nitrogen, the reaction was stirred at 90°C under nitrogen atmosphere for 3 hours. The reaction mixture was cooled, added water and extracted with DCM/MeOH(10:1). The organic phases were combined, dried and concentrated, and the residue was purified by column chromatography (mobile phase: DCM/MeOH 10/0 to 10/1) to give compound 109 (190 mg, 70%). LC-MS (ESI): m/z 678.1 (M+H) ⁺.

### Example 107: Synthetic route of compound 110

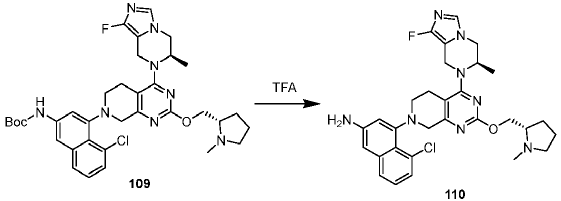

### Synthesis of compound 110

To a reaction flask were added 109 (180 mg, 0.27 mmol), TFA (3 mL) and DCM (6 mL). Afer degassed and purged with nitrogen, the resulting mixture was stirred at room temperature under nitrogen atmosphere for 3 hours. The reaction was concentrated, basified by adding a solution of ammonia in methanol, concentrated and the residue was purified by column chromatography (mobile phase:DCM/MeOH 10/0 to 10/1) to give compound 110 (110 mg, 72%). LC-MS (ESI): m/z 577.3 (M+H) ⁺.

### Example 108: Synthetic route of compound 111

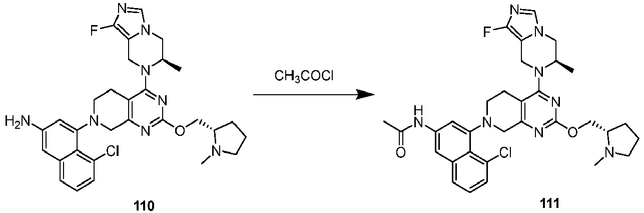

### Synthesis of compound 111

To a reaction flask were added 110 (15 mg, 0.026 mmol), DCM (6 mL) and DIPEA (8 mg, 0.06 mmol). After degassed and purged with nitrogen, the mixture was added acetyl chloride (3 mg, 0.04 mmol) at 0°C under nitrogen atmosphere and stirred at room temperature for 2 hours. The reaction was basified by adding a solution of ammonia in methanol, concentrated and the residue was purified by column chromatography (mobile phase: DCM/MeOH= 10/0 to 10/1) to give compound 111 (6 mg, 37%). LC-MS (ESI): m/z 619.2 (M+H) ⁺.

### Example 109: Synthetic route of compound 112

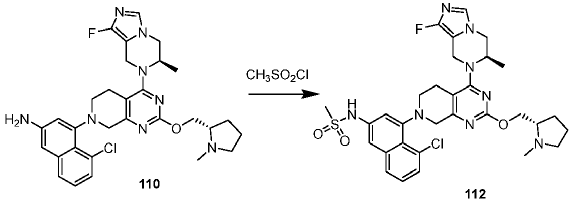

### Synthesis of compound 112

To a reaction flask were added 110 (15 mg, 0.026 mmol), DCM (6 mL) and DIPEA (8 mg, 0.06 mmol). Afer degassed and purged with nitrogen, the resulting mixture was added methanesulfonyl chloride (5 mg, 0.04 mmol) at 0°C under nitrogen atmosphere. The reaction was stirred at room temperature under nitrogen atmosphere for 3 hours. The reaction was basified by adding a solution of ammonia in methanol, concentrated and the residue was purified by column chromatography (mobile phase: DCM/MeOH= 10/0 to 10/1) to give compound 112 (5 mg, 29%). LC-MS (ESI): m/z 655.2 (M+H) ⁺.

### Example 110: Synthetic route of compound 113

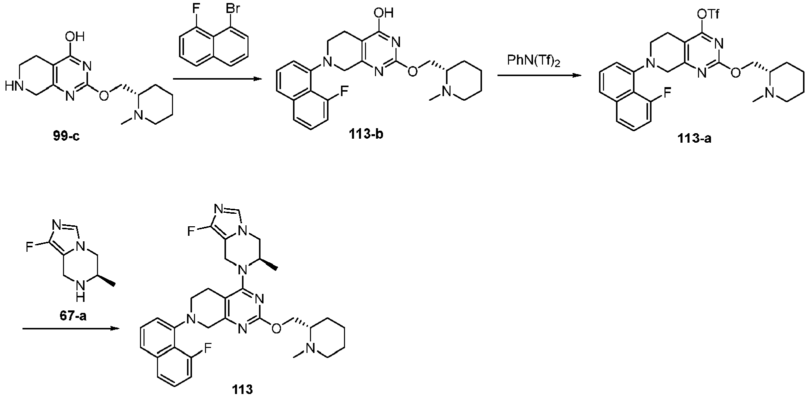

### Synthesis of compound 113-b

To a three-necked flask were added 99-c (417 mg, 1.5 mmol), 1-bromo-8-fluoronaphthalene (338 mg, 1.5 mmol), anhydrous toluene (3 mL), tridibenzylideneacetone dipalladium (41 mg, 0.04 mmol), 4,5-bis(diphenylphosphino)-9,9-dimethylxanthene (43 mg, 0.08 mmol) and sodium tert-butoxide (360 mg, 3.75 mmol). After degassed and purged with nitrogen for three times, the resulting mixture was stirred at 80 °C for 2 hours. Cooled to room temperature, the reaction was quenched by adding ammonium chloride solution(10 mL) while stirring, was added ethyl acetate(10 mL ) to separate the organic layer. The organic layers were washed with brine, dried over anhydrous sodium sulfate, filtered, and the filtrate was concentrated to dryness to give compound 113-b (750 mg, 100%) . LC-MS (ESI): m/z = 423.3 (M+H)⁺.

### Synthesis of compound 113-a

113-b (630 mg, 1.50 mmol, crude), PhN(Tf)₂ (803 mg, 2.25 mmol), anhydrous dichloromethane (5 mL), DBU (228 mg, 1.5 mmol) and DMAP (9 mg, 0.075 mmol) were added to a three-necked flask while keeping the internal temperature below 10 °C during the addition. After degassed and purged with nitrogen for three times, the resulting mixture was stirred at 25°C for 2 hours. The reaction was quenched by adding ice-cold water (10 mL) while stirring, and was added DCM to separate the organic layer. The organic phase was washed with brine, dried over anhydrous sodium sulfate, filtered and the filter cake was washed with dichloromethane twice, the filtrate was concentrated to dryness, and the residue was purified by column chromatography (mobile phase: methanol/dichloromethane = 0/100 to 5/95) to give compound 113-a (445mg, 53%). LC-MS (ESI): m/z = 555.2 (M+H)⁺.

### Synthesis of compound 113

113-a (443 mg, 0.80 mmol), DMSO (3 mL), 67-a (149 mg, 0.96 mmol) and DIPEA (134 mg, 1.04 mmol) were combined in a three-necked flask. After degassed and purged with nitrogen for three times, the resulting mixture was stirred at 45 °C for 24 hours. A large amount of gray solid was precipitated after the reaction was added water (15 mL) at room temperature. The mixture was filtered and the filter cake was purified by column chromatography (mobile phase: ammonia-methanol/dichloromethane = 0/100 to 7/95) and freeze-dried to give compound 113 (88 mg, 20%). LC-MS (ESI): m/z = 560.3 (M+H)⁺; ¹H NMR (400 MHz, CDCl₃) *δ* 7.64 (d, J = 7.6 Hz, 1H), 7.56 (d, J = 8.4 Hz, 1H), 7.46-7.39 (m, 2H), 7.18-7.09 (m, 3H), 5.05-4.11 (m, 8H), 3.97-3.94 (m, 1H), 3.80-3.65 (m, 1H), 3.43-3.34 (m, 1H), 3.20-2.78 (m, 2H), 2.65-2.40 (m, 5H), 2.22(brs, 1H), 1.93-1.89 (m, 1H), 1.82-1.78 (m, 1H), 1.60-1.45 (m, 2H), 1.40-1.25 (m, 5H).

### Synthetic methods for similar compounds:

| Compound | Structure | Synthetic method |
|---|---|---|
| **106A** | 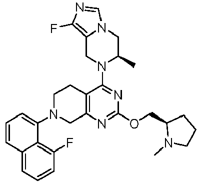 | Synthesized according to compound **106,** *N*-methyl-D-prolinol was used instead of *N-*methyl-L-prolinol. |
| **114** | 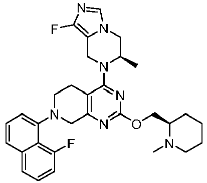 | Synthesized according to compound 113, (*R*)-1-Methylpiperidine-2-methanol was used instead of (*S*)-1-methylpiperidine-2-methanol. |

### Example 111: Synthetic route of compound 115

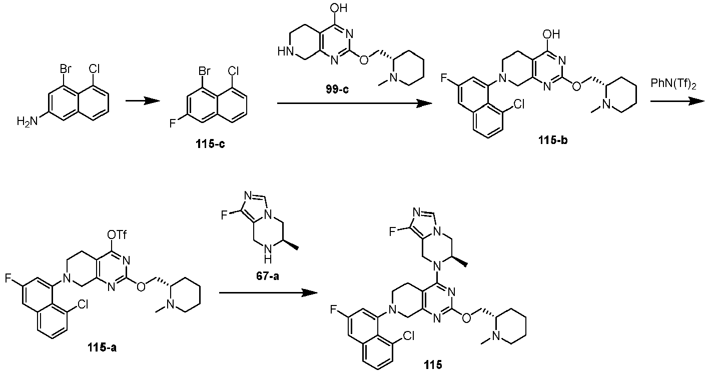

### Synthesis of compound 115-c

To a reaction flask were added 1-bromo-3-amino-8-chloronaphthalene (500 mg, 1.95 mmol) and 70% aqueous hydrogen fluoride pyridine solution (5 mL, 38.88 mmol). After degassed and purged with nitrogen for three times, the mixture was added NaNO₂ (175mg, 2.53mmol) and stirred at 80°C under nitrogen atmosphere for 12 hours. The reaction was concentrated, added water, extracted with EA. The organic phases were combined, dried and concentrated, and the residue was purified by column chromatography (mobile phase: PE/EA 10/0 to 10/1) to give compound 115-c (270 mg, 53%).

### Synthesis of compound 115-b

To a reaction flask were added 99-c (205 mg, 0.74 mmol), toluene (40 mL), BINAP (76 mg, 0.12 mmol), Pd₂(dba)₃ (56 mg, 0.06 mmol), sodium *tert*-butoxide (295 mg, 3.07 mmol) and 115-c (159 mg, 0.61 mmol ). The resulting mixture was stirred at 110°C under nitrogen atmosphere for 3 hours. The reaction mixture was filtered, the filatrate was concentrated to dryness, and the residue was purified by column chromatography (mobile phase: methanol (containing NH₃)/dichloromethane 1/20 to 1/10) and concentrated to give compound 115-b (150 mg, 53%) . LC-MS (ESI): m/z 457.3 (M+H) ⁺.

### Synthesis of compound 115-a

To a reaction flask were added 115-b (150 mg, 0.33 mmol), DBU (50 mg, 0.33 mmol), DMAP (1 mg, 0.01 mmol) and dichloromethane (15 mL). The resulting mixture was stirred at room temperature under nitrogen atmosphere for 10 min, then was added *N-*phenylbis(trifluoromethanesulfonyl)imide (176 mg, 0.49 mmol) . After addition, the reaction continued stirring for 180 min. The reaction was quenched with saturated sodium bicarbonate (100 mL), extracted with dichloromethane (100 mL*3), and the organic phase was concentrated and the residue was purified by column chromatography (mobile phase: methanol/dichloromethane 1/30 to 1/10) to give compound 115-a (120 mg, 62%). LC-MS (ESI): m/z 590.2 (M+H) ⁺.

### Synthesis of compound 115

To a reaction vial were added 115-a (60 mg, 0.10 mmol), DMAC (3 mL), 67-a (21 mg, 0.13 mmol) and DIPEA (80 mg, 0.62 mmol), and the resulting mixture was stirred at 100°C under nitrogen atmosphere for 1.5 hours. The reaction was quenched with water (20 mL), extracted with ethyl acetate, concentrated and the residue was purified by column chromatography (mobile phase: methanol (with NH₃/dichloromethane= 1/20 to 1/10), concentrated to obtain compound 115 (3.3 mg, 5%). LC-MS (ESI): m/z 594.3 (M+H) ⁺.

### Example 112: Synthetic route of compound 116

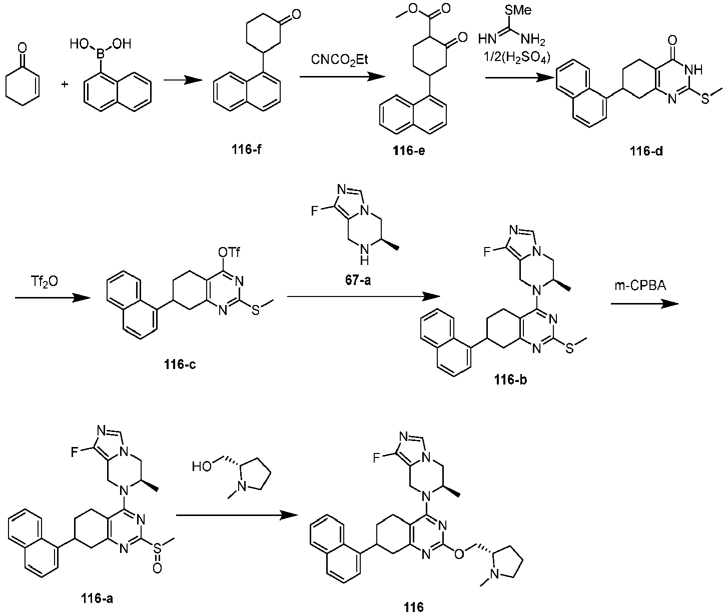

### Synthesis of compound 116-f

1-boronaphthalene (2.9 g, 16.86 mmol), cyclooctadiene rhodium dimer chloride (0.50 g, 1.01 mmol), sodium bicarbonate (4.25 g, 50.58 mmol), 1,4-dioxane (10 mL) and water (80 mL) were combined in a reaction flask, and the resulting mixture was stirred at room temperature under argon atmosphere for 10 min. After added cyclohexenone (4.05 g, 42.15 mmol), the reaction was stirred at 65°C under argon atmosphere for 12 hours. The mixture was filtered, and the organic solvent was concentrated, the aqueous phase was extracted with ethyl acetate, purified by column chromatography (mobile phase: petroleum ether/ethyl acetate 5/1 to 1/1) and concentrated to give compound 116-f (3.1 g, 82%).

### Synthesis of compound 116-e

To a reaction flask were added 116-f (2.8 g, 12.48 mmol) and tetrahydrofuran (120 mL) and the resulting mixture was stirred at -78°C under nitrogen atmosphere for 30 min. LDA (7.49 mL, 14.98 mmol) was added dropwise slowly to the mixture and the reaction was stirred at -78°C for 60 min. Ethyl cyanoformate (1.85 g, 18.73 mmol) was added to the reaction and the reaction was stirred for 2 hours. The reaction was quenched by adding diluted hydrochloric acid (10 mL) and water (100 mL), extracted with ethyl acetate (100 mL*3), and the organic phases was concentrated and the residue was purified by column chromatography (mobile phase: petroleum ether/ethyl acetate 10/1 to 1/1) to give compound 116-e (2.3 g, 56%).

### Synthesis of compound 116-d

A mixture of 116-e (920 mg, 3.19 mmol) and 2-methyl-2-mercapto-urea sulfate (1100 mg, 3.96 mmol) in methanol (3 mL) in a reaction vial was stired at 0 °C, then was added sodium methoxide (1300 mg, 24.06 mmol). The resulting mixture was slowly warmed to room temperature under nitrogen atmosphere and stirred at room temperature for 2 hours. The reaction mixture was concentrated and quenched by adding diluted hydrochloric acid (20 mL) (pH was adjusted to about 3) and the solid was filtered to give compound 116-d (710 mg, 71%). LC-MS (ESI): m/z 323.1 (M+H) ⁺.

### Synthesis of compound 116-c

To a reaction flask were added 116-d (400 mg, 1.24 mmol), DIPEA (1000 mg, 7.74 mmol) and dichloromethane (60 mL) and the resulting mixture was stirred under an ice-salt bath (-5°C) for 15 min. The mixture was added Tf₂O (620 mg, 2.20 mmol) dropwise slowly and stirred for 60 min after addition. The organic phase was concentrated and the residue was purified by column chromatography (mobile phase: petroleum ether/ethyl acetate 5/1) to give compound 116-c (320 mg, 57%). LC-MS (ESI): m/z 455.1 (M+H)⁺.

### Synthesis of compound 116-b

To a reaction vial were added 116-c (280 mg, 0.62 mmol), DMAC (5 mL), 67-a (143 mg, 0.92 mmol) and DIPEA (398 mg, 3.08 mmol) and the resulting mixture was stirred at 100°C under nitrogen atmosphere for 12 hours. The reaction mixture was quenched with water (50 mL), extracted with ethyl acetate, the organic layers were concentrated to dryness and residue was purified by column chromatography (mobile phase: ethyl acetate/petroleum ether 115 to pure ethyl acetate) and concentrated to give compound 116-b (190 mg, 67%). LC-MS (ESI): m/z 460.2(M+H) ⁺.

### Synthesis of compound 116-a

Compound 116-b (190 mg, 0.41 mmol) was dissolved in ethyl acetate (20 mL). After stirred at 0 °C for 10 min, the resulting mixture was added m-CPBA (160 mg, 0.50 mmol, 85% purity). After stirred at 0 °C for 20 min, the mixture was washed with saturated sodium bicarbonate solution (30 mL) and the organic phase was concentrated to give compound 116-a (190 mg, 97%). LC-MS (ESI): m/z 476.2 (M+H)⁺.

### Synthesis of compound 116

N-methyl-L-prolinol (34 mg, 0.34 mmol) and *tert*-butanol sodium (32 mg, 0.34 mmol) were added sequentially to a solution of compound 116-a (80 mg, 0.17 mmol) in toluene ( 10 mL) while cooling in an ice-cold water bath. The mixture was stirred under nitrogen atmosphere in an ice-cold water bath for 0.5 hour. The crude product was purified by column chromatography (mobile phase: methanol (with ammonia)/dichloromethane 1/30 to 1/10) to give compound 116 (50 mg, 57%). LC-MS (ESI): m/z 527.3 (M+H)⁺; ¹H NMR (400MHz, CD₃OD): δ8.22 (1H, t, *J =* 8.0Hz), 7.89 (1H, d, *J* = 8.4Hz), 7.76 (1H, d, *J* = 7.6Hz), 7.25-7.60 (5H, m), 4.50-4.86 (2H, m), 4.28-4.46 (3H, m), 3.95-4.18 (2H, m), 3.05-3.20 (2H, m), 2.62-2.97 (4H, m), 2.53 (3H, d, *J =* 18.4Hz), 2.33-2.53 (1H, m), 2.22-2.33 (1H, m), 1.96-2.17 (2H, m), 1.62-1.95 (3H, m), 1.40 (2H, d, *J =* 5.6Hz), 1.28 (2H, d, *J =* 6.8Hz).

### Example 113: Synthetic route of compound 117

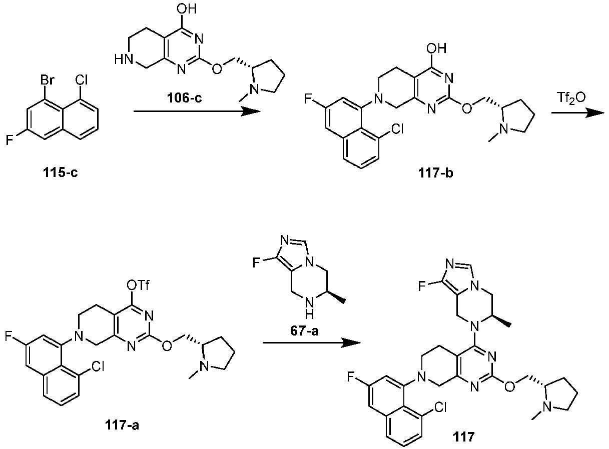

### Synthesis of compound 117-b

To a reaction flask were added 106-c (80 mg, 0.30 mmol), 115-c (86 mg, 0.33 mmol), toluene (10 mL), sodium *tert*-butoxide (86 mg, 0.90 mmol), Pd₂(dba)₃ (27 mg, 0.03 mmol) and BINAP (37 mg, 0.06 mmol). After degassed and purged with nitrogen, the resulting mixture was stirred at 100°C for 8 hours. The reaction mixture was concentrated and the residue was purified by column chromagraphy (mobile phase: DCM/MeOH 10/0 to 10/1) to give compound 117-b (80 mg, 60%). LC-MS (ESI): m/z 443.5 (M+H)⁺.

### Synthesis of compound 117-a

To a reaction flask were added 117-b (80 mg, 0.18 mmol), DIPEA (70 mg, 0.54 mmol) and DCM (20 mL). After degassed and purged with nitrogen, the resulting mixture was added Tf₂O (101 mg, 0.36 mmol) dropwise at -10°C. The reaction was stirred at -10°C for 1 hour, quenched with water, extracted with DCM. The organic phases were combined, dried and concentrated, and the residue was purified by column chromatography (mobile phase: DCM/MeOH 10/0 to 10/1) to give compound 117-a (70 mg, 67%). LC-MS (ESI): m/z 575.5 (M+H)⁺.

### Synthesis of compound 117

To a reaction flask were added 117-a (70 mg, 0.12 mmol), 67-a (37 mg, 0.24 mmol), DIPEA (46 mg, 0.36 mmol) and DMSO (3 mL). After degassed and purged with nitrogen, the resulting mixture was stirred at 70°C under nitrogen atmosphere for 8 hours. Cooled, the reaction was added water and extracted with a solution of DCM/MeOH(10:1), the organic phases were combined, dried and concentrated, and the residue was purified by column chromatography (mobile phase: DCM/MeOH = 10/0 to 10/1) to give compound 117 (19 mg, 27%). LC-MS (ESI): m/z 580.8 (M+H)⁺.

### Synthetic methods for similar compounds:

| Compounds | Structure | Synthetic methods |
|---|---|---|
| **115A** | 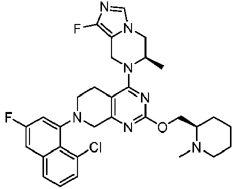 | Synthesized according to synthesis of compound **115,** (*R*)-1-methylpiperidine-2-methanol was used instead of (*S*)-1-methylpiperidine-2-methanol. |
| **117A** | 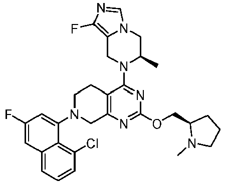 | Synthesized according to synthesis of compound **117,** *N*-methyl-D-prolinol was used instead of *N*-methyl-L-prolinol. |

### Example 114: Synthetic route of compound 118

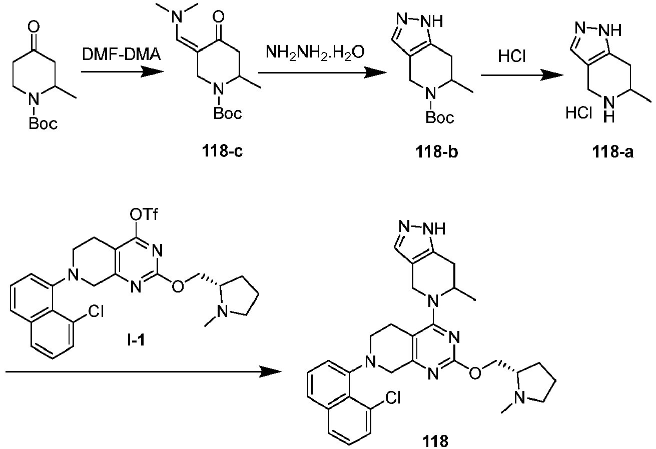

### Synthesis of compound 118-c

Compound 1-*tert*-butoxycarbonyl-2-methyl-piperidone (2.0 g, 9.38 mmoL) was dissolved in DMF-DMA(20 mL) at room temperature and the reaction mixture was stirred at 100°C under nitrogen atmosphere overnight. Cooled to room temperature, the reaction mixture was quenched with water(100 mL), extracted with ethyl acetate (100 mL*2). The organic phase was washed with brine (100 mL*3), dried over anhydrous sodium sulfate, filtered, and concentrated at reduced pressure to give the crude compound 118-c (2.5 g). The crude product was used directly in the next step without purification.

### Synthesis of compound 118-b

Hydrazine hydrate (800 µL, 2.98 mmoL) was added to a solution of the crude compound 118-c (800 mg, 2.98 mmoL) in ethanol(25 mL) at room temperature, and the resulting mixture was stirred at 90°C under nitrogen atmosphere for 2 hours. Cooled to room temperature, the reaction mixture was concentrated at reduced pressure to give the crude compound. The crude product was purified by a flash column chromatography (DCM/MeOH=30/1) to give compound 118-b (265 mg, 37%). LC-MS (ESI): m/z = 238.2 [M+1]⁺.

### Synthesis of compound 118-a

A solution of hydrogen chloride in 1,4-dioxane (4 M, 5 mL, 20 mmoL) was added to a soluton of compound 118-b (55 mg, 0.23 mmoL) in DCM (2 mL) at room temperature and and the reaction mixture was stirred at room temperature under nitrogen atmosphere overnight. The reaction mixture was concentrated at reduced pressure to give the crude compound 118-a (40 mg). LC-MS (ESI): m/z = 138.2 [M+1]⁺.

### Synthesis of compound 118

118-a (39 mg, 0.28 mmoL) and DIPEA (98 µL, 0.59 mmoL) were added sequentially to a soluton of compound 1-1 (55 mg, 0.10 mmoL) in DMAC (3 mL) at room temperature, and the resulting mixture was stirred at 80°C under nitrogen atmosphere for 3 hours. The reaction mixture was purified by pre-HPLC (basic method) to give compound 118 (26 mg, 46%). LC-MS (ESI): m/z = 544.2 [M+1]⁺.

### Synthetic methods for similar compounds

| compound | Structure | Synthetic method | MS (M+H)⁺ |
|---|---|---|---|
| Comparative compound 2' | 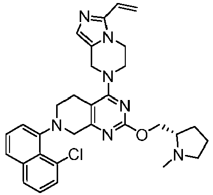 | Synthesized according to synthesis of compound **2**, 5,6,7,8-tetrahydroimidazo[1,5-a]pyrazine was used instead of 3-vinyl-5,6,7,8-tetrahydroimidazo[1,5-a]pyrazine instead of 5,6,7,8-tetrahydroimidazo[1,5-a]pyrazine. | 556.3 |
| Comparative compound 3' | 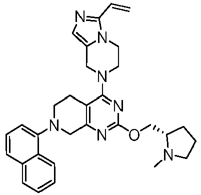 | Synthesized according to synthesis of compound **106,** 1-bromonaphthalene was used instead of 1-bromo-8-fluoronaphthalene, and 3-vinyl-5,6,7,8-tetrahydroimidazo[1,5-a]pyrazine was used instead of 67-a. | 522.3 |

### Effect Example 1 Proliferation inhibition of compounds on RAS cell lines measured by CTG

NCI-H358 is a human non-small cell lung cancer cell line with KRAS G12C mutation; PANC-1 and AsPC-1 are pancreatic cancer cell lines with KRAS G12D mutation; AGS is a gastric cancer cell line with KRAS G12D mutation; A427 is a lung cancer cell line with KRAS G12D mutation; THP-1 is a leukemia cell line with NRAS G12D mutation; Hs 578T is a breast cancer cell line with HRAS G12D mutation; SW480 is a colon cancer cell lines with KRAS G12V mutation; A375 is a wild type malignant melanoma cell line. The proliferation inhibitory effect of the compounds on various mutations was evaluated by determining the proliferation inhibitory activity of the compounds on these cell lines.

The assay was conducted on either 384-well plates or 96-well plates. The procedures are as follows:
The cell suspensions were added to 384- or 96-well plates (384-well plate: 40 µL; 96-well plate: 100 µL) except for peripheral wells. The plates were incubated in a carbon dioxide incubator overnight. Prepared compounds (10 concentration gradients by serial 3-fold dilution) were added to the wells. The cell plates were incubated in a carbon dioxide incubator for 120 hours. CellTiter Glo reagent (384 well plate: 25 µL; 96 well plate: 100 µL) was added to the 384- or 96-well plates. The plates were shaken for 10 minutes away from light, and incubated for 10 minutes. The plates were read by EnVision system. Inhibition curves were plotted using XLFit and IC₅₀ values were calculated. The activity results for representative compounds are shown in Tables 1-3 below. "IC₅₀ > 10 µM" is denoted by "*", "10 µM ≥ IC₅₀ > 1 µM" is denoted by "**", "1 µM ≥ IC₅₀> 100 nM" is denoted by "***", and "IC₅₀ ≤ 100 nM" is denoted by "****".

**Table 1 Proliferation inhibitory activity of representative compounds of the present disclosure on RAS cells**

| **Compound No.** | **IC₅₀ (H358 )** | **IC₅₀ (AsPC-1)** | **IC₅₀ (AGS)** | **IC₅₀ (A427)** | **IC₅₀ (Hs 578T)** | **IC₅₀ (THP-1)** | **IC₅₀ (A375)** |
|---|---|---|---|---|---|---|---|
| **1** | *** | *** | - | *** | - | - | *** |
| **2** | **** (0.063 ) | *** (0.139 ) | **** (0.092 ) | **** (0.070 ) | *** (0.237 ) | **** (0.067 ) | *** (0.167 ) |
| **3** | *** | ** | - | - | - | - | ** |
| **4** | ** | ** | --- | *** | --- | --- | *** |
| **5** | ** | ** | --- | --- | --- | --- | ** |
| **6** | *** | *** | --- | *** | --- | --- | *** |
| **7** | *** | ** | --- | *** | --- | --- | *** |
| **8** | ** | ** | --- | *** | --- | --- | *** |
| **9** | **** | **** | **** | **** | --- | --- | *** |
| **10** | *** | *** | --- | *** | --- | --- | *** |
| **11** | *** | *** | --- | *** | --- | --- | *** |
| **14** | *** | *** | --- | *** | --- | --- | *** |
| **16** | *** | **** | --- | **** | --- | --- | *** |
| **17** | *** | *** | --- | *** | --- | --- | *** |
| **18** | **** | *** | **** | --- | *** | *** | *** |
| **20** | *** | ** | *** | --- | ** | *** | ** |
| **21** | **** | *** | **** | --- | *** | *** | *** |
| **22** | **** | **** | **** | --- | *** | **** | **** |
| **23** | *** | *** | *** | --- | ** | *** | *** |
| **24** | *** | ** | ** | --- | * | *** | *** |
| **27** | *** | *** | *** | --- | ** | *** | ** |
| **28** | *** | *** | *** | --- | ** | *** | *** |
| **29** | **** | **** | **** | --- | *** | *** | *** |
| **30** | *** | *** | *** | --- | ** | *** | *** |
| **31** | *** | *** | *** | --- | ** | *** | ** |
| **32** | *** | ** | ** | --- | ** | ** | ** |
| **33** | **** | *** | **** | --- | *** | **** | *** |
| **34** | *** | *** | *** | --- | ** | *** | ** |
| **35** | *** | ** | ** | --- | ** | ** | ** |
| **40** | *** | --- | *** | --- | --- | --- | *** |
| **41** | *** | --- | **** | --- | --- | --- | *** |
| **Comparati ve compound 1'** | * (17.169 ) | * (39.690 ) | --- | --- | --- | --- | * (22.715 ) |

Comparative compound 1' is:

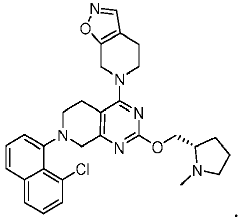

### Effect Example 2

**Table 2 Proliferation inhibitory activity of compound 2 on RAS cells (procedures are as described above)**

| **Cell line** | **IC₅₀ H358 (G12C)** | **IC₅₀ A549 (G12S)** | **IC₅₀ AGS (G12D)** | **IC₅₀ RPMI 8226 (G12A)** | **IC₅₀ SW480 (G12V)** | **IC₅₀ NCI-H647 (G13D)** | **IC₅₀ PANC-1 (G12D)** | **IC₅₀ A375 (wide type)** |
|---|---|---|---|---|---|---|---|---|
| **Compound 2 IC₅₀ (µM)** | 0.063 | 0.253 | 0.092 | 0.128 | 0.098 | 0.115 | 0.193 | 0.167 |
| **Compound 22 IC₅₀ (µM)** | 0.073 | --- | 0.054 | --- | --- | --- | --- | 0.096 |
| **Compound 44 IC₅₀ (µM)** | 0.033 | --- | 0.024 | --- | 0.025 | --- | --- | 0.036 |
| **Comparative compound 2' IC₅₀ (µM)** | 1.230 | --- | 1.480 | --- | --- | --- | --- | 1.611 |
| **Comparative compound 3' IC₅₀ (µM)** | 1.044 | --- | 1.183 | --- | --- | --- | --- | 1.503 |

**Table 3 Proliferation inhibitory activity of representative compounds of the present disclosure on RAS cells (procedures are as described above)**

| **Compo und No.** | IC₅₀ (H358 ) | IC₅₀ (AGS) | IC₅₀ (SW480) | IC₅₀ (A375) |
|---|---|---|---|---|
| 43 | *** | *** | --- | --- |
| 44 | **** | **** | **** | **** |
| 49 | *** | *** | ** | ** |
| 50 | ** | ** | ** | ** |
| 52 | *** | *** | ** | ** |
| 53 | ** | ** | ** | ** |
| 57 | **** | **** | **** | *** |
| 58 | **** | **** | **** | *** |
| 59 | *** | *** | **** | *** |
| 60 | *** | *** | *** | ** |
| 63 | **** | **** | **** | *** |
| 64 | **** | **** | **** | **** |
| 66 | **** | **** | *** | *** |
| 67 | **** | **** | **** | **** |
| 68 | **** | **** | **** | *** |
| 69 | **** | **** | **** | **** |
| 70 | **** | **** | *** | *** |
| 71 | *** | *** | *** | ** |
| 74 | *** | *** | *** | ** |
| 77 | **** | **** | **** | **** |
| 78 | **** | **** | **** | **** |
| 79 | --- | **** | **** | **** |
| 80 | --- | **** | **** | **** |
| 82 | *** | **** | *** | *** |
| 83 | **** | **** | **** | *** |
| 84 | ** | *** | *** | ** |
| 85 | *** | *** | *** | ** |
| 86 | **** | **** | **** | **** |
| 87 | **** | **** | **** | **** |
| 88 | **** | **** | **** | *** |
| 89 | ** | ** | ** | ** |
| 91 | **** | **** | **** | **** |
| 92 | **** | **** | **** | **** |
| 93 | **** | **** | **** | **** |
| 94 | ** | *** | ** | ** |
| 96 | --- | **** | **** | --- |
| 97 | **** | **** | **** | *** |
| 98 | **** | **** | **** | **** |
| 99 | **** | **** | **** | **** |
| 101 | **** | **** | **** | **** |
| 102 | **** | **** | **** | **** |
| 103 | **** | **** | **** | **** |
| 104 | *** | *** | *** | *** |
| 105 | **** | **** | **** | **** |
| 106 | **** | **** | **** | **** |
| 107 | **** | **** | **** | **** |
| 108 | *** | **** | **** | *** |
| 109 | ** | *** | *** | ** |
| 110 | **** | **** | **** | **** |
| 111 | ** | ** | *** | ** |
| 112 | ** | *** | *** | ** |
| 113 | **** | **** | **** | **** |
| 115 | **** | **** | **** | **** |
| 116 | *** | **** | **** | *** |
| 117 | **** | **** | **** | **** |
| 118 | *** | **** | **** | *** |

The results show that such structures have obvious inhibitory activity on various RAS, and are prospective for applications in preventing and treating RAS -related diseases.

Although specific embodiments of the present disclosure have been described above, it will be appreciated by those skilled in the art that these embodiments are merely illustrative and that many changes or modifications can be made to these embodiments without departing from the principles and spirit of the present disclosure. The scope of protection of the present disclosure is therefore defined by the appended claims.

## Claims

1. A nitrogen-containing heterocyclic compound of formula I, a pharmaceutically acceptable salt thereof, a stereoisomer thereof, a tautomer thereof or an isotopically labeled compound thereof: 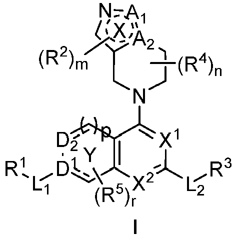
wherein "-̅ -̅ -̅" represents a single or double bond; 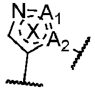
is nitrogen-containing 5-membered heteroaryl; A₁ is CH, O or N; A₂ is C or N;
m is 0, 1 or 2;
R² is -CN, C₁₋₆ alkyl, C₂₋₆ alkenyl, C₂₋₆ alkynyl, C₁₋₆ alkyl substituted with one or more R²⁻¹, halogen, -OR^{2a}, -C(=O)R^{2b}, -NR^{2c1}R^{2c2}, -C(=O)OR^{2d}, -C(=O)NR^{2e1}R^{2e2}, C₃₋₁₀ cycloalkyl, C₃₋₁₀ cycloalkyl substituted with one or more R²⁻², "4- to 10-membered heterocycloalkyl containing 1-3 heteroatoms independently selected from O and N", "4- to 10-membered heterocycloalkyl containing 1-3 heteroatoms independently selected from O and N" substituted with one or more R²⁻³, C₆₋₂₀ aryl, C₆₋₂₀ aryl substituted with one or more R²⁻⁴, "5- to 12-membered heteroaryl containing 1-4 heteroatoms independently selected from O, S and N", or "5- to 12-membered heteroaryl containing 1-4 heteroatoms independently selected from O, S and N" substituted with one or more R²⁻⁵; provided that when multiple substituents are present, the substituents are the same or different;
R²¹, R²⁻², R²⁻³, R²⁻⁴ and R²⁻⁵ are independently halogen, hydroxyl, cyano, C₁₋₆ alkyl, C₂₋₆ alkenyl, C₂₋₆ alkynyl, C₁₋₆ alkyl-O-, -C(=O)R³¹, -NR³²R³³, -C(=O)OR³⁴ or -C(=O)NR³⁵R³⁶;
R^{2a}, R^{2b}, R^{2c1}, R^{2c2}, R^{2d}, R^{2e1} and R^{2e2} are independently hydrogen or C₁₋₆ alkyl;
R³¹, R³², R³³, R³⁴, R³⁵ and R³⁶ are independently hydrogen or C₁₋₆ alkyl;
n is 0, 1, 2, 3, 4, 5 or 6;
R⁴ is independently C₁₋₆ alkyl, C₁₋₆ alkyl substituted with one or more R⁴⁻¹, C₁₋₆ alkyl-O-, O=, - C(=O)OR^{4a} or -C(=O)NR^{4b}R^{4c}; or, when n is 2, 3, 4, 5 or 6, two optional R⁴ are connected, together with the atoms on the ring to which they are attached, independently form 3- to 8-membered carbocyclic ring or 3- to 8-membered heterocyclic ring containing 1-3 heteroatoms independently selected from O, S and N;
R⁴⁻¹ is independently halogen, cyano, hydroxyl, C₁₋₆ alkyl-O-, -NR⁴ⁱR^{4j}, -C(=O)OR^{4d} or - C(=O)NR^{4e}R^{4f};
R^{4a}, R^{4b}, R^{4c}, R^{4d}, R^{4e}, R^{4f}, R⁴ⁱ and R^{4j} are independently hydrogen or C₁₋₆ alkyl;
p is 0 or 1; 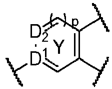
is phenyl, "5- to 7-membered heterocycloalkenyl containing 1-3 heteroatoms independently selected from O, S and N", "5- to 7-membered heteroaryl containing 1-3 heteroatoms independently selected from O, S and N" or 5- to 7-membered cycloalkenyl; wherein D¹ is C, CH or N; D₂ is 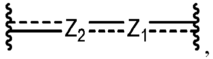 wherein Z₁ and Z₂ are independently a bond, CH, CH₂, O, S, N or NH;
r is 0, 1, 2, 3, 4, 5 or 6;
R⁵ is independently halogen or C₁₋₆ alkyl;
X¹ and X² are independently CR^{b} or N, and X¹ and X² are not both CR^{b};
L₁ is a bond, -C(=O)- or C₁₋₆ alkylene;
R¹ is C₆₋₂₀ aryl, C₈₋₁₁ benzocycloalkenyl, "5- to 12-membered heteroaryl containing 1-4 heteroatoms independently selected from O, S and N", C₆₋₂₀ aryl substituted with one or more R¹⁻¹ or "5- to 12-membered heteroaryl containing 1-4 heteroatoms independently selected from O, S and N" substituted with one or more R¹⁻²; provided that when multiple substituents are present, the substituents are the same or different;
R^{b}, R¹⁻¹ and R¹⁻² are independently halogen, -OR^{c}, cyano, -C(=O)R¹¹, -NR¹²R¹³, -C(=O)OR¹⁴, - C(=O)NR¹⁵R¹⁶, C₁₋₆ alkyl, C₂₋₆ alkenyl, C₂₋₆ alkynyl, C₃₋₁₀ cycloalkyl, "5- to 7-membered heterocycloalkyl containing 1 or 2 heteroatoms independently selected from O and N", C₆₋₂₀ aryl, "5- to 7-membered heteroaryl containing 1 or 2 heteroatoms independently selected from O and N", C₁₋₆ alkyl substituted with one or more R¹⁻¹⁻¹, C₁₋₆ alkyl-O substituted with one or more R¹⁻¹⁻², C₃₋₁₀ cycloalkyl substituted with one or more R¹⁻¹⁻³, "5- to 7-membered heterocycloalkyl containing 1 or 2 heteroatoms independently selected from O and N" substituted with one or more R¹⁻¹⁻⁴, C₆₋₂₀ aryl substituted with one or more R¹⁻¹⁻⁵, or "5- to 7-membered heteroaryl containing 1 or 2 heteroatoms independently selected from O and N" substituted with one or more R^{1-1-6,} provided that when multiple substituents are present, the substituents are the same or different; or, when the number of R¹⁻¹ or R¹⁻² is more than one, two optional R¹⁻¹ or R¹⁻² are connected, together with the atoms on the ring to which they are attached, independently form 3- to 8-membered cyclic olefin;
R^{c}, R¹² and R¹³ are independently hydrogen, C₁₋₆ alkyl, -C(=O)R^{c1}, -C(=O)OR^{c2}, -C(=O)NR^{c3}R^{c4} or -SO₂R^{c5}; R^{c1}, R^{c2}, R^{c3}, R^{c4} and R^{c5} are independently hydrogen, C₁₋₆ alkyl, C₃₋₁₀ cycloalkyl, "5-to 7-membered heterocycloalkyl containing 1 or 2 heteroatoms independently selected from O and N", C₆₋₂₀ aryl, "5- to 7-membered heteroaryl containing 1 or 2 heteroatoms independently selected from O and N", C₁₋₆ alkyl substituted with one or more R⁴⁻¹⁻¹, C₃₋₁₀ cycloalkyl substituted with one or more R⁴⁻¹⁻², "5- to 7-membered heterocycloalkyl containing 1 or 2 heteroatoms independently selected from O and N" substituted with one or more R⁴⁻¹⁻³, C₆₋₂₀ aryl substituted with one or more R⁴⁻¹⁻⁴, or, "5- to 7-membered heteroaryl containing 1 or 2 heteroatoms independently selected from O and N" substituted with one or more R⁴⁻¹⁻⁵; provided that when multiple substituents are present, the substituents are the same or different;
R¹⁻¹⁻¹, R¹⁻¹⁻², R¹⁻¹⁻³, R¹⁻¹⁻⁴, R¹⁻¹⁻⁵, R¹⁻¹⁻⁶, R⁴⁻¹⁻¹, R⁴⁻¹⁻², R⁴⁻¹⁻³, R⁴⁻¹⁻⁴ and R⁴⁻¹⁻⁵ are independently cyano, halogen, hydroxyl, C₁₋₆ alkyl-O-, C₁₋₆ alkyl, -C(=O)R²¹, -NR²²R²³, -C(=O)OR²⁴ or -C(=O)NR²⁵R²⁶;
R¹¹, R²¹, R²², R²³, R¹⁴, R²⁴, R¹⁵, R²⁵, R¹⁶ and R²⁶ are independently hydrogen or C₁₋₆ alkyl;
L₂ is a bond, C₁₋₆ alkylene, -C(=O)-, -O(R^{L-1})ₙ₁-, -S(R^{L-2})ₙ₂- or -NR^{L-3}(R^{L-4})ₙ₃-; R^{L-1}, R^{L-2} and R^{L-4} are independently C₁₋₆ alkylene; R^{L-3} is hydrogen or C₁₋₆ alkyl; n1, n2 and n3 are independently 0 or 1;
R³ is C₃₋₁₂ cycloalkyl, C₃₋₁₂ cycloalkyl substituted with one or more R³⁻¹, "4- to 12-membered heterocycloalkyl containing 1-3 heteroatoms independently selected from O, S and N", "4- to 12-membered heterocycloalkyl containing 1-3 heteroatoms independently selected from O, S and N" substituted with one or more R³⁻², C₁₋₆ alkyl, C₁₋₆ alkyl substituted with one or more R³⁻³, -OR^{d}, - SR^{d1}, -NR^{e1}R^{e2} or -C(=O)NR^{e3}R^{e4}; provided that when multiple substituents are present, the substituents are the same or different;
R³⁻¹, R³⁻² and R³⁻³ are independently C₁₋₆ alkyl, C₁₋₆ alkyl substituted with one or more R³⁻¹⁻¹, hydroxyl, C1-6 alkyl-O-, halogen, O=, -NR^{e5}R^{e6} or -C(=O)NR^{e7}R^{e8};
R^{d}, R^{d1}, R^{e1}, R^{e2}, R^{e3} and R^{e4} are independently hydrogen, C₁₋₆ alkyl, C₃₋₁₀ cycloalkyl, "4- to 10-membered heterocycloalkyl containing 1-3 heteroatoms independently selected from O and N", or C₁₋₆ alkyl substituted with one or more R³⁻¹⁻².
R³⁻¹⁻¹ and R³⁻¹⁻² are independently deuterium, cyano, halogen, hydroxyl, C₁₋₆ alkyl-O-, -C(=O)R^{e9}, -NR^{e10}R^{e11}, -C(=O)OR^{e12} or ^{_}C(=O)NR^{e13}R^{e14}.
R^{e5}, R^{e6}, R^{e7}, R^{e8}, R^{e9}, R^{e10}, R^{e11}, R^{e12}, R^{e13} and R^{e14} are independently hydrogen or C₁₋₆ alkyl.

2. The nitrogen-containing heterocyclic compound of formula I, the pharmaceutically acceptable salt thereof, the stereoisomer thereof, the tautomer thereof or the isotopically labeled compound thereof according to claim 1, wherein m is 0 or 1;
and/or, in 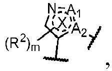 A₁ is CH or N, and A₂ is N; or, A₁ is O, and A₂ is C; or, A₁ is NH, and A₂ is C;
and/or, R² is -CN, C₁₋₆ alkyl, C₁₋₆ alkyl substituted with one or more R²⁻¹, halogen, -NR^{2c1}R^{2c2}, - C(=O)NR^{2e1}R^{2e2}, C₃₋₁₀ cycloalkyl, C₆₋₂₀ aryl, or "5- to 12-membered heteroaryl containing 1-4 heteroatoms independently selected from O, S and N";
and/or, R²⁻¹ is hydroxyl;
and/or, R^{2c1}, R^{2c2}, R^{2e1} and R^{2e2} are independently hydrogen;
and/or, n is 0 or 1;
and/or, R⁴ is independently C₁₋₆ alkyl, C₁₋₆ alkyl substituted with one or more R⁴⁻¹, or C₁₋₆ alkyl-O-;
and/or, R⁴⁻¹ is independently cyano;
and/or, D¹ is C or N;
and/or, in D₂, either of Z₁ and Z₂ is CH, CH₂, O, S or N, and the other is a bond;
and/or, r is 0 or 1;
and/or, 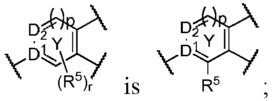
and/or, when p is 1, 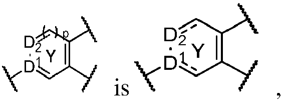 which is phenyl, "6-membered heterocycloalkenyl containing 1-2 heteroatoms independently selected from O and N" or "6-membered heteroaryl containing 1-2 heteroatoms independently selected from O, S and N"; when p is 0, 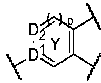 is thiophenyl;
and/or, R⁵ is independently halogen;
and/or, X¹ and X² are independently N;
and/or, L₁ is a bond or -C(=O)-;
and/or, R¹ is C₆₋₂₀ aryl substituted with one or more R¹⁻¹, C₈₋₁₁ benzocycloalkenyl, "5- to 12-membered heteroaryl containing 1-4 heteroatoms independently selected from O, S and N", or "5-to 12-membered heteroaryl containing 1-4 heteroatoms independently selected from O, S and N" substituted with one or more R¹⁻²;
and/or, R¹⁻¹ is independently halogen, -NR¹²R¹³, -OR^{c}, C₁₋₆ alkyl, C₂₋₆ alkynyl, C₁₋₆ alkyl substituted with one or more R¹⁻¹⁻¹, C₃₋₁₀ cycloalkyl, or C₁₋₆ alkyl-O- substituted with one or more R¹⁻¹⁻², or when the number of R¹⁻¹ is more than one, two optional R¹⁻¹ are connected, together with the atoms on the ring to which they are attached, independently form 3- to 8-membered cyclic olefin;
and/or, R^{c} is hydrogen, C₁₋₆ alkyl, -C(=O)R^{c1}, -C(=O)OR^{c2} or -C(=O)NR^{c3}R^{c4} ;
and/or, R¹² and R¹³ are independently hydrogen, -C(=O)R^{c1}, -C(=O)OR^{c2} or -SO₂R^{c5};
and/or, R¹⁻¹⁻¹ is independently halogen;
and/or, R¹⁻¹⁻² is independently C₁₋₆ alkyl-O-;
and/or, R¹⁻² is independently C₁₋₆ alkyl;
and/or, in R^{c}, R^{c1} is independently C₁₋₆ alkyl, C₁₋₆ alkyl substituted with one or more R⁴⁻¹⁻¹, or C₆₋₂₀ aryl substituted with one or more R⁴⁻¹⁻⁴; R⁴⁻¹⁻¹ is -NR²²R²³, R²² and R²³ are independently hydrogen; R⁴⁻¹⁻⁴ is independently -NR²²R²³ or C₁₋₆ alkyl, R²² and R²³ are independently hydrogen;
and/or, in R^{c}, R^{c2} is C₁₋₆ alkyl;
and/or, in R^{c}, R^{c3} and R^{c4} are independently hydrogen, C₁₋₆ alkyl, C₆₋₂₀ aryl substituted with one or more R⁴⁻¹⁻⁴, R⁴⁻¹⁻⁴ is independently C₁₋₆ alkyl;
and/or, in R¹² and R¹³, R^{c1}, R^{c2} and R^{c5} are independently C₁₋₆ alkyl;
and/or, L₂ is a bond or -O(R^{L-1})ₙ₁-;
and/or, R^{L-1} is independently C₁₋₆ alkylene;
and/or, n1 is 0 or 1;
and/or, R³ is C₃₋₁₂ cycloalkyl substituted with one or more R³⁻¹, "4- to 12-membered heterocycloalkyl containing 1-3 heteroatoms independently selected from O, S and N", "4- to 12-membered heterocycloalkyl containing 1-3 heteroatoms independently selected from O, S and N" substituted with one or more R³⁻², C₁₋₆ alkyl or -NR^{e1}R^{e2};
and/or, R³⁻¹ is independently -NR^{e5}R^{e6} or C₁₋₆ alkyl substituted with one or more R³⁻¹⁻¹, R³⁻¹⁻¹ is independently -NR^{e10}R^{e11}; R^{e5}, R^{e6}, R^{e10} and R^{e11} are independently C₁₋₆ alkyl;
and/or, R³⁻² is C₁₋₆ alkyl, halogen, -NR^{e5}R^{e6}, O= or C₁₋₆ alkyl substituted with one or more R³⁻¹⁻¹, R^{e5} and R^{e6} are independently C₁₋₆ alkyl; R³⁻¹⁻¹ is halogen or deuterium;
and/or, R^{e1} and R^{e2} are independently C₁₋₆ alkyl;
and/or, when n is 1, 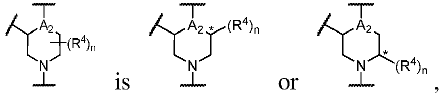 wherein 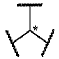 represents R configuration, S configuration or a mixture thereof.

3. The nitrogen-containing heterocyclic compound of formula I, the pharmaceutically acceptable salt thereof, the stereoisomer thereof, the tautomer thereof or the isotopically labeled compound thereof according to claim 1, wherein
in 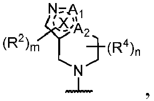 m and n are independently 0 or 1, and 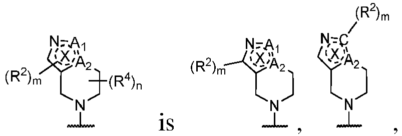 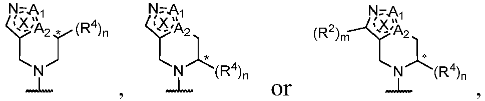 wherein 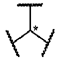 represents R configuration, S configuration or a mixture thereof;
and/or, when 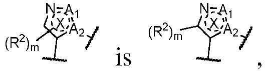 R² is -CN, C₁₋₆ alkyl, halogen, -C(=O)NR^{2e1}R^{2e2}, C₃₋₁₀ cycloalkyl, C₆₋₂₀ aryl or 5- to 12-membered heteroaryl containing 1-4 heteroatoms independently selected from O, S and N; when 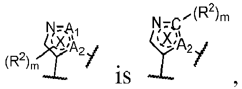 R² is -CN, C₁₋₆ alkyl, C₁₋₆ alkyl substituted with one or more R²¹, halogen, -NR^{2c1}R^{2c2}, C₃₋₁₀ cycloalkyl or C₆₋₂₀ aryl;
and/or, when 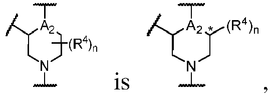 and n is 1, R⁴ is C₁₋₆ alkyl substituted with one or more R⁴⁻¹, or C₁₋₆ alkyl-O-; when 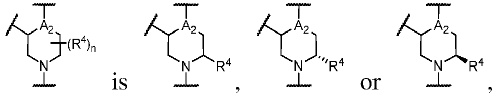 and n is 1, R⁴ is C₁₋₆ alkyl;
and/or, D¹ is C, and in D₂, either of Z₁ and Z₂ is CH or N, and the other is a bond; or D¹ is CH, and in D₂, either of Z₁ and Z₂ is O or CH₂, and the other is a bond; or D¹ is N, and in D₂, either of Z₁ and Z₂ is CH₂, and the other is a bond;
and/or, in 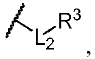 when L₂ is a bond, R³ is "4- to 12-membered heterocycloalkyl containing 1-3 heteroatoms independently selected from O, S and N"; or, when L₂ is -O(R^{L-1})ₙ₁-, R³ is C₃₋₁₂ cycloalkyl substituted with one or more R³⁻¹, "4- to 12-membered heterocycloalkyl containing 1-3 heteroatoms independently selected from O, S and N" substituted with one or more R³⁻², C₁₋₆ alkyl or -NR^{e1}R^{e2};
and/or, when R³ is "4- to 12-membered heterocycloalkyl containing 1-3 heteroatoms independently selected from O, S and N" substituted with one or more R³⁻², R³⁻² is C₁₋₆ alkyl;
and/or, in 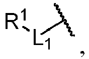 when L₁ is a bond, R¹ is C₆₋₂₀ aryl substituted with one or more R¹⁻¹, C₈₋₁₁ benzocycloalkenyl, 5- to 12-membered heteroaryl containing 1-4 heteroatoms independently selected from O, S and N, or 5- to 12-membered heteroaryl containing 1-4 heteroatoms independently selected from O, S and N substituted with one or more R¹⁻²; or, when L₁ is -C(=O)-, R¹ is C₆₋₂₀ aryl substituted with one or more R¹⁻¹.

4. The nitrogen-containing heterocyclic compound of formula I, the pharmaceutically acceptable salt thereof, the stereoisomer thereof, the tautomer thereof or the isotopically labeled compound thereof according to any one of claims 1-3, wherein
when R² is C₁₋₆ alkyl or C₁₋₆ alkyl substituted with one or more R²⁻¹, the C₁₋₆ alkyl and the C₁₋₆ alkyl in the C₁₋₆ alkyl substituted with one or more R²⁻¹ are methyl, ethyl, n-propyl, isopropyl, n-butyl, sec-butyl, isobutyl or tert-butyl, for example, methyl;
and/or, when R² is C₂₋₆ alkenyl, the C₂₋₆ alkenyl is vinyl, propenyl or allyl;
and/or, when R² is C₂₋₆ alkynyl, the C₂₋₆ alkynyl is C₂₋₃ alkynyl, for example, ethynyl, propynyl or propargyl;
and/or, when R² is halogen, the halogen is fluorine, chlorine, bromine or iodine, for example, bromine;
and/or, when R² is C₃₋₁₀ cycloalkyl or C₃₋₁₀ cycloalkyl substituted with one or more R²⁻², the C₃₋₁₀ cycloalkyl and the C₃₋₁₀ cycloalkyl in the C₃₋₁₀ cycloalkyl substituted with one or more R²⁻² are cyclohexyl, cyclopentyl, cyclobutyl or cyclopropyl, for example, cyclopropyl;
and/or, when R² is "4- to 10-membered heterocycloalkyl containing 1-3 heteroatoms independently selected from O and N" or "4- to 10-membered heterocycloalkyl containing 1-3 heteroatoms independently selected from O and N" substituted with one or more R²⁻³, the "4- to 10-membered heterocycloalkyl containing 1-3 heteroatoms independently selected from O and N" and the "4- to 10-membered heterocycloalkyl containing 1-3 heteroatoms independently selected from O and N" in the "4- to 10-membered heterocycloalkyl containing 1-3 heteroatoms independently selected from O and N" substituted with one or more R²⁻³ are 4- to 6-membered heterocycloalkyl containing 1 heteroatom of O or N;
and/or, when R² is C₆₋₂₀ aryl or C₆₋₂₀ aryl substituted with one or more R²⁻⁴, the C₆₋₂₀ aryl and the C₆₋₂₀ aryl in the C₆₋₂₀ aryl substituted with one or more R²⁻⁴ are C₆₋₁₀ aryl, for example, phenyl or naphthyl, for another example, phenyl;
and/or, when R² is "5- to 12-membered heteroaryl containing 1-4 heteroatoms independently selected from O, S and N" or "5- to 12-membered heteroaryl containing 1-4 heteroatoms independently selected from O, S and N" substituted with one or more R²⁻⁵, the "5- to 12-membered heteroaryl containing 1-4 heteroatoms independently selected from O, S and N" and the "5- to 12-membered heteroaryl containing 1-4 heteroatoms independently selected from O, S and N" in the "5- to 12-membered heteroaryl containing 1-4 heteroatoms independently selected from O, S and N" substituted with one or more R²⁻⁵ are 5- to 6-membered heteroaryl containing 1 heteroatom of O, S or N, for example, pyridinyl, for another example, 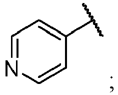
and/or, when R²⁻¹, R²⁻², R²⁻³, R²⁻⁴ and R²⁻⁵ are independently halogen, the halogen is fluorine, chlorine, bromine or iodine;
and/or, when R²⁻¹, R²⁻², R²⁻³, R²⁻⁴ and R²⁻⁵ are independently C₁₋₆ alkyl, the C₁₋₆ alkyl is methyl, ethyl, n-propyl, isopropyl, n-butyl, sec-butyl, isobutyl or tert-butyl, for example, methyl;
and/or, when R²⁻¹, R²⁻², R²⁻³, R²⁻⁴ and R²⁻⁵ are independently C₂₋₆ alkenyl, the C₂₋₆ alkenyl is vinyl, propenyl or allyl;
and/or, when R²⁻¹, R²⁻², R²⁻³, R²⁻⁴ and R²⁻⁵ are independently C₂₋₆ alkynyl, the C₂₋₆ alkynyl is ethynyl, propynyl or propargyl;
and/or, when R²⁻¹, R²⁻², R²⁻³, R²⁻⁴ and R²⁻⁵ are independently C₁₋₆ alkyl-O-, the C₁₋₆ alkyl in the C₁₋₆ alkyl-O- is methyl, ethyl, n-propyl, isopropyl, n-butyl, *sec-*butyl, isobutyl or *tert-*butyl*,* for example, methyl;
and/or, when R^{2a}, R^{2b}, R^{2c1}, R^{2c2}, R^{2d}, R^{2e1} and R^{2e2} are independently C₁₋₆ alkyl, the C₁₋₆ alkyl is methyl, ethyl, n-propyl, isopropyl, n-butyl, *sec-*butyl, isobutyl or *tert*-butyl, for example, methyl;
and/or, when R³¹, R³², R³³, R³⁴, R³⁵ and R³⁶ are independently C₁₋₆ alkyl, the C₁₋₆ alkyl is methyl, ethyl, n-propyl, isopropyl, n-butyl, *sec*-butyl, isobutyl, or *tert*-butyl, for example, methyl;
and/or, when R⁴ is C₁₋₆ alkyl or C₁₋₆ alkyl substituted with one or more R⁴⁻¹, the C₁₋₆ alkyl and the C₁₋₆ alkyl in the C₁₋₆ alkyl substituted with one or more R⁴⁻¹ are methyl, ethyl, n-propyl, isopropyl, n-butyl, *sec-*butyl, isobutyl or *tert*-butyl, for example, methyl;
and/or, when R⁴ is C₁₋₆ alkyl-O-, the C₁₋₆ alkyl in the C₁₋₆ alkyl-O- is methyl, ethyl, n-propyl, isopropyl, n-butyl, *sec-*butyl, isobutyl or *tert*-butyl, for example, methyl;
and/or, when n is 2, 3, 4, 5 or 6, and two optional R⁴ are connected, together with the atoms on the ring to which they are attached, independently form 3- to 8-membered carbocyclic ring, the 3- to 8-membered carbocyclic ring is 3- to 6-membered carbocyclic ring, and the carbocyclic ring is a monocyclic or bridged cycloalkyl;
and/or, when n is 2, 3, 4, 5 or 6, and two optional R⁴ are connected, together with the atoms on the ring to which they are attached, independently form "3- to 8-membered heterocyclic ring containing 1-3 heteroatoms independently selected from O, S and N", the "3- to 8-membered heterocyclic ring containing 1-3 heteroatoms independently selected from O, S and N" is 3- to 6-membered heterocyclic ring containing 1 heteroatom of O, S or N;
and/or, when R⁴⁻¹ is independently halogen, the halogen is fluorine, chlorine, bromine or iodine;
and/or, when R⁴⁻¹ is independently C₁₋₆ alkyl-O-, the C₁₋₆ alkyl in the C₁₋₆ alkyl-O- is methyl, ethyl, n-propyl, isopropyl, n-butyl, sec-butyl, isobutyl or *tert*-butyl;
and/or, when R^{4a}, R^{4b}, R^{4c}, R^{4d}, R^{4e}, R^{4f}, R⁴ⁱ and R^{4j} are independently C₁₋₆ alkyl, the C₁₋₆ alkyl is methyl, ethyl, n-propyl, isopropyl, n-butyl, sec-butyl, isobutyl or tert-butyl;
and/or, when 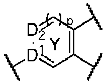 is "5- to 7-membered heterocycloalkenyl containing 1-3 heteroatoms independently selected from O, S and N", the "5- to 7-membered heterocycloalkenyl containing 1-3 heteroatoms independently selected from O, S and N" is 6-membered heterocycloalkenyl containing 1-2 heteroatoms independently selected from O and N, for example, "6-membered heterocycloalkenyl containing 1 heteroatom being O or N", for another example, 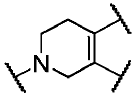 or 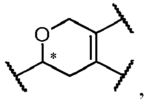 and for yet another example, 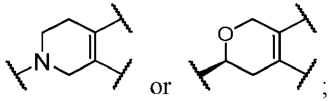
and/or, when 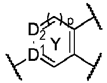 is "5- to 7-membered heteroaryl containing 1-3 heteroatoms independently selected from O, S and N", the "5- to 7-membered heteroaryl containing 1-3 heteroatoms independently selected from O, S" and N is "6-membered heteroaryl containing 1-2 heteroatoms independently selected from O, S and N", for example, pyridinyl, for another example, 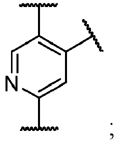
and/or, when 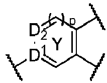 is 5- to 7-membered cycloalkenyl, the 5- to 7-membered cycloalkenyl is cyclopentenyl or cyclohexenyl;
and/or, when R⁵ is independently halogen, the halogen is fluorine, chlorine, bromine or iodine, for example, fluorine;
and/or, when R⁵ is independently C₁₋₆ alkyl, the C₁₋₆ alkyl is methyl, ethyl, n-propyl, isopropyl, n-butyl, *sec-*butyl, isobutyl or *tert*-butyl;
and/or, when L₁ is C₁₋₆ alkylene, the C₁₋₆ alkylene is -CH₂-, -CH₂CH₂-, -CH₂CH₂CH₂-, - CH(CH₃)CH₂-, -CH₂CH₂CH₂CH₂-, -CH(CH₃)CH₂CH₂-, -CH₂CH(CH₃)CH₂- or -C(CH₃)₂CH₂-;
and/or, when R¹ is C₆₋₂₀ aryl or C₆₋₂₀ aryl substituted with one or more R¹⁻¹, the C₆₋₂₀ aryl and the C₆₋₂₀ aryl in the C₆₋₂₀ aryl substituted with one or more R¹⁻¹ are C₆₋₁₀ aryl, for example, phenyl or naphthyl;
and/or, when R¹ is C₈₋₁₁ benzocycloalkenyl, the C₈₋₁₁ benzocycloalkenyl is benzocyclobutenyl, benzocyclopentenyl or benzocyclohexenyl;
and/or, when R¹ is 5- to 12-membered heteroaryl containing "1-4 heteroatoms independently selected from O, S and N" or "5- to 12-membered heteroaryl containing 1-4 heteroatoms independently selected from O, S and N" substituted with one or more R¹⁻², the "5- to 12-membered heteroaryl containing 1-4 heteroatoms independently selected from O, S and N" and the "5- to 12-membered heteroaryl containing 1-4 heteroatoms independently selected from O, S and N" in the "5- to 12-membered heteroaryl containing 1-4 heteroatoms independently selected from O, S and N" substituted with one or more R¹⁻² are "5- to 9-membered heteroaryl containing 1-2 heteroatoms independently selected from O, S and N", for example, "5- to 9-membered heteroaryl containing 1 heteroatom being N", for another example, quinolinyl, and for yet another example, 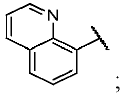 for another example, "9-membered heteroaryl containing 2 heteroatoms independently selected from O, S and N", for another example, indazolyl, and for yet another example, 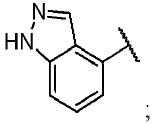
and/or, when R^{b}, R¹⁻¹ and R¹⁻² are independently halogen, the halogen is fluorine, chlorine, bromine or iodine, for example, chlorine or bromine;
and/or, when R^{b}, R¹⁻¹ and R¹⁻² are independently C₁₋₆ alkyl or C₁₋₆ alkyl substituted with one or more R¹⁻¹⁻¹, the C₁₋₆ alkyl and the C₁₋₆ alkyl in the C₁₋₆ alkyl substituted with one or more R¹⁻¹⁻¹ are methyl, ethyl, n-propyl, isopropyl, n-butyl, sec-butyl, isobutyl or tert-butyl;
and/or, when R^{b}, R¹⁻¹ and R¹⁻² are independently C₁₋₆ alkyl substituted with one or more R¹⁻¹⁻¹, the more R¹⁻¹⁻¹ is two or three R¹⁻¹⁻¹;
and/or, when R^{b}, R¹⁻¹ and R¹⁻² are independently C₂₋₆ alkenyl, the C₂₋₆ alkenyl is vinyl, propenyl or allyl;
and/or, when R^{b}, R¹⁻¹ and R¹⁻² are independently C₂₋₆ alkynyl, the C₂₋₆ alkynyl is ethynyl, propynyl or propargyl;
and/or, when R^{b}, R¹⁻¹ and R¹⁻² are independently C₁₋₆ alkyl-O- substituted with one or more R¹⁻¹⁻², the C₁₋₆ alkyl in the C₁₋₆ alkyl-O- and the C₁₋₆ alkyl-O- substituted with one or more R¹⁻¹⁻² are methyl, ethyl, n-propyl, isopropyl, n-butyl, *sec*-butyl, isobutyl or *tert*-butyl;
and/or, when R^{b}, R¹⁻¹ and R¹⁻² are independently C₃₋₁₀ cycloalkyl or C₃₋₁₀ cycloalkyl substituted with one or more R¹⁻¹⁻³, the C₃₋₁₀ cycloalkyl and the C₃₋₁₀ cycloalkyl in the C₃₋₁₀ cycloalkyl substituted with one or more R¹⁻¹⁻³ are cyclohexyl, cyclopentyl, cyclobutyl or cyclopropyl, for example, cyclopropyl;
and/or, when R^{b}, R¹⁻¹ and R¹⁻² are independently "5- to 7-membered heterocycloalkyl containing 1 or 2 heteroatoms independently selected from O and N" or "5- to 7-membered heterocycloalkyl containing 1 or 2 heteroatoms independently selected from O and N" substituted with one or more R¹⁻¹⁻⁴, the "5- to 7-membered heterocycloalkyl containing 1 or 2 heteroatoms independently selected from O and N" and the "5- to 7-membered heterocycloalkyl containing 1 or 2 heteroatoms independently selected from O and N" in the "5- to 7-membered heterocycloalkyl containing 1 or 2 heteroatoms independently selected from O and N" substituted with one or more R¹⁻¹⁻⁴ are 5- to 6-membered heterocycloalkyl containing 1 heteroatom of O or N;
and/or, when R^{b}, R¹⁻¹ and R¹⁻² are independently C₆₋₂₀ aryl or C₆₋₂₀ aryl substituted with one or more R¹⁻¹⁻⁵, the C₆₋₂₀ aryl and the C₆₋₂₀ aryl in the C₆₋₂₀ aryl substituted with one or more R¹⁻¹⁻⁵ are C₆₋₁₀ aryl, for example, phenyl or naphthyl;
and/or, when R^{b}, R¹⁻¹ and R¹⁻² are independently "5- to 7-membered heteroaryl containing 1 or 2 heteroatoms independently selected from O and N" or "5- to 7-membered heteroaryl containing 1 or 2 heteroatoms independently selected from O and N" substituted with one or more R¹⁻¹⁻⁶, the "5- to 7-membered heteroaryl containing 1 or 2 heteroatoms independently selected from O and N" and "the 5- to 7-membered heteroaryl containing 1 or 2 heteroatoms independently selected from O and N" in the "5- to 7-membered heteroaryl containing 1 or 2 heteroatoms independently selected from O and N" substituted with one or more R¹⁻¹⁻⁶ are "5- to 6-membered heteroaryl containing 1 heteroatom being O or N";
and/or, when two optional R¹⁻¹or R¹⁻² are connected, together with the atoms on the ring to which they are attached, independently form 3- to 8-membered cyclic olefin, the 3- to 8-membered cyclic olefin is cyclobutene, cyclopentene or cyclohexene;
and/or, when R^{c}, R¹² and R¹³ are independently C₁₋₆ alkyl, the C₁₋₆ alkyl is methyl, ethyl, n-propyl, isopropyl, n-butyl, *sec-*butyl, isobutyl or *tert*-butyl;
and/or, when R^{c1}, R^{c2}, R^{c3}, R^{c4} and R^{c5} are independently C₁₋₆ alkyl or C₁₋₆ alkyl substituted with one or more R⁴⁻¹⁻¹, the C₁₋₆ alkyl and the C₁₋₆ alkyl in the C₁₋₆ alkyl substituted with one or more R⁴⁻¹⁻¹ are methyl, ethyl, n-propyl, isopropyl, n-butyl, sec-butyl, isobutyl or *tert*-butyl;
and/or, when R^{c1}, R^{c2}, R^{c3}, R^{c4} and R^{c5} are independently C₃₋₁₀ cycloalkyl or C₃₋₁₀ cycloalkyl substituted with one or more R⁴⁻¹⁻², the C₃₋₁₀ cycloalkyl and the C₃₋₁₀ cycloalkyl in the C₃₋₁₀ cycloalkyl substituted with one or more R⁴⁻¹⁻² are cyclohexyl, cyclopentyl, cyclobutyl or cyclopropyl;
and/or, when R^{c1}, R^{c2}, R^{c3}, R^{c4} and R^{c5} are independently C₆₋₂₀ aryl or C₆₋₂₀ aryl substituted with one or more R⁴⁻¹⁻⁴, the C₆₋₂₀ aryl and the C₆₋₂₀ aryl in the C₆₋₂₀ aryl substituted with one or more R⁴⁻¹⁻⁴ are C₆₋₁₀ aryl, for example, phenyl or naphthyl;
and/or, when R¹⁻¹⁻¹, R¹⁻¹⁻², R¹⁻¹⁻³, R¹⁻¹⁻⁴, R¹⁻¹⁻⁵, R¹⁻¹⁻⁶, R⁴⁻¹⁻¹, R⁴⁻¹⁻², R⁴⁻¹⁻³, R⁴⁻¹⁻⁴ and R⁴⁻¹⁻⁵ are independently halogen, the halogen is fluorine, chlorine, bromine or iodine, for example, fluorine;
and/or, when R¹⁻¹⁻¹, R¹⁻¹⁻² , R¹⁻¹⁻³, R¹⁻¹⁻⁴, R¹⁻¹⁻⁵, R¹⁻¹⁻⁶, R⁴⁻¹⁻¹, R⁴⁻¹⁻², R⁴⁻¹⁻³, R⁴⁻¹⁻⁴ and R⁴⁻¹⁻⁵ are independently C₁₋₆ alkyl-O-, the C₁₋₆ alkyl in the C₁₋₆ alkyl-O- is methyl, ethyl, n-propyl, isopropyl, n-butyl, *sec*-butyl, isobutyl or *tert*-butyl;
and/or, when R¹⁻¹⁻¹, R¹⁻¹⁻², R¹⁻¹⁻³, R¹⁻¹⁻⁴, R¹⁻¹⁻⁵, R¹⁻¹⁻⁶, R⁴⁻¹⁻¹, R⁴⁻¹⁻², R⁴⁻¹⁻³, R⁴⁻¹⁻⁴ and R⁴⁻¹⁻⁵ are independently C₁₋₆ alkyl, the C₁₋₆ alkyl is methyl, ethyl, n-propyl, isopropyl, n-butyl, *sec*-butyl, isobutyl or *tert*-butyl;
and/or, when R¹¹, R²¹, R²², R²³, R¹⁴, R²⁴, R¹⁵, R²⁵, R¹⁶ and R²⁶ are independently C₁₋₆ alkyl, the C₁₋₆ alkyl is methyl, ethyl, n-propyl, isopropyl, n-butyl, sec-butyl, isobutyl or *tert*-butyl;
and/or, when L₂ is C₁₋₆ alkylene, the C₁₋₆ alkylene is -CH₂-, -CH₂CH₂-, -CH₂CH₂CH₂-, - CH(CH₃)CH₂-, -CH₂CH₂CH₂CH₂-, -CH(CH₃)CH₂CH₂-, -CH₂CH(CH₃)CH₂- or -C(CH₃)₂CH₂-;
and/or, when R^{L-1}, R^{L-2} or R^{L-4} is independently C₁₋₆ alkylene, the C₁₋₆ alkylene is -CH₂-, -CH₂CH₂-, - CH₂CH₂CH₂-, -CH(CH₃)CH₂-, -CH₂CH₂CH₂CH₂-, -CH(CH₃)CH₂CH₂-, -CH₂CH(CH₃)CH₂- or - C(CH₃)₂CH₂-, for example, -CH₂-, -CH₂CH₂-, -CH₂CH₂CH₂- or -CH(CH₃)CH₂-;
and/or, when R^{L-3} is C₁₋₆ alkyl, the C₁₋₆ alkyl is methyl, ethyl, n-propyl, isopropyl, n-butyl, *sec-*butyl, isobutyl or tert-butyl;
and/or, when R³ is C₃₋₁₂ cycloalkyl or C₃₋₁₂ cycloalkyl substituted with one or more R³⁻¹, the C₃₋₁₂ cycloalkyl and the C₃₋₁₂ cycloalkyl in the C₃₋₁₂ cycloalkyl substituted with one or more R³⁻¹ is C₃₋₁₀ cycloalkyl, for example, cyclopropyl;
and/or, when R³ is C₃₋₁₂ cycloalkyl or C₃₋₁₂ cycloalkyl substituted with one or more R³⁻¹, the C₃₋₁₂ cycloalkyl and the C₃₋₁₂ cycloalkyl in the C₃₋₁₂ cycloalkyl substituted with one or more R³⁻¹ is a monocyclic alkyl, a bridged cycloalkyl or a spiral cycloalkyl;
and/or, when R³ is "4- to 12-membered heterocycloalkyl containing 1-3 heteroatoms independently selected from O, S and N", the "4- to 12-membered heterocycloalkyl containing 1-3 heteroatoms independently selected from O, S and N" is "5- to 8-membered heterocycloalkyl containing 1-2 heteroatoms independently selected from O and N";
and/or, when R³ is "4- to 12-membered heterocycloalkyl containing 1-3 heteroatoms independently selected from O, S and N", the "4- to 12-membered heterocycloalkyl containing 1-3 heteroatoms independently selected from O, S and N" is a monocyclic cycloalkyl, a spiral cycloalky or a fused cycloalkyl; for example, azetidinyl, pyrrolidinyl, tetrahydrofuryl, hexahydro-1*H*-pyrrolizinyl, 7-azaspiro[3.5]nonanyl, 3-azaspiro[5.5]undecanyl, morphinyl or piperidinyl, for another example, 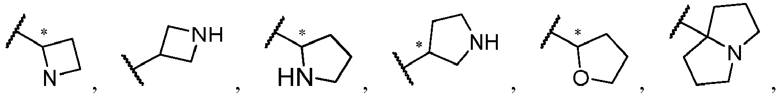 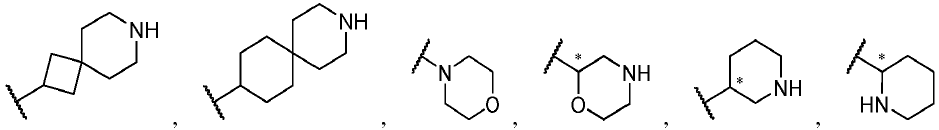 or 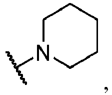 and for yet another example, 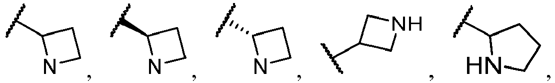 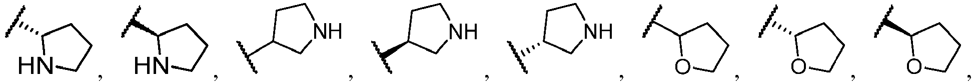 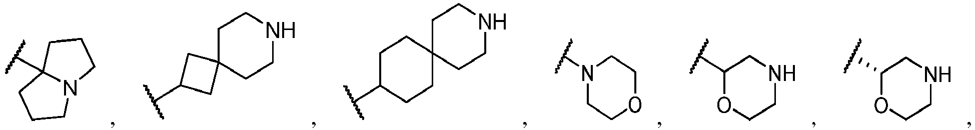 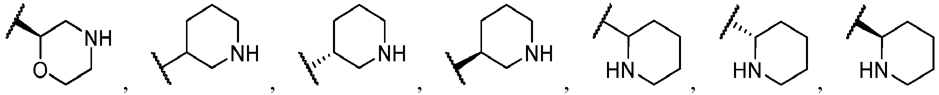 or 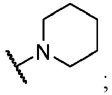
and/or, when R³ is "4- to 12-membered heterocycloalkyl containing 1-3 heteroatoms independently selected from O, S and N" substituted with one or more R³⁻², the "4- to 12-membered heterocycloalkyl containing 1-3 heteroatoms independently selected from O, S and N" in the "4- to 12-membered heterocycloalkyl containing 1-3 heteroatoms independently selected from O, S and N" substituted with one or more R³⁻² is "5- to 11-membered heterocycloalkyl containing 1-2 heteroatoms independently selected from O and N";
and/or, when R³ is "4- to 12-membered heterocycloalkyl containing 1-3 heteroatoms independently selected from O, S and N" substituted with one or more R³⁻², the "4- to 12-membered heterocycloalkyl containing 1-3 heteroatoms independently selected from O, S and N" is a monocyclic alkyl, a spiral cycloalky or fused cycloalkyl; the "4- to 12-membered heterocycloalkyl containing 1-3 heteroatoms independently selected from O, S and N" substituted with one or more R³⁻² is, for example, 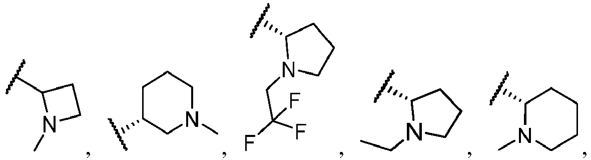 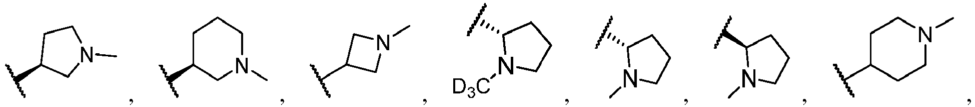 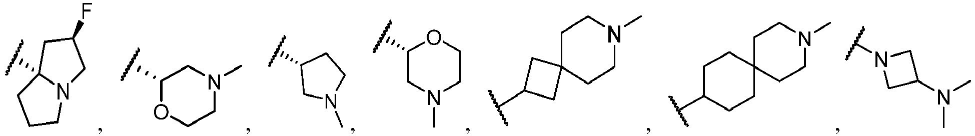 or 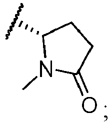
and/or, when R³ is C₁₋₆ alkyl or C₁₋₆ alkyl substituted with one or more R³⁻³, the C₁₋₆ alkyl and the C₁₋₆ alkyl in the C₁₋₆ alkyl substituted with one or more R³⁻³ are methyl, ethyl, n-propyl, isopropyl, n-butyl, *sec-*butyl, isobutyl or *tert*-butyl, for example, methyl;
and/or, when R³⁻¹, R³⁻² and R³⁻³ are independently C₁₋₆ alkyl or C₁₋₆ alkyl substituted with one or more R³⁻¹⁻¹, the C₁₋₆ alkyl and the C₁₋₆ alkyl in the C₁₋₆ alkyl substituted with one or more R³⁻¹⁻¹ are methyl, ethyl, n-propyl, isopropyl, n-butyl, sec-butyl, isobutyl or *tert*-butyl, for example, methyl;
and/or, when R³⁻¹, R³⁻² and R³⁻³ are independently C₁₋₆ alkyl-O-, the C₁₋₆ alkyl in the C₁₋₆ alkyl-O-is methyl, ethyl, n-propyl, isopropyl, n-butyl, *sec*-butyl, isobutyl or *tert*-butyl;
and/or, when R³⁻¹, R³⁻² and R³⁻³ are independently halogen, the halogen is fluorine, chlorine, bromine or iodine, for example, fluorine;
and/or, when R^{d}, R^{d1}, R^{e1}, R^{e2}, R^{e3} and R^{e4} are independently C₁₋₆ alkyl or C₁₋₆ alkyl substituted with one or more R³⁻¹⁻², the C₁₋₆ alkyl and the C₁₋₆ alkyl in the C₁₋₆ alkyl substituted with one or more R³⁻¹⁻² are methyl, ethyl, n-propyl, isopropyl, *n*-butyl, *sec*-butyl, isobutyl or *tert*-butyl, for example, methyl;
and/or, when R^{d}, R^{d1}, R^{e1}, R^{e2}, R^{e3} and R^{e4} are independently C₃₋₁₀ cycloalkyl, the C₃₋₁₀ cycloalkyl is cyclohexyl, cyclopentyl, cyclobutyl or cyclopropyl, for example, cyclopropyl;
and/or, when R^{d}, R^{d1}, R^{e1}, R^{e2}, R^{e3} and R^{e4} are independently "4- to 10-membered heterocycloalkyl containing 1-3 heteroatoms independently selected from O and N", the "4- to 10-membered heterocycloalkyl containing 1-3 heteroatoms independently selected from O and N" is "4- to 6-membered heterocycloalkyl containing 1-2 heteroatoms independently selected from O and N";
and/or, when R³⁻¹⁻¹ and R³⁻¹⁻² are independently halogen, the halogen is fluorine, chlorine, bromine or iodine;
and/or, when R³⁻¹⁻¹ and R³⁻¹⁻² are independently C₁₋₆ alkyl-O-, the C₁₋₆ alkyl in the C₁₋₆ alkyl-O- is methyl, ethyl, n-propyl, isopropyl, n-butyl, *sec*-butyl, isobutyl or tert-butyl;
and/or, when R^{e5}, R^{e6}, R^{e7}, R^{e8}, R^{e9}, R^{e10}, R^{e11}, R^{e12}, R^{e13} and R^{e14} are independently C₁₋₆ alkyl, the C₁₋₆ alkyl is methyl, ethyl, n-propyl, isopropyl, n-butyl, *sec*-butyl, isobutyl or *tert*-butyl*.*

5. The nitrogen-containing heterocyclic compound of formula I, the pharmaceutically acceptable salt thereof, the stereoisomer thereof, the tautomer thereof or the isotopically labeled compound thereof according to claim 1, wherein 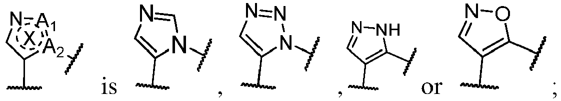
and/or, R² is -CH₃, 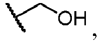 -NH₂, -CN, 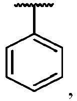 -F, -Br, -Cl, 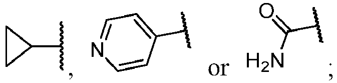
and/or, R⁴ is -CH₃, 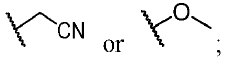
and/or, 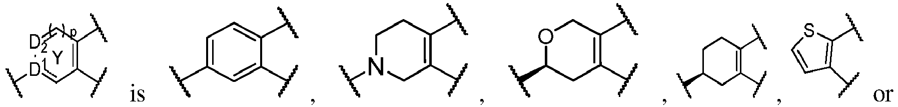 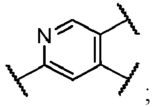
and/or, R¹ is 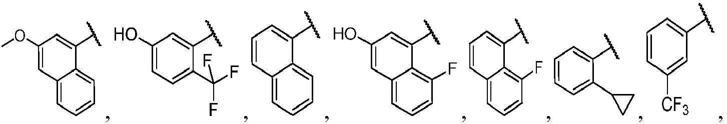 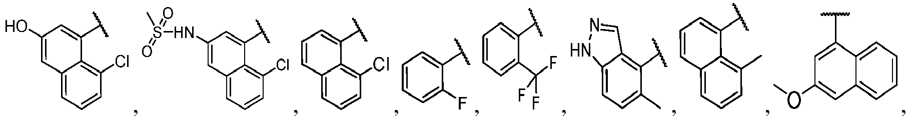 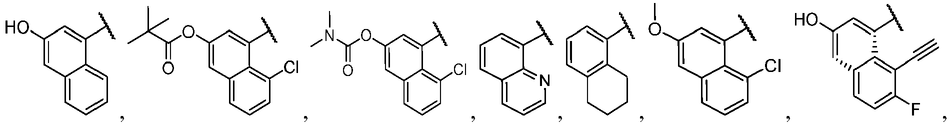 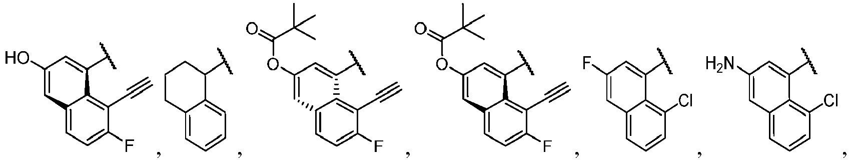 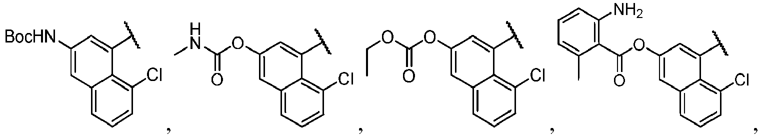 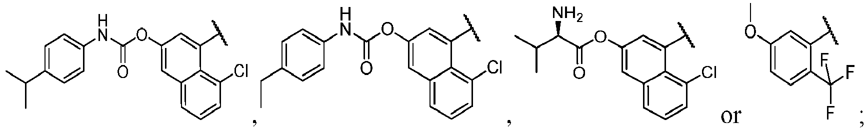
and/or, L₂ is a bond, 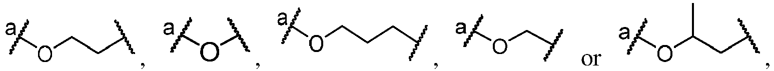 with position a connected to position b of ring 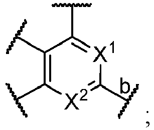
and/or, R³ is methyl,

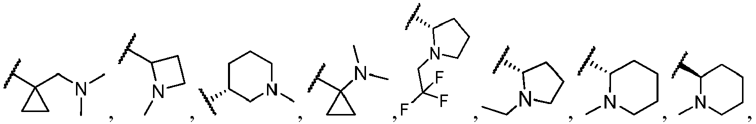

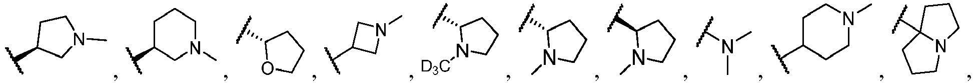

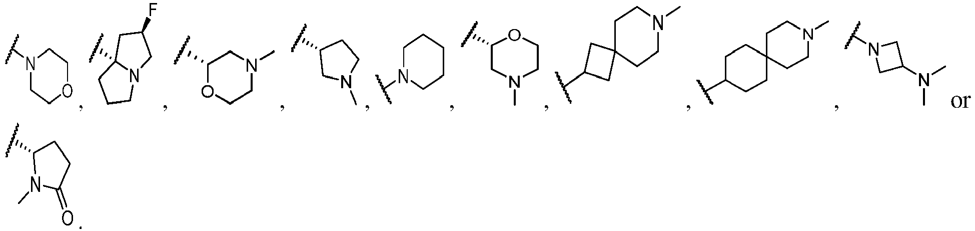

6. The nitrogen-containing heterocyclic compound of formula I, the pharmaceutically acceptable salt thereof, the stereoisomer thereof, the tautomer thereof or the isotopically labeled compound thereof according to claim 5, wherein 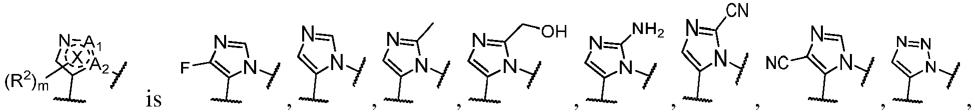 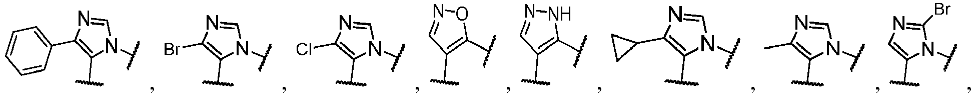 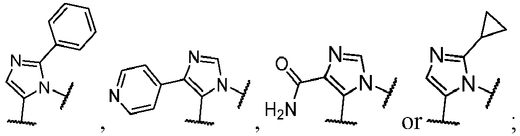
and/or, 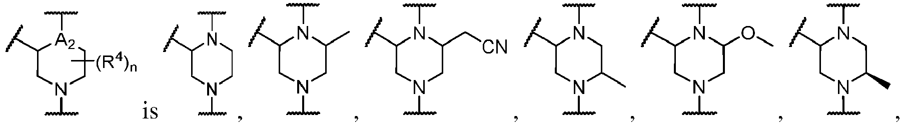 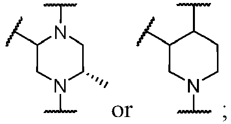
and/or, 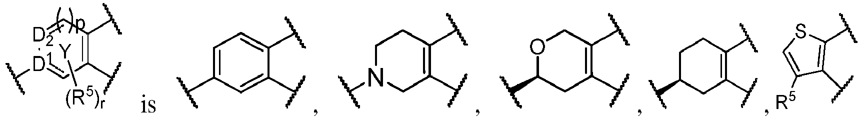 or 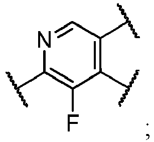
and/or, 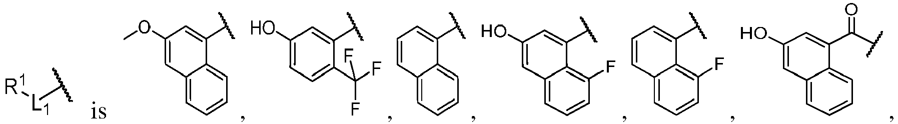 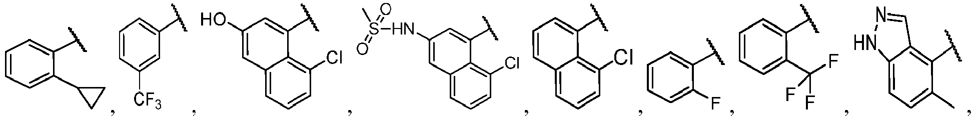 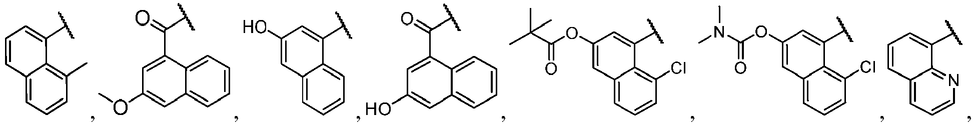 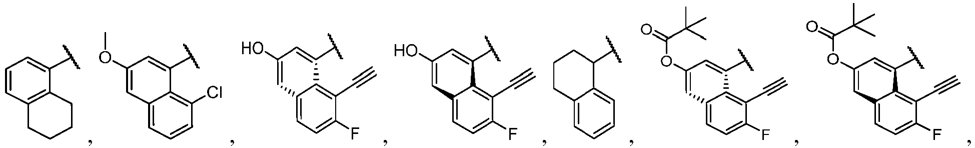 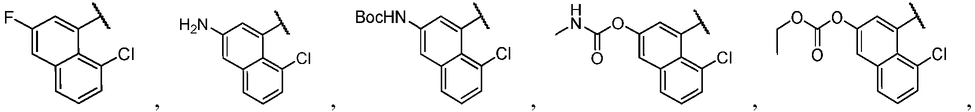 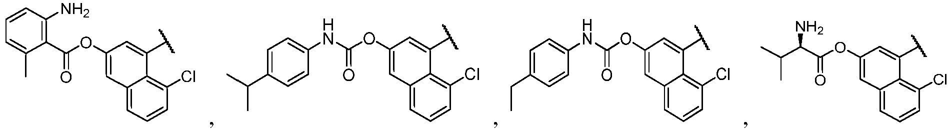 or 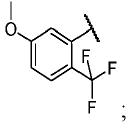
and/or, 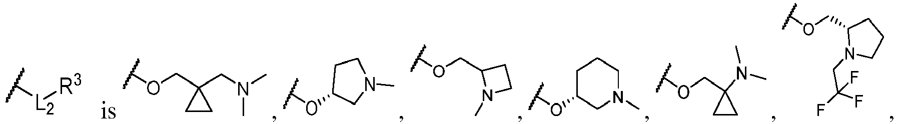 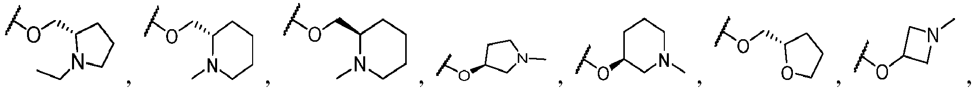 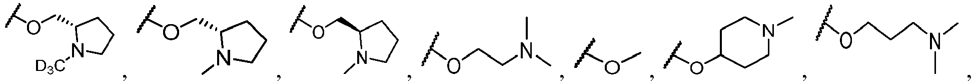 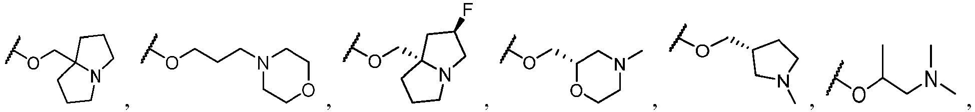 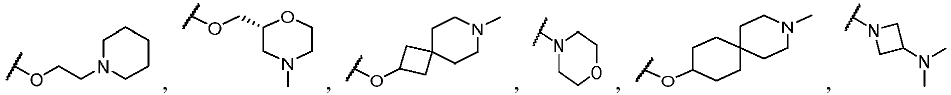 or

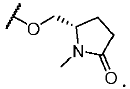

7. The nitrogen-containing heterocyclic compound of formula I, the pharmaceutically acceptable salt thereof, the stereoisomer thereof, the tautomer thereof or the isotopically labeled compound thereof according to claim 6, wherein 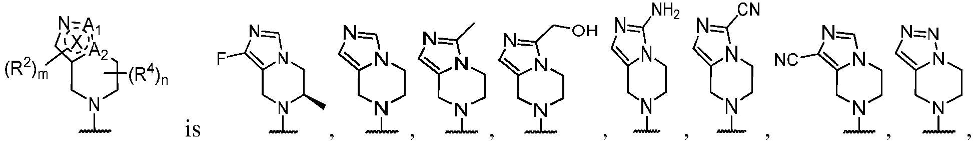 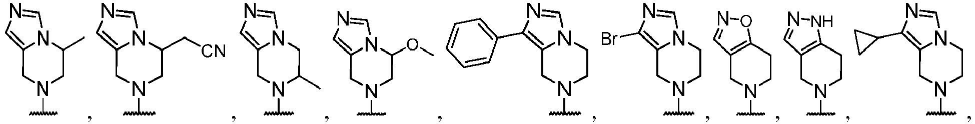 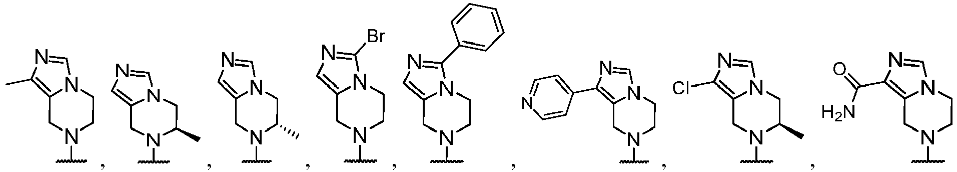 or 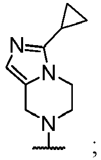
and/or, 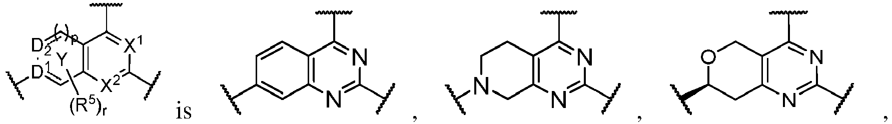 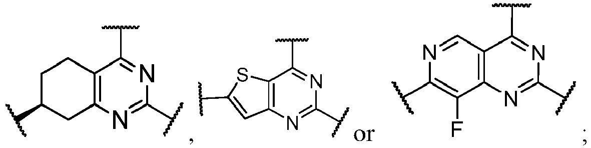
and/or, R¹, R¹⁻¹ and R¹⁻² are independently hydroxyl, F, Cl, CF₃, methyl, methyl-O- , cyclopropyl, isopropyl, NH₂-, 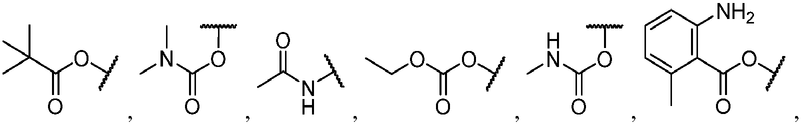 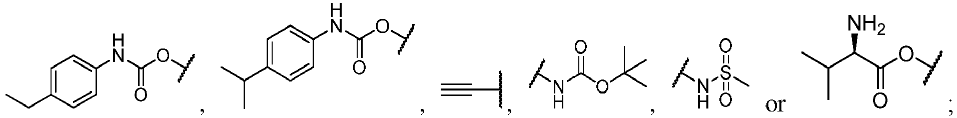
and/or, R³⁻¹, R³⁻² and R³⁻³ are independently F, O=, 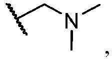 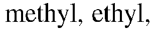 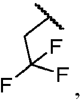 -CD₃ or - N(CH₃)₂.

8. The nitrogen-containing heterocyclic compound of formula I, the pharmaceutically acceptable salt thereof, the stereoisomer thereof, the tautomer thereof or the isotopically labeled compound thereof according to claim 1, wherein the nitrogen-containing heterocyclic compound of formula I, having the structure shown below: 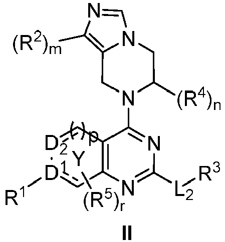
wherein, "-̅ -̅ -̅" represents a single or double bond;
m is 0 or 1;
R² is -CN, C₁₋₆ alkyl, halogen, C₃₋₁₀ cycloalkyl or C₆₋₂₀ aryl;
n is 0 or 1;
R⁴ is independently C₁₋₆ alkyl, or C₁₋₆ alkyl substituted with one or more R⁴⁻¹;
R⁴⁻¹ is independently halogen;
p is 0 or 1; 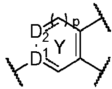 is phenyl, "6-membered heterocycloalkenyl containing 1 heteroatom selected from O and N", cyclohexenyl, pyridyl or thiophenyl; wherein D¹ is C, CH or N; D₂ is 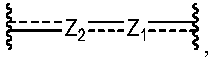 wherein Z₁ and Z₂ are independently a bond, CH, CH₂, O, S or N;
r is 0 or 1;
R⁵ is independently halogen or C₁₋₂ alkyl;
R¹ is C₆₋₂₀ aryl, "5- to 12-membered heteroaryl containing 1-4 heteroatoms independently selected from O, S and N", C₆₋₂₀ aryl substituted with one or more R¹⁻¹ or "5- to 12-membered heteroaryl containing 1-4 heteroatoms independently selected from O, S and N" substituted with one or more R¹⁻²; provided that when multiple substituents are present, the substituents are the same or different;
R¹⁻¹ and R¹⁻² are independently halogen, -OR^{c}, -NR¹²R¹³, C₁₋₆ alkyl, C₁₋₆ alkyl-O substituted with one or more R¹⁻¹⁻²; provided that when multiple substituents are present, the substituents are the same or different; R¹⁻¹⁻² is C₁₋₆ alkyl-O-;
R^{c} is hydrogen, C₁₋₆ alkyl, -C(=O)R^{c1}, -C(=O)OR^{c2}, or -C(=O)NR^{c3}R^{c4}; R^{c1} is C₁₋₆ alkyl, C₁₋₆ alkyl substituted with -NH₂, C₆₋₂₀ aryl, or, C₆₋₂₀ aryl substituted with one or more R⁴⁻¹⁻⁴; R^{c3} and R^{c4} are independently C₆₋₂₀ aryl, or, C₆₋₂₀ aryl substituted with one or more R⁴⁻¹⁻⁴.
R⁴⁻¹⁻⁴ is halogen, C₁₋₆ alkyl or -NH₂;
R^{c2} is C₁₋₆ alkyl;
R¹² and R¹³ are independently hydrogen, C₁₋₆ alkyl, -C(=O)R^{c1}, or, -SO₂R^{c5}; R^{c1} and R^{c5} are independently C₁₋₆ alkyl;
L₂ is -O(R^{L-1})ₙ₁-; R^{L-1} is C₁₋₆ alkylene; n1 is 0 or 1;
R³ is "4- to 12-membered heterocycloalkyl containing 1-3 heteroatoms independently selected from O, S and N", "4- to 12-membered heterocycloalkyl containing 1-3 heteroatoms independently selected from O, S and N" substituted with one or more R³⁻², or -NR^{e1}R^{e2}; provided that when multiple substituents are present, the substituents are the same or different;
R³⁻² is independently C₁₋₆ alkyl, C₁₋₆ alkyl substituted with one or more R³⁻¹⁻¹ or halogen;
R³⁻¹⁻¹ is independently deuterium or halogen;
R^{e1} and R^{e2} are independently hydrogen or C₁₋₆ alkyl.

9. The nitrogen-containing heterocyclic compound of formula I, the pharmaceutically acceptable salt thereof, the stereoisomer thereof, the tautomer thereof or the isotopically labeled compound thereof according to claim 8, wherein the nitrogen-containing heterocyclic compound of formula I, having the structure shown below: 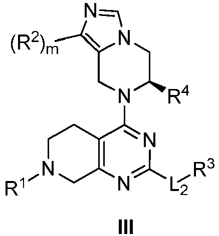
m is 0 or 1;
R² is -CN, C₁₋₃ alkyl or halogen;
R⁴ is independently C₁₋₆ alkyl;
R¹ is naphthyl, quinolinyl, naphthyl substituted with one or more R¹⁻¹ or, quinolinyl substituted with one or more R¹⁻²; provided that when multiple substituents are present, the substituents are the same or different;
R¹⁻¹ and R¹⁻² are independently halogen, -OR^{c}, -NR¹²R¹³, C₁₋₆ alkyl, or C₁₋₆ alkyl-O substituted with one or more R¹⁻¹⁻²; provided that when multiple substituents are present, the substituents are the same or different;
R¹⁻¹⁻² is C₁₋₆ alkyl-O-;
R^{c} is hydrogen, C₁₋₆ alkyl, -C(=O)R^{c1}, -C(=O)OR^{c2}, or -C(=O)NR^{c3}R^{c4}; R^{c1} is C₁₋₆ alkyl, C₁₋₆ alkyl substituted with -NH₂, C₆₋₂₀ aryl, or, C₆₋₂₀ aryl substituted with one or more R⁴⁻¹⁻⁴; R^{c3} and R^{c4} are independently C₆₋₂₀ aryl, or, C₆₋₂₀ aryl substituted with one or more R⁴⁻¹⁻⁴; R⁴⁻¹⁻⁴ is independently halogen, C₁₋₆ alkyl or -NH₂; R^{c2} is C₁₋₆ alkyl;
R¹² and R¹³ are independently hydrogen, C₁₋₆ alkyl, -C(=O)R^{c1} or -SO₂R^{c5}; R^{c1} and R^{c5} are independently C₁₋₆ alkyl;
L₂ is -O(R^{L-1})ₙ₁-; R^{L-1} is C₁₋₆ alkylene; n1 is 1;
R³ is "5- to 6-membered heterocycloalkyl containing 1 heteroatom of N" substituted with one or
more R³⁻², or -NR^{e1}R^{e2};
R³⁻² is independently C₁₋₂ alkyl, or C₁₋₆ alkyl substituted with one or more R³⁻¹⁻¹;
R³⁻¹⁻¹ is independently deuterium;
R^{e1} and R^{e2} are independently hydrogen or C₁₋₆ alkyl.

10. The nitrogen-containing heterocyclic compound of formula I, the pharmaceutically acceptable salt thereof, the stereoisomer thereof, the tautomer thereof or the isotopically labeled compound thereof according to claim 8, wherein the nitrogen-containing heterocyclic compound of formula I, having the structure shown below: 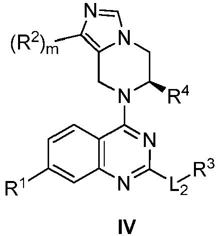
m is 0 or 1;
R² is -CN, C₁₋₃ alkyl or halogen;
R⁴ is C₁₋₆ alkyl;
R¹ is naphthyl, quinolinyl, naphthyl substituted with one or more R¹⁻¹ or, quinolinyl substituted with one or more R¹⁻²; provided that when multiple substituents are present, the substituents are the same or different;
R¹⁻¹ and R¹⁻² are independently halogen, -OR^{c}, -NR¹²R¹³, C₁₋₆ alkyl, or, C₁₋₆ alkyl-O substituted with one or more R¹⁻¹⁻²; provided that when multiple substituents are present, the substituents are the same or different;
R¹⁻¹⁻² is C₁₋₆ alkyl-O-;
R^{c} is hydrogen, C₁₋₆ alkyl, -C(=O)R^{c1}, -C(=O)OR^{c2}, or -C(=O)NR^{c3}R^{c4}; R^{c1} is C₁₋₆ alkyl, C₁₋₆ alkyl substituted with -NH₂, C₆₋₂₀ aryl, or, C₆₋₂₀ aryl substituted with one or more R⁴⁻¹⁻⁴; R^{c3} and R^{c4} are independently C₆₋₂₀ aryl, or, C₆₋₂₀ aryl substituted with one or more R⁴⁻¹⁻⁴; R⁴⁻¹⁻⁴ is halogen, C₁₋₆ alkyl or -NH₂; R^{c2} is C₁₋₆ alkyl;
R¹² and R¹³ are independently hydrogen, C₁₋₆ alkyl, -C(=O)R^{c1}, or, -SO₂R^{c5}; R^{c1} and R^{c5} are independently C₁₋₆ alkyl;
L₂ is -O(R^{L-1})ₙ₁-; R^{L-1} is C₁₋₆ alkylene; n1 is 1;
R³ is "5- to 6-membered heterocycloalkyl containing 1heteroatom of N" substituted with R³⁻², or -NR^{e1}R^{e2};
R³⁻² is C₁₋₂ alkyl;
R^{e1} and R^{e2} are independently hydrogen or C₁₋₆ alkyl.

11. The nitrogen-containing heterocyclic compound of formula I, the pharmaceutically acceptable salt thereof, the stereoisomer thereof, the tautomer thereof or the isotopically labeled compound thereof according to claim 1, wherein the nitrogen-containing heterocyclic compound of formula I has any one of the following structures: 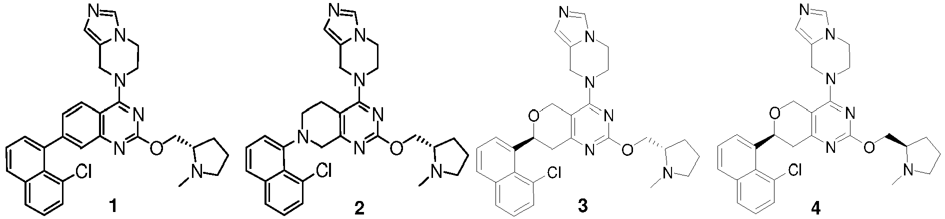 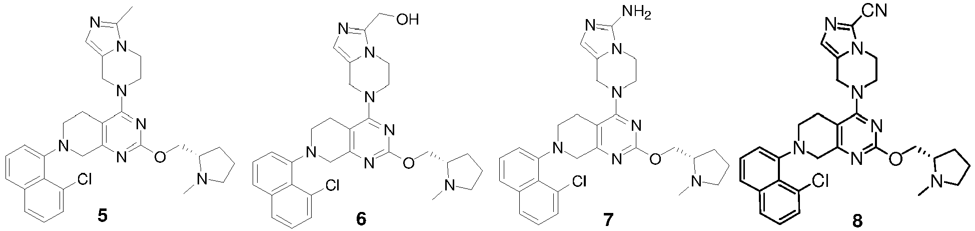 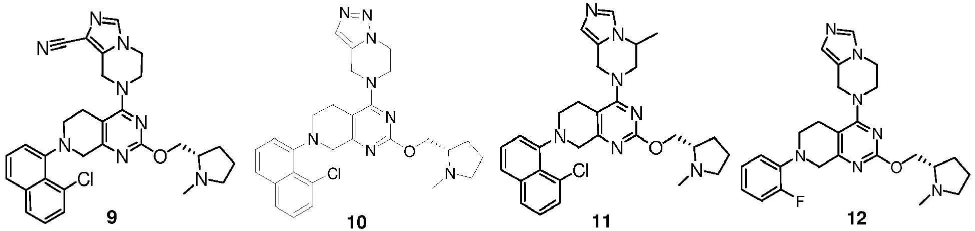 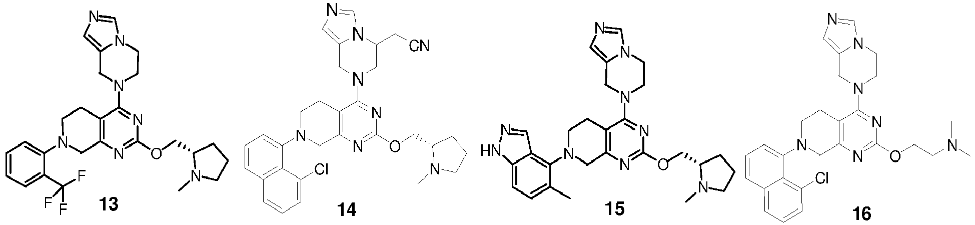 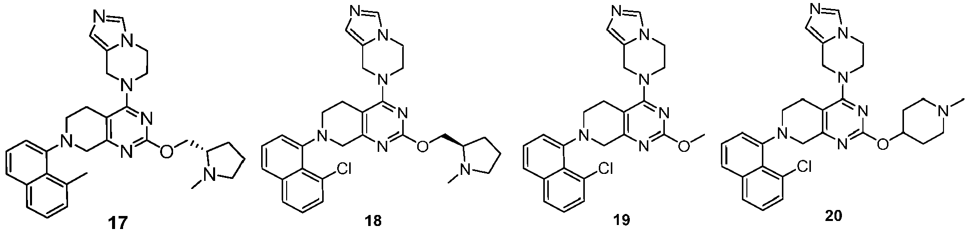 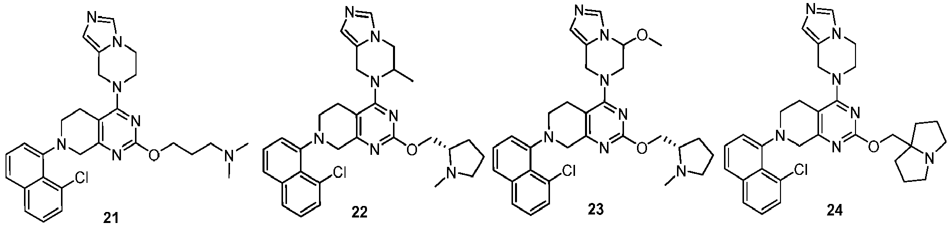 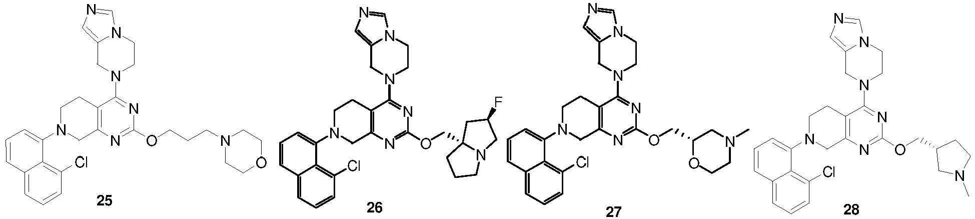 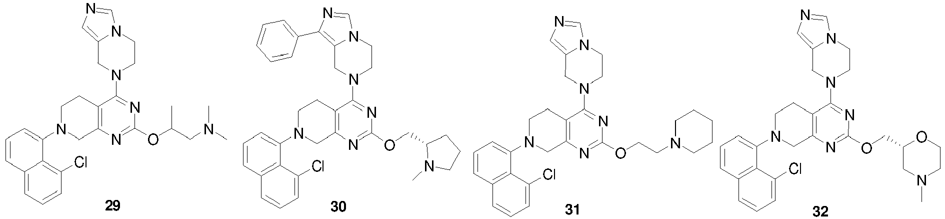 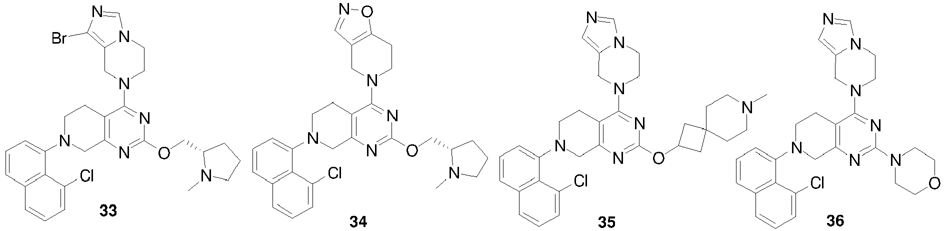 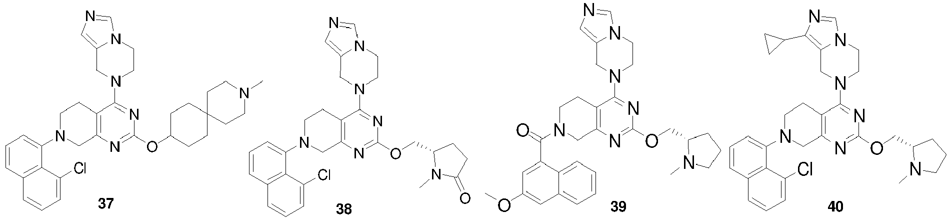 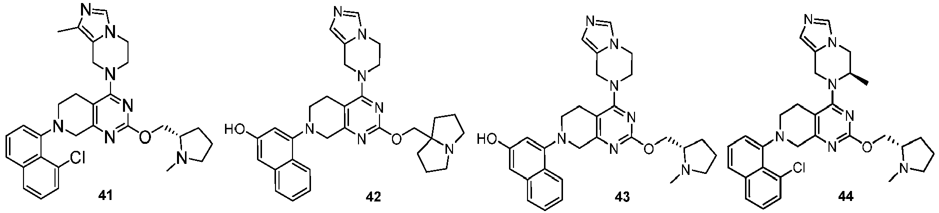 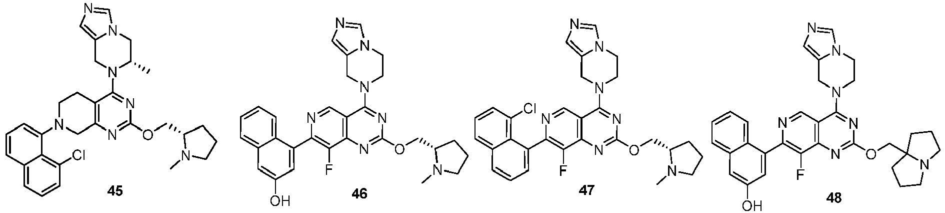 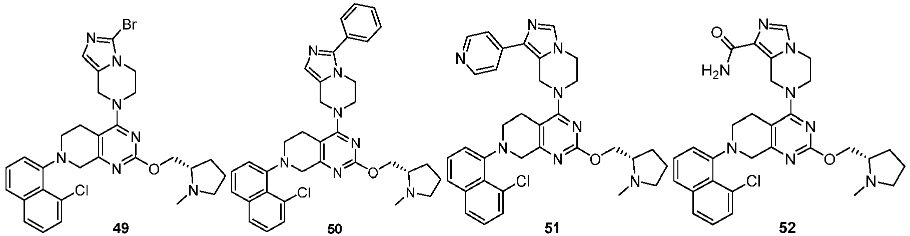 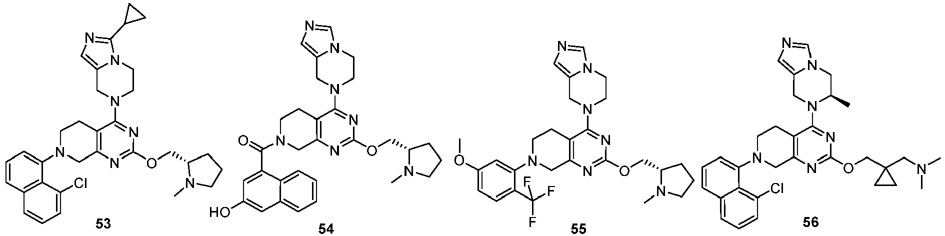 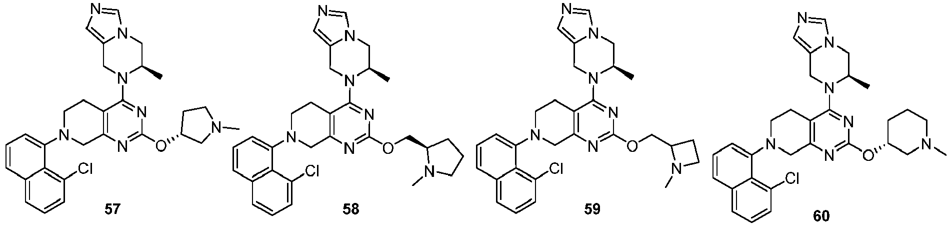 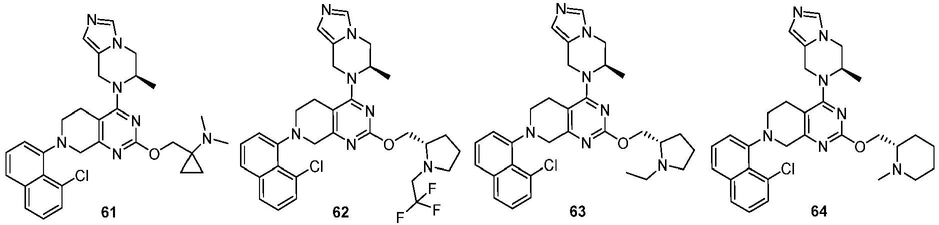 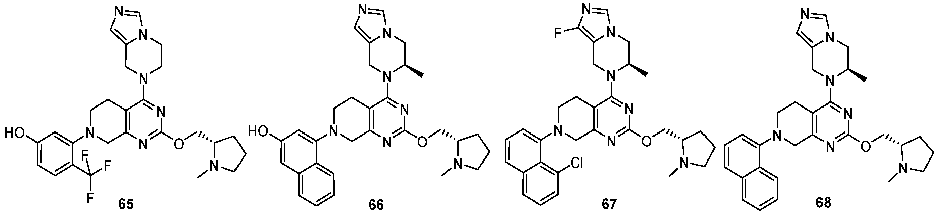 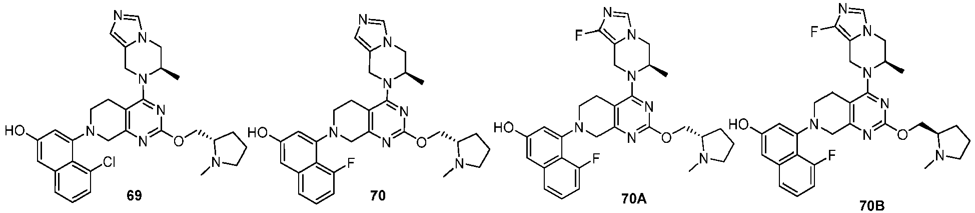 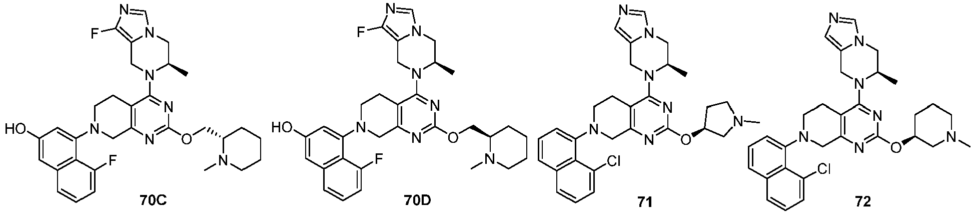 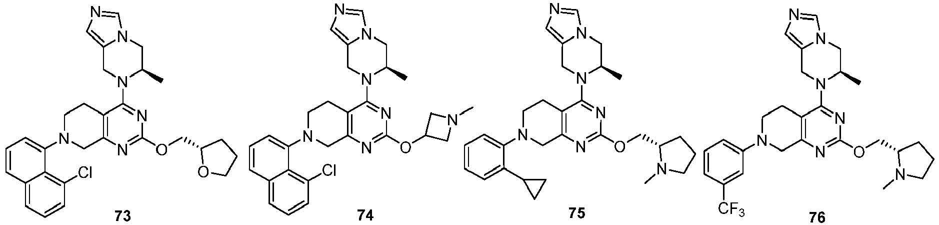 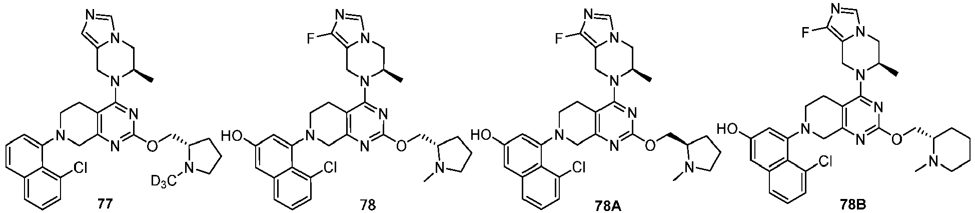 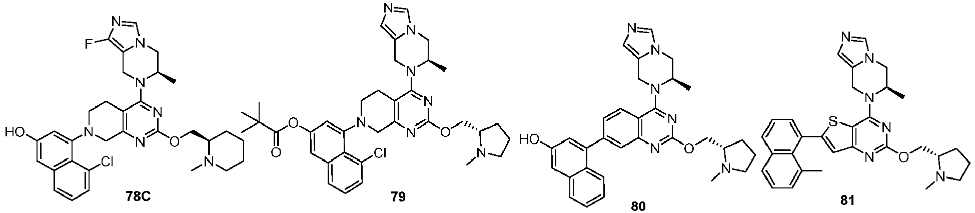 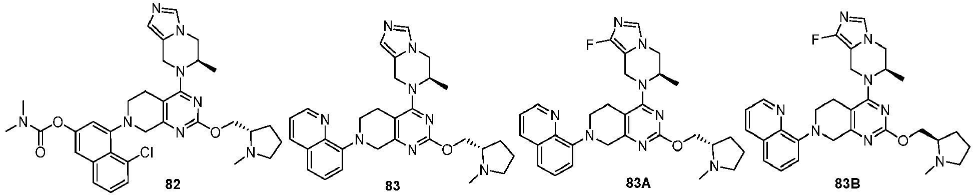 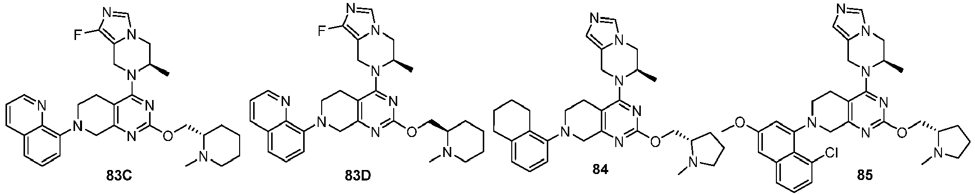 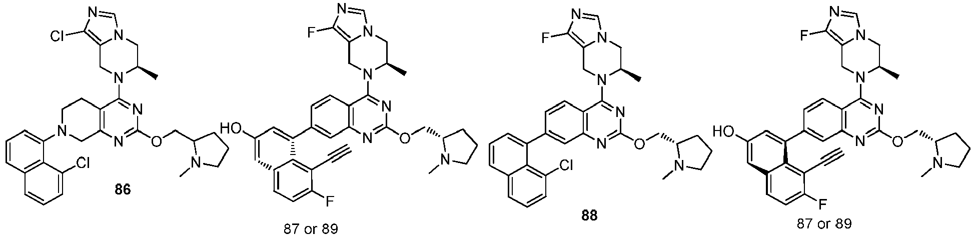 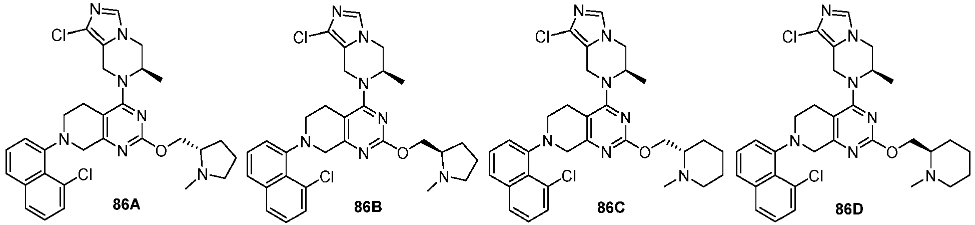 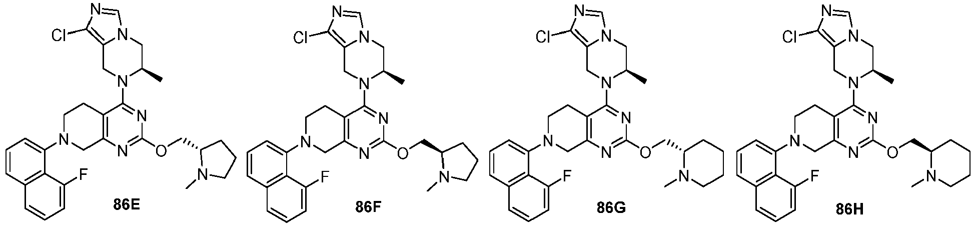 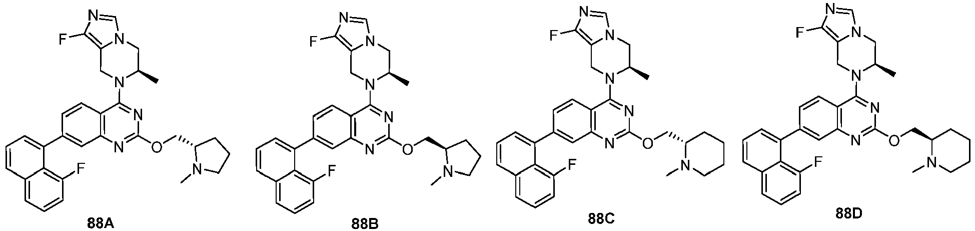 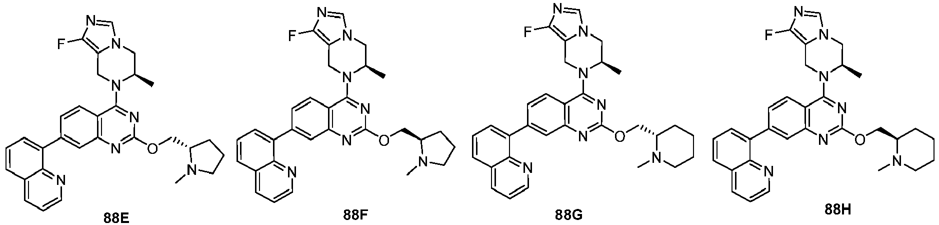 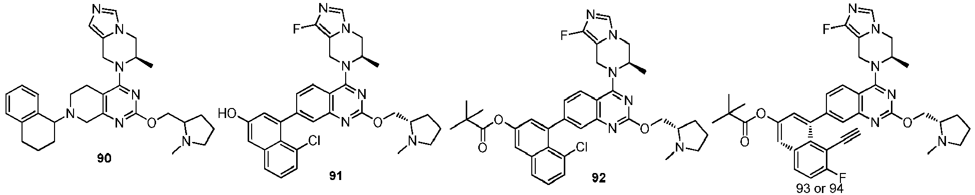 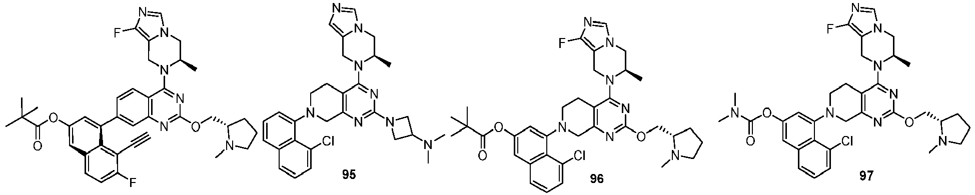 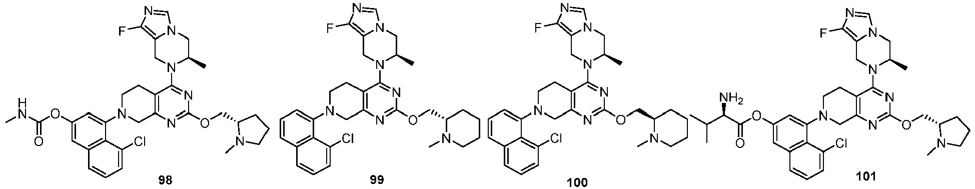 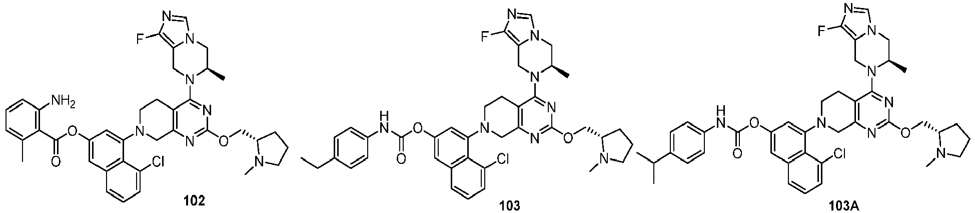 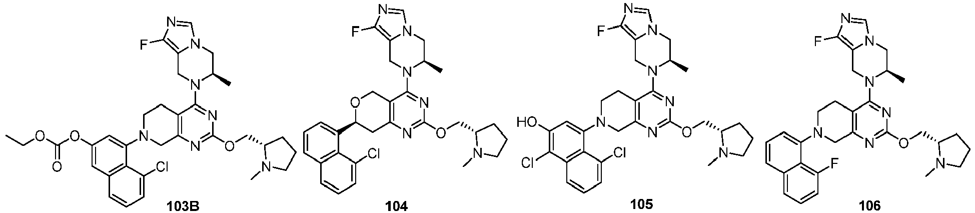 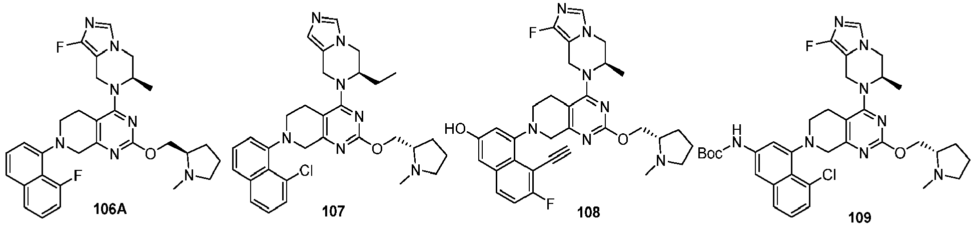 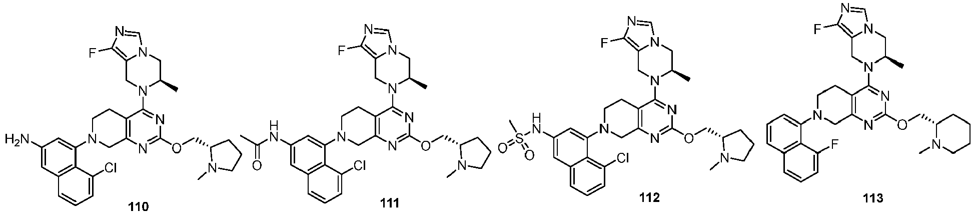 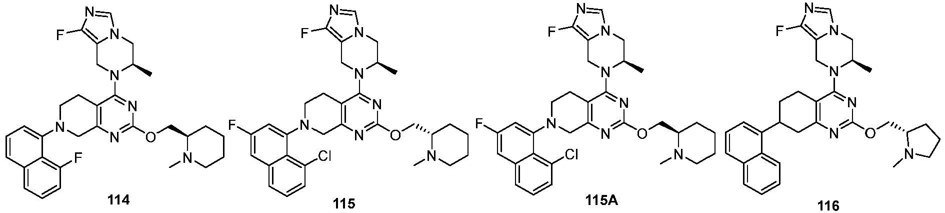 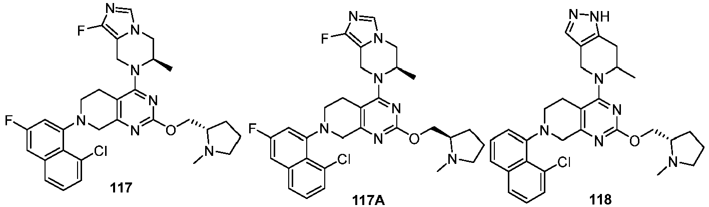 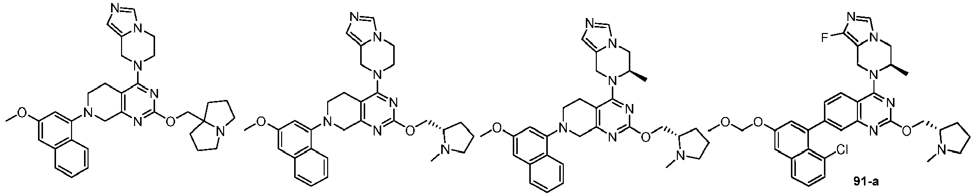 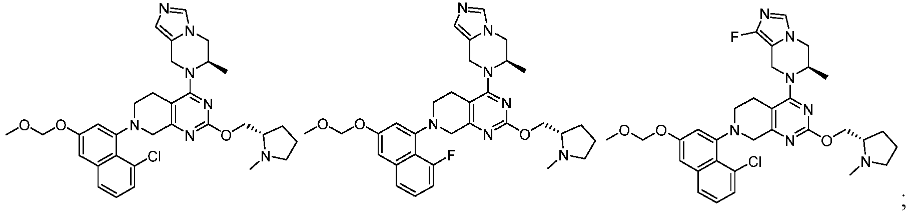 the pharmaceutically acceptable salt of the nitrogen-containing heterocyclic compound of formula I is trifluoroacetate having any one of the following structures: 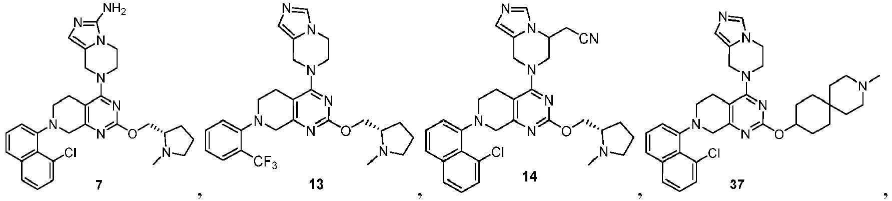 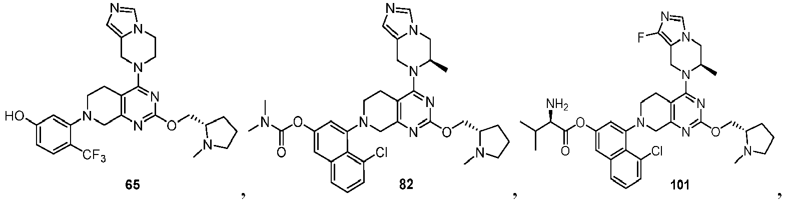 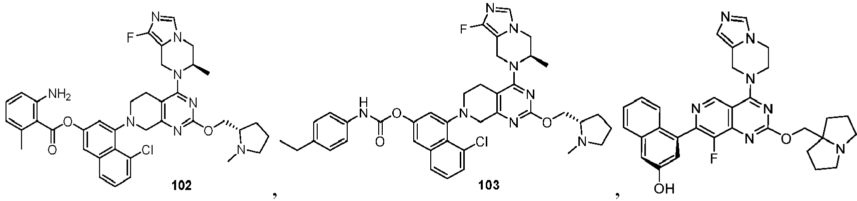 or 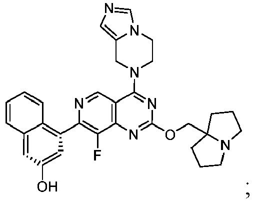 or, the pharmaceutically acceptable salt of the nitrogen-containing heterocyclic compound of formula I is formate having the following structure: 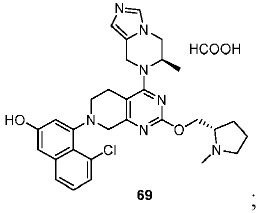 the stereoisomer of the nitrogen-containing heterocyclic compound of formula I has any one of the following structures:

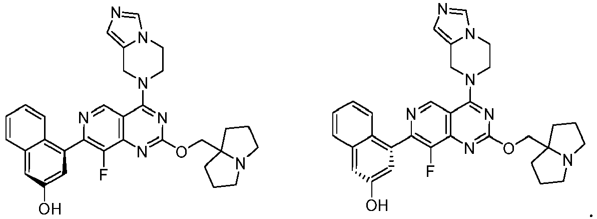

12. A method for preparing the nitrogen-containing heterocyclic compound of formula I according to any one of claims 1-11, wherein the method has any one of the following routes: 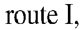 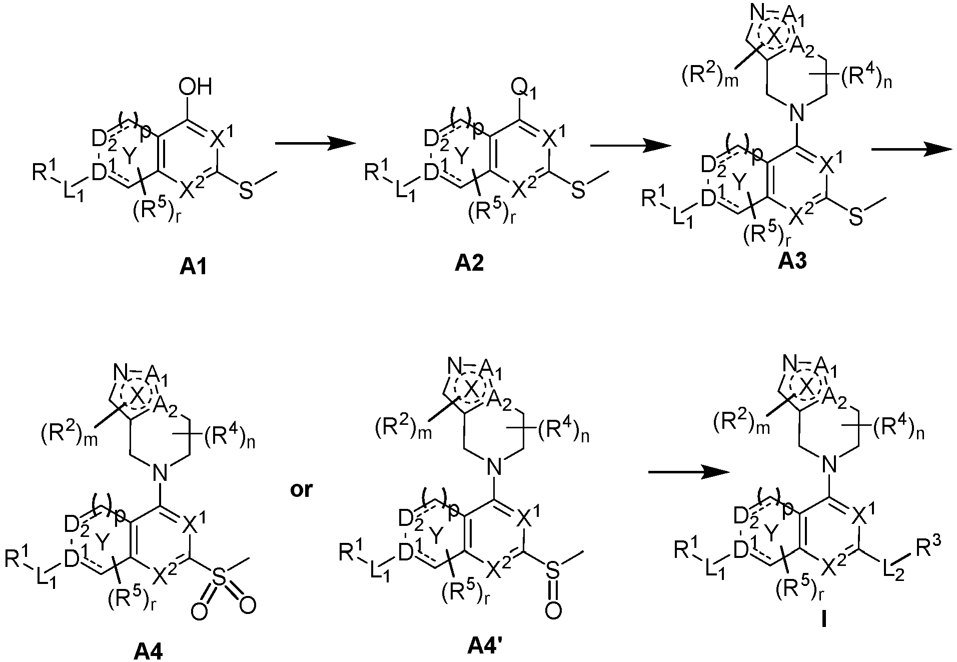 wherein R¹, R², R³, R⁴, R⁵, A₁, A₂, D¹, D₂, L₁, L₂, X¹, X², m, n, p and r are as defined in claim 1, and Q₁ is a leaving group; the route I comprises the following steps: converting hydroxyl of compound A1 into a leaving group to obtain A2, converting A2 into A3 by nucleophilic substitution reaction, oxidizing A3 into A4 or A4', and converting A4 or A4' into compound I by nucleophilic substitution reaction; 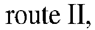 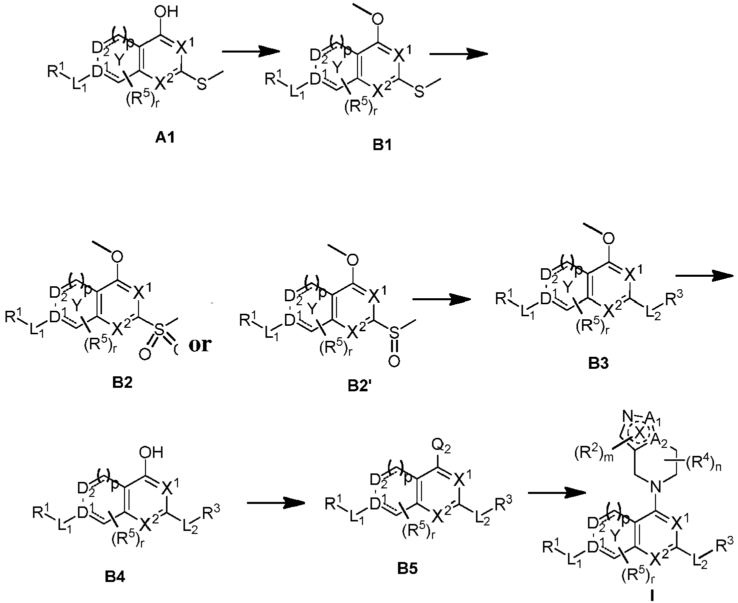 wherein R¹, R², R³, R⁴, R⁵, A₁, A₂, D¹, D₂, L₁, L₂, X¹, X², m, n, p and r are as defined in claim 1, and Q₂ is a leaving group; the route II comprises the following steps: protecting compound A1 with Me group to obtain B1, oxidizing B1 to obtain B2 or B2', converting B2 or B2' into B3 by nucleophilic substitution reaction, converting B3 into B4 by demethylation protection reaction, converting hydroxyl in B4 into a leaving group to obtain B5, and converting B5 into compound I by nucleophilic substitution reaction; 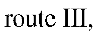 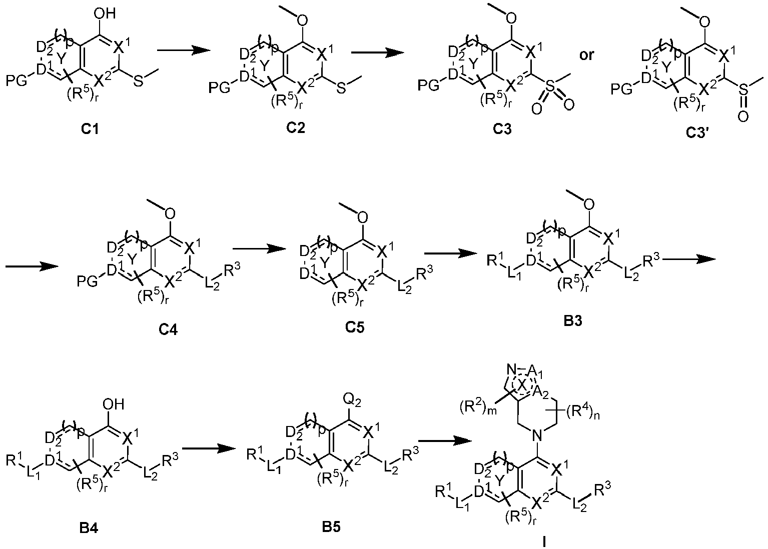 wherein R¹, R², R³, R⁴, R⁵, A₁, A₂, D¹, D₂, L₁, L₂, X¹, X², m, n, p and r are as defined in claim 1, Q₂ is independently a leaving group, and PG is H or an amino protecting group; the route III comprises the following steps: protecting compound C1 with Me group to obtain C2, oxidizing C2 to obtain C3 or C3', converting C3 or C3' into C4 by nucleophilic substitution reaction, removing the protecting group from C4 to obtain C5, converting C5 into B3 by nucleophilic substitution or coupling reaction, converting B3 into B4 by demethylation protection, converting hydroxyl in B4 into a leaving group to obtain B5, and converting B5 into compound I by nucleophilic substitution reaction; 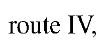 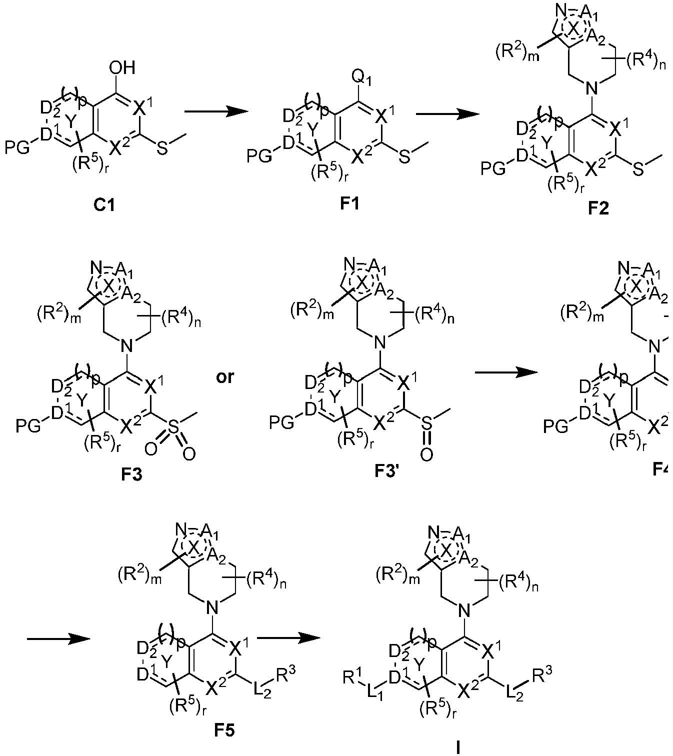 wherein R¹, R², R³, R⁴, R⁵, A₁, A₂, D¹, D₂, L₁, L₂, X¹, X², m, n, p and r are as defined in claim 1, Q₁ is independently a leaving group, and PG is H or an amino protecting group; the route IV comprises the following steps: converting hydroxyl in compound C1 into a leaving group to obtain F1, converting F1 into F2 by nucleophilic substitution reaction, oxidizing F2 to obtain F3 or F3', converting F3 or F3' into F4 by nucleophilic substitution reaction, removing the protecting group from F4 to obtain F5, and converting F5 into compound I by nucleophilic substitution or coupling reaction; 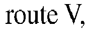 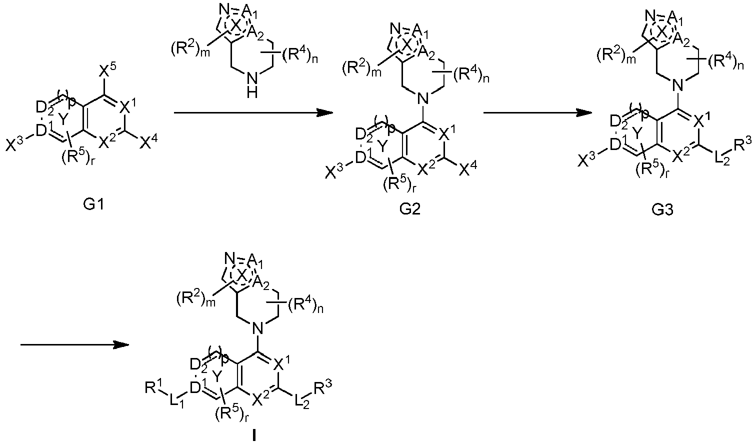 wherein R¹, R², R³, R⁴, R⁵, A₁, A₂, D¹, D₂, L₁, L₂, X¹, X², m, n, p and r are as defined in claim 1, and X³, X⁴ and X⁵ are independently a leaving group; the route V comprises the following steps: converting compound G1 into G2 by nucleophilic substitution reaction, converting G2 into G3 by nucleophilic substitution reaction, and converting G3 into compound I by nucleophilic substitution or coupling reaction; 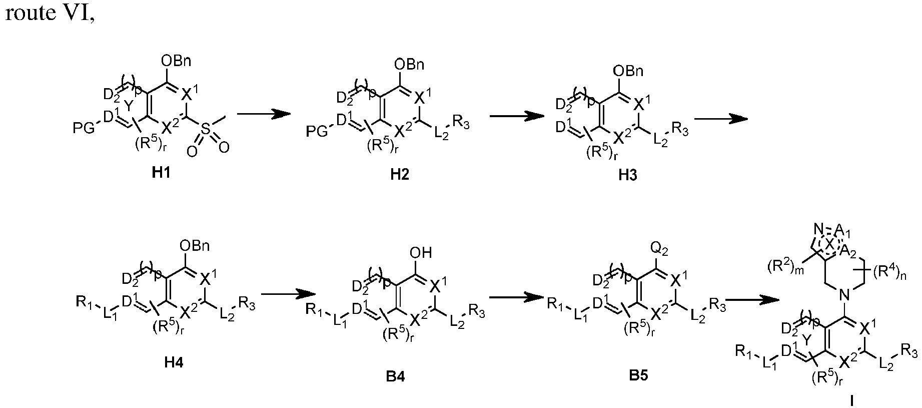 wherein R¹, R², R³, R⁴, R⁵, A₁, A₂, D¹, D₂, L₁, L₂, X¹, X², m, n, p and r are as defined in claim 1, and Q₂ is a leaving group; the route VI comprises the following steps: converting compound H1 into H2 by nucleophilic substitution reaction, converting H2 into H3 by removing protective group, converting H3 into compound H4 by nucleophilic substitution or coupling reaction, converting H4 into B4 by removing benzyl group, converting hydroxyl in B4 into a leaving group to obtain B5, and converting B5 into compound I by nucleophilic substitution reaction;

13. A compound of formula T1, T2, T3, T4, T5, T6, T7, T8, T2' or T6': 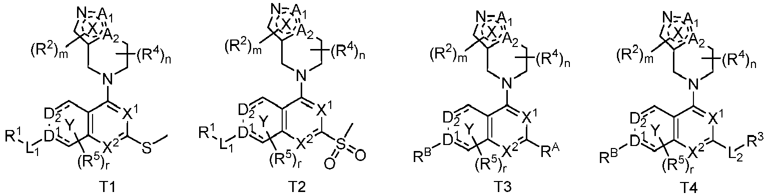 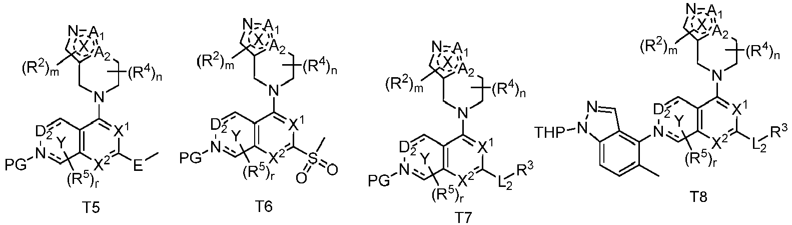 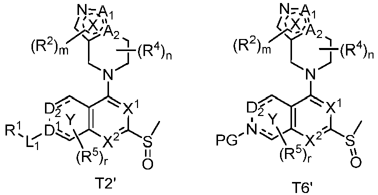 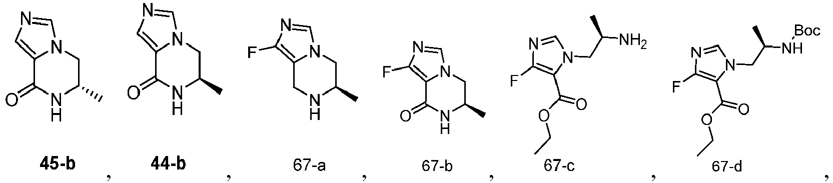 Wherein, A₁, A₂, R², R⁴, X¹, X², L₂, R³, D¹, D₂, L₁, R¹, R⁵, m, n, p and r are defined as claim 1-11; R^{A} and R^{B} are independently a leaving group, such as Cl, Br or OTf; PG is H or an amino protecting group, the amino protecting group such as THP, Boc or Cbz; E is O or S; for example, the compound of T1, T2, T3, T4, T5, T6, T7, T8, T2', T6', has any one of the following structures:

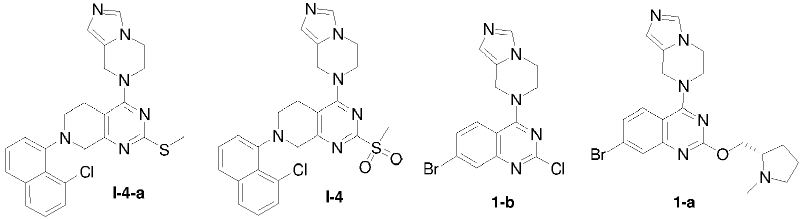

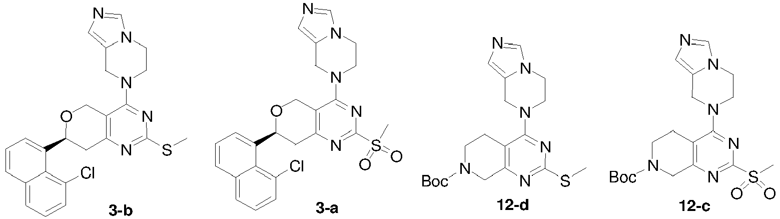

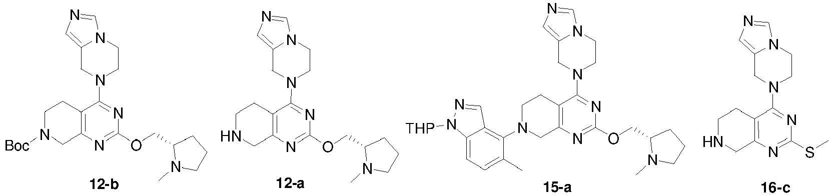

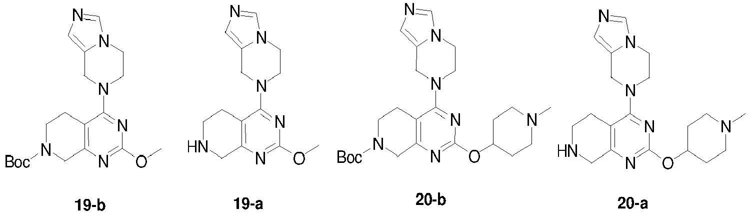

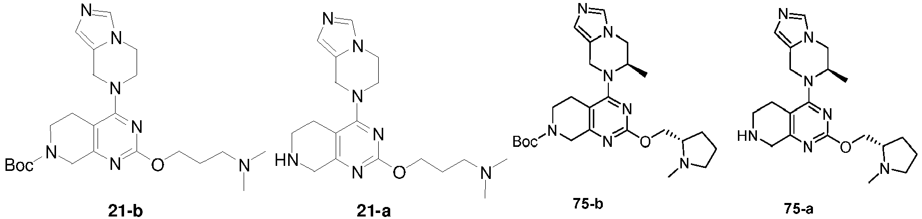

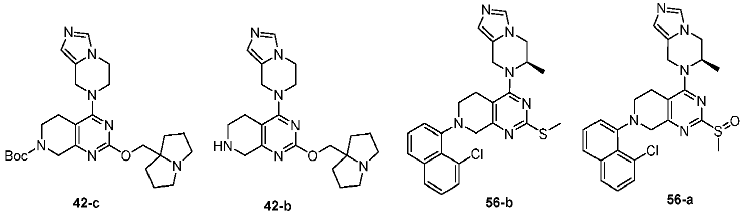

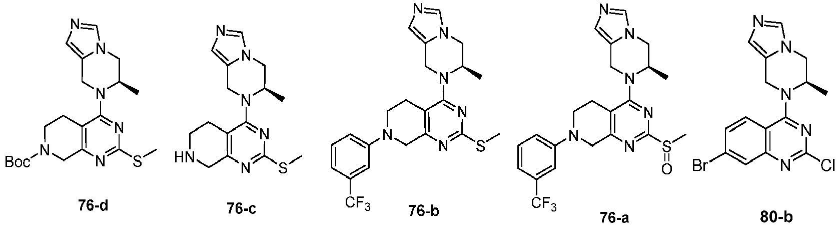

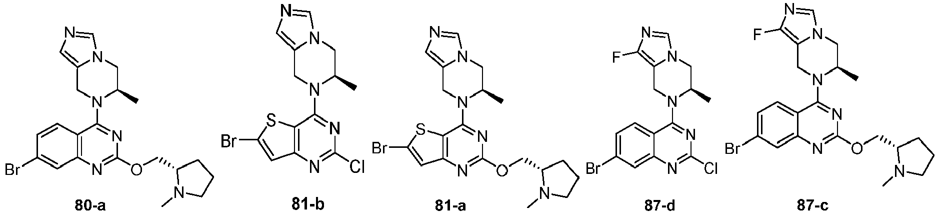

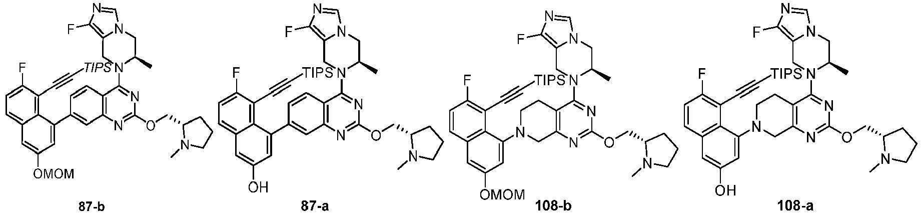

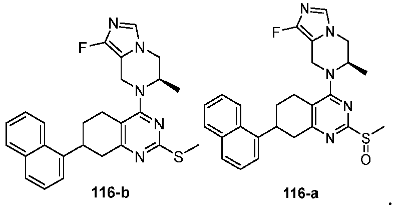

14. A pharmaceutical composition comprising a substance A and a pharmaceutically acceptable excipient, wherein the substance A is a therapeutically effective amount of the nitrogen-containing heterocyclic compound of formula I, the pharmaceutically acceptable salt thereof, the stereoisomer thereof, the tautomer thereof or the isotopically labeled compound thereof according to any one of claims 1-11.

15. A use of substance A in manufacturing an RAS inhibitor, wherein the substance A is a therapeutically effective amount of the nitrogen-containing heterocyclic compound of formula I, the pharmaceutically acceptable salt thereof, the stereoisomer thereof, the tautomer thereof or the isotopically labeled compound thereof according to any one of claims 1-11; The RAS is wild type RAS or mutated forms of RAS; The mutated forms of RAS such as a KRAS, HRAS or NRAS mutation; wherein, the KRAS mutation can be a G12, G13 or Q61 mutation, for example, KRAS G12C, KRAS G12D, KRAS G12S, KRAS G12A, KRAS G12V or KRAS G13D, and for another example, KRAS G12C, KRAS G12D or KRAS G12V; the HRAS mutation can be a G12, G13 or Q61 mutation, for example, HRAS G12C, HRAS G12D, HRAS G12S, HRAS G12A, HRAS G12V or HRAS G13D; the NRAS mutation can be a G12, G13 or Q61 mutation, for example, NRAS G12C, NRAS G12D, NRAS G12S, NRAS G12A, NRAS G12V or NRAS G13D; the mutated forms of RAS is, for another example, KRAS G12C.

16. A use of substance A in manufacturing a medicament, wherein the medicament is used for treating and/or preventing an RAS-related disease; The substance A is a therapeutically effective amount of the nitrogen-containing heterocyclic compound of formula I, the pharmaceutically acceptable salt thereof, the stereoisomer thereof, the tautomer thereof or the isotopically labeled compound thereof according to any one of claims 1-11;
The RAS is wild type RAS or mutated forms of RAS; The mutated forms of RAS such as a KRAS, HRAS or NRAS mutation; wherein, the KRAS mutation for example, KRAS G12C, KRAS G12D, KRAS G12S, KRAS G12A, KRAS G12V or KRAS G13D, and for another example, KRAS G12C, KRAS G12D or KRAS G12V; the HRAS mutation can be a G12, G13 or Q61 mutation, for example, HRAS G12C, HRAS G12D, HRAS G12S, HRAS G12A, HRAS G12V or HRAS G13D; the NRAS mutation can be a G12, G13 or Q61 mutation, for example, NRAS G12C, NRAS G12D, NRAS G12S, NRAS G12A, NRAS G12V or NRAS G13D; the mutated forms of RAS is, for another example, KRAS G12C;
and/or, the RAS-related disease for example cancer, the cancer is selected from the group consisting of colon cancer, appendiceal cancer, pancreatic cancer, MYH-related polyposis, hematologic cancer, breast cancer, endometrial cancer, gallbladder cancer, bile duct cancer, prostate cancer, lung cancer, brain cancer, ovarian cancer, cervical cancer, testicular cancer, kidney cancer, head or neck cancer, bone cancer, skin cancer, rectal cancer, liver cancer, esophageal cancer, stomach cancer, thyroid cancer, bladder cancer, lymphoma, leukemia and melanoma

17. A use of substance A in manufacturing a medicament, wherein the medicament is used for treating and/or preventing cancer; The substance A is the nitrogen-containing heterocyclic compound of formula I, the pharmaceutically acceptable salt thereof, the stereoisomer thereof, the tautomer thereof or the isotopically labeled compound thereof according to any one of claims 1-11; Preferably, the cancer is defined as cancer in claim 16.

18. The nitrogen-containing heterocyclic compound of formula I, the pharmaceutically acceptable salt thereof, the stereoisomer thereof, the tautomer thereof or the isotopically labeled compound thereof according to claim 1, wherein, nitrogen-containing heterocyclic compound of formula I is defined as solution 1, solution 2 or solution 3:
solution 1:
A nitrogen-containing heterocyclic compound of formula I, a pharmaceutically acceptable salt thereof, a stereoisomer thereof, a tautomer thereof or an isotopically labeled compound thereof: 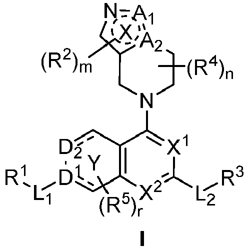
wherein "=-=" represents a single or double bond; 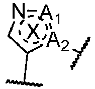 is nitrogen-containing 5-membered heteroaryl; A₁ is CH, O or N; A₂ is C or N;
m is 0, 1 or 2;
R² is -CN, C₁₋₆ alkyl, C₂₋₆ alkenyl, C₂₋₆ alkynyl, C₁₋₆ alkyl substituted with one or more R²⁻¹, halogen, -OR^{2a}, -C(=O)R^{2b}, -NR^{2c1}R^{2c2}, -C(=O)OR^{2d}, -C(=O)NR^{2e1}R^{2e2}, C₃₋₁₀ cycloalkyl, C₃₋₁₀ cycloalkyl substituted with one or more R²⁻², "4- to 10-membered heterocycloalkyl containing 1-3 heteroatoms independently selected from O and N", "4- to 10-membered heterocycloalkyl containing 1-3 heteroatoms independently selected from O and N" substituted with one or more R²⁻³, C₆₋₂₀ aryl, C₆₋₂₀ aryl substituted with one or more R²⁻⁴, "5- to 12-membered heteroaryl containing 1-4 heteroatoms independently selected from O, S and N", or "5- to 12-membered heteroaryl containing 1-4 heteroatoms independently selected from O, S and N" substituted with one or more R²⁻⁵; provided that when multiple substituents are present, the substituents are the same or different;
R²⁻¹, R²⁻², R²⁻³, R²⁻⁴ and R²⁻⁵ are independently halogen, hydroxyl, cyano, C₁₋₆ alkyl, C₂₋₆ alkenyl, C₂₋₆ alkynyl, C₁₋₆ alkyl-O-, -C(=O)R³¹, -NR³²R³³, -C(=O)OR³⁴ or -C(=O)NR³⁵R³⁶;
R^{2a}, R^{2b}, R^{2c1}, R^{2c2}, R^{2d}, R^{2e1} and R^{2e2} are independently hydrogen or C₁₋₆ alkyl;
R³¹, R³², R³³, R³⁴, R³⁵ and R³⁶ are independently hydrogen or C₁₋₆ alkyl;
n is 0, 1, 2, 3, 4, 5 or 6;
R⁴ is independently C₁₋₆ alkyl, C₁₋₆ alkyl substituted with one or more R⁴⁻¹, C₁₋₆ alkyl-O-, O=, - C(=O)OR^{4a} or -C(=O)NR^{4b}R^{4c}; or, when n is 2, 3, 4, 5 or 6, two optional R⁴ are connected, together with the atoms on the ring to which they are attached, independently form 3- to 8-membered carbocyclic ring or 3- to 8-membered heterocyclic ring containing 1-3 heteroatoms independently selected from O, S and N;
R⁴⁻¹ is independently halogen, cyano, hydroxyl, C₁₋₆ alkyl-O-, -NR⁴ⁱR^{4j}, -C(=O)OR^{4d} or - C(=O)NR^{4e}R^{4f};
R^{4a}, R^{4b}, R^{4C}, R^{4d}, R^{4e}, R^{4f}, R⁴ⁱ and R^{4j} are independently hydrogen or C₁₋₆ alkyl; 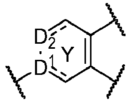 is phenyl, "5- to 7-membered heterocycloalkenyl containing 1-3 heteroatoms selected from O, S and N", "5- to 7-membered heteroaryl containing 1-3 heteroatoms independently selected from O, S and N" or 5- to 7-membered cycloalkenyl; wherein D¹ is C, CH or N; D₂ is 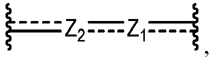 wherein Z₁ and Z₂ are independently a bond, CH, CH₂, O, S, N or NH;
r is 0, 1, 2, 3, 4, 5 or 6;
R⁵ is independently halogen or C₁₋₆ alkyl;
X¹ and X² are independently CR^{b} or N, and X¹ and X² are not both CR^{b};
L₁ is a bond, -C(=O)- or C₁₋₆ alkylene;
R¹ is C₆₋₂₀ aryl, "5- to 12-membered heteroaryl containing 1-4 heteroatoms independently selected from O, S and N", C₆₋₂₀ aryl substituted with one or more R¹⁻¹ or "5- to 12-membered heteroaryl containing 1-4 heteroatoms independently selected from O, S and N and substituted with one or more R¹⁻²"; provided that when multiple substituents are present, the substituents are the same or different;
R^{b}, R¹⁻¹ and R¹⁻² are independently halogen, hydroxyl, cyano, -C(=O)R¹¹, -NR¹²R¹³, -C(=O)OR¹⁴, -C(=O)NR¹⁵R¹⁶, C₁₋₆ alkyl, C₂₋₆ alkenyl, C₂₋₆ alkynyl, C₁₋₆ alkyl-O-, C₃₋₁₀ cycloalkyl, "5- to 7-membered heterocycloalkyl containing 1 or 2 heteroatoms independently selected from O and N", C₆₋₂₀ aryl, "5- to 7-membered heteroaryl containing 1 or 2 heteroatoms independently selected from O and N", C₁₋₆ alkyl substituted with one or more R¹⁻¹⁻¹, C₁₋₆ alkyl-O substituted with one or more R¹⁻¹⁻², C₃₋₁₀ cycloalkyl substituted with one or more R¹⁻¹⁻³, "5- to 7-membered heterocycloalkyl containing 1 or 2 heteroatoms independently selected from O and N substituted with one or more R¹⁻¹⁻⁴, C₆₋₂₀ aryl substituted with one or more R¹⁻¹⁻⁵, or "5- to 7-membered heteroaryl containing 1 or 2 heteroatoms independently selected from O and N" substituted with one or more R¹⁻¹⁻⁶; provided that when multiple substituents are present, the substituents are the same or different;
R¹⁻¹⁻¹, R¹⁻¹⁻², R¹⁻¹⁻³, R¹⁻¹⁻⁴, R¹⁻¹⁻⁵ and R¹⁻¹⁻⁶ are independently cyano, halogen, hydroxyl, C₁₋₆ alkyl-O-, C₁₋₆ alkyl, -C(=O)R²¹, -NR²²R²³, -C(=O)OR²⁴ or -C(=O)NR²⁵R²⁶,
R¹¹, R²¹, R¹², R²², R¹³, R²³, R¹⁴, R²⁴, R¹⁵, R²⁵, R¹⁶ and R²⁶ are independently hydrogen or C₁₋₆ alkyl;
L₂ is a bond, C₁₋₆ alkylene, -C(=O)-, -O(R^{L-1})ₙ₁-, -S(R^{L-2})ₙ₂- or -NR^{L-3}(R^{L-4})ₙ₃-; R^{L-1}, R^{L-2} and R^{L-4} are independently C₁₋₆ alkylene; R^{L-3} is hydrogen or C₁₋₆ alkyl; n1, n2 and n3 are independently 0 or 1;
R³ is C₃₋₁₂ cycloalkyl, C₃₋₁₂ cycloalkyl substituted with one or more R³⁻¹, "4- to 12-membered heterocycloalkyl containing 1-3 heteroatoms independently selected from O, S and N", "4- to 12-membered heterocycloalkyl containing 1-3 heteroatoms independently selected from O, S and N" substituted with one or more R³⁻², C₁₋₆ alkyl, C₁₋₆ alkyl substituted with one or more R³⁻³, -OR^{d}, - SR^{d1}, -NR^{e1}R^{e2} or -C(=O)NR^{e3}R^{e4}; provided that when multiple substituents are present, the substituents are the same or different;
R³⁻¹, R³⁻² and R³⁻³ are independently C₁₋₆ alkyl, C₁₋₆ alkyl substituted with one or more R³⁻¹⁻¹, hydroxyl, C₁₋₆ alkyl-O-, halogen, O=, -NR^{e5}R^{e6} or -C(=O)NR^{e7}R^{e8};
R^{d}, R^{d1}, R^{e1}, R^{e2}, R^{e3} and R^{e4} are independently hydrogen, C₁₋₆ alkyl, C₃₋₁₀ cycloalkyl, "4- to 10-membered heterocycloalkyl containing 1-3 heteroatoms independently selected from O and N", or C₁₋₆ alkyl substituted with one or more R³⁻¹⁻²;
R³⁻¹⁻¹ and R³⁻¹⁻² are independently cyano, halogen, hydroxyl, C₁₋₆ alkyl-O-, -C(=O)R^{e9}, -NR^{e10}R^{e11}, -C(=O)OR^{e12} or ^{_}C(=O)NR^{e13}R^{e14};
R^{e5}, R^{e6}, R^{e7}, R^{e8}, R^{e9}, R^{e10}, R^{e11}, R^{e12}, R^{e13} and R^{e14} are independently hydrogen or C₁₋₆ alkyl;
Solution 2:
A nitrogen-containing heterocyclic compound of formula I, a pharmaceutically acceptable salt thereof, a stereoisomer thereof, a tautomer thereof or an isotopically labeled compound thereof: 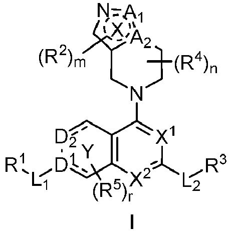
wherein "=-=" represents a single or double bond; 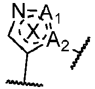 is nitrogen-containing 5-membered heteroaryl; A₁ is CH, O or N; A₂ is C or N;
m is 0, 1 or 2;
R² is -CN, C₁₋₆ alkyl, C₂₋₆ alkenyl, C₂₋₆ alkynyl, C₁₋₆ alkyl substituted with one or more R²⁻¹, halogen, -OR^{2a}, -C(=O)R^{2b}, -NR^{2c1}R^{2c2}, -C(=O)OR^{2d}, -C(=O)NR^{2e1}R^{2e2}, C₃₋₁₀ cycloalkyl, C₃₋₁₀ cycloalkyl substituted with one or more R²⁻², "4- to 10-membered heterocycloalkyl containing 1-3 heteroatoms independently selected from O and N", "4- to 10-membered heterocycloalkyl containing 1-3 heteroatoms independently selected from O and N" substituted with one or more R²⁻³, C₆₋₂₀ aryl, C₆₋₂₀ aryl substituted with one or more R²⁻⁴, "5- to 12-membered heteroaryl containing 1-4 heteroatoms independently selected from O, S and N", or "5- to 12-membered heteroaryl containing 1-4 heteroatoms independently selected from O, S and N" substituted with one or more R²⁻⁵; provided that when multiple substituents are present, the substituents are the same or different;
R²⁻¹, R²⁻², R²⁻³, R²⁻⁴ and R²⁻⁵ are independently halogen, hydroxyl, cyano, C₁₋₆ alkyl, C₂₋₆ alkenyl, C₂₋₆ alkynyl, C₁₋₆ alkyl-O-, -C(=O)R³¹, -NR³²R³³, -C(=O)OR³⁴ or -C(=O)NR³⁵R³⁶,
R^{2a}, R^{2b}, R^{2c1}, R^{2c2}, R^{2d}, R^{2e1} and R^{2e2} are independently hydrogen or C₁₋₆ alkyl;
R³¹, R³², R³³, R³⁴, R³⁵ and R³⁶ are independently hydrogen or C₁₋₆ alkyl;
n is 0, 1, 2, 3, 4, 5 or 6;
each R⁴ is independently C₁₋₆ alkyl, C₁₋₆ alkyl substituted with one or more R⁴⁻¹, C₁₋₆ alkyl-O-, O=, -C(=O)OR^{4a} or -C(=O)NR^{4b}R^{4c}; or, when n is 2, 3, 4, 5 or 6, two optional R⁴ are connected, together with the atoms on the ring to which they are attached, independently form 3- to 8-membered carbocyclic ring or 3- to 8-membered heterocyclic ring containing 1-3 heteroatoms independently selected from O, S and N;
R⁴⁻¹ is independently halogen, cyano, hydroxyl, C₁₋₆ alkyl-O-, -NR⁴ⁱR^{4j}, -C(=O)OR^{4d} or - C(=O)NR^{4e}R^{4f};
R^{4a}, R^{4b}, R^{4c}, R^{4d}, R^{4e}, R^{4f}, R⁴ⁱ and R^{4j} are independently hydrogen or C₁₋₆ alkyl; 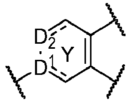 is phenyl, "5- to 7-membered heterocycloalkenyl containing 1-3 heteroatoms independently selected from O, S and N", "5- to 7-membered heteroaryl containing 1-3 heteroatoms independently selected from O, S and N" or 5- to 7-membered cycloalkenyl; wherein D¹ is C, CH or N; D₂ is 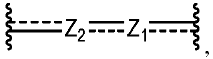 wherein Z₁ and Z₂ are independently a bond, CH, CH₂, O, S, N or NH;
r is 0, 1, 2, 3, 4, 5 or 6;
R⁵ is independently halogen or C₁₋₆ alkyl;
X¹ and X² are independently CR^{b} or N, and X¹ and X² are not both CR^{b};
L₁ is a bond, -C(=O)- or C₁₋₆ alkylene;
R¹ is C₆₋₂₀ aryl, "5- to 12-membered heteroaryl containing 1-4 heteroatoms independently selected from O, S and N", C₆₋₂₀ aryl substituted with one or more R¹⁻¹ or "5- to 12-membered heteroaryl containing 1-4 heteroatoms independently selected from O, S and N and substituted with one or more R¹⁻²"; provided that when multiple substituents are present, the substituents are the same or different;
R^{b}, R¹⁻¹ and R¹⁻² are independently halogen, hydroxyl, cyano, -C(=O)R¹¹, -NR¹²R¹³, -C(=O)OR¹⁴, -C(=O)NR¹⁵R¹⁶, C₁₋₆ alkyl, C₂₋₆ alkenyl, C₂₋₆ alkynyl, C₁₋₆ alkyl-O-, C₃₋₁₀ cycloalkyl, "5- to 7-membered heterocycloalkyl containing 1 or 2 heteroatoms independently selected from O and N", C₆₋₂₀ aryl, "5- to 7-membered heteroaryl containing 1 or 2 heteroatoms independently selected from O and N", C₁₋₆ alkyl substituted with one or more R¹⁻¹⁻¹, C₁₋₆ alkyl-O substituted with one or more R¹⁻¹⁻², C₃₋₁₀ cycloalkyl substituted with one or more R¹⁻¹⁻³, "5- to 7-membered heterocycloalkyl containing 1 or 2 heteroatoms independently selected from O and N" substituted with one or more R¹⁻¹⁻⁴, C₆₋₂₀ aryl substituted with one or more R¹⁻¹⁻⁵, or "5- to 7-membered heteroaryl containing 1 or 2 heteroatoms independently selected from O and N" substituted with one or more R¹⁻¹⁻⁶; provided that when multiple substituents are present, the substituents are the same or different;
R¹⁻¹⁻¹, R¹⁻¹⁻², R¹⁻¹⁻³, R¹⁻¹⁻⁴, R¹⁻¹⁻⁵ and R¹⁻¹⁻⁶ are independently cyano, halogen, hydroxyl, C₁₋₆ alkyl-O-, C₁₋₆ alkyl, -C(=O)R²¹, -NR²²R²³, -C(=O)OR²⁴ or -C(=O)NR²⁵R²⁶;
R¹¹, R²¹, R¹², R²², R¹³, R²³, R¹⁴, R²⁴, R¹⁵, R²⁵, R¹⁶ and R²⁶ are independently hydrogen or C₁₋₆ alkyl;
L₂ is a bond, C₁₋₆ alkylene, -C(=O)-, -O(R^{L-1})ₙ₁-, -S(R^{L-2})ₙ₂- or -NR^{L-3}(R^{L-4})ₙ₃-, R^{L-1}, R^{L-2} and R^{L-4} are independently C₁₋₆ alkylene; R^{L-3} is hydrogen or C₁₋₆ alkyl; n1, n2 and n3 are independently 0 or 1;
R³ is C₃₋₁₂ cycloalkyl, C₃₋₁₂ cycloalkyl substituted with one or more R³⁻¹, "4- to 12-membered heterocycloalkyl containing 1-3 heteroatoms independently selected from O, S and N", "4- to 12-membered heterocycloalkyl containing 1-3 heteroatoms independently selected from O, S and N" substituted with one or more R³⁻², C₁₋₆ alkyl, C₁₋₆ alkyl substituted with one or more R³⁻³, -OR^{d}, - SR^{d1}, -NR^{e1}R^{e2} or -C(=O)NR^{e3}R^{e4}; provided that when multiple substituents are present, the substituents are the same or different;
R³⁻¹, R³⁻² and R³⁻³ are independently C₁₋₆ alkyl, C₁₋₆ alkyl substituted with one or more R³⁻¹⁻¹, hydroxyl, C₁₋₆ alkyl-O-, halogen, O=, -NR^{e5}R^{e6} or -C(=O)NR^{e7}R^{e8};
R^{d}, R^{d1}, R^{e1}, R^{e2}, R^{e3} and R^{e4} are independently hydrogen, C₁₋₆ alkyl, C₃₋₁₀ cycloalkyl, "4- to 10-membered heterocycloalkyl containing 1-3 heteroatoms independently selected from O and N", or C₁₋₆ alkyl substituted with one or more R³⁻¹⁻².
R³⁻¹⁻¹ and R³⁻¹⁻² are independently deuterium, cyano, halogen, hydroxyl, C₁₋₆ alkyl-O-, -C(=O)R^{e9}, -NR^{e10}R^{e11}, -C(=O)OR^{e12} or -C(=O)NR^{e13}R^{e14};
R^{e5}, R^{e6}, R^{e7}, R^{e8}, R^{e9}, R^{e10}, R^{e11}, R^{e12}, R^{e13} and R^{e14} are independently hydrogen or C₁₋₆ alkyl;
Solution 3:
A nitrogen-containing heterocyclic compound of formula I, a pharmaceutically acceptable salt thereof, a stereoisomer thereof, a tautomer thereof or an isotopically labeled compound thereof: 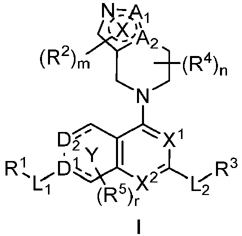
wherein "=-=" represents a single or double bond; 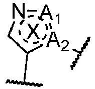
is nitrogen-containing 5-membered heteroaryl; A₁ is CH, O or N; A₂ is C or N;
m is 0, 1 or 2;
R² is -CN, C₁₋₆ alkyl, C₂₋₆ alkenyl, C₂₋₆ alkynyl, C₁₋₆ alkyl substituted with one or more R²⁻¹, halogen, -OR^{2a}, -C(=O)R^{2b}, -NR^{2c1}R^{2c2}, -C(=O)OR^{2d}, -C(=O)NR^{2e1}R^{2e2}, C₃₋₁₀ cycloalkyl, C₃₋₁₀ cycloalkyl substituted with one or more R²⁻², "4- to 10-membered heterocycloalkyl containing 1-3 heteroatoms independently selected from O and N", "4- to 10-membered heterocycloalkyl containing 1-3 heteroatoms independently selected from O and N" substituted with one or more R²⁻³, C₆₋₂₀ aryl, C₆₋₂₀ aryl substituted with one or more R²⁻⁴, "5- to 12-membered heteroaryl containing 1-4 heteroatoms independently selected from O, S and N", or "5- to 12-membered heteroaryl containing 1-4 heteroatoms independently selected from O, S and N" substituted with one or more R²⁻⁵; provided that when multiple substituents are present, the substituents are the same or different;
R²⁻¹, R²⁻², R²⁻³, R²⁻⁴ and R²⁻⁵ are independently halogen, hydroxyl, cyano, C₁₋₆ alkyl, C₂₋₆ alkenyl, C₂₋₆ alkynyl, C₁₋₆ alkyl-O-, -C(=O)R³¹, -NR³²R³³, -C(=O)OR³⁴ or -C(=O)NR³⁵R³⁶,
R^{2a}, R^{2b}, R^{2C1}, R^{2c2}, R^{2d}, R^{2e1} and R^{2e2} are independently hydrogen or C₁₋₆ alkyl;
R³¹, R³², R³³, R³⁴, R³⁵ and R³⁶ are independently hydrogen or C₁₋₆ alkyl;
n is 0, 1, 2, 3, 4, 5 or 6;
each R⁴ is independently C₁₋₆ alkyl, C₁₋₆ alkyl substituted with one or more R⁴⁻¹, C₁₋₆ alkyl-O-, O=, -C(=O)OR^{4a} or -C(=O)NR^{4b}R^{4c}; or, when n is 2, 3, 4, 5 or 6, two optional R⁴ are connected, together with the atoms on the ring to which they are attached, independently form 3- to 8-membered carbocyclic ring or 3- to 8-membered heterocyclic ring containing 1-3 heteroatoms independently selected from O, S and N;
R⁴⁻¹ is independently halogen, cyano, hydroxyl, C₁₋₆ alkyl-O-, -NR⁴ⁱR^{4j}, -C(=O)OR^{4d} or - C(=O)NR^{4e}R^{4f};
R^{4a}, R^{4b}, R^{4e}, R^{4d}, R^{4e}, R^{4f}, R⁴ⁱ and R^{4j} are independently hydrogen or C₁₋₆ alkyl; 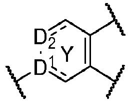
is phenyl, "5- to 7-membered heterocycloalkenyl containing 1-3 heteroatoms independently selected from O, S and N", "5- to 7-membered heteroaryl containing 1-3 heteroatoms independently selected from O, S and N" or 5- to 7-membered cycloalkenyl; wherein D¹ is C, CH or N; D₂ is 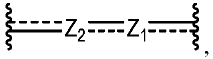 wherein Z₁ and Z₂ are independently a bond, CH, CH₂, O, S, N or NH;
r is 0, 1, 2, 3, 4, 5 or 6;
R⁵ is independently halogen or C₁₋₆ alkyl;
X¹ and X² are independently CR^{b} or N, and X¹ and X² are not both CR^{b};
L₁ is a bond, -C(=O)- or C₁₋₆ alkylene;
R¹ is C₆₋₂₀ aryl, C₈₋₁₁ benzocycloalkenyl, "5- to 12-membered heteroaryl containing 1-4 heteroatoms independently selected from O, S and N", C₆₋₂₀ aryl substituted with one or more R¹⁻¹ or "5- to 12-membered heteroaryl containing 1-4 heteroatoms independently selected from O, S and N" substituted with one or more R¹⁻²; provided that when multiple substituents are present, the substituents are the same or different;
R^{b}, R¹⁻¹ and R¹⁻² are independently halogen, -OR^{c}, cyano, -C(=O)R¹¹, -NR¹²R¹³, -C(=O)OR¹⁴, - C(=O)NR¹⁵R¹⁶, C₁₋₆ alkyl, C₂₋₆ alkenyl, C₂₋₆ alkynyl, C₃₋₁₀ cycloalkyl, "5- to 7-membered heterocycloalkyl containing 1 or 2 heteroatoms independently selected from O and N", C₆₋₂₀ aryl, "5- to 7-membered heteroaryl containing 1 or 2 heteroatoms independently selected from O and N", C₁₋₆ alkyl substituted with one or more R¹⁻¹⁻¹, C₁₋₆ alkyl-O substituted with one or more R¹⁻¹⁻², C₃₋₁₀ cycloalkyl substituted with one or more R¹⁻¹⁻³, "5- to 7-membered heterocycloalkyl containing 1 or 2 heteroatoms independently selected from O and N" substituted with one or more R¹⁻¹⁻⁴, C₆₋₂₀ aryl substituted with one or more R¹⁻¹⁻⁵, or "5- to 7-membered heteroaryl containing 1 or 2 heteroatoms independently selected from O and N" substituted with one or more R¹⁻¹⁻⁶; provided that when multiple substituents are present, the substituents are the same or different; or, when the number of R¹⁻¹ or R¹⁻² is more than one, two optional R¹⁻¹ or R¹⁻² are connected, together with the atoms on the ring to which they are attached, independently form 3- to 8-membered cyclic olefin;
R^{c}, R¹² and R¹³ are independently hydrogen, C₁₋₆ alkyl, -C(=O)R^{c1}, -C(=O)OR^{c2}, or - C(=O)NR^{c3}R^{c4}; R^{c1}, R^{c2}, R^{c3} and R^{c4} are independently hydrogen, C₁₋₆ alkyl, C₃₋₁₀ cycloalkyl, "5-to 7-membered heterocycloalkyl containing 1 or 2 heteroatoms independently selected from O and N", C₆₋₂₀ aryl, "5- to 7-membered heteroaryl containing 1 or 2 heteroatoms independently selected from O and N", C₁₋₆ alkyl substituted with one or more R⁴⁻¹⁻¹, C₃₋₁₀ cycloalkyl substituted with one or more R⁴⁻¹⁻², "5- to 7-membered heterocycloalkyl containing 1 or 2 heteroatoms independently selected from O and N" substituted with one or more R⁴⁻¹⁻³, C₆₋₂₀ aryl substituted with one or more R⁴⁻¹⁻⁴, or, "5- to 7-membered heteroaryl containing 1 or 2 heteroatoms independently selected from O and N" substituted with one or more R⁴⁻¹⁻⁵; provided that when multiple substituents are present, the substituents are the same or different;
R¹⁻¹⁻¹, R¹⁻¹⁻² , R¹⁻¹⁻³, R¹⁻¹⁻⁴, R¹⁻¹⁻⁵, R¹⁻¹⁻⁶, R⁴⁻¹⁻¹, R⁴⁻¹⁻², R⁴⁻¹⁻³, R⁴⁻¹⁻⁴ and R⁴⁻¹⁻⁵ are independently cyano, halogen, hydroxyl, C₁₋₆ alkyl-O-, C₁₋₆ alkyl, -C(=O)R²¹, -NR²²R²³, -C(=O)OR²⁴ or -C(=O)NR²⁵R²⁶;
R¹¹, R²¹, R²², R²³, R¹⁴, R²⁴, R¹⁵, R²⁵, R¹⁶ and R²⁶ are independently hydrogen or C₁₋₆ alkyl;
L₂ is a bond, C₁₋₆ alkylene, -C(=O)-, -O(R^{L-1})ₙ₁-, -S(R^{L-2})ₙ₂- or -NR^{L-3}(R^{L-4})ₙ₃-; R^{L-1}, R^{L-2} and R^{L-4} are independently C₁₋₆ alkylene; R^{L-3} is hydrogen or C₁₋₆ alkyl; n1, n2 and n3 are independently 0 or 1;
R³ is C₃₋₁₂ cycloalkyl, C₃₋₁₂ cycloalkyl substituted with one or more R³⁻¹, "4- to 12-membered heterocycloalkyl containing 1-3 heteroatoms independently selected from O, S and N", "4- to 12-membered heterocycloalkyl containing 1-3 heteroatoms independently selected from O, S and N" substituted with one or more R³⁻², C₁₋₆ alkyl, C₁₋₆ alkyl substituted with one or more R³⁻³, -OR^{d}, - SR^{d1}, -NR^{e1}R^{e2} or -C(=O)NR^{e3}R^{e4}; provided that when multiple substituents are present, the substituents are the same or different;
R³⁻¹, R³⁻² and R³⁻³ are independently C₁₋₆ alkyl, C₁₋₆ alkyl substituted with one or more R³⁻¹⁻¹, hydroxyl, C1-6 alkyl-O-, halogen, O=, -NR^{e5}R^{e6} or -C(=O)NR^{e7}R^{e8};
R^{d}, R^{d1}, R^{e1} R^{e2}, R^{e3} and R^{e4} are independently hydrogen, C₁₋₆ alkyl, C₃₋₁₀ cycloalkyl, "4- to 10-membered heterocycloalkyl containing 1-3 heteroatoms independently selected from O and N", or C₁₋₆ alkyl substituted with one or more R³⁻¹⁻².
R³⁻¹⁻¹ and R³⁻¹⁻² are independently deuterium, cyano, halogen, hydroxyl, C₁₋₆ alkyl-O-, -C(=O)R^{e9}, -NR^{e10}R^{e11}, -C(=O)OR^{e12} or ^{_}C(=O)NR^{e13}R^{e14}.
R^{e5}, R^{e6}, R^{e7}, R^{e8}, R^{e9}, R^{e10}, R^{e11}, R^{e12}, R^{e13} and R^{e14} are independently hydrogen or C₁₋₆ alkyl.
